(19) Europäisches Patentamt
European Patent Office
Office européen des brevets

(11) EP 4 606 800 A1

(12) **EUROPEAN PATENT APPLICATION**
published in accordance with Art. 153(4) EPC

(43) Date of publication:
**27.08.2025 Bulletin 2025/35**

(21) Application number: **23879134.7**

(22) Date of filing: **18.10.2023**

(51) International Patent Classification (IPC):
***C07D 455/03*** *(2006.01)* ***C07D 498/14*** *(2006.01)*
***A61K 31/4375*** *(2006.01)* ***A61P 25/00*** *(2006.01)*

(52) Cooperative Patent Classification (CPC):
**A61K 31/4375; A61P 25/00; C07D 455/03; C07D 498/14**

(86) International application number:
**PCT/CN2023/125192**

(87) International publication number:
**WO 2024/083146 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.10.2022 CN 202211273550**
**25.08.2023 CN 202311089455**

(71) Applicant: **Beijing Tide Pharmaceutical Co., Ltd.**
**Beijing 100176 (CN)**

(72) Inventors:
- **LI, Wenming**
  **Beijing 100176 (CN)**
- **LI, Xiaobo**
  **Beijing 100176 (CN)**
- **GUO, Guangzhu**
  **Beijing 100176 (CN)**
- **PEI, Xiaolin**
  **Beijing 100176 (CN)**
- **ZHANG, Yu**
  **Beijing 100176 (CN)**
- **WANG, Jingying**
  **Beijing 100176 (CN)**
- **LI, Xinchun**
  **Beijing 100176 (CN)**
- **YU, Xiaolei**
  **Beijing 100176 (CN)**
- **PENG, Lijiao**
  **Beijing 100176 (CN)**
- **ZHOU, Liying**
  **Beijing 100176 (CN)**
- **GAN, Leling**
  **Beijing 100176 (CN)**
- **LI, Eryao**
  **Beijing 100176 (CN)**
- **YU, Guokun**
  **Beijing 100176 (CN)**
- **LIU, Yanan**
  **Beijing 100176 (CN)**

(74) Representative: **Novitas Patent AB**
**P.O. Box 55557**
**102 04 Stockholm (SE)**

# (54) COMPOUND BASED ON ORYDALMINE STRUCTURE AND USE THEREOF

(57) The present invention relates to a compound of general formula (A) and a pharmaceutical composition thereof. The compound of formula (A) provided in the present invention can be used for preventing and/or treating diseases related to the central nervous system such as pain, drug addiction and depression. The present invention also relates to the preparation and the use of the compound.

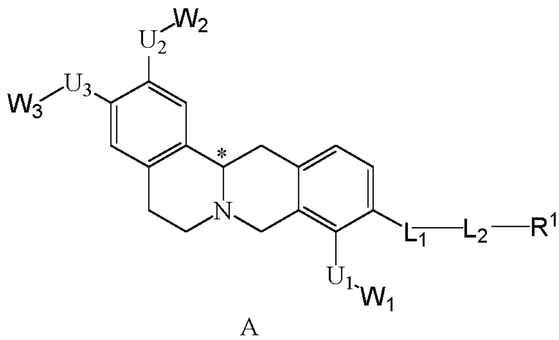

A

EP 4 606 800 A1

## Description

The present disclosure claims priority from the Chinese Patent Application No. 202211273550.1 filed on October 18, 2022 and the Chinese Patent Application No. 202311089455.0 filed on August 25, 2023, both of which are incorporated herein by reference in their entirety.

## TECHNICAL FIELD

The present disclosure relates to the technical field of medicine, especially to a compound based on corydalmine structure and use thereof.

## BACKGROUND

L-corydalmine (l-DL) is a known compound having analgesic, drug rehabilitation, and anti-drug addiction effects, with a chemical name of (S)-2,3,9-trimethoxy-5,8,13,13a-tetrahydro-6H-isoquinolino[3,2-a]isoquinolin-10-ol and a structure of:

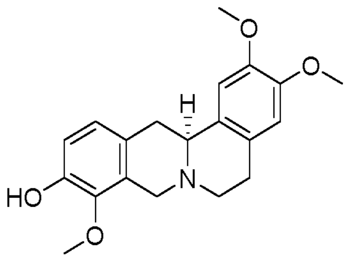

.

Corydalmine, as one of the effective alkaloid ingredients in the traditional Chinese herb *Rhizoma Corydalis,* has extremely low natural content, which limits its use in the pharmaceutical field. Thus, there is an urgent need to develop other active compounds based on the corydalmine structure.

## SUMMARY

The present disclosure aims to provide a compound based on corydalmine structure, which has a good analgesic effect and may be used to prepare analgesic drugs.

In an aspect, the present disclosure provides a compound of Formula I, or a pharmaceutically acceptable salt, ester, stereoisomer, polymorph, solvate, N-oxide, isotope-labeled compound, metabolite, or pro-drug thereof, and a mixture thereof:

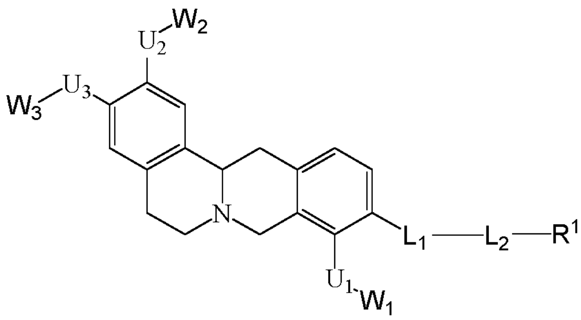

I

wherein $L_1$ is selected from a single bond, -O-, -NH-, -S- or -P(O)OH-; alternatively a single bond, -O- and -NH-;
$L_2$ is selected from a single bond, -O-, -NH-, -C(O)-, -C(O)-O-, $C_{1-18}$ alkylene (alternatively $C_{1-12}$ alkylene, and yet alternatively $C_{1-6}$ alkylene), $C_{3-10}$ cycloalkylene (alternatively $C_{3-8}$ cycloalkylene, and yet alternatively $C_{3-6}$ cycloalkylene), $C_{6-30}$ arylene (alternatively $C_{6-18}$ arylene, and yet alternatively $C_{6-12}$ arylene), $C_{3-30}$ heteroarylene (alternatively $C_{3-18}$ heteroarylene, and yet alternatively $C_{3-12}$ heteroarylene), -$C_{1-18}$ alkylene-O- (alternatively -$C_{1-12}$ alkylene-O-, and yet alternatively -$C_{1-6}$ alkylene-O-), -$C_{6-30}$ arylene-O- (alternatively -$C_{6-18}$ arylene-O-, and yet alternatively -$C_{6-12}$ arylene-O-), -$C_{3-30}$ heteroarylene-O- (alternatively -$C_{3-18}$ heteroarylene-O-, and yet alternatively

$-C_{3-12}$ heteroarylene-O-)-C(O)-$C_{1-18}$ alkyl-, $C_{1-30}$ alkoxy or $-X_1$-PRa(O)-$X_2$-; the Ra is each independently selected from hydrogen, hydroxyl, halogen (alternatively F, Cl or Br), $C_{6-30}$ aryl (alternatively $C_{6-18}$ aryl, and yet alternatively $C_{6-12}$ aryl), $C_{3-30}$ heteroaryl (alternatively $C_{3-18}$ heteroaryl, and yet alternatively $C_{3-12}$ heteroaryl), $C_{1-30}$ hydrocarbon radical (alternatively $C_{1-30}$ alkyl, and yet alternatively $C_{1-18}$ alkyl, and further alternatively $C_{1-6}$ alkyl), $C_{3-10}$ cycloalkyl (alternatively $C_{3-8}$ cycloalkyl, and yet alternatively $C_{3-6}$ cycloalkyl), $C_{1-30}$ alkoxy (alternatively $C_{1-18}$ alkoxy, and yet alternatively $C_{1-6}$ alkoxy), $C_{6-30}$ aryl-O- (alternatively $C_{6-18}$ aryl-O-, and yet alternatively $C_{6-12}$ aryl-O-), $C_{3-30}$ heteroaryl-O- (alternatively $C_{3-18}$ heteroaryl-O-, and yet alternatively $C_{3-12}$ heteroaryl-O-), $C_{6-30}$ aryl-$C_{1-18}$ alkylene-O- (alternatively $C_{6-18}$ aryl-$C_{1-12}$ alkylene-O-, and yet alternatively $C_{6-12}$ aryl-$C_{1-6}$ alkylene-O-), $C_{3-30}$ heteroaryl-$C_{1-18}$ alkylene-O- (alternatively $C_{3-18}$ heteroaryl-$C_{1-12}$ alkylene-O-, and yet alternatively $C_{3-12}$ heteroaryl-$C_{1-6}$ alkylene-O-), -O-$C_{6-30}$ aryl-$C_{1-18}$ alkylene- (alternatively -O-$C_{6-18}$ aryl-$C_{1-12}$ alkylene-), or -O-$C_{3-30}$ heteroaryl-$C_{1-18}$ alkylene- (alternatively -O-$C_{3-18}$ heteroaryl-$C_{1-12}$ alkylene-); the Ra and $X_2$ may be connected to form a ring; the $X_1$ is selected from a single bond, -O-, $-C_{1-18}$ alkylene-O- (alternatively $-C_{1-12}$ alkylene-O-, and yet alternatively $-C_{1-6}$ alkylene-O-), $-C_{6-30}$ arylene-O- (alternatively $-C_{6-18}$ arylene-O-, and yet alternatively $-C_{6-12}$ arylene-O-), or $-C_{3-30}$ heteroarylene-O- (alternatively $-C_{3-18}$ heteroarylene-O-, and yet alternatively $-C_{3-12}$ heteroarylene-O-);

the $X_2$ is selected from a single bond, -O-- or -NR*-; wherein R* is selected from H, $C_{1-6}$ alkyl, or $C_{1-6}$ alkoxy;

$R^1$ is absent, or selected from hydrogen, hydroxyl, unsubstituted or Rb-substituted amino, unsubstituted or Rb-substituted $C_{1-30}$ hydrocarbon radical (alternatively $C_{1-30}$ alkyl or $C_{2-30}$ alkenyl, and yet alternatively $C_{1-18}$ alkyl or $C_{2-18}$ alkenyl, and further alternatively $C_{1-6}$ alkyl or $C_{2-12}$ alkenyl, wherein the alkenyl has a double-bond number of 1-10, alternatively 1-6), unsubstituted or Rb-substituted $C_{3-10}$ cycloalkyl (alternatively $C_{3-8}$ cycloalkyl, and yet alternatively $C_{3-6}$ cycloalkyl), unsubstituted or Rb-substituted $C_{1-30}$ alkoxy (alternatively $C_{1-20}$ alkoxy, and yet alternatively $C_{1-12}$ alkoxy), unsubstituted or Rb-substituted $C_{12-30}$ condensed cyclic group (alternatively $C_{12-17}$ condensed cyclic group, and yet alternatively $C_{12-17}$ condensed cyclic group containing at least one heteroatom, and further alternatively $C_{12-17}$ tetracondensed cyclic group containing at least one heteroatom), $C_{1-18}$ alkyl-$C_{6-30}$ arylene-$C_{1-18}$ alkylene- (alternatively $C_{1-12}$ alkyl-$C_{6-18}$ arylene-$C_{1-12}$ alkyl-) which is unsubstituted or has a hydrogen atom replaced with Rc in the alkyl, $C_{1-12}$ alkyl-O-C(O)-$C_{1-12}$ alkylene-imino-, which is unsubstituted or has a hydrogen atom replaced with Rd in the alkyl, $C_{6-30}$ aryl-$C_{1-18}$ alkylene- (alternatively $C_{6-18}$ aryl-$C_{1-12}$ alkylene-), -O-C(O)-$Y_1$, $-C_{1-12}$ alkylene-C(O)-O-$Y_2$ which is unsubstituted or has a hydrogen atom replaced with Rd in the alkylene, -O-$C_{1-12}$ alkylene-O-C(O)-$Y_3$ which is unsubstituted or has a hydrogen atom replaced with Rd in the alkylene, -C=CH-$Y_4$, $-Y_5$-$Y_6$-$Y_7$, or $-CHR^{\#}R^{\#\#}$;

wherein $R^{\#}$ and $R^{\#\#}$ are each independently selected from H, hydroxyl, amino, halogen, alkyl, cycloalkyl, aryl, heteroaryl,

$-C(O)-OR^z$, $-OC(O)-OR^2$, $-C_{1-18}$ alkylene-C(O)-$OR^2$, $-C_{1-18}$ alkylene-$R^2$, or $-C_{6-10}$ aryl-$C_{1-6}$ alkyl;

$R^2$ is selected from H, hydroxyl, amino, halogen, alkyl, cycloalkyl, aryl, heteroaryl, unsubstituted or Rb-substituted $C_{12-30}$ condensed cyclic group, or -NHC(NH)NH-;

the Rb is each independently selected from $C_{1-30}$ alkoxy (alternatively $C_{1-20}$ alkoxy, yet alternatively $C_{1-12}$ alkoxy, and further alternatively $C_{1-3}$ alkoxy), $C_{6-30}$ aryl (alternatively $C_{6-18}$ aryl, yet alternatively $C_{6-12}$ aryl), $C_{3-30}$ heteroaryl (alternatively $C_{3-18}$ heteroaryl, yet alternatively $C_{3-12}$ heteroaryl), or $C_{1-30}$ alkyl (alternatively $C_{1-18}$ alkyl, yet alternatively $C_{1-6}$ alkyl), which are unsubstituted or substituted with halogen (alternatively F, Cl, or Br);

the Rc is each independently selected from $C_{3-10}$ cycloalkyl (alternatively $C_{3-8}$ cycloalkyl, and yet alternatively $C_{3-6}$ cycloalkyl, and further alternatively cyclopentyl) which is unsubstituted or substituted with hydroxyl, oxo, or thioxo;

the Rd is each independently selected from $C_{1-18}$ alkyl (alternatively $C_{1-12}$ alkyl, and yet alternatively $C_{1-6}$ alkyl), $C_{1-30}$ alkoxy (alternatively $C_{1-20}$ alkoxy, and yet alternatively $C_{1-12}$ alkoxy, and further alternatively $C_{1-3}$ alkoxy), $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with halogen (alternatively F, Cl or Br);

the $Y_1$ is selected from $C_{1-30}$ alkyl (alternatively $C_{1-12}$ alkyl, and yet alternatively $C_{1-6}$ alkyl), $C_{3-10}$ cycloalkyl (alternatively $C_{3-8}$ cycloalkyl, and yet alternatively $C_{3-6}$ cycloalkyl), $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with Re; the Re is each independently selected from $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with halogen (alternatively F, Cl or Br);

the $Y_2$ is selected from unsubstituted or Rf-substituted $C_{12-30}$ condensed cyclic group (alternatively $C_{12-17}$ condensed cyclic group, and yet alternatively $C_{12-17}$ condensed cyclic group containing at least one heteroatom, and further alternatively $C_{12-17}$ tetracondensed cyclic group containing at least one heteroatom); wherein the Rf is each independently selected from $C_{1-30}$ alkoxy (alternatively $C_{1-20}$ alkoxy, and yet alternatively $C_{1-12}$ alkoxy, and further alternatively $C_{1-3}$ alkoxy), or $C_{6-30}$ aryl (alternatively $C_{6-18}$ aryl, and yet alternatively $C_{6-12}$ aryl), which are unsubstituted or substituted with halogen (alternatively F, Cl or Br);

the $Y_3$ is selected from $C_{1-18}$ alkyl (alternatively $C_{1-12}$ alkyl, and yet alternatively $C_{1-6}$ alkyl) or -O-$C_{1-18}$ alkyl (alternatively -O-$C_{1-12}$ alkyl, and yet alternatively -O-$C_{1-6}$ alkyl);

the $Y_4$ is selected from $C_{6-30}$ aryl (alternatively $C_{6-18}$ aryl, and yet alternatively $C_{6-12}$ aryl) or $C_{3-30}$ heteroaryl (alternatively $C_{3-18}$ heteroaryl, and yet alternatively $C_{3-12}$ heteroaryl), which are unsubstituted or substituted with Rg, wherein the Rg is each independently selected from hydroxyl and $C_{1-30}$ alkoxy (alternatively $C_{1-20}$ alkoxy, and yet alternatively $C_{1-12}$ alkoxy, and further alternatively $C_{1-3}$ alkoxy);

the $Y_5$ is selected from a single bond, unsubstituted or amino-substituted $C_{1-18}$ alkylene (alternatively $C_{1-12}$ alkylene, and yet alternatively $C_{3-6}$ alkylene);

the $Y_6$ is selected from a single bond, -NH-C(NH)-, $-(O-CH_2-CH_2)_m-$ or $-CH_2-(O-CH_2-CH_2)_n-$, and the *m* and the *n* are each independently selected from any natural number of 2-20 (alternatively any natural number of 2-10);

the $Y_7$ is selected from amino, $C_{1-30}$ alkoxy (alternatively $C_{1-20}$ alkoxy, and yet alternatively $C_{1-12}$ alkoxy, and further alternatively $C_{1-3}$ alkoxy) or hydroxyl;

When the $L_1$ and the $L_2$ are both selected from a single bond, the $R^1$ is not hydroxyl;

$U_1$, $U_2$ and $U_3$ are each independently selected from -O-C(O)-, -NH-C(O)-, $-O-CH_2-O-$ or -O-; alternatively -O-; or an available ring atom on $U_2$ and an available ring atom on $U_3$ are connected via Z group to form a 4- to 8-membered ring, alternatively a 5- to 6-membered ring,

Z is selected from a single bond; $NR^3$; $C_{1-3}$ alkylene in which 1 or 2 $CH_2$ are optionally independently replaced with a group selected from O, S and $NR^3$; and $C_{2-3}$ alkenylene in which any CH for forming the C=C double bond is optionally replaced with N;

$W_1$, $W_2$ and $W_3$ are each independently selected from $C_{1-18}$ alkyl (alternatively $C_{1-12}$ alkyl, and yet alternatively $C_{1-6}$ alkyl) or $-(O-C_{2-6}\ alkylene)_p$ (alternatively $-(O-C_{2-4}\ alkylene)_p$, and yet alternatively $-(O-CH_2-CH_2)_p$), wherein the p is selected from any natural number of 1-18 (alternatively any natural number of 1-10).

The above alkyl, alkylene, alkenyl, alkenylene, alkynyl, cyclic hydrocarbon radical, heterocyclyl, aryl, heteroaryl, and aralkyl at each occurrence are each optionally substituted with 1, 2, 3 or more $R^3$, wherein the $R^3$ at each occurrence is independently selected from halogen, cyano, nitro, $C_{1-6}$ alkyl, $C_{3-10}$ cyclic hydrocarbon radical, 3- to 10-membered heterocyclyl, $C_{6-10}$ aryl, 5- to 14-membered heteroaryl, $C_{6-12}$ aralkyl; and wherein the alkyl, alkylene, cyclic hydrocarbon radical, heterocyclyl, aryl, heteroaryl, and aralkyl in relation to the substituent $R^3$ are further optionally substituted with 1, 2, 3 or more substituents independently selected from halogen, OH, oxo, amino, cyano, nitro, $C_{1-6}$ alkyl, $C_{1-6}$ haloalkyl, $C_{3-6}$ cyclic hydrocarbon radical, 3- to 10-membered heterocyclyl, $C_{6-10}$ aryl, 5- to 14-membered heteroaryl, and $C_{6-12}$ aralkyl.

In the present disclosure, the number of the substituents, such as, Ra, Rb, Rc, Rd, Re, Rf, Rg, methoxy, halogen, amino, etc., may be one or more, which is not limited in the present disclosure.

In some embodiments of the present disclosure, in the compound of Formula I, the heteroatoms in the heteroaryl, the heteroarylene, and the $C_{12-17}$ condensed cyclic group containing at least one heteroatom are each independently selected from O, N and S, alternatively N;

alternatively, the number of heteroatoms in the heteroaryl, the heteroarylene, and the $C_{12-17}$ condensed cyclic group containing at least one heteroatom are 1-3, alternatively 1-2;

alternatively, the $C_{12-17}$ tetracondensed cyclic group containing at least one heteroatom is

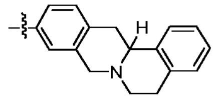

In a second aspect, the present disclosure provides a composition including the above-mentioned compound, or a stereoisomer, pharmaceutically acceptable salt, ester, optical isomer, polymorph, solvate, N-oxide, isotope-labeled compound, metabolite, chelate, complex, inclusion or prodrug thereof.

In a third aspect, the present disclosure provides use of the above-mentioned compound or a stereoisomer, pharmaceutically acceptable salt, ester, optical isomer, polymorph, solvate, N-oxide, isotope-labeled compound, metabolite, chelate, complex, inclusion or prodrug thereof, or the composition in the manufacture of a medicament for preventing and/or treating a central nervous system (CNS)-associated disease.

In a fourth aspect, the present disclosure provides use of the above-mentioned compound or a stereoisomer, pharmaceutically acceptable salt, ester, optical isomer, polymorph, solvate, N-oxide, isotope-labeled compound, metabolite, chelate, complex, inclusion or prodrug thereof, or the composition of claim 42 in the manufacture of a medicament for analgesia, anti-depression, or anti-addiction.

In some alternative embodiments of the present disclosure, the drug is used for pains caused by postoperative trauma, postoperative incision, or organ metastasis of cancer.

In a fifth aspect, the present disclosure provides a method for analgesia, anti-depression, or anti-drug addiction including administering to a subject in need thereof an effective dose of the compound, or a stereoisomer, pharmaceutically acceptable salt, ester, optical isomer, polymorph, solvate, N-oxide, isotope-labeled compound, metabolite, chelate, complex, inclusion or prodrug thereof, or the composition, wherein the administration is carried out by ways of oral, rectal, nasal, topical, or parenteral administration.

Definitions

Chemical Definitions

Hereinafter the definitions of specific functional groups and chemical terms are described in more detail.

When a numerical range is listed, it is intended to encompass each and every value and sub-range within the range. For example, "$C_{1-6}$ alkyl" includes $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$, and $C_{5-6}$ alkyl.

"$C_{1-30}$ hydrocarbon radical" refers to a group only including carbon and hydrogen atoms, which may be saturated or unsaturated, e.g., "$C_{1-30}$ alkyl", "$C_{2-30}$ alkenyl", and "$C_{2-30}$ alkynyl".

"$C_{1-30}$ alkyl" refers to a linear or branched saturated hydrocarbon radical having 1 to 30 carbon atoms. In some embodiments, $C_{l-18}$ alkyl and $C_{1-12}$ alkyl are alternative. In some embodiments, $C_{1-6}$ alkyl and $C_{1-4}$ alkyl are yet alternative. In some embodiments, $C_{1-4}$ alkyl and $C_{1-2}$ alkyl are still yet alternative. Examples of $C_{1-6}$ alkyl include: methyl ($C_1$), ethyl ($C_2$), n-propyl ($C_3$), isopropyl ($C_3$), n-butyl ($C_4$), tert-butyl ($C_4$), sec-butyl ($C_4$), isobutyl ($C_4$), n-pentyl ($C_5$), 3-pentyl ($C_5$), pentyl ($C_5$), neopentyl ($C_5$), 3-methyl-2-butyl ($C_5$), tert-pentyl ($C_5$), and n-hexyl ($C_6$). The term "$C_{1-6}$ alkyl" further includes heteroalkyl in which one or more (e.g., 1, 2, 3 or 4) carbon atoms are replaced with a heteroatom (e.g., oxygen, sulfur, nitrogen, boron, silicon, or phosphorus). The alkyl group may be optionally substituted with one or more substituents, e.g., with 1 to 5 substituents, 1 to 3 substituents, or 1 substituent. Conventional alkyl abbreviations include Me ($-CH_3$), Et ($-CH_2CH_3$), iPr ($-CH(CH_3)_2$), nPr ($-CH_2CH_2CH_3$), n-Bu ($-CH_2CH_2CH_2CH_3$), or i-Bu ($-CH_2CH(CH_3)_2$).

"$C_{2-30}$ alkenyl" refers to a linear or branched hydrocarbon radical having 2 to 30 carbon atoms and at least one carbon-carbon double bond. In some embodiments, $C_{2-18}$ alkenyl and $C_{2-12}$ alkenyl are alternative. In some embodiments, $C_{2-6}$ alkenyl, $C_{2-4}$ alkenyl, and $C_{2-3}$ alkenyl are yet alternative. Examples of $C_{2-6}$ alkenyl include: ethenyl ($C_2$), 1-propenyl ($C_3$), 2-propenyl ($C_3$), 1-butenyl ($C_4$), 2-butenyl ($C_4$), butadienyl ($C_4$), pentenyl ($C_5$), pentadienyl (Cs), hexenyl ($C_6$), or the like. The term "$C_{2-6}$ alkenyl" further includes heteroalkenyl in which one or more (e.g., 1, 2, 3 or 4) carbon atoms are replaced with a heteroatom (e.g., oxygen, sulfur, nitrogen, boron, silicon, or phosphorus). The alkenyl group may be optionally substituted with one or more substituents, e.g., 1 to 5 substituents, 1 to 3 substituents, or 1 substituent.

"$C_{2-30}$ alkynyl" refers to a linear or branched hydrocarbon radical having 2 to 30 carbon atoms, at least one carbon-carbon triple bond, and optionally one or more carbon-carbon double bonds. In some embodiments, $C_{2-18}$ alkynyl and $C_{2-12}$ alkynyl are alternative. In some embodiments, $C_{2-6}$ alkynyl and $C_{2-4}$ alkynyl are yet alternative. Examples of $C_{2-6}$ alkynyl include, but are not limited to: ethynyl ($C_2$), 1-propynyl ($C_3$), 2-propynyl ($C_3$), 1-butynyl ($C_4$), 2-butynyl ($C_4$), pentynyl ($C_5$), hexynyl ($C_6$), etc. The term "$C_{2-6}$ alkynyl" further includes heteroalkynyl in which one or more (e.g., 1, 2, 3 or 4) carbon atoms are replaced with a heteroatom (e.g., oxygen, sulfur, nitrogen, boron, silicon, or phosphorus). The alkynyl group may be optionally substituted with one or more substituents, e.g., 1 to 5 substituents, 1 to 3 substituents, or 1 substituent.

"$C_{1-30}$ alkylene", "$C_{2-30}$ alkenylene", and "$C_{2-30}$ alkynylene" respectively refer to divalent groups formed by removal of another hydrogen from $C_{1-30}$ alkyl, $C_{2-30}$ alkenyl, and $C_{2-30}$ alkynyl, and may be substituted or unsubstituted. In some embodiments, $C_{2-8}$ alkylene, $C_{3-7}$ alkylene, $C_{1-6}$ alkylene, $C_{4-6}$ alkylene, $C_{1-4}$ alkylene, $C_{2-4}$ alkylene, and $C_{1-3}$ alkylene are alternative. In some embodiments, $C_{2-18}$ alkenylene, $C_{2-12}$ alkenylene, $C_{2-6}$ alkenylene, and $C_{2-4}$ alkenylene are alternative. In some embodiments, $C_{2-18}$ alkynylene, $C_{2-12}$ alkynylene, $C_{2-6}$ alkynylene, and $C_{2-4}$ alkynylene are alternative. The unsubstituted alkylene groups include, but are not limited to: methylene ($-CH_2-$), ethylene ($-CH_2CH_2-$), propylene ($-CH_2CH_2CH_2-$), butylene ($-CH_2CH_2CH_2CH_2-$), pentylene ($-CH_2CH_2CH_2CH_2CH_2-$), hexylene ($-CH_2CH_2CH_2CH_2CH_2CH_2-$), etc. Exemplary substituted alkylene groups, e.g., those substituted with one or more alkyl (methyl), include, but are not limited to: substituted methylene ($-CH(CH_3)-$, $-C(CH_3)_2-$), substituted ethylene ($-CH(CH_3)CH_2-$, $-CH_2CH(CH_3)-$, $-C(CH_3)_2CH_2-$, $-CH_2C(CH_3)_2-$), substituted propylene ($-CH(CH_3)CH_2CH_2-$, $-CH_2CH(CH_3)CH_2-$, $-CH_2CH_2CH(CH_3)-$, $-C(CH_3)_2CH_2CH_2-$, $- CH_2C(CH_3)_2CH_2-$, $-CH_2CH_2C(CH_3)_2-$), etc.

"Halo" or "halogen" refers to fluorine (F), chlorine (Cl), bromine (Br), and iodine (I).

Thus, "$C_{1-6}$ haloalkyl" refers to the "$C_{1-6}$ alkyl" substituted with one or more halogen groups. In some embodiments, $C_{1-4}$ haloalkyl is particularly alternative, and $C_{1-2}$ haloalkyl is yet alternative. Exemplary haloalkyl groups include, but are not limited to: $-CF_3$, $-CH_2F$, $-CHF_2$, $-CHFCH_2F$, $-CH_2CHF_2$, $-CF_2CF_3$, $-CCl_3$, $-CH_2Cl$, $- CHCl_2$, 2,2,2-trifluoro-1,1-dimethyl-ethyl, etc. The haloalkyl groups may be substituted at any available point of attachment, e.g., with 1 to 5 substituents, 1 to 3 substituents or 1 substituent.

"$C_{1-30}$ alkoxy" refers to -OR group, wherein R is the $C_{1-30}$ alkyl as defined above. In some embodiments, $C_{1-18}$ alkoxy and $C_{1-12}$ alkoxy are alternative. In some embodiments, $C_{1-6}$ alkoxy and $C_{1-4}$ alkoxy are yet alternative.

"$C_{1-30}$ alkyleneoxy" refers to a divalent group formed by removal of another hydrogen from the $C_{1-30}$ alkoxy, and may be substituted or unsubstituted. In some embodiments, $C_{1-18}$ alkyleneoxy and $C_{1-12}$ alkyleneoxy are alternative. In

some embodiments, $C_{1-6}$ alkyleneoxy and $C_{1-4}$ alkyleneoxy are yet alternative.

"$C_{1-6}$ haloalkoxy" refers to the "$C_{1-6}$ alkoxy" substituted with one or more halogen groups. In some embodiments, $C_{1-4}$ haloalkoxy is particularly alternative, and $C_{1-2}$ haloalkoxy is yet alternative. Exemplary haloalkoxy groups include, but are not limited to: $-OCF_3$, $-OCH_2F$, $-OCHF_2$, $-OCHFCH_2F$, $-OCH_2CHF_2$, $-OCF_2CF_3$, $-OCCl_3$, $-OCH_2Cl$, $-OCHCl_2$, 2,2,2-trifluoro-1,1-dimethyl-ethoxy, etc. The haloalkoxy groups may be substituted at any available point of attachment, e.g., with 1 to 5 substituents, 1 to 3 substituents or 1 substituent.

"$C_{3-10}$ cycloalkyl" refers to a non-aromatic cyclic hydrocarbon radical having 3 to 10 ring carbon atoms and zero heteroatoms. In some embodiments, $C_{3-8}$ cycloalkyl, $C_{3-7}$ cycloalkyl, and $C_{3-5}$ cycloalkyl are particularly alternative, and $C_{5-6}$ cycloalkyl is yet alternative. The cycloalkyl groups further include a ring system in which the cycloalkyl ring is fused with one or more aryl or heteroaryl groups, and the point of attachment is on the cycloalkyl ring, and in such case, the carbon number continues to represent the carbon number in the cycloalkyl system. Exemplary cycloalkyl groups include, but are not limited to: cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), etc. The cycloalkyl group may be optionally substituted with one or more substituents, e.g., 1 to 5 substituents, 1 to 3 substituents, or 1 substituent.

"3- to 10-membered heterocyclyl" refers to a group of 3- to 10-membered, saturated or unsaturated, non-aromatic ring system having ring carbon atoms and 1 to 4 ring heteroatoms, wherein each of heteroatoms is independently selected from nitrogen, oxygen, sulfur, boron, phosphorus, and silicon. In the heterocyclyl groups containing one or more nitrogen atoms, the point of attachment may be carbon or nitrogen atom, as long as the valence permits. In some embodiments, 4- to 8-membered heterocyclyl is alternative, which is a 4- to 8-membered non-aromatic ring system having ring carbon atoms and 1 to 3 ring heteroatoms; in some other embodiments, 4-to 7-membered heterocyclyl is alternative, which is a 4- to 7-membered non-aromatic ring system having ring carbon atoms and 1 to 3 ring heteroatoms; and 5- to 6-membered heterocyclyl is yet alternative, which is a 5- to 6-membered non-aromatic ring system having ring carbon atoms and 1 to 3 ring heteroatoms. The heterocyclyl also includes a ring system wherein the heterocyclyl described above is fused with one or more cycloalkyl groups, wherein the point of attachment is on the cycloalkyl ring, or the heterocyclyl described above is fused with one or more aryl or heteroaryl groups, wherein the point of attachment is on the heterocyclyl ring; and in such cases, the number of ring members continues to represent the number of ring members in the heterocyclyl ring system. Exemplary 3-membered heterocyclyl groups containing one heteroatom include, but are not limited to, aziridinyl, oxiranyl and thiiranyl (thiorenyl). Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocycly groups containing one heteroatom include, but are not limited to, azepanyl, oxepanyl and thiepanyl. Exemplary 5-membered heterocyclyl groups fused with a $C_6$ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a $C_6$ aryl (also referred as 6,6-bicyclic heterocyclyl herein) include, but are not limited to, tetrahydroquinolinyl, tetrahydroisoquinolinyl, etc. The heterocyclyl group also includes a bridged ring or spiro ring formed by the above heterocyclyl group and a cycloalkyl, heterocyclyl, aryl or heteroaryl group sharing one or two atoms, where the shared atom can be a carbon or nitrogen atom as long as the valence permits. The heterocyclyl group can be optionally substituted with one or more substituents, for example, substituted with 1 to 5 substituents, 1 to 3 substituents or 1 substituent.

"$C_{6-30}$ aryl" refers to a group of monocyclic or polycyclic (e.g., bicyclic) 4n+2 aromatic ring system (e.g., having 6 or 10 shared $\pi$ electrons in a cyclic array) having 6-30 ring carbon atoms and zero heteroatoms. In some embodiments, $C_{6-18}$ aryl and $C_{6-12}$ aryl are alternative, and $C_{6-10}$ aryl are yet alternative. In some embodiments, the aryl has six ring carbon atoms ("$C_6$ aryl"; e.g., phenyl). In some embodiments, the aryl has ten ring carbon atoms ("$C_{10}$ aryl"; e.g., naphthyl, e.g., 1-naphthyl and 2-naphthyl). The aryl groups further include a ring system in which the aryl ring is fused with one or more cycloalkyl or heterocyclyl, and the point of attachment is on the aryl ring, and in such case, the number of carbon atoms continues to represent the number of carbon atoms in the aryl ring system. The aryl groups may be optionally substituted with one or more substituents, e.g., with 1 to 5 substituents, 1 to 3 substituents, or 1 substituent.

"$C_{3-30}$ heteroaryl" refers to a group of monocyclic or bicyclic 4n+2 aromatic ring system (e.g., having 6 or 10 shared $\pi$ electrons in a cyclic array) containing 3-30 ring carbon atoms and at least one ring heteroatom, and "5- to 14-membered heteroaryl" refers to a group of 5- to 14-membered monocyclic or bicyclic 4n+2 aromatic ring system containing

ring carbon atoms and 1-5 ring heteroatoms, wherein each of heteroatoms is independently selected from nitrogen, oxygen, and sulfur. In heteroaryl groups containing one or more nitrogen atoms, the point of attachment may be carbon or nitrogen atom as long as the valence permits. The heteroaryl bicyclic system may include one or more heteroatoms in one or both of the rings. The heteroaryl groups further include a ring system in which the heteroaryl ring is fused with one or more cycloalkyl or heterocyclyl, and the point of attachment is on the heteroaryl ring, and in such case, the number of carbon atoms continues to represent the number of carbon atoms in the heteroaryl ring system. In some embodiments, $C_{3-18}$ heteroaryl and $C_{3-12}$ heteroaryl are alternative, and $C_{3-10}$ heteroaryl and $C_{3-6}$ heteroaryl are yet alternative. Exemplary 5-membered heteroaryl groups containing one heteroatom (*i.e.,* $C_4$ heteroaryl) include, but are not limited to, pyrrolyl, furyl and thienyl. Exemplary 5-membered heteroaryl groups containing two heteroatoms (*i.e.,* $C_3$ heteroaryl) include, but are not limited to, imidazolyl, pyrazolyl, oxazolyl, isoxazolyl, thiazolyl, and isothiazolyl. Exemplary 5-membered heteroaryl groups containing three heteroatoms (*i.e.,* $C_2$ heteroaryl) include, but are not limited to, triazolyl, oxadiazolyl (e.g., 1,2,4-oxadiazolyl), and thiadiazolyl. Exemplary 5-membered heteroaryl groups containing four heteroatoms (*i.e.,* $C_1$ heteroaryl) include, but are not limited to, tetrazolyl. Exemplary 6-membered heteroaryl groups containing one heteroatom (*i.e.,* $C_5$ heteroaryl) include, but are not limited to, pyridyl. Exemplary 6-membered heteroaryl groups containing two heteroatoms include, but are not limited to: pyridazinyl, pyrimidinyl and pyrazinyl. Exemplary 6-membered heteroaryl groups containing three or four heteroatoms include, but are not limited to: triazinyl or tetrazinyl, respectively. Exemplary 7-membered heteroaryl groups containing one heteroatom (*i.e.,* $C_6$ heteroaryl) include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolinyl, isoquinolinyl, cinnolinyl, quinoxalinyl, phthalazinyl and quinazolinyl. The heteroaryl group may be optionally substituted with one or more substituents, e.g., with 1 to 5 substituents, 1 to 3 substituents, or 1 substituent.

"$C_{3-10}$ cycloalkylene", "3- to 10-membered heterocyclylene", "$C_{6-30}$ arylene", and "$C_{3-30}$ heteroarylene" refer to divalent group formed by removal of another hydrogen from the $C_{3-10}$ cycloalkyl, 3- to 10-membered heterocyclyl, $C_{6-30}$ aryl, and $C_{3-30}$ heteroaryl, and may be substituted or unsubstituted.

The ring-forming groups such as cycloalkyl, heterocyclyl, aryl, and heteroaryl as defined above are collectively called "cyclic group".

The groups such as alkyl, alkenyl, alkynyl, cycloalkyl, heterocyclyl, aryl, and heteroaryl as defined herein are optionally substituted groups.

Exemplary substituents on carbon atoms include, but are not limited to, halogen, -CN, $-NO_2$, $-N_3$, $-SO_2H$, $-SO_3H$, -OH, $-OR^{aa}$, $-ON(R^{bb})_2$, $-N(R^{bb})_2$, $-N(R^{bb})_3^+X^-$, $-N(OR^{cc})R^{bb}$, -SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, $-C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, $-C(=NR^{bb})R^{aa}$, $-C(=NR^{bb})OR^{aa}$, $-OC(=NR^{bb})R^{aa}$, $-OC(=NR^{bb})OR^{aa}$, $-C(=NR^{bb})N(R^{bb})_2$, $-OC(=NR^{bb})N(R^{bb})_2$, $-NR^{bb}C(=NR^{bb})N(R^{bb})_2$, $-C(=O)NR^{bb}SO_2R^{aa}$, $-NR^{bb}SO_2R^{aa}$, $-SO_2N(R^{bb})_2$, $-SO_2R^{aa}$, $-SO_2OR^{aa}$, $-OSO_2R^{aa}$, $-S(=O)R^{aa}$, $-OS(=O)R^{aa}$, $-Si(R^{aa})_3$, $-OSi(R^{aa})_3$, $-C(=S)N(R^{bb})_2$, $-C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, $-SC(=O)R^{aa}$, $-P(=O)_2R^{aa}$, $-OP(=O)_2R^{aa}$, $-P(=O)(R^{aa})_2$, $-OP(=O)(^{Raa})^2$, $-OP(=O)(OR^{cc})_2$, $-P(=O)_2N(R^{bb})_2$, $-OP(=O)_2N(R^{bb})_2$, $-P(=O)(NR^{bb})_2$, $-OP(=O)(NR^{bb})_2$, $-NR^{bb}P(=O)(OR^{cc})_2$, $-NR^{bb}P(=O)(NR^{bb})_2$, $-P(R^{cc})_2$, $-P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, alkyl, haloalkyl, alkenyl, alkynyl, cycloalkyl, heterocyclyl, aryl and heteroaryl, wherein each of the alkyl, alkenyl, alkynyl, cycloalkyl, heterocyclyl, aryl and heteroaryl is independently substituted with 0, 1, 2, 3, 4 or 5 $R^{dd}$ groups;

or two geminal hydrogen on a carbon atom are replaced with =O, =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=NR^{bb}$ or $=NOR^{cc}$ groups;

each of the $R^{aa}$ is independently selected from alkyl, haloalkyl, alkenyl, alkynyl, cycloalkyl, heterocyclyl, aryl and heteroaryl, or two of the $R^{aa}$ groups are combined to form a heterocyclyl or heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups;

each of the $R^{bb}$ is independently selected from hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, cycloalkyl, heterocyclyl, aryl and heteroaryl, or two $R^{bb}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups;

each of the $R^{cc}$ is independently selected from hydrogen, alkyl, haloalkyl, alkenyl, alkynyl, cycloalkyl, heterocyclyl, aryl and heteroaryl, or two $R^{cc}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups;

each of the $R^{dd}$ is independently selected from halogen, -CN, $-NO_2$, $-N_3$, $-SO_2H$, $-SO_3H$, -OH, $-OR^{ee}$, - $ON(R^{ff})_2$, $-N(R^{ff})_2$, $-N(R^{ff})_3^+X^-$, $-N(OR^{ee})R^{ff}$, -SH, $-SR^{ee}$, $-SSR^{ee}$, $-C(=O)R^{ee}$, $-CO_2H$, $-CO_2R^{ee}$, $-OC(=O)R^{ee}$, - $OCO_2R^{ee}$, $-C(=O)N(R^{ff})_2$, $-OC(=O)N(R^{ff})_2$, $-NR^{ff}C(=O)R^{ee}$, $-NR^{ff}CO_2R^{ee}$, $-NR^{ff}C(=O)N(R^{ff})_2$, $-C(=NR^{ff})OR^{ee}$, - $OC(=NR^{ff})R^{cc}$, $-OC(=NR^{ff})OR^{ee}$, $-C(=NR^{ff})N(R^{ff})_2$, $-OC(=NR^{ff})N(R^{ff})_2$, $-NR^{ff}C(=NR^{ff})N(R^{ff})_2$, $-NR^{ff}SO_2R^{ee}$, - $SO_2N(R^{ff})_2$, $-SO_2R^{cc}$, $-SO_2OR^{ee}$, $-OSO_2R^{ee}$, $-S(=O)R^{ee}$, $-Si(R^{ee})_3$, $-OSi(R^{ee})_3$, $-C(=S)N(R^{ff})_2$, $-C(=O)SR^{ee}$, - $C(=S)SR^{ee}$, $-SC(=S)SR^{ee}$, $-P(=O)_2R^{ee}$, $-P(=O)(R^{ee})_2$, $-OP(=O)(R^{cc})_2$, $-OP(=O)(OR^{cc})_2$, alkyl, haloalkyl, alkenyl, alkynyl, cycloalkyl, heterocyclyl, aryl, and heteroaryl, wherein, each of the alkyl, alkenyl, alkynyl, cycloalkyl, heterocyclyl, aryl and heteroaryl is independently substituted with 0, 1, 2, 3, 4 or 5 $R^{gg}$ groups, or two geminal $R^{dd}$ substituents can be combined to form=O or =S;

each of the $R^{ee}$ is independently selected from alkyl, haloalkyl, alkenyl, alkynyl, cycloalkyl, aryl, heterocyclyl, and heteroaryl, wherein, each of the alkyl, alkenyl, alkynyl, cycloalkyl, heterocyclyl, aryl and heteroaryl is independently substituted with 0, 1, 2, 3, 4 or 5 $R^{gg}$ groups;

each of the $R^{ff}$ is independently selected from hydrogen, alkyl, haloalkyl, alkenyl, alkynyl, cycloalkyl, heterocyclyl, aryl and heteroaryl, or two $R^{ff}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{gg}$ groups;

each of the $R^{gg}$ is independently halogen, -CN, $-NO_2$, $-N_3$, $-SO_2H$, $-SO_3H$, -OH, $-OC_{1-6}$ alkyl, $-ON(C_{1-6}$ alkyl$)_2$, $-N(C_{1-6}$ alkyl$)_2$, $-N(C_{1-6}$ alkyl$)_3^+X^-$, $-NH(C_{1-6}$ alkyl$)_2^+X^-$, $-NH_2(C_{1-6}$ alkyl$)^+X^-$, $-NH_3^+X^-$, $-N(OC_{1-6}$ alkyl$)(C_{1-6}$ alkyl), $-N(OH)(C_{1-6}$ alkyl), -NH(OH), -SH, $-SC_{1-6}$ alkyl, $-SS(C_{1-6}$ alkyl), $-C(=O)(C_{1-6}$ alkyl), $-CO_2H$, $-CO_2(C_{1-6}$ alkyl), - $OC(=O)(C_{1-6}$ alkyl), $-OCO_2(C_{1-6}$ alkyl), $-C(=O)NH_2$, $-C(=O)N(C_{1-6}$ alkyl$)_2$, $-OC(=O)NH(C_{1-6}$ alkyl), - $NHC(=O)(C_{1-6}$ alkyl), $-N(C_{1-6}$ alkyl) $C(=O)(C_{1-6}$ alkyl), $-NHCO_2(C_{1-6}$ alkyl), $-NHC(=O)N(C_{1-6}$ alkyl$)_2$, - $NHC(=O)NH(C_{1-6}$ alkyl), $-NHC(=O)NH_2$, -C(=NH) $O(C_{1-6}$ alkyl), $-OC(=NH)(C_{1-6}$ alkyl), $-OC(=NH)OC_{1-6}$ alkyl, $-C(=NH)N(C_{1-6}$ alkyl$)_2$, $-C(=NH)NH(C_{1-6}$ alkyl), -C(=NH) $NH_2$, $-OC(=NH)N(C_{1-6}$ alkyl$)_2$, $-OC(NH)NH(C_{1-6}$ alkyl), $-OC(NH)NH_2$, $-NHC(NH)N(C_{1-6}$ alkyl$)_2$, $-NHC(=NH)NH_2$, $-NHSO_2(C_{1-6}$ alkyl), $-SO_2N(C_{1-6}$ alkyl$)_2$, - $SO_2NH(C_{1-6}$ alkyl), $-SO_2NH_2$, $-SO_2C_{1-6}$ alkyl, $-SO_2OC_{1-6}$ alkyl, $-OSO_2C_{1-6}$ alkyl, $-SOC_{1-6}$ alkyl, $-Si(C_{1-6}$ alkyl$)_3$, - $OSi(C_{1-6}$ alkyl$)_3$, $-C(=S)N(C_{1-6}$ alkyl$)_2$, $C(=S)NH(C_{1-6}$ alkyl), $C(=S)NH_2$, -C(=O) $S(C_{1-6}$ alkyl), $-C(=S)SC_{1-6}$ alkyl, $-SC(=S)SC_{1-6}$ alkyl, $-P(=O)_2(C_{1-6}$ alkyl), $-P(=O)(C_{1-6}$ alkyl$)_2$, $-OP(=O)(C_{1-6}$ alkyl$)_2$, $-OP(=O)(OC_{1-6}$ alkyl$)_2$, $C_{1-6}$ alkyl, $C_{1-6}$ haloalkyl, $C_2$-$C_6$ alkenyl, $C_2$-$C_6$ alkynyl, $C_3$-$C_7$ cycloalkyl, $C_6$-$C_{10}$ aryl, $C_3$-$C_7$ heterocyclyl, or $C_5$-$C_{10}$ heteroaryl; or two geminal $R^{gg}$ substituents may combine to form =O or =S; wherein, $X^-$ is a counter-ion.

Exemplary substituents on nitrogen atoms include, but are not limited to, hydrogen, -OH, $-OR^{aa}$, $-N(R^{cc})_2$, - CN, $-C(=O)R^{aa}$, $-C(=O)N(R^{cc})_2$, $-CO_2R^{aa}$, $-SO_2R^{aa}$, $-C(=NR^{bb})R^{aa}$, $-C(=NR^{cc})OR^{aa}$, $-C(=NR^{cc})N(R^{cc})_2$, - $SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, - $P(=O)_2N(R^{cc})_2$, $-P(=O)(NR^{cc})_2$, alkyl, haloalkyl, alkenyl, alkynyl, cycloalkyl, heterocyclyl, aryl and heteroaryl, or two $R^{cc}$ groups attached to a nitrogen atom combine to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups, and wherein $R^{aa}$, $R^{bb}$, $R^{cc}$ and $R^{dd}$ are as described herein.

Other Definitions

The term "treating" as used herein relates to reversing, alleviating or inhibiting the progression or prevention of the disorders or conditions to which the term applies, or of one or more symptoms of such disorders or conditions. The noun "treatment" as used herein relates to the action of treating, which is a verb, and the latter is as just defined.

The term "pharmaceutically acceptable salt" as used herein refers to those carboxylate and amino acid addition salts of the compounds of the present disclosure, which are suitable for the contact with patients' tissues within a reliable medical judgment, and do not produce inappropriate toxicity, irritation, allergy, etc. They are commensurate with a reasonable benefit/risk ratio, and are effective for their intended use. The term includes, if possible, the zwitterionic form of the compounds of the disclosure.

The pharmaceutically acceptable base addition salts are formed with metals or amines, such as alkali metal and alkaline earth metal hydroxides or organic amines. Examples of the metals used as cations include sodium, potassium, magnesium, calcium, etc. Examples of suitable amines are N,N'-dibenzylethylenediamine, chloroprocaine, choline, diethanolamine, ethylenediamine, N-methylglucamine and procaine.

The base addition salt of the acidic compound can be prepared by contacting the free acid form with a sufficient amount of the required base to form a salt in a conventional manner. The free acid can be regenerated by contacting the salt form with an acid in a conventional manner and then isolating the free acid. The free acid forms are somewhat different from their respective salt forms in their physical properties, such as solubility in polar solvents. But for the purposes of the present disclosure, the salts are still equivalent to their respective free acids.

The salts can be prepared from the inorganic acids, which include sulfates, pyrosulfates, bisulfates, sulfites,

bisulfites, nitrates, phosphates, monohydrogen phosphates, dihydrogen phosphates, metaphosphates, pyrophosphates, chlorides, bromides and iodides. Examples of the acids include hydrochloric acid, nitric acid, sulfuric acid, hydrobromic acid, hydroiodic acid, phosphoric acid, etc. The representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthalate, methanesulfonate, glucoheptanate, lactobionate, lauryl sulfonate, isethionate, etc. The salts can also be prepared from the organic acids, which include aliphatic monocarboxylic and dicarboxylic acids, phenyl-substituted alkanoic acids, hydroxyalkanoic acids, alkanedioic acid, aromatic acids, aliphatic and aromatic sulfonic acids, etc. The representative salts include acetate, propionate, octanoate, isobutyrate, oxalate, malonate, succinate, suberate, sebacate, fumarate, maleate, mandelate, benzoate, chlorobenzoate, methyl benzoate, dinitrobenzoate, naphthoate, besylate, tosylate, phenylacetate, citrate, lactate, maleate, tartrate, methanesulfonate, etc. The pharmaceutically acceptable salts can include cations based on alkali metals and alkaline earth metals, such as sodium, lithium, potassium, calcium, magnesium, etc., as well as non-toxic ammonium, quaternary ammonium, and amine cations including, but not limited to, ammonium, tetramethylammonium, tetraethylammonium, methylamine, dimethylamine, trimethylamine, triethylamine, ethylamine, etc. Salts of amino acids are also included, such as arginine salts, gluconates, galacturonates, etc. (for example, see Berge S. M. et al., "Pharmaceutical Salts," J. Pharm. Sci., 1977; 66: 1- 19 for reference).

"Subject in need thereof" to which administration is contemplated include, but are not limited to, humans (e.g., males or females of any age group, e.g., paediatric subjects (e.g., infants, children, adolescents) or adult subjects (e.g., young adults, middle-aged adults or older adults)) and/or non-human animals, such as mammals, e.g., primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "human", "patient", "subject in need thereof" and "subject" can be used interchangeably herein.

"Disease," "disorder," and "condition" can be used interchangeably herein.

Unless indicated, otherwise the term "treatment" as used herein includes the effect on a subject who is suffering from a particular disease, disorder, or condition, which reduces the severity of the disease, disorder, or condition, or delays or slows the progression of the disease, disorder or condition ("therapeutic treatment"). The term also includes the effect that occurs before the subject begins to suffer from a specific disease, disorder or condition ("prophylactic treatment").

Generally, "effective dose" or "effective amount" of a compound refers to an amount sufficient to elicit a target biological response. As understood by those skilled in the art, the effective amount of the compound of the disclosure can vary depending on the following factors, such as the desired biological endpoint, the pharmacokinetics of the compound, the diseases being treated, the mode of administration, and the age, health status and symptoms of the subjects. The effective amount includes therapeutically effective amount and prophylactically effective amount.

Unless indicated, otherwise the "therapeutically effective amount" of the compound as used herein is an amount sufficient to provide therapeutic benefits in the course of treating a disease, disorder or condition, or to delay or minimize one or more symptoms associated with the disease, disorder or condition. The therapeutically effective amount of a compound refers to the amount of the therapeutic agent that, when used alone or in combination with other therapies, provides a therapeutic benefit in the treatment of a disease, disorder or condition. The term "therapeutically effective amount" can include an amount that improves the overall treatment, reduces or avoids the symptoms or causes of the disease or condition, or enhances the therapeutic effect of other therapeutic agents.

Unless indicated, otherwise the "prophylactically effective amount" of the compound as used herein is an amount sufficient to prevent a disease, disorder or condition, or an amount sufficient to prevent one or more symptoms associated with a disease, disorder or condition, or an amount sufficient to prevent the recurrence of a disease, disorder or condition. The prophylactically effective amount of a compound refers to the amount of a therapeutic agent that, when used alone or in combination with other agents, provides a prophylactic benefit in the prevention of a disease, disorder or condition. The term "prophylactically effective amount" can include an amount that improves the overall prevention, or an amount that enhances the prophylactic effect of other preventive agents.

"Combination" and related terms refer to the simultaneous or sequential administration of the compounds of the present disclosure and other therapeutic agents. For example, the compounds of the present disclosure can be administered simultaneously or sequentially in separate unit dosage with other therapeutic agents, or simultaneously in a single unit dosage with other therapeutic agents.

## DETAILED DESCRIPTION

In the present disclosure, "the compound of the present disclosure" refers to a compound of the following Formula (A) or Formula (I) (including sub-general formulae), or a pharmaceutically acceptable salt, ester, stereoisomer, polymorph, solvate, N-oxide, isotope-labeled compound, metabolite, or pro-drug thereof.

In the present disclosure, compounds are named using standard nomenclature. For compounds having an asymmetric center, it should be understood, unless otherwise stated, that all optical isomers and mixtures thereof are

included. Furthermore, unless otherwise specified, all isomer compounds and carbon-carbon double bonds included in the present disclosure may occur in the form of Z and E. For compounds that can exist in different tautomeric forms, a described compound is not limited to any specific tautomer but is intended to encompass all tautomeric forms.

In some embodiments, the present disclosure relates to a compound of Formula A, or a pharmaceutically acceptable salt, ester, stereoisomer, polymorph, solvate, N-oxide, isotope-labeled compound, metabolite, or prodrug thereof, and a mixture thereof:

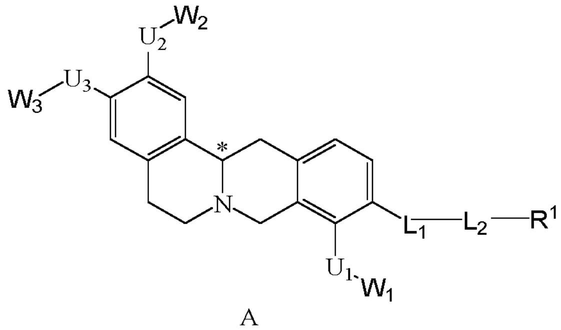

A

wherein

* represents a chiral center, and is selected from (S)-configuration or (R)-configuration;
$L_1$ is selected from a single bond, -O-, -NH-, -S- or -P(O)OH-; alternatively a single bond, -O-, -NH-;
$L_2$ is selected from a single bond, -O-, -NH-, -C(O)-, -C(O)-O-, -C(O)NH-, -OC(O)-,
$C_{l-18}$ alkylene (alternatively $C_{1-12}$ alkylene, and yet alternatively $C_{1-6}$ alkylene),
$C_{3-10}$ cycloalkylene (alternatively $C_{3-8}$ cycloalkylene, and yet alternatively $C_{3-6}$ cycloalkylene),
$C_{6-30}$ arylene (alternatively $C_{6-18}$ arylene, and yet alternatively $C_{6-12}$ arylene),
$C_{3-30}$ heteroarylene (alternatively $C_{3-18}$ heteroarylene, and yet alternatively $C_{3-12}$ heteroarylene),
-$C_{1-18}$ alkylene-O- (alternatively -$C_{1-12}$ alkylene-O-, and yet alternatively -$C_{1-6}$ alkylene-O-),
-$C_{6-30}$ arylene-O- (alternatively -$C_{6-18}$ arylene-O-, and yet alternatively -$C_{6-12}$ arylene-O-),
-$C_{3-30}$ heteroarylene-O- (alternatively -$C_{3-18}$ heteroarylene-O-, and yet alternatively -$C_{3-12}$ heteroarylene-O-),
-C(O)-$C_{1-18}$ alkylene-,
-C(O)-$C_{1-18}$ alkylene-NHC(O)-$C_{1-18}$ alkylene-,
-C(O)-$C_{1-18}$ alkylene-C(O)NH-$C_{1-18}$ alkylene-,
$C_{1-30}$ alkyleneoxy, or
-$X_1$-PRa(O)-$X_2$-; the Ra is each independently selected from hydrogen, hydroxyl, halogen (alternatively F, Cl or Br), $C_{6-30}$ aryl (alternatively $C_{6-18}$ aryl, and yet alternatively $C_{6-12}$ aryl), $C_{3-30}$ heteroaryl (alternatively $C_{3-18}$ heteroaryl, and yet alternatively $C_{3-12}$ heteroaryl), $C_{1-30}$ hydrocarbon radical (alternatively $C_{1-30}$ alkyl, and yet alternatively $C_{1-18}$ alkyl, and further alternatively $C_{1-6}$ alkyl), $C_{3-10}$ cycloalkyl (alternatively $C_{3-8}$ cycloalkyl, and yet alternatively $C_{3-6}$ cycloalkyl), $C_{1-30}$ alkoxy (alternatively $C_{1-18}$ alkoxy, and yet alternatively $C_{1-6}$ alkoxy), $C_{6-30}$ aryl-O- (alternatively $C_{6-18}$ aryl-O-, and yet alternatively $C_{6-12}$ aryl-O-), $C_{3-30}$ heteroaryl-O- (alternatively $C_{3-18}$ heteroaryl-O-, and yet alternatively $C_{3-12}$ heteroaryl-O-), $C_{6-30}$ aryl-$C_{1-18}$ alkylene-O- (alternatively $C_{6-18}$ aryl-$C_{1-12}$ alkylene-O-, and yet alternatively $C_{6-12}$ aryl-$C_{1-6}$ alkylene-O-), $C_{3-30}$ heteroaryl-$C_{1-18}$ alkylene-O- (alternatively $C_{3-18}$ heteroaryl-$C_{1-12}$ alkylene-O-, and yet alternatively $C_{3-12}$ heteroaryl-$C_{1-6}$ alkylene-O-), -O-$C_{6-30}$ aryl-$C_{1-18}$ alkylene- (alternatively -O-$C_{6-18}$ aryl-$C_{1-12}$ alkylene-) or -O-$C_{3-30}$ heteroaryl-$C_{1-18}$ alkylene- (alternatively -O-$C_{3-18}$ heteroaryl-$C_{1-12}$ alkylene-); the Ra and $X_2$ may be connected to form a ring;
the $X_1$ is selected from a single bond, -O-, -$C_{1-18}$ alkylene-O- (alternatively -$C_{1-12}$ alkylene-O-, and yet alternatively -$C_{1-6}$ alkylene-O-), -$C_{6-30}$ arylene-O- (alternatively -$C_{6-18}$ arylene-O-, and yet alternatively -$C_{6-12}$ arylene-O-), or -$C_{3-30}$ heteroarylene-O- (alternatively -$C_{3-18}$ heteroarylene-O-, and yet alternatively -$C_{3-12}$ heteroarylene-O-);
the $X_2$ is selected from a single bond, -O- or -NR*-; wherein R* is selected from H, $C_{1-6}$ alkyl, and $C_{1-6}$ alkoxy;
the $L_2$ is optionally substituted with 1, 2, 3, 4, or 5 substituents selected from halogen, OH, CN, C(O)OH, C(O)O-$C_{1-6}$ alkyl, $C_{1-6}$ alkyl, $C_{1-6}$ haloalkyl, $C_{1-6}$ alkoxy, or $C_{1-6}$ haloalkoxy;
$R^1$ is selected from absent or hydrogen, hydroxyl, amino unsubstituted or substituted with 1 or 2 Rb, unsubstituted or Rb-substituted $C_{1-30}$ hydrocarbon radical (alternatively $C_{1-30}$ alkyl or $C_{2-30}$ alkenyl, yet alternatively $C_{l-18}$ alkyl or $C_{2-18}$ alkenyl, further alternatively $C_{1-6}$ alkyl or $C_{2-12}$ alkenyl, wherein the alkenyl has a double-bond number of 1-10, alternatively 1-6), $C_{3-10}$ cycloalkyl (alternatively $C_{3-8}$ cycloalkyl, yet alternatively $C_{3-6}$ cycloalkyl) unsubstituted or substituted with 1, 2, 3, 4 or 5 Rb, unsubstituted or Rb-substituted $C_{1-30}$ alkoxy (alternatively $C_{1-20}$ alkoxy, yet alternatively $C_{1-12}$ alkoxy), $C_{12-30}$ condensed cyclic group (alternatively $C_{12-17}$ condensed cyclic group, yet alter-

natively $C_{12-17}$ condensed cyclic group containing at least one heteroatom, further alternatively $C_{12-17}$ tetracondensed cyclic group containing at least one heteroatom) unsubstituted or substituted with 1, 2, 3, 4 or 5 Rb, $C_{1-18}$ alkyl-$C_{6-30}$ arylene-$C_{1-18}$ alkylene- (alternatively $C_{1-12}$ alkyl-$C_{6-18}$ arylene-$C_{1-12}$ alkylene-) which is unsubstituted or has a hydrogen atom replaced with Rc in the alkyl, $C_{1-12}$ alkyl-O-C(O)-$C_{1-12}$ alkylene-imino-, which is unsubstituted or has a hydrogen atom replaced with Rd in the alkyl, $C_{6-30}$ aryl-$C_{1-18}$ alkylene- (alternatively $C_{6-18}$ aryl-$C_{1-12}$ alkylene-), -O-C(O)-$Y_1$, -$C_{1-12}$ alkylene-C(O)-O-$Y_2$ which is unsubstituted or has a hydrogen atom replaced with Rd in the alkylene, -O-$C_{1-12}$ alkylene-O-C(O)-$Y_3$ which is unsubstituted or has a hydrogen atom replaced with Rd in the alkylene, -C=CH-$Y_4$ or -$Y_5$-$Y_6$-$Y_7$, -$CHR^{\#}R^{\#\#}$, -Z-$C_{6-30}$ aryl (alternatively -Z-$C_{6-18}$ aryl, yet alternatively -Z-$C_{6-12}$ aryl, e.g., -Z-phenyl) optionally substituted with *x* A; wherein $R^{\#}$ and $R^{\#\#}$ are each independently selected from H, hydroxyl, amino, halogen, alkyl, cycloalkyl, aryl, heteroaryl,

-C(O)-$OR^2$, -OC(O)-$OR^2$, -$C_{1-18}$ alkylene-C(O)-$OR^2$, -$C_{1-18}$ alkylene-$R^2$, or -$C_{6-10}$ aryl-$C_{1-6}$ alkyl;

$R^2$ is selected from H, hydroxyl, amino, halogen, alkyl, cycloalkyl, aryl, heteroaryl, unsubstituted or Rb-substituted $C_{12-30}$ condensed cyclic group, or -NHC(NH)NH-;

the Rb is each independently selected from $C_{1-30}$ alkoxy (alternatively $C_{1-20}$ alkoxy, yet alternatively $C_{1-12}$ alkoxy, and further alternatively $C_{1-3}$ alkoxy), $C_{6-30}$ aryl (alternatively $C_{6-18}$ aryl, yet alternatively $C_{6-12}$ aryl), $C_{3-30}$ heteroaryl (alternatively $C_{3-18}$ heteroaryl, yet alternatively $C_{3-12}$ heteroaryl), or $C_{1-30}$ alkyl (alternatively $C_{1-18}$ alkyl, yet alternatively $C_{1-6}$ alkyl), which are unsubstituted or substituted with halogen (alternatively F, Cl, or Br);

the Rc is each independently selected from $C_{3-10}$ cycloalkyl (alternatively $C_{3-8}$ cycloalkyl, and yet alternatively $C_{3-6}$ cycloalkyl, and further alternatively cyclopentyl) which is unsubstituted or substituted with hydroxyl, oxo, or thioxo;

the Rd is each independently selected from $C_{1-18}$ alkyl (alternatively $C_{1-12}$ alkyl, and yet alternatively $C_{1-6}$ alkyl), $C_{1-30}$ alkoxy (alternatively $C_{1-20}$ alkoxy, and yet alternatively $C_{1-12}$ alkoxy, and further alternatively $C_{1-3}$ alkoxy), $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with halogen (alternatively F, Cl or Br);

the $Y_1$ is selected from $C_{1-30}$ alkyl (alternatively $C_{1-12}$ alkyl, and yet alternatively $C_{1-6}$ alkyl), $C_{3-10}$ cycloalkyl (alternatively $C_{3-8}$ cycloalkyl, and yet alternatively $C_{3-6}$ cycloalkyl), $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with Re; the Re is each independently selected from $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with halogen (alternatively F, Cl or Br);

the $Y_2$ is selected from unsubstituted or Rf-substituted $C_{12-30}$ condensed cyclic group (alternatively $C_{12-17}$ condensed cyclic group, and yet alternatively $C_{12-17}$ condensed cyclic group containing at least one heteroatom, and further alternatively $C_{12-17}$ tetracondensed cyclic group containing at least one heteroatom); wherein the Rf is each independently selected from $C_{1-30}$ alkoxy (alternatively $C_{1-20}$ alkoxy, and yet alternatively $C_{1-12}$ alkoxy, and further alternatively $C_{1-3}$ alkoxy), or $C_{6-30}$ aryl (alternatively $C_{6-18}$ aryl, and yet alternatively $C_{6-12}$ aryl), which are unsubstituted or substituted with halogen (alternatively F, Cl or Br);

the $Y_3$ is selected from $C_{1-18}$ alkyl (alternatively $C_{1-12}$ alkyl, and yet alternatively $C_{1-6}$ alkyl) or -O-$C_{1-18}$ alkyl (alternatively -O-$C_{1-12}$ alkyl, and yet alternatively -O-$C_{1-6}$ alkyl);

the $Y_4$ is selected from $C_{6-30}$ aryl (alternatively $C_{6-18}$ aryl, and yet alternatively $C_{6-12}$ aryl) or $C_{3-30}$ heteroaryl (alternatively $C_{3-18}$ heteroaryl, and yet alternatively $C_{3-12}$ heteroaryl), which are unsubstituted or substituted with Rg, wherein the Rg is each independently selected from hydroxyl and $C_{1-30}$ alkoxy (alternatively $C_{1-20}$ alkoxy, and yet alternatively $C_{1-12}$ alkoxy, and further alternatively $C_{1-3}$ alkoxy);

the $Y_5$ is selected from a single bond, unsubstituted or amino-substituted $C_{1-18}$ alkylene (alternatively $C_{1-12}$ alkylene, and yet alternatively $C_{3-6}$ alkylene);

the $Y_6$ is selected from a single bond, -NH-C(NH)-, $-(O-CH_2-CH_2)_m-$ or $-CH_2-(O-CH_2-CH_2)_n-$, and the *m* and the *n* are each independently selected from any natural number of 2-20 (alternatively any natural number of 2-10);

the $Y_7$ is selected from amino, $C_{1-30}$ alkoxy (alternatively $C_{1-20}$ alkoxy, and yet alternatively $C_{1-12}$ alkoxy, and further alternatively $C_{1-3}$ alkoxy) or hydroxyl;

*x* is selected from 0, 1, 2, 3, 4, or 5;

Z is selected from a chemical bond, -O-, -NH-, -$C_{1-12}$ alkylene-, -$C_{1-12}$ alkylene-O-$C_{1-12}$ alkylene-, -$C_{1-12}$ alkylene-NH-$C_{1-12}$ alkylene-, -$C_{1-18}$ alkylene-C(O)-$C_{1-12}$ alkylene-, -$C_{1-10}$ alkylene-C(O)O-$C_{1-10}$ alkylene-, -$C_{1-10}$ alkylene-NHC(O)-$C_{1-10}$ alkylene-, -$C_{1-10}$ alkylene-NHC(O)O-$C_{1-10}$ alkylene-, -$C_{1-10}$ alkylene-C(O)NH-$C_{1-10}$ alkylene- or -$C_{1-10}$ alkylene-OC(O)NH-$C_{1-10}$ alkylene-, alternatively a chemical bond or -$C_{1-6}$ alkylene-NHC(O)-$C_{1-6}$ alkylene-;

the Z is optionally substituted with 1, 2, 3, 4, or 5 substituents selected from halogen, -C(O)OH, -C(O)O-$C_{1-6}$ alkyl, -C(O)-$C_{1-6}$ alkyl, -O-$C_{1-6}$ alkyl, $C_{1-6}$ alkyl, or $C_{1-6}$ haloalkyl;

A is independently selected from H, halogen, OH, -$L_A$-$C_{3-10}$ cycloalkyl, -$L_A$-3- to 10-membered heterocyclyl, -$L_A$-$C_{6-12}$ aryl or -$L_A$-5- to 12-membered heteroaryl, alternatively halogen, $C_{6-10}$ aryl or -NH-$C_{6-10}$ aryl;

$L_A$ is selected from a chemical bond, -NH-, -O-, -C(O)-, -C(O)O-, -NHC(O)-, -NHC(O)O-, -OC(O)NH- or - C(O)NH-;

the A is optionally further substituted with halogen, $C_{1-6}$ alkyl or $C_{1-6}$ haloalkyl;

when the $L_1$ and the $L_2$ are both selected from a single bond, the $R^1$ is not hydroxyl;
$U_1$, $U_2$ and $U_3$ are each independently selected from -O-C(O)-, -NH-C(O)-, -O-$CH_2$-O-, and -O-; or an available ring atom on $U_2$ and an available ring atom on $U_3$ are connected via Q group to form a 4- to 8-membered ring, alternatively a 5- to 6-membered ring,
Q is selected from a single bond; $NR^3$; $C_{1-3}$ alkylene in which 1 or 2 $CH_2$ are optionally independently replaced with a group selected from O, S and $NR^3$; and $C_{2-3}$ alkenylene in which any CH for forming the C=C double bond is optionally replaced with N;
$W_1$, $W_2$ and $W_3$ are each independently selected from $C_{1-18}$ alkyl (alternatively $C_{1-12}$ alkyl, and yet alternatively $C_{1-6}$ alkyl) or -(O-$C_{2-6}$ alkylene)$_p$ (alternatively -(O-$C_{2-4}$ alkylene)$_p$, and yet alternatively -(O-$CH_2$-$CH_2$)$_p$), wherein the *p* is selected from any natural number of 1-18 alternatively any natural number of 1-10;
the above alkyl, alkylene, alkenyl, alkenylene, alkynyl, cyclic hydrocarbon radical, heterocyclyl, aryl, heteroaryl, and aralkyl at each occurrence are each optionally substituted with 1, 2, 3 or more $R^3$, wherein the $R^3$ at each occurrence is independently selected from halogen, cyano, nitro, $C_{1-6}$ alkyl, $C_{3-10}$ cyclic hydrocarbon radical, 3- to 10-membered heterocyclyl, $C_{6-10}$ aryl, 5- to 14-membered heteroaryl, $C_{6-12}$ aralkyl; and wherein the alkyl, alkylene, cyclic hydrocarbon radical, heterocyclyl, aryl, heteroaryl, and aralkyl in relation to the substituent $R^3$ are further optionally substituted with 1, 2, 3 or more substituents independently selected from halogen, OH, oxo, amino, cyano, nitro, $C_{1-6}$ alkyl, $C_{1-6}$ haloalkyl, $C_{3-6}$ cyclic hydrocarbon radical, 3- to 10-membered heterocyclyl, $C_{6-10}$ aryl, 5- to 14-membered heteroaryl, and $C_{6-12}$ aralkyl.

In some embodiments, the present disclosure relates to a compound of Formula A-1 or A-2, or a pharmaceutically acceptable salt, ester, stereoisomer, polymorph, solvate, N-oxide, isotope-labeled compound, metabolite, or pro-drug thereof, and a mixture thereof:

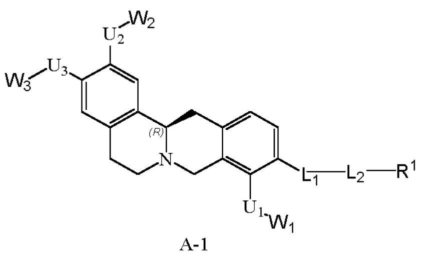

A-1

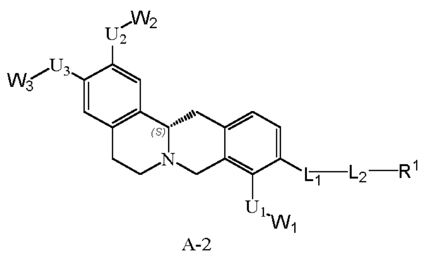

A-2

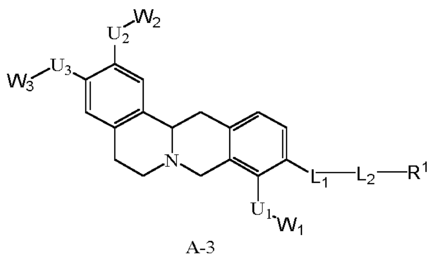

A-3

wherein various variables are as defined herein.

$L_1$

In an embodiment, $L_1$ is a single bond; in another embodiment, $L_1$ is -O-; in another embodiment, $L_1$ is -NH-; in another embodiment, $L_1$ is -S-; and in another embodiment, $L_1$ is -P(O)OH-.

$L_2$

In an embodiment, $L_2$ is a single bond; in another embodiment, $L_2$ is -O-; in another embodiment, $L_2$ is -NH-; in another embodiment, $L_2$ is -C(O)-; in another embodiment, $L_2$ is -C(O)-O-; in another embodiment, $L_2$ is - C(O)NH-; in another embodiment, $L_2$ is -OC(O)-; in another embodiment, $L_2$ is $C_{1-18}$ alkylene; in another embodiment, $L_2$ is $C_{1-12}$ alkylene; in another embodiment, $L_2$ is $C_{1-6}$ alkylene; in another embodiment, $L_2$ is $C_{3-10}$ cycloalkylene; in another embodiment, $L_2$ is $C_{3-8}$ cycloalkylene; in another embodiment, $L_2$ is $C_{3-6}$ cycloalkylene; in another embodiment, $L_2$ is $C_{6-30}$ arylene; in another embodiment, $L_2$ is $C_{6-18}$ arylene; in another embodiment, $L_2$ is $C_{6-12}$ arylene; in another embodiment, $L_2$ is $C_{3-30}$ heteroarylene; in another embodiment, $L_2$ is $C_{3-18}$ heteroarylene; in another embodiment, $L_2$ is $C_{3-12}$

heteroarylene; in another embodiment, $L_2$ is -$C_{1-18}$ alkylene-O-; in another embodiment, $L_2$ is -$C_{1-12}$ alkylene-O-; in another embodiment, $L_2$ is -$C_{1-6}$ alkylene-O-; in another embodiment, $L_2$ is -$C_{6-30}$ arylene-O-; in another embodiment, $L_2$ is -$C_{6-18}$ arylene-O-; in another embodiment, $L_2$ is -$C_{6-12}$ arylene-O-; in another embodiment, $L_2$ is -$C_{3-30}$ heteroarylene-O-; in another embodiment, $L_2$ is -$C_{3-18}$ heteroarylene-O; in another embodiment, $L_2$ is -$C_{3-12}$ heteroarylene-O-, e.g., -$C_{3-6}$ heteroarylene-O-; in another embodiment, $L_2$ is -C(O)-$C_{1-18}$ alkylene-; in another embodiment, $L_2$ is -C(O)-$C_{1-12}$ alkylene-; in another embodiment, $L_2$ is -C(O)-$C_{1-6}$ alkylene-; in another embodiment, $L_2$ is -C(O)-$C_{1-18}$ alkylene-NHC(O)-$C_{1-18}$ alkylene-; in another embodiment, $L_2$ is -C(O)-$C_{1-12}$ alkylene-NHC(O)-$C_{1-12}$ alkylene-; in another embodiment, $L_2$ is -C(O)-$C_{1-6}$ alkylene-NHC(O)-$C_{1-6}$ alkylene-; in another embodiment, $L_2$ is -C(O)-$C_{1-18}$ alkylene-C(O)NH-$C_{1-18}$ alkylene-; in another embodiment, $L_2$ is -C(O)-$C_{1-12}$ alkylene-C(O)NH-$C_{1-12}$ alkylene-; in another embodiment, $L_2$ is -C(O)-$C_{1-6}$ alkylene-C(O)NH-$C_{1-6}$ alkylene-; in another embodiment, $L_2$ is $C_{1-30}$ alkyleneoxy; in another embodiment, $L_2$ is $C_{1-18}$ alkyleneoxy; in another embodiment, $L_2$ is $C_{1-12}$ alkyleneoxy; in another embodiment, $L_2$ is $C_{1-6}$ alkyleneoxy; and in another embodiment, $L_2$ is -$X_1$-PRa(O)-$X_2$-.

In an embodiment, $L_2$ is unsubstituted; and in another embodiment, $L_2$ is optionally substituted with 1, 2, 3, 4, or 5 substituents selected from halogen, OH, CN, C(O)OH, C(O)O-$C_{1-6}$ alkyl, $C_{1-6}$ alkyl, $C_{1-6}$ haloalkyl, $C_{1-6}$ alkoxy, or $C_{1-6}$ haloalkoxy.

In a specific embodiment, $L_2$ is -$C_{1-12}$ alkylene-, and the alkylene may be further substituted with $C_{1-6}$ alkyl.

In a specific embodiment, $L_2$ is -$CH_2$-; in another specific embodiment, $L_2$ is -$CH(CH_3)$-; and in another specific embodiment, $L_2$ is

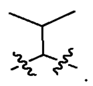

$L_3$

In an embodiment, $L_3$ is a chemical bond; in another embodiment, $L_3$ is $C_{1-12}$ alkylene; in another embodiment, $L_3$ is -$C_{1-6}$- alkylene-; in another embodiment, $L_3$ is -$CH_2$-; in another embodiment, $L_3$ is - $CH_2CH_2CH_2$-; and in another embodiment, $L_3$ is -$CH_2CH_2CH_2CH_2CH_2CH_2$-.

$X_1$

In an embodiment, $X_1$ is a single bond; in another embodiment, $X_1$ is -O-; in another embodiment, $X_1$ is -$C_{1-18}$ alkylene-O-, alternatively -$C_{1-12}$ alkylene-O-, and yet alternatively -$C_{1-6}$ alkylene-O-; in another embodiment, $X_1$ is -$C_{6-30}$ arylene-O-, alternatively -$C_{6-18}$ arylene-O-, and yet alternatively -$C_{6-12}$ arylene-O-; and in another embodiment, $X_1$ is -$C_{3-30}$ heteroarylene-O-, alternatively -$C_{3-18}$ heteroarylene-O-, and yet alternatively -$C_{3-12}$ heteroarylene-O-.

$X_2$

In an embodiment, $X_2$ is a single bond; in another embodiment, $X_2$ is -O-; and in another embodiment, $X_2$ is -NR*-.

In an embodiment, R* is selected from H, $C_{1-6}$ alkyl, $C_{1-6}$ alkoxy.

Ra

In an embodiment, Ra is hydrogen; in another embodiment, Ra is hydroxyl; in another embodiment, Ra is halogen, alternatively F, Cl or Br; in another embodiment, Ra is $C_{6-30}$ aryl, alternatively $C_{6-18}$ aryl, and yet alternatively $C_{6-12}$ aryl; in another embodiment, Ra is $C_{3-30}$ heteroaryl, alternatively $C_{3-18}$ heteroaryl, and yet alternatively $C_{3-12}$ heteroaryl; in another embodiment, Ra is $C_{1-30}$ hydrocarbon radical, alternatively $C_{1-30}$ alkyl, and yet alternatively $C_{1-18}$ alkyl, and further alternatively $C_{1-6}$ alkyl, e.g., methyl or ethyl; in another embodiment, Ra is $C_{3-10}$ cycloalkyl, alternatively $C_{3-8}$ cycloalkyl, and yet alternatively $C_{3-6}$ cycloalkyl; in another embodiment, Ra is $C_{1-30}$ alkoxy, alternatively $C_{1-18}$ alkoxy, and yet alternatively $C_{1-6}$ alkoxy; in another embodiment, Ra is $C_{6-30}$ aryl-O-, alternatively $C_{6-18}$ aryl-O-, and yet alternatively $C_{6-12}$ aryl-O-; in another embodiment, Ra is $C_{3-30}$ heteroaryl-O-, alternatively $C_{3-18}$ heteroaryl-O-, and yet alternatively $C_{3-12}$ heteroaryl-O-; in another embodiment, Ra is $C_{6-30}$ aryl-$C_{1-18}$ alkylene-O-, alternatively $C_{6-18}$ aryl-$C_{1-12}$ alkylene-O-, and yet alternatively $C_{6-12}$ aryl-$C_{1-6}$ alkylene-O-; in another embodiment, Ra is $C_{3-30}$ heteroaryl-$C_{1-18}$ alkylene-O-, alternatively $C_{3-18}$ heteroaryl-$C_{1-12}$ alkylene-O-, and yet alternatively $C_{3-12}$ heteroaryl-$C_{1-6}$ alkylene-O-; in another embodiment, Ra is -O-$C_{6-30}$ aryl-$C_{1-18}$ alkylene-, alternatively -O-$C_{6-18}$ aryl-$C_{1-12}$ alkylene-; in another embodiment, Ra is -O-$C_{3-30}$ heteroaryl-$C_{1-18}$ alkylene-, alternatively -O-$C_{3-18}$ heteroaryl-$C_{1-12}$ alkylene-; and in another embodiment, Ra is the instance that Ra and $X_2$ may be connected to form a ring.

$R^1$

In an embodiment, $R^1$ is absent; in another embodiment, $R^1$ is hydrogen; in another embodiment, $R^1$ is hydroxyl; in another embodiment, $R^1$ is amino unsubstituted or substituted with 1 or 2 Rb; in another embodiment, $R^1$ is unsubstituted or Rb-substituted $C_{1-30}$ hydrocarbon radical; in another embodiment, $R^1$ is $C_{1-30}$ alkyl or $C_{2-30}$ alkenyl; in another embodiment, $R^1$ is $C_{1-18}$ alkyl or $C_{2-18}$ alkenyl; in another embodiment, $R^1$ is $C_{1-6}$ alkyl or $C_{2-12}$ alkenyl, wherein the alkenyl has a double-bond number of 1-10, alternatively 1-6; in another embodiment, $R^1$ is $C_{3-10}$ cycloalkyl, alternatively $C_{3-8}$ cycloalkyl, and yet alternatively $C_{3-6}$ cycloalkyl, which are unsubstituted or substituted with 1, 2, 3, 4, or 5 Rb; in another embodiment, $R^1$ is unsubstituted or Rb-substituted $C_{1-30}$ alkoxy, alternatively $C_{1-20}$ alkoxy, and yet alternatively $C_{1-12}$ alkoxy; in another embodiment, $R^1$ is $C_{12-30}$ condensed cyclic group, alternatively $C_{12-17}$ condensed cyclic group, and yet alternatively $C_{12-17}$ condensed cyclic group containing at least one heteroatom, and further alternatively $C_{12-17}$ tetracondensed cyclic group containing at least one heteroatom, e.g.,

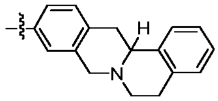

,

which are unsubstituted or substituted with 1, 2, 3, 4, or 5 Rb; in another embodiment, $R^1$ is oxocyclopentyl-$C_{1-6}$ alkylene-$C_{6-12}$ arylene-$C_{1-6}$ alkylene-; in another embodiment, $R^1$ is hydroxylcyclopentyl-$C_{1-6}$ alkylene-$C_{6-12}$ arylene-$C_{1-6}$ alkylene-; in another embodiment, $R^1$ is $C_{1-18}$ alkyl-$C_{6-30}$ arylene-$C_{1-18}$ alkylene-, alternatively $C_{1-12}$ alkyl-$C_{6-18}$ arylene-$C_{1-12}$ alkylene-, which is unsubstituted or has a hydrogen atom replaced with Rc in the alkyl; in another embodiment, $R^1$ is $C_{1-12}$ alkyl-O-C(O)-$C_{1-12}$ alkylene-imino-, which is unsubstituted or has a hydrogen atom replaced with Rd in the alkyl; in another embodiment, $R^1$ is $C_{6-30}$ aryl-$C_{1-18}$ alkylene-, alternatively $C_{6-18}$ aryl-$C_{1-12}$ alkylene-; in another embodiment, $R^1$ is -O-C(O)-$Y_1$; in another embodiment, $R^1$ is -$C_{1-12}$ alkylene-C(O)-O-$Y_2$ which is unsubstituted or has a hydrogen atom replaced with Rd in the alkylene; in another embodiment, $R^1$ is -O-$C_{1-12}$ alkylene-O-C(O)-$Y_3$ which is unsubstituted or has a hydrogen atom replaced with Rd in the alkylene; in another embodiment, $R^1$ is -C=CH-$Y_4$; in another embodiment, $R^1$ is -$Y_5$-$Y_6$-$Y_7$; in another embodiment, $R^1$ is -CHR$^{\#}$R$^{\#\#}$; in another embodiment, $R^1$ is -Z-$C_{6-30}$ aryl, alternatively -Z-$C_{6-18}$ aryl, and yet alternatively -Z-$C_{6-12}$ aryl, e.g., -Z-phenyl, which is optionally substituted with x A.

Among others, $R^{\#}$ and $R^{\#\#}$ are each independently selected from H, hydroxyl, amino, halogen, alkyl, cycloalkyl, aryl, heteroaryl, -C(O)-OR$^2$, -OC(O)-OR$^2$, -$C_{1-18}$ alkylene-C(O)-OR$^2$, -$C_{1-18}$ alkylene-R$^2$, or -$C_{6-10}$ aryl-$C_{1-6}$ alkyl.

In an embodiment, when the $L_1$ and $L_2$ are both selected from a single bond, $R^1$ is not hydroxyl.

In a specific embodiment, $R^1$ is H; in another specific embodiment, $R^1$ is -OH; in another specific embodiment, $R^1$ is -$NH_2$; in another specific embodiment, $R^1$ is -$OCH_3$; in another specific embodiment, $R^1$ is methyl; in another specific embodiment, $R^1$ is ethyl; in another specific embodiment, $R^1$ is iso-propyl; in another specific embodiment, $R^1$ is cyclopropyl; in another specific embodiment, $R^1$ is -$(CH_2)_pCH_3$; in another specific embodiment, $R^1$ is -$(CH_2)_pOCH_3$; in another specific embodiment, $R^1$ is -$(CH_2)_pNH_2$; in another specific embodiment, $R^1$ is -$(CH_2)_pOH$; in another specific embodiment, $R^1$ is -$(OCH_2CH_2)qCH_3$; in another specific embodiment, $R^1$ is -$(OCH_2CH_2)qOCH_3$; in another specific embodiment, $R^1$ is -$(OCH_2CH_2)qOH$; in another specific embodiment, $R^1$ is -$(OCH_2CH_2)qNH_2$; in another specific embodiment, $R^1$ is phenyl; in another specific embodiment, $R^1$ is

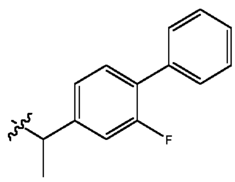

;

in another specific embodiment, $R^1$ is

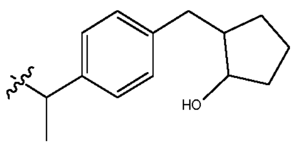

;

in another specific embodiment, $R^1$ is

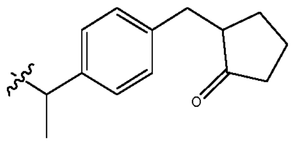
;

in another specific embodiment, $R^1$ is

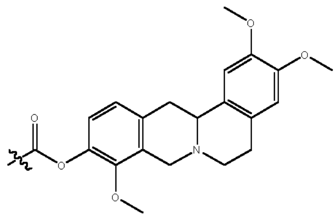
;

in another specific embodiment, $R^1$ is

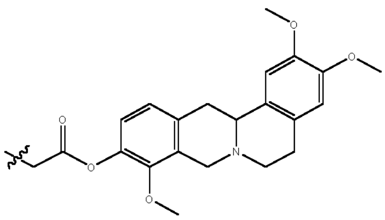
;

in another specific embodiment, $R^1$ is

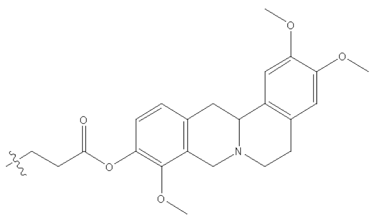
;

in another specific embodiment, $R^1$ is

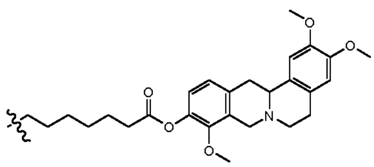
;

in another specific embodiment, $R^1$ is

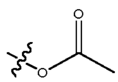
;

in another specific embodiment, $R^1$ is

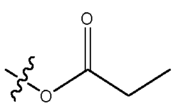
;

in another specific embodiment, $R^1$ is

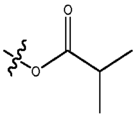;

in another specific embodiment, $R^1$ is

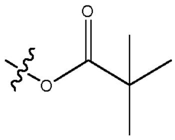;

in another specific embodiment, $R^1$ is

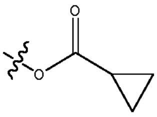;

in another specific embodiment, $R^1$ is

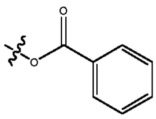;

in another specific embodiment, $R^1$ is

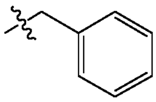;

in another specific embodiment, $R^1$ is

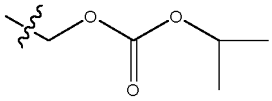;

in another specific embodiment, $R^1$ is

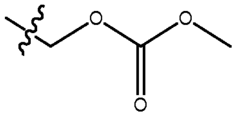;

in another specific embodiment, $R^1$ is

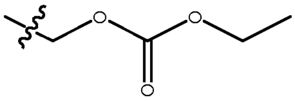;

in another specific embodiment, $R^1$ is

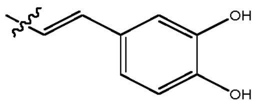

;

in another specific embodiment, $R^1$ is

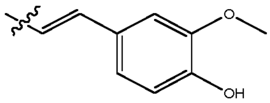

;

in another specific embodiment, $R^1$ is

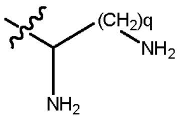

(such as,

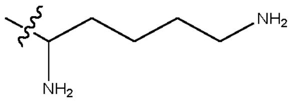

);

in another specific embodiment, $R^1$ is

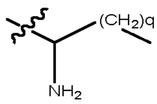

;

in another specific embodiment, $R^1$ is

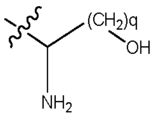

;

in another specific embodiment, $R^1$ is

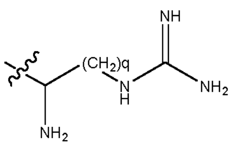

(such as,

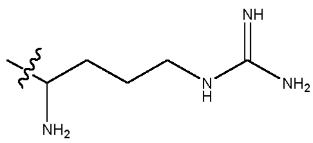

);

in another specific embodiment, $R^1$ is

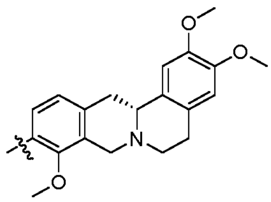
;

in another specific embodiment, $R^1$ is

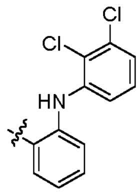
;

in another specific embodiment, $R^1$ is

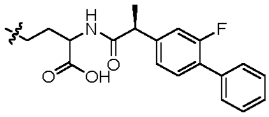
;

in another specific embodiment, $R^1$ is

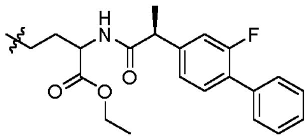
;

in another specific embodiment, $R^1$ is

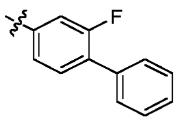
;

in another specific embodiment, $R^1$ is

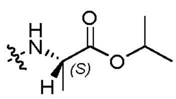
;

in another specific embodiment, $R^1$ is $-CH_2$-phenyl; in another specific embodiment, $R^1$ is

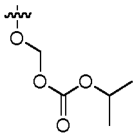
;

in another specific embodiment, $R^1$ is

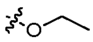
;

in another specific embodiment, $R^1$ is

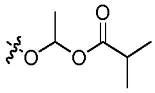

;

in another specific embodiment, $R^1$ is $C_7$ alkyl; in another specific embodiment, $R^1$ is $C_8$ alkyl; in another specific embodiment, $R^1$ is $C_9$ alkyl; in another specific embodiment, $R^1$ is $C_{10}$ alkyl; in another specific embodiment, $R^1$ is $C_{11}$ alkyl; in another specific embodiment, $R^1$ is $C_{12}$ alkyl; in another specific embodiment, $R^1$ is $C_{13}$ alkyl; in another specific embodiment, $R^1$ is $C_{14}$ alkyl; in another specific embodiment, $R^1$ is $C_{15}$ alkyl; in another specific embodiment, $R^1$ is $C_{16}$ alkyl; in another specific embodiment, $R^1$ is $C_{17}$ alkyl; in another specific embodiment, $R^1$ is $C_{18}$ alkyl; in another specific embodiment, $R^1$ is $C_{19}$ alkyl; in another specific embodiment, $R^1$ is $C_{20}$ alkyl; in another specific embodiment, $R^1$ is $C_{21}$ alkyl; in another specific embodiment, $R^1$ is $C_{22}$ alkyl; in another specific embodiment, $R^1$ is $C_{23}$ alkyl; in another specific embodiment, $R^1$ is $C_{24}$ alkyl; in another specific embodiment, $R^1$ is $C_{25}$ alkyl; in another specific embodiment, $R^1$ is

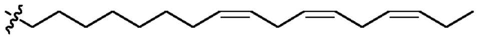

;

in another specific embodiment, $R^1$ is

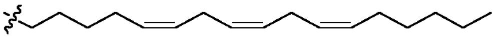

;

in another specific embodiment, $R^1$ is

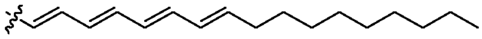

;

in another specific embodiment, $R^1$ is

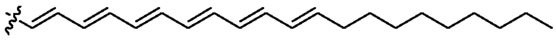

;

in another specific embodiment, $R^1$ is

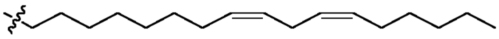

;

in another specific embodiment, $R^1$ is

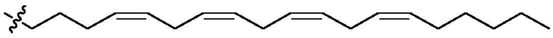

;

in another specific embodiment, $R^1$ is

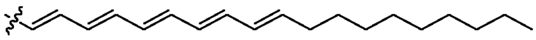

;

in another specific embodiment, $R^1$ is

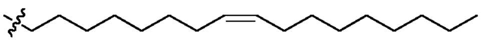

;

in another specific embodiment, $R^1$ is

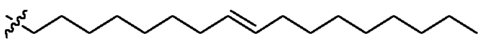

;

in another specific embodiment, $R^1$ is

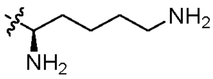

;

in another specific embodiment, $R^1$ is

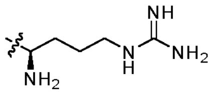

;

in another specific embodiment, $R^1$ is

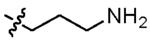

;

and in another specific embodiment, $R^1$ is

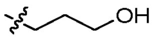

.

$R^2$

In an embodiment, $R^2$ is H; in another embodiment, $R^2$ is hydroxyl; in another embodiment, $R^2$ is amino; in another embodiment, $R^2$ is halogen; in another embodiment, $R^2$ is alkyl; in another embodiment, $R^2$ is cycloalkyl; in another embodiment, $R^2$ is aryl; in another embodiment, $R^2$ is heteroaryl; in another embodiment, $R^2$ is unsubstituted or Rb-substituted $C_{12-30}$ condensed cyclic group; and in another embodiment, $R^2$ is -NHC(NH)NH-.

Rb

In an embodiment, Rb is $C_{1-30}$ alkoxy; in another embodiment, Rb is $C_{1-20}$ alkoxy; in another embodiment, Rb is $C_{1-12}$ alkoxy; in another embodiment, Rb is $C_{1-3}$ alkoxy; in another embodiment, Rb is $C_{6-30}$ aryl; in another embodiment, Rb is $C_{6-18}$ aryl; in another embodiment, Rb is $C_{6-12}$ aryl; in another embodiment, Rb is $C_{3-30}$ heteroaryl; in another embodiment, Rb is $C_{3-18}$ heteroaryl; in another embodiment, Rb is $C_{3-12}$ heteroaryl; in another embodiment, Rb is $C_{1-30}$ alkyl; in another embodiment, Rb is $C_{1-18}$ alkyl; and in another embodiment, Rb is $C_{1-6}$ alkyl.

In an embodiment, Rb is unsubstituted; and in another embodiment, Rb is substituted with halogen (alternatively F, Cl or Br).

Rc

In an embodiment, Rc is $C_{3-10}$ cycloalkyl; in another embodiment, Rc is $C_{3-8}$ cycloalkyl; in another embodiment, Rc is $C_{3-6}$ cycloalkyl; and in another embodiment, Rc is cyclopentyl.

In an embodiment, Rc is unsubstituted; and in another embodiment, Rc is substituted with hydroxyl; and in another embodiment, Rc is substituted with oxo or thioxo.

Rd

In an embodiment, Rd is $C_{1-18}$ alkyl; in another embodiment, Rd is $C_{1-12}$ alkyl; in another embodiment, Rd is $C_{1-6}$ alkyl; in another embodiment, Rd is $C_{1-30}$ alkoxy; in another embodiment, Rd is $C_{1-20}$ alkoxy; in another embodiment, Rd is $C_{1-12}$ alkoxy; in another embodiment, Rd is $C_{1-3}$ alkoxy; in another embodiment, Rd is $C_{6-30}$ aryl; in another embodiment, Rd is $C_{6-18}$ aryl; in another embodiment, Rd is $C_{6-12}$ aryl; in another embodiment, Rd is $C_{3-30}$ heteroaryl; in another embodiment, Rd is $C_{3-18}$ heteroaryl; and in another embodiment, Rd is $C_{3-12}$ heteroaryl.

In an embodiment, Rd is unsubstituted; and in another embodiment, Rd is substituted with halogen (alternatively F, Cl or Br).

$Y_1$

In an embodiment, $Y_1$ is $C_{1-30}$ alkyl; in another embodiment, $Y_1$ is $C_{1-12}$ alkyl; in another embodiment, $Y_1$ is $C_{1-6}$ alkyl; in another embodiment, $Y_1$ is $C_{3-10}$ cycloalkyl; in another embodiment, $Y_1$ is $C_{3-8}$ cycloalkyl; in another embodiment,

$Y_1$ is $C_{3-6}$ cycloalkyl; in another embodiment, $Y_1$ is $C_{6-30}$ aryl; in another embodiment, $Y_1$ is $C_{6-18}$ aryl; in another embodiment, $Y_1$ is $C_{6-12}$ aryl; in another embodiment, $Y_1$ is $C_{3-30}$ heteroaryl; in another embodiment, $Y_1$ is $C_{3-18}$ heteroaryl; and in another embodiment, $Y_1$ is $C_{3-12}$ heteroaryl.

In an embodiment, $Y_1$ is unsubstituted; and in another embodiment, $Y_1$ is substituted with Re; and in another embodiment, $Y_1$ is substituted with $C_{1-6}$ alkyl.

Re

In an embodiment, Re is $C_{6-30}$ aryl; in another embodiment, Re is $C_{6-18}$ aryl; in another embodiment, Re is $C_{6-12}$ aryl; in another embodiment, Re is $C_{3-30}$ heteroaryl; in another embodiment, Re is $C_{3-1S}$ heteroaryl; and in another embodiment, Re is $C_{3-12}$ heteroaryl.

In an embodiment, Re is unsubstituted; and in another embodiment, Re is substituted with halogen (alternatively F, Cl or Br).

$Y_2$

In an embodiment, $Y_2$ is $C_{12-30}$ condensed cyclic group; in another embodiment, $Y_2$ is $C_{12-17}$ condensed cyclic group; in another embodiment, $Y_2$ is $C_{12-17}$ condensed cyclic group containing at least one heteroatom; and in another embodiment, $Y_2$ is $C_{12-17}$ tetracondensed cyclic group containing at least one heteroatom, e.g.,

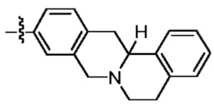
;

In an embodiment, $Y_2$ is unsubstituted; and in another embodiment, $Y_2$ is substituted with Rf.

Rf

In an embodiment, Rf is $C_{1-30}$ alkoxy; in another embodiment, Rf is $C_{1-20}$ alkoxy; in another embodiment, Rf is $C_{1-12}$ alkoxy; in another embodiment, Rf is $C_{1-3}$ alkoxy; in another embodiment, Rf is $C_{6-30}$ aryl; in another embodiment, Rf is $C_{6-18}$ aryl; and in another embodiment, Rf is $C_{6-12}$ aryl.

In an embodiment, Rf is unsubstituted; and in another embodiment, Rf is substituted with halogen (alternatively F, Cl or Br).

$Y_3$

In an embodiment, $Y_3$ is $C_{1-18}$ alkyl; in another embodiment, $Y_3$ is $C_{1-12}$ alkyl; in another embodiment, $Y_3$ is $C_{1-6}$ alkyl; in another embodiment, $Y_3$ is -O-$C_{1-18}$ alkyl; in another embodiment, $Y_3$ is -O-$C_{1-12}$ alkyl; and in another embodiment, $Y_3$ is -O-$C_{1-6}$ alkyl.

$Y_4$

In an embodiment, $Y_4$ is $C_{6-30}$ aryl; in another embodiment, $Y_4$ is $C_{6-18}$ aryl; in another embodiment, $Y_4$ is $C_{6-12}$ aryl; in another embodiment, $Y_4$ is $C_{3-30}$ heteroaryl; in another embodiment, $Y_4$ is $C_{3-18}$ heteroaryl; in another embodiment, $Y_4$ is $C_{3-12}$ heteroaryl; and in another embodiment, $Y_4$ is $C_{3-6}$ heteroaryl.

In an embodiment, $Y_4$ is unsubstituted; and in another embodiment, $Y_4$ is substituted with Rg.

Rg

In an embodiment, Rg is hydroxyl; in another embodiment, Rg is $C_{1-30}$ alkoxy; in another embodiment, Rg is $C_{1-20}$ alkoxy; in another embodiment, Rg is $C_{1-12}$ alkoxy; and in another embodiment, Rg is $C_{1-3}$ alkoxy.

$Y_5$

In an embodiment, $Y_5$ is a single bond; in another embodiment, $Y_5$ is unsubstituted or amino-substituted $C_{1\ 18}$ alkylene; in another embodiment, $Y_5$ is unsubstituted or amino-substituted $C_{1-12}$ alkylene; and in another embodiment, $Y_5$ is unsubstituted or amino-substituted $C_{3-6}$ alkylene.

$Y_6$

In an embodiment, $Y_6$ is a single bond; in another embodiment, $Y_6$ is -NH-C(NH)-; in another embodiment, $Y_6$ is $-(O-CH_2-CH_2)_m-$; and in another embodiment, $Y_6$ is $-CH_2-(O-CH_2-CH_2)_n-$.

*m* and *n* are each independently selected from any natural number of 2-20, alternatively any natural number of 2-10.

$Y_7$

In an embodiment, $Y_7$ is amino; in another embodiment, $Y_7$ is $C_{1-30}$ alkoxy; in another embodiment, $Y_7$ is $C_{1-20}$ alkoxy; in another embodiment, $Y_7$ is $C_{1-12}$ alkoxy; in another embodiment, $Y_7$ is $C_{1-3}$ alkoxy; and in another embodiment, $Y_7$ is hydroxyl.

Z

In an embodiment, Z is a chemical bond; in another embodiment, Z is -O-; in another embodiment, Z is - NH-; in another embodiment, Z is $C_{1-30}$ alkylene; in another embodiment, Z is $C_{1-18}$ alkylene; in another embodiment, Z is $-C_{1-12}$ alkylene-; in another embodiment, Z is $-C_{1-6}$ alkylene-; in another embodiment, Z is $-C_{1-12}$ alkylene-O-$C_{1-12}$ alkylene-; in another embodiment, Z is $-C_{1-12}$ alkylene-NH-$C_{1-12}$ alkylene-; in another embodiment, Z is $-C_{1-6}$ alkylene-NH-$C_{1-6}$ alkylene-; in another embodiment, Z is $-C_{1-18}$ alkylene-C(O)-$C_{1-12}$ alkylene-; in another embodiment, Z is $-C_{1-10}$ alkylene-C(O)O-$C_{1-10}$ alkylene-; in another embodiment, Z is $-C_{1-10}$ alkylene-NHC(O)-$C_{1-10}$ alkylene-; in another embodiment, Z is $-C_{1-6}$ alkylene-NHC(O)-$C_{1-6}$ alkylene-; in another embodiment, Z is $-C_{1-4}$ alkylene-NHC(O)-$C_{1-4}$ alkylene-; in another embodiment, Z is $-C_{1-10}$ alkylene-NHC(O)O-$C_{1-10}$ alkylene-; in another embodiment, Z is $-C_{1-10}$ alkylene-C(O)NH-$C_{1-10}$ alkylene-; in another embodiment, Z is $-C_{1-6}$ alkylene-C(O)NH-$C_{1-6}$ alkylene-; in another embodiment, Z is $-C_{1-4}$ alkylene-C(O)NH-$C_{1-4}$ alkylene-; in another embodiment, Z is $-C_{1-10}$ alkylene-OC(O)NH-$C_{1-10}$ alkylene-; in another embodiment, Z is $C_{3-10}$ cycloalkylene; in another embodiment, Z is $C_{6-30}$ arylene; in another embodiment, Z is $C_{3-30}$ heteroarylene; and in another embodiment, Z is -C=CH-.

In an embodiment, Z is unsubstituted; and in another embodiment, Z is optionally substituted with 1, 2, 3, 4, or 5 substituents selected from halogen, -C(O)OH, -C(O)O-$C_{1-6}$ alkyl, -C(O)-$C_{1-6}$ alkyl, -C(O)-$C_{1-4}$ alkyl, -O-$C_{1\ 6}$ alkyl, $C_{1-6}$ alkyl or $C_{1-6}$ haloalkyl.

A

In an embodiment, A is H; in another embodiment, A is halogen; in another embodiment, A is OH; in another embodiment, A is $-L_A-C_{3-10}$ cycloalkyl; in another embodiment, A is $-L_A$-3- to 10-membered heterocyclyl; in another embodiment, A is $-L_A-C_{6-12}$ aryl; in another embodiment, A is $-L_A$-5- to 12-membered heteroaryl; in another embodiment, A is $C_{6-10}$ aryl; in another embodiment, A is -NH-$C_{6-10}$ aryl; and in another embodiment, A is Re-substituted $C_{1-18}$ alkyl.

In a specific embodiment, A is F; in another specific embodiment, A is OH; in another specific embodiment, A is phenyl; in another specific embodiment, A is

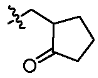

;

and in another specific embodiment, A is

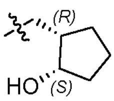

.

In an embodiment, A is unsubstituted; and in another embodiment, A is optionally further substituted with halogen, $C_{1-6}$ alkyl or $C_{1-6}$ haloalkyl.

B

In an embodiment, B is H; in another embodiment, B is halogen; in another embodiment, B is OH; in another embodiment, B is $-L_A-C_{3-10}$ cycloalkyl; in another embodiment, B is $-L_A$-3- to 10-membered heterocyclyl; in another embodiment, B is $C_{6-30}$ aryl; in another embodiment, B is $-L_A-C_{6-12}$ aryl; in another embodiment, B is - $L_A$-5- to 12-membered heteroaryl; in another embodiment, B is $C_{6-10}$ aryl; and in another embodiment, B is -NH-$C_{6-10}$ aryl.

B is optionally further substituted with halogen, $C_{1-6}$ alkyl or $C_{1-6}$ haloalkyl;

*x* and *y*

In an embodiment, x is selected from 0, 1, 2, 3, 4 and 5.
In an embodiment, y is selected from 0, 1 and 2.
In an embodiment, x + *y* is not 0.

$L_A$

In an embodiment, $L_A$ is a chemical bond; in another embodiment, $L_A$ is -NH-; in another embodiment, $L_A$ is -O-; in another embodiment, $L_4$ is -C(O)-; in another embodiment, $L_A$ is -C(O)O-; in another embodiment, $L_A$ is -NHC(O)-; in another embodiment, $L_A$ is -NHC(O)O-; in another embodiment, $L_A$ is -OC(O)NH-; and in another embodiment, $L_A$ is -C(O)NH-.

$U_1$, $U_2$ and $U_3$

In an embodiment, $U_1$ is -O-C(O)-; in another embodiment, $U_1$ is -NH-C(O)-; in another embodiment, $U_1$ is -O-$CH_2$-O-; and in another embodiment, $U_1$ is -O-.
In an embodiment, $U_2$ is -O-C(O)-; in another embodiment, $U_2$ is -NH-C(O)-; in another embodiment, $U_2$ is -O-$CH_2$-O-; and in another embodiment, $U_2$ is -O-.
In an embodiment, $U_3$ is -O-C(O)-; in another embodiment, $U_3$ is -NH-C(O)-; in another embodiment, $U_3$ is -O-$CH_2$-O-; and in another embodiment, $U_3$ is -O-.
In an embodiment, an available ring atom on $U_2$ and an available ring atom on $U_3$ are connected via Q group to form a 4- to 8-membered ring, alternatively a 5- to 6-membered ring.

Q

In an embodiment, Q is a single bond; in another embodiment, Q is $NR^3$; in another embodiment, Q is $C_{1-3}$ alkylene in which 1 or 2 $CH_2$ are optionally replaced with group selected from O, S and $NR^3$; and in another embodiment, Q is $C_{2-3}$ alkenylene in which any CH for forming C=C double bond is optionally replaced with N.

$W_1$, $W_2$ and $W_3$

In an embodiment, $W_1$ is $C_{1-18}$ alkyl; in another embodiment, $W_1$ is $C_{1-12}$ alkyl; in another embodiment, $W_1$ is $C_{1-6}$ alkyl; in another embodiment, $W_1$ is $C_{1-4}$ alkyl, alternatively methyl; in another embodiment, $W_1$ is -(O-$C_{2-6}$ alkylene)$_p$; in another embodiment, $W_1$ is -(O-$C_{2-4}$ alkylene)$_p$; and in another embodiment, $W_1$ is -(O-$CH_2$-$CH_2$)$_p$.
*p* is selected from any natural number of 1-18, alternatively any natural number of 1-10.

$M_1$

In an embodiment, $M_1$ is a single bond; and in another embodiment, $M_1$ is -C(O)-.

$M_4$

In an embodiment, $M_4$ is a chemical bond; in another embodiment, $M_4$ is -O-; in another embodiment, $M_4$ is -NH-; in another embodiment, $M_4$ is -C(O)-; in another embodiment, $M_4$ is -C(O)O-, -OC(O)-; in another embodiment, $M_4$ is -NHC(O)O-; in another embodiment, $M_4$ is -OC(O)NH-; in another embodiment, $M_4$ is -$C_{1-10}$ alkylene-; in another embodiment, $M_4$ is -$C_{1-6}$ alkylene-; and in another embodiment, $M_4$ is -$C_{1-4}$ alkylene-.

Ring G

In an embodiment, the ring G is $C_{6-12}$ aryl; in another embodiment, the ring G is $C_{6-10}$ aryl; in another embodiment, and the ring G is phenyl.

*r, t, s, k, m* and *n*

In an embodiment, *r* is selected from 0, 1, 2, 3, 4, 5, 6, 7, 8, 9 and 10.
In an embodiment, t is selected from 0, 1, 2, 3, 4, 5, 6, 7, 8, 9 and 10.
In an embodiment, s is selected from 0, 1, 2, 3, 4, 5, 6, 7, 8, and 9.
In an embodiment, k is selected from 0, 1, 2, 3, 4, and 5.
In an embodiment, *m* is selected from 2, 3, 4, 5, 6, 7, 8, 9, 10, 11, 12, 13, 14 and 15, alternatively 6, 7, 8, 9 or 10, and yet alternatively 7, 8 or 9.
In an embodiment, n is selected from 2, 3, 4, 5, 6, 7, 8, 9, 10, 11, 12, 13, 14 and 15, alternatively 6, 7, 8, 9 or 10, and yet alternatively 7, 8 or 9.
In an embodiment, n is selected from any natural number of 2-20, alternatively any natural number of 2-10.
The above alkyl, alkylene, alkenyl, alkenylene, alkynyl, cyclic hydrocarbon radical, heterocyclyl, aryl, heteroaryl and aralkyl at each occurrence are each optionally substituted with 1, 2, 3 or more $R^3$.

$R^3$

In an embodiment, $R^3$ is halogen; in another embodiment, $R^3$ is cyano; in another embodiment, $R^3$ is nitro; in another embodiment, $R^3$ is $C_{1-6}$ alkyl; in another embodiment, $R^3$ is $C_{3-10}$ cyclic hydrocarbon radical; in another embodiment, $R^3$ is 3- to 10-membered heterocyclyl; in another embodiment, $R^3$ is $C_{6-10}$ aryl; in another embodiment, $R^3$ is 5- to 14-membered heteroaryl; and in another embodiment, $R^3$ is $C_{6-12}$ aralkyl.
In an embodiment, the alkyl, alkylene, cyclic hydrocarbon radical, heterocyclyl, aryl, heteroaryl and aralkyl of $R^3$ are further optionally substituted with 1, 2, 3 or more substituents independently selected from: halogen, OH, oxo, amino, cyano, nitro, $C_{1-6}$ alkyl, $C_{1-6}$ haloalkyl, $C_{3-6}$ cyclic hydrocarbon radical, 3- to 10-membered heterocyclyl, $C_{6-10}$ aryl, 5- to 14-membered heteroaryl, and $C_{6-12}$ aralkyl.
Any technical solution of the above any specific embodiments or any combination thereof may be combined with any technical solution of other specific embodiments or any combination thereof. For example, any technical solution of $L_1$ or any combination thereof may be combined with any technical solution of $L_2$, $L_3$, $X_1$, $X_2$, $R^1$-$R^3$, Ra-Rg, $Y_1$-$Y_7$, Z, A, B, x, y, $L_A$, $U_1$-$U_3$, $W_1$-$W_3$, Q, $M_1$, $M_4$, ring G, r, t, s, k, m, n, etc., or any combination thereof. The present disclosure is intended to encompass all the combinations of these technical solutions, which are not listed one by one due to due to space limitations.
In some embodiments, the present disclosure relates to a compound of Formula I, or a pharmaceutically acceptable salt, ester, stereoisomer, polymorph, solvate, N-oxide, isotope-labeled compound, metabolite, or pro-drug thereof, and a mixture thereof:

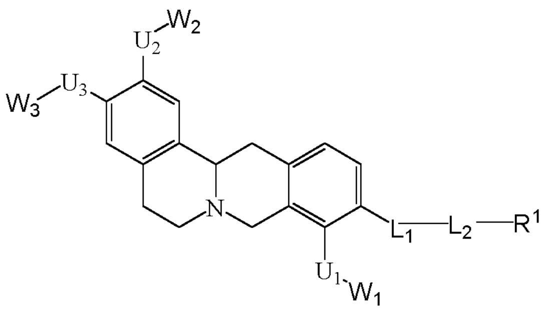

I

wherein

$L_1$ is selected from a single bond, -O-, -NH-, -S- or -P(O)OH-; alternatively a single bond, -O-, -NH-;
$L_2$ is selected from a single bond, -O-, -NH-, -C(O)-, -C(O)-O-,
$C_{1-18}$ alkylene (alternatively $C_{1-12}$ alkylene, and yet alternatively $C_{1-6}$ alkylene),
$C_{3-10}$ cycloalkylene (alternatively $C_{3-8}$ cycloalkylene, and yet alternatively $C_{3-6}$ cycloalkylene),
$C_{6-30}$ arylene (alternatively $C_{6-18}$ arylene, and yet alternatively $C_{6-12}$ arylene),
$C_{3-30}$ heteroarylene (alternatively $C_{3-18}$ heteroarylene, and yet alternatively $C_{3-12}$ heteroarylene),
-$C_{1-18}$ alkylene-O- (alternatively -$C_{1-12}$ alkylene-O-, and yet alternatively -$C_{1-6}$ alkylene-O-),
-$C_{6-30}$ arylene-O- (alternatively -$C_{6-18}$ arylene-O-, and yet alternatively -$C_{6-12}$ arylene-O-),
-$C_{3-30}$ heteroarylene-O- (alternatively -$C_{3-18}$ heteroarylene-O-, and yet alternatively -$C_{3-12}$ heteroarylene-O-),
-C(O)-$C_{1-18}$ alkyl-,

$C_{1-30}$ alkoxy, or

$-X_1$-PRa(O)-$X_2$-; the Ra is each independently selected from hydrogen, hydroxyl, halogen (alternatively F, Cl or Br), $C_{6-30}$ aryl (alternatively $C_{6-18}$ aryl, and yet alternatively $C_{6-12}$ aryl), $C_{3-30}$ heteroaryl (alternatively $C_{3-18}$ heteroaryl, and yet alternatively $C_{3-12}$ heteroaryl), $C_{1-30}$ hydrocarbon radical (alternatively $C_{1-30}$ alkyl, and yet alternatively $C_{1-18}$ alkyl, and further alternatively $C_{1-6}$ alkyl), $C_{3-10}$ cycloalkyl (alternatively $C_{3-8}$ cycloalkyl, and yet alternatively $C_{3-6}$ cycloalkyl), $C_{1-30}$ alkoxy (alternatively $C_{1-18}$ alkoxy, and yet alternatively $C_{1-6}$ alkoxy), $C_{6-30}$ aryl-O- (alternatively $C_{6-18}$ aryl-O-, and yet alternatively $C_{6-12}$ aryl-O-), $C_{3-30}$ heteroaryl-O- (alternatively $C_{3-18}$ heteroaryl-O-, and yet alternatively $C_{3-12}$ heteroaryl-O-), $C_{6-30}$ aryl-$C_{1-18}$ alkylene-O- (alternatively $C_{6-18}$ aryl-$C_{1-12}$ alkylene-O-, and yet alternatively $C_{6-12}$ aryl-$C_{1-6}$ alkylene-O-), $C_{3-30}$ heteroaryl-$C_{1-18}$ alkylene-O- (alternatively $C_{3-18}$ heteroaryl-$C_{1-12}$ alkylene-O-, and yet alternatively $C_{3-12}$ heteroaryl-$C_{1-6}$ alkylene-O-), -O-$C_{6-30}$ aryl-$C_{1-18}$ alkylene- (alternatively -O-$C_{6-18}$ aryl-$C_{1-12}$ alkylene-) or -O-$C_{3-30}$ heteroaryl-$C_{1-18}$ alkylene- (alternatively -O-$C_{3-18}$ heteroaryl-$C_{1-12}$ alkylene-); the Ra and $X_2$ may be connected to form a ring;

the $X_1$ is selected from a single bond, -O-, -$C_{1-18}$ alkylene-O- (alternatively -$C_{1-12}$ alkylene-O-, and yet alternatively -$C_{1-6}$ alkylene-O-), -$C_{6-30}$ arylene-O- (alternatively -$C_{6-18}$ arylene-O-, and yet alternatively -$C_{6-12}$ arylene-O-), or -$C_{3-30}$ heteroarylene-O- (alternatively -$C_{3-18}$ heteroarylene-O-, and yet alternatively -$C_{3-12}$ heteroarylene-O-);

the $X_2$ is selected from a single bond, -O- or -NR*-; wherein R* is selected from H, $C_{1-6}$ alkyl, and $C_{1-6}$ alkoxy;

$R^1$ is selected from absent and hydrogen, hydroxyl, unsubstituted or Rb-substituted amino, unsubstituted or Rb-substituted $C_{1-30}$ hydrocarbon radical (alternatively $C_{1-30}$ alkyl or $C_{2-30}$ alkenyl, and yet alternatively $C_{1-18}$ alkyl or $C_{2-18}$ alkenyl, and further alternatively $C_{1-6}$ alkyl or $C_{2-12}$ alkenyl, wherein the alkenyl has a double-bond number of 1-10, alternatively 1-6), unsubstituted or Rb-substituted $C_{3-10}$ cycloalkyl (alternatively $C_{3-8}$ cycloalkyl, and yet alternatively $C_{3-6}$ cycloalkyl), unsubstituted or Rb-substituted $C_{1-30}$ alkoxy (alternatively $C_{1-20}$ alkoxy, and yet alternatively $C_{1-12}$ alkoxy), unsubstituted or Rb-substituted $C_{12-30}$ condensed cyclic group (alternatively $C_{12-17}$ condensed cyclic group, and yet alternatively $C_{12-17}$ condensed cyclic group containing at least one heteroatom, and further alternatively $C_{12-17}$ tetracondensed cyclic group containing at least one heteroatom), $C_{1-18}$ alkyl-$C_{6-30}$ arylene-$C_{1-18}$ alkylene- (alternatively $C_{1-12}$ alkyl-$C_{6-18}$ arylene-$C_{1-12}$ alkylene-) which is unsubstituted or has a hydrogen atom replaced with Rc in the alkyl, $C_{1-12}$ alkyl-O-C(O)-$C_{1-12}$ alkylene-imino-, which is unsubstituted or has a hydrogen atom replaced with Rd in the alkyl, $C_{6-30}$ aryl-$C_{1-18}$ alkylene- (alternatively $C_{6-18}$ aryl-$C_{1-12}$ alkylene-), -O-C(O)-$Y_1$, -$C_{1-12}$ alkylene-C(O)-O-$Y_2$ which is unsubstituted or has a hydrogen atom replaced with Rd in the alkylene, -O-$C_{1-12}$ alkylene-O-C(O)-$Y_3$ which is unsubstituted or has a hydrogen atom replaced with Rd in the alkylene, -C=CH-$Y_4$, or -$Y_5$-$Y_6$-$Y_7$, -CHR$^{\#}$R$^{\#\#}$;

wherein R$^{\#}$ and R$^{\#\#}$ are each independently selected from H, hydroxyl, amino, halogen, alkyl, cycloalkyl, aryl, heteroaryl,

-C(O)-OR$^2$, -OC(O)-OR$^2$, -$C_{1-18}$ alkylene-C(O)-OR$^2$, -$C_{1-18}$ alkylene-R$^2$, or -$C_{6-10}$ aryl-$C_{1-6}$ alkyl;

$R^2$ is selected from H, hydroxyl, amino, halogen, alkyl, cycloalkyl, aryl, heteroaryl, unsubstituted or Rb-substituted $C_{12-30}$ condensed cyclic group, or -NHC(NH)NH-;

the Rb is each independently selected from $C_{1-30}$ alkoxy (alternatively $C_{1-20}$ alkoxy, yet alternatively $C_{1-12}$ alkoxy, and further alternatively $C_{1-3}$ alkoxy), $C_{6-30}$ aryl (alternatively $C_{6-18}$ aryl, yet alternatively $C_{6-12}$ aryl), $C_{3-30}$ heteroaryl (alternatively $C_{3-18}$ heteroaryl, yet alternatively $C_{3-12}$ heteroaryl), or $C_{1-30}$ alkyl (alternatively $C_{1-18}$ alkyl, yet alternatively $C_{1-6}$ alkyl), which are unsubstituted or substituted with halogen (alternatively F, Cl, or Br);

the Rc is each independently selected from $C_{3-10}$ cycloalkyl (alternatively $C_{3-8}$ cycloalkyl, and yet alternatively $C_{3-6}$ cycloalkyl, and further alternatively cyclopentyl) which is unsubstituted or substituted with hydroxyl, oxo, or thioxo;

the Rd is each independently selected from $C_{1-18}$ alkyl (alternatively $C_{1-12}$ alkyl, and yet alternatively $C_{1-6}$ alkyl), $C_{1-30}$ alkoxy (alternatively $C_{1-20}$ alkoxy, and yet alternatively $C_{1-12}$ alkoxy, and further alternatively $C_{1-3}$ alkoxy), $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with halogen (alternatively F, Cl or Br);

the $Y_1$ is selected from $C_{1-30}$ alkyl (alternatively $C_{1-12}$ alkyl, and yet alternatively $C_{1-6}$ alkyl), $C_{3-10}$ cycloalkyl (alternatively $C_{3-8}$ cycloalkyl, and yet alternatively $C_{3-6}$ cycloalkyl), $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with Re; the Re is each independently selected from $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with halogen (alternatively F, Cl or Br);

the $Y_2$ is selected from unsubstituted or Rf-substituted $C_{12-30}$ condensed cyclic group (alternatively $C_{12-17}$ condensed cyclic group, and yet alternatively $C_{12-17}$ condensed cyclic group containing at least one heteroatom, and further alternatively $C_{12-17}$ tetracondensed cyclic group containing at least one heteroatom); wherein the Rf is each independently selected from $C_{1-30}$ alkoxy (alternatively $C_{1-20}$ alkoxy, and yet alternatively $C_{1-12}$ alkoxy, and further alternatively $C_{1-3}$ alkoxy), or $C_{6-30}$ aryl (alternatively $C_{6-18}$ aryl, and yet alternatively $C_{6-12}$ aryl), which are unsubstituted or substituted with halogen (alternatively F, Cl or Br);

the $Y_3$ is selected from $C_{1-18}$ alkyl (alternatively $C_{1-12}$ alkyl, and yet alternatively $C_{1-6}$ alkyl) or -O-$C_{1-18}$ alkyl

(alternatively -O-$C_{1-12}$ alkyl, and yet alternatively -O-$C_{1-6}$ alkyl);

the $Y_4$ is selected from $C_{6-30}$ aryl (alternatively $C_{6-18}$ aryl, and yet alternatively $C_{6-12}$ aryl) or $C_{3-30}$ heteroaryl (alternatively $C_{3-18}$ heteroaryl, and yet alternatively $C_{3-12}$ heteroaryl), which are unsubstituted or substituted with Rg, wherein the Rg is each independently selected from hydroxyl and $C_{1-30}$ alkoxy (alternatively $C_{1-20}$ alkoxy, and yet alternatively $C_{1-12}$ alkoxy, and further alternatively $C_{1-3}$ alkoxy);

the $Y_5$ is selected from a single bond, unsubstituted or amino-substituted $C_{1-18}$ alkylene (alternatively $C_{1-12}$ alkylene, and yet alternatively $C_{3-6}$ alkylene);

the $Y_6$ is selected from a single bond, -NH-C(NH)-, -(O-$CH_2$-$CH_2$)$_m$- or -$CH_2$-(O-$CH_2$-$CH_2$)$_n$-, and the *m* and the *n* are each independently selected from any natural number of 2-20 (alternatively any natural number of 2-10);

the $Y_7$ is selected from amino, $C_{1-30}$ alkoxy (alternatively $C_{1-20}$ alkoxy, and yet alternatively $C_{1-12}$ alkoxy, and further alternatively $C_{1-3}$ alkoxy) or hydroxyl;

when the $L_1$ and the $L_2$ are both selected from a single bond, the $R^1$ is not hydroxyl;

$U_1$, $U_2$ and $U_3$ are each independently selected from -O-C(O)-, -NH-C(O)-, -O-$CH_2$-O-, and -O-; or an available ring atom on $U_2$ and an available ring atom on $U_3$ are connected via Q group to form a 4- to 8-membered ring, alternatively a 5- to 6-membered ring,

Q is selected from a single bond; $NR^3$; $C_{1-3}$ alkylene in which 1 or 2 $CH_2$ are optionally independently replaced with a group selected from O, S and $NR^3$; and $C_{2-3}$ alkenylene in which any CH for forming the C=C double bond is optionally replaced with N;

$W_1$, $W_2$ and $W_3$ are each independently selected from $C_{1-18}$ alkyl (alternatively $C_{1-12}$ alkyl, and yet alternatively $C_{1-6}$ alkyl) or -(O-$C_{2-6}$ alkylene)$_p$ (alternatively -(O-$C_{2-4}$ alkylene)$_p$, and yet alternatively -(O-$CH_2$-$CH_2$)$_p$), wherein the *p* is selected from any natural number of 1-18 alternatively any natural number of 1-10;

the above alkyl, alkylene, alkenyl, alkenylene, alkynyl, cyclic hydrocarbon radical, heterocyclyl, aryl, heteroaryl, and aralkyl at each occurrence are each optionally substituted with 1, 2, 3 or more $R^3$, wherein the $R^3$ at each occurrence is independently selected from halogen, cyano, nitro, $C_{1-6}$ alkyl, $C_{3-10}$ cyclic hydrocarbon radical, 3- to 10-membered heterocyclyl, $C_{6-10}$ aryl, 5- to 14-membered heteroaryl, $C_{6-12}$ aralkyl; and wherein the alkyl, alkylene, cyclic hydrocarbon radical, heterocyclyl, aryl, heteroaryl, and aralkyl in relation to the substituent $R^3$ are further optionally substituted with 1, 2, 3 or more substituents independently selected from halogen, OH, oxo, amino, cyano, nitro, $C_{1-6}$ alkyl, $C_{1-6}$ haloalkyl, $C_{3-6}$ cyclic hydrocarbon radical, 3- to 10-membered heterocyclyl, $C_{6-10}$ aryl, 5- to 14-membered heteroaryl, and $C_{6-12}$ aralkyl.

In some embodiments of the present disclosure, the compound is selected from the compound of Formula II:

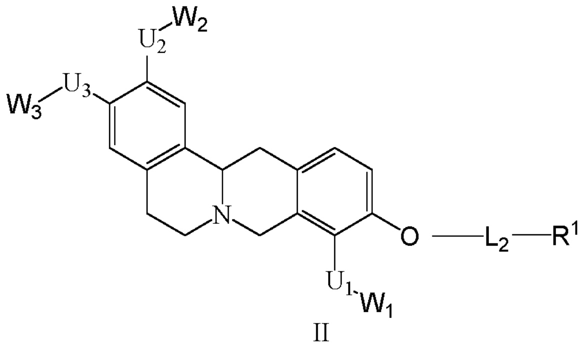

II

the $L_2$ is selected from a single bond, -C(O)-, -C(O)-O-, $C_{1-18}$ alkylene, -$C_{1-18}$ alkylene-O-, -C(O)-$C_{1-18}$ alkyl-, $C_{1-30}$ alkoxy, or -$X_1$-PRa(O)-$X_2$-; the Ra is each independently selected from hydrogen, hydroxyl, halogen, $C_{6-30}$ aryl, $C_{3-30}$ heteroaryl, $C_{1-30}$ hydrocarbon radical, $C_{3-10}$ cycloalkyl, $C_{1-30}$ alkoxy, $C_{6-30}$ aryl-O-, $C_{3-30}$ heteroaryl-O-, $C_{6-30}$ aryl-$C_{1-18}$ alkylene-O-, $C_{3-30}$ heteroaryl-$C_{1-18}$ alkylene-O-, -O-$C_{6-30}$ aryl-$C_{1-18}$ alkylene- or -O-$C_{3-30}$ heteroaryl-$C_{1-18}$ alkylene-; the Ra and $X_2$ may be connected to form a ring;

the $X_1$ is selected from a single bond or -$C_{1-18}$ alkylene-O-;

the $X_2$ is selected from a single bond, -O- or -NR*- (e.g., -NH-);

the $R^1$ is selected from hydrogen, hydroxyl, amino, $C_{1-30}$ hydrocarbon radical, $C_{1-30}$ alkoxy, unsubstituted or Rb-substituted $C_{12-30}$ condensed cyclic group, $C_{1-18}$ alkyl-$C_{6-30}$ arylene-$C_{1-18}$ alkylene- which is unsubstituted or has a hydrogen atom replaced with Rc in the alkyl, $C_{1-12}$ alkyl-O-C(O)-$C_{1-12}$ alkylene-imino-, $C_{6-30}$ aryl-$C_{1-18}$ alkylene-, -O-C(O)-$Y_1$, -$C_{1-12}$ alkylene-C(O)-O-$Y_2$, -O-$C_{1-12}$ alkylene-O-C(O)-$Y_3$, -C=CH-$Y_4$, or -$Y_5$-$Y_6$-$Y_7$, - $CHR^{\#}R^{\#\#}$;

wherein $R^{\#}$ and $R^{\#\#}$ are each independently selected from H, hydroxyl, amino, halogen, alkyl, cycloalkyl, aryl, heteroaryl,

-C(O)-$OR^2$, -OC(O)-$OR^2$, -$C_{1-18}$ alkylene-C(O)-$OR^2$, -$C_{1-18}$ alkylene-$R^2$, or -$C_{6-10}$ aryl-$C_{1-6}$ alkyl;

$R^2$ is selected from H, hydroxyl, amino, halogen, alkyl, cycloalkyl, aryl, heteroaryl, unsubstituted or Rb-substituted $C_{12-30}$ condensed cyclic group, or -NHC(NH)NH-;
the Rb is each independently selected from $C_{1-30}$ alkoxy, $C_{6-30}$ aryl, $C_{3-30}$ heteroaryl, or $C_{1-30}$ alkyl which are unsubstituted or substituted with halogen;
the Rc is each independently selected from $C_{3-10}$ cycloalkyl unsubstituted or substituted with hydroxyl, oxo, or thioxo;
the $Y_1$ is selected from $C_{1-30}$ alkyl, $C_{3-10}$ cycloalkyl, $C_{6-30}$ aryl, or $C_{3-30}$ heteroaryl which are unsubstituted or substituted with Re; wherein the Re is each independently selected from $C_{6-30}$ aryl unsubstituted or substituted with halogen;
the $Y_2$ is selected from unsubstituted or Rf-substituted $C_{12-30}$ condensed cyclic group; wherein the Rf is each independently selected from $C_{1-30}$ alkoxy;
the $Y_3$ is selected from $C_{1-18}$ alkyl or -O-$C_{1-18}$ alkyl;
the $Y_4$ is selected from $C_{6-30}$ aryl or $C_{3-30}$ heteroaryl which are unsubstituted or substituted with Rg, wherein the Rg is each independently selected from hydroxyl or $C_{1-30}$ alkoxy;
the $Y_5$ is selected from a single bond, unsubstituted or amino-substituted $C_{1-18}$ alkylene;
the $Y_6$ is selected from a single bond, -NH-C(NH)-, $-(O-CH_2-CH_2)_m-$ or $-CH_2-(O-CH_2-CH_2)_n-$, wherein the *m* and the *n* are each independently selected from any natural number of 2-20;
the $Y_7$ is selected from amino, $C_{1-30}$ alkoxy or hydroxyl.

In some embodiments of the present disclosure, the compound is selected from the compound of Formula I-1:

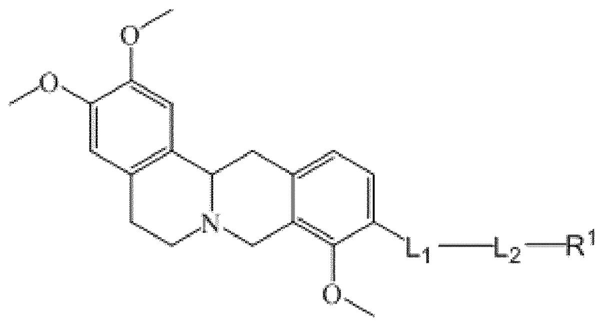

I-1

wherein $L_1$ is selected from a single bond, -O-, -NH-, -S- or -P(O)OH-; alternatively a single bond, -O- and -NH-;
the $L_2$ is selected from a single bond, -C(O)-, -C(O)-O-, $-C_{1-18}$ alkylene-, $-C_{1-18}$ alkylene-O- (e.g., $-(CH2)_{1-5}-O-$), -C(O)-$C_{1-18}$ alkyl-, $C_{1-30}$ alkoxy (e.g., - $(OCH_2CH_2)_{2-8}$-), or $-X_1-PRa(O)-X_2-$;
the Ra is each independently selected from hydrogen, hydroxyl, halogen, $C_{6-30}$ aryl, $C_{3-30}$ heteroaryl, $C_{1-30}$ hydrocarbon radical, $C_{3-10}$ cycloalkyl, $C_{1-30}$ alkoxy, $C_{6-30}$ aryl-O-, $C_{3-30}$ heteroaryl-O-, $C_{6-30}$ aryl-$C_{1-18}$ alkylene-O-, $C_{3-30}$ heteroaryl-$C_{1-18}$ alkylene-O-, -O-$C_{6-30}$ aryl-$C_{1-18}$ alkylene- or -O-$C_{3-30}$ heteroaryl-$C_{1-18}$ alkylene-; wherein an available ring atom on the Ra may be directly connected with $X_2$ to form a 4- to 7-membered ring, alternatively a 5- or 6-membered ring;
the $X_1$ is selected from a single bond, -O- or $-C_{1-18}$ alkylene-O-; alternatively a single bond, -O- or $-(CH_2)_{1-3}O-$ (e.g., $-CH_2O-$);
the $X_2$ is selected from a single bond, -O- or -NR*-; wherein R* is selected from H or $C_{1-6}$ alkyl (e.g., methyl, ethyl, isopropyl, etc.);
the $R^1$ is absent, or selected from hydrogen, hydroxyl, amino, $C_{1-30}$ hydrocarbon radical (in which the $C_{1-30}$ hydrocarbon radical contains 0-10 double bonds or triple bonds, alternatively 0-6 double bonds or triple bonds), $C_{1-30}$ alkoxy, unsubstituted or Rb-substituted $C_{12-30}$ condensed cyclic group, $C_{1-18}$ alkyl-$C_{6-30}$ arylene-$C_{1-18}$ alkylene- which is unsubstituted or has a hydrogen atom replaced with Rc in the alkyl, $C_{1-12}$ alkyl-O-C(O)-$C_{1-12}$ alkylene-imino-, $C_{6-30}$ aryl-$C_{1-18}$ alkylene-, -O-C(O)-$Y_1$, $-C_{1-12}$ alkylene-C(O)-O-$Y_2$, -O-$C_{1-12}$ alkylene-O-C(O)-$Y_3$, -CH=CH-$Y_4$, or $-Y_5-Y_6-Y_7$, $-CHR^{\#}R^{\#\#}$;
wherein $R^{\#}$ and $R^{\#\#}$ are each independently selected from H, hydroxyl, amino, halogen, alkyl, cycloalkyl, aryl, heteroaryl,
-C(O)-$OR^2$, -OC(O)-$OR^2$, $-C_{1-18}$ alkylene-C(O)-$OR^2$, $-C_{1-18}$ alkylene-$R^2$, or $-C_{6-10}$ aryl-$C_{1-6}$ alkyl;
$R^2$ is selected from H, hydroxyl, amino, halogen, alkyl, cycloalkyl, aryl, heteroaryl, unsubstituted or Rb-substituted $C_{12-30}$ condensed cyclic group, or -NHC(NH)NH-;
the Rb is each independently selected from $C_{1-30}$ alkoxy, $C_{6-30}$ aryl, $C_{3-30}$ heteroaryl, or $C_{1-30}$ alkyl which are unsubstituted or substituted with halogen;

the Rc is each independently selected from $C_{3-10}$ cycloalkyl unsubstituted or substituted with hydroxyl, oxo, or thioxo; the $Y_1$ is selected from $C_{1-30}$ alkyl, $C_{3-10}$ cycloalkyl, $C_{6-30}$ aryl, or $C_{3-30}$ heteroaryl which are unsubstituted or substituted with Re; wherein the Re is each independently selected from $C_{6-30}$ aryl unsubstituted or substituted with halogen;
the $Y_2$ is selected from unsubstituted or Rf-substituted $C_{12-30}$ condensed cyclic group; wherein the Rf is each independently selected from $C_{1-30}$ alkoxy;
the $Y_3$ is selected from $C_{1-18}$ alkyl or -O-$C_{1-18}$ alkyl;
the $Y_4$ is selected from $C_{6-30}$ aryl or $C_{3-30}$ heteroaryl which are unsubstituted or substituted with Rg, wherein the Rg is each independently selected from hydroxyl or $C_{1-30}$ alkoxy;
the $Y_5$ is selected from a single bond, unsubstituted or amino-substituted $C_{1-18}$ alkylene;
the $Y_6$ is selected from a single bond, -NH-C(NH)-, $-(O-CH_2-CH_2)_m$- or $-CH_2-(O-CH_2-CH_2)_n$-, wherein the *m* and the *n* are each independently selected from any natural number of 2-20;
the $Y_7$ is selected from amino, $C_{1-30}$ alkoxy or hydroxyl.

In some embodiments of the present disclosure, in the compound of Formula II, the $L_2$ is selected from a single bond, -C(O)-, -C(O)-O-, $C_{1-6}$ alkylene, -$C_{1-6}$ alkylene-O- or -$X_1$-PRa(O)-$X_2$-; the Ra is each independently selected from hydrogen, hydroxyl, $C_{6-12}$ aryl-O-, or $C_{6-12}$ aryl-$C_{1-6}$ alkylene-O-, -O-$C_{6-30}$ aryl-$C_{1-18}$ alkylene- or - O-$C_{3-30}$ heteroaryl-$C_{1-18}$ alkylene-; the Ra and $X_2$ may be connected to form a ring;

the $X_1$ is selected from a single bond or -$C_{1-6}$ alkylene-O-;
the $X_2$ is selected from a single bond, -O-, -NH-;
the $R^1$ is selected from absent, hydrogen, hydroxyl, amino, $C_{1-30}$ alkyl, $C_{1-30}$ alkoxy, unsubstituted or methoxy-substituted $C_{12-17}$ tetracondensed cyclic group containing at least one heteroatom, oxocyclopentyl-$C_{1-6}$ alkylene-$C_{6-12}$ arylene-$C_{1-6}$ alkylene-, hydroxylcyclopentyl-$C_{1-6}$ alkylene-$C_{6-12}$ arylene-$C_{1-6}$ alkylene-, $C_{1-6}$ alkyl-O-C(O)-$C_{1-6}$ alkylene-imino-, $C_{6-12}$ aryl-$C_{1-6}$ alkylene-, -O-C(O)-$Y_1$, -$C_{1-12}$ alkylene-C(O)-O-$Y_2$, -O-$C_{1-12}$ alkylene-O-C(O)-$Y_3$, -C=CH-$Y_4$, or -$Y_5$-$Y_6$-$Y_7$, -$CHR^{\#}R^{\#\#}$;
wherein $R^{\#}$ and $R^{\#\#}$ are each independently selected from H, hydroxyl, amino, halogen, alkyl, cycloalkyl, aryl, heteroaryl,
-C(O)-$OR^2$, -OC(O)-$OR^2$, -$C_{1-18}$ alkylene-C(O)-$OR^2$, -$C_{1-18}$ alkylene-$R^2$, or -$C_{6-10}$ aryl-$C_{1-6}$ alkyl;
$R^2$ is selected from H, hydroxyl, amino, halogen, alkyl, cycloalkyl, aryl, heteroaryl, unsubstituted or Rb-substituted $C_{12-30}$ condensed cyclic group, or -NHC(NH)NH-;
the $Y_1$ is selected from $C_{1-6}$ alkyl, $C_{3-6}$ cycloalkyl or $C_{6-12}$ aryl which are unsubstituted or substituted with Re; wherein the Re is each independently selected from unsubstituted or halogen-substituted $C_{6-12}$ aryl;
the $Y_3$ is selected from $C_{1-6}$ alkyl or -O-$C_{1-6}$ alkyl;
the $Y_4$ is selected from unsubstituted or Rg-substituted $C_{6-12}$ aryl, wherein the Rg is each independently selected from hydroxyl or methoxy;
the $Y_5$ is selected from a single bond, unsubstituted or amino-substituted $C_{1-6}$ alkylene;
the $Y_6$ is selected from a single bond, -NH-C(NH)-, $-(O-CH_2-CH_2)_m$- or $-CH_2-(O-CH_2-CH_2)_n$-, wherein the *m* and the *n* are each independently selected from any natural number of 2-10;
the $Y_7$ is selected from amino, $C_{1-6}$ alkoxy or hydroxyl.

In some embodiments, the $R^1$ is selected from absent, hydrogen, hydroxyl, amino, aryl (e.g., phenyl), $C_{1-30}$ alkyl, $C_{1-30}$ alkoxy (e.g., $-OCH_3$), unsubstituted or Rb-substituted $C_{12-30}$ condensed cyclic group, $-(CH_2)_{1-10}$-C(O)-$OC_{12-30}$ condensed cyclic group, -O-C(O)-$Y_1$, -$C_{1-12}$ alkylene-C(O)-O-$Y_2$, -O-$C_{1-12}$ alkylene-O-C(O)-$Y_3$, -C=CH-$Y_4$, or -$Y_5$-$Y_6$-$Y_7$, -$CHR^{\#}R^{\#\#}$, alternatively the structures of H, -OH, $-NH_2$, $-OCH_3$, methyl, ethyl, isopropyl, cyclopropyl, $-(CH_2)_pCH_3$, $-(CH_2)_pOCH_3$, $-(CH_2)_pNH_2$, $-(CH_2)_pOH$, $-(OCH_2CH_2)qCH_3$, $-(OCH_{2C}H_2)qOCH_3$, - $(OCH_2CH_2)qOH$, $-(OCH_2CH_2)qNH_2$, phenyl,

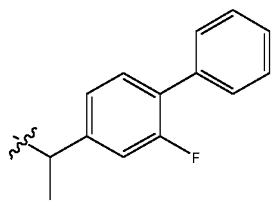 , 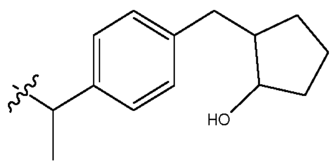 ,

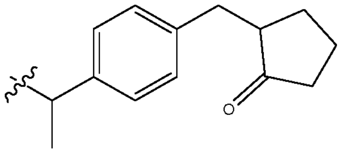, 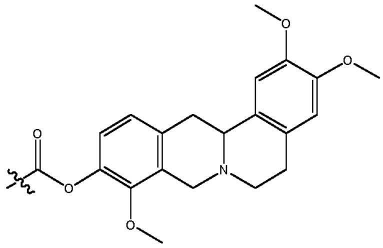, 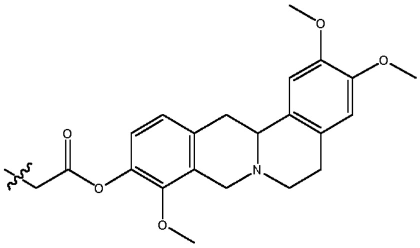,

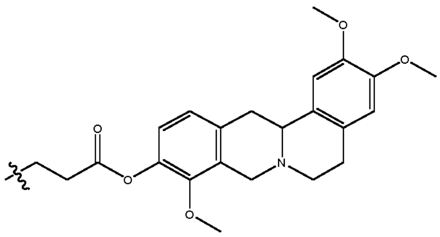, 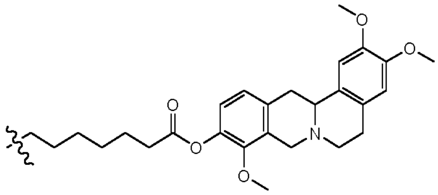, 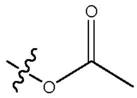,

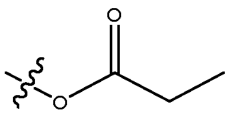, 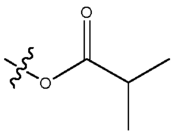, 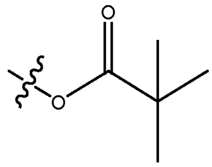, 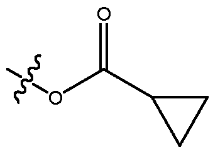, 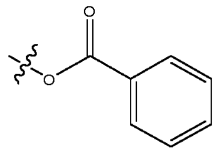,

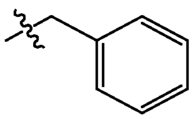, 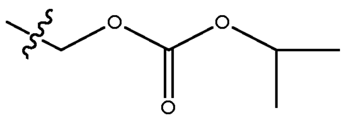, 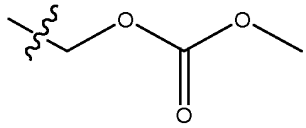, 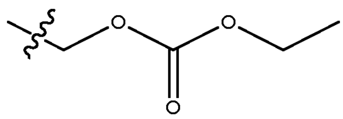,

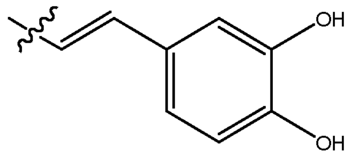, 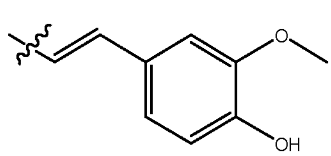, 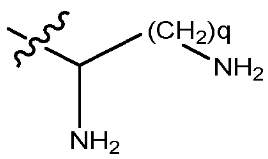

(e.g.,

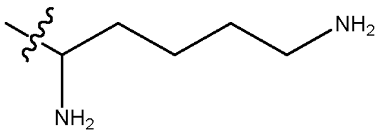), 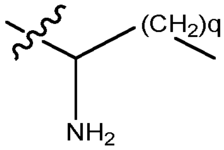, 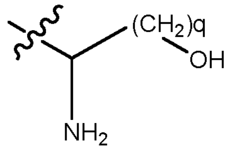,

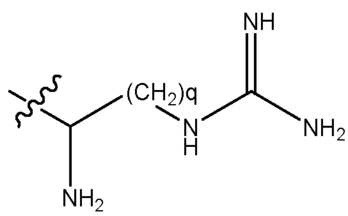

(e.g.,

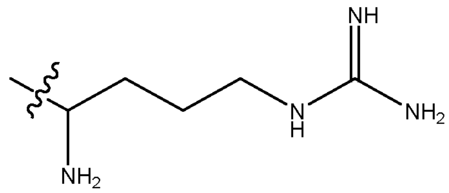

);

wherein *p* is selected from 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30;

*q* is selected from 0, 1, 2, 3, 4, 5, 6, 7, 8, 9, 10, 11, 12, 13, 14, or 15.

The oxocyclopentyl of the present disclosure refers to

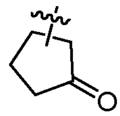

,

and the hydroxylcyclopentyl of the present disclosure refers to

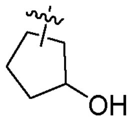

.

In some embodiments of the present disclosure, in the compound of Formula II, the $L_2$ is selected from a single bond, -C(O)-, -C(O)-O-, $C_{1-6}$ alkylene, -$C_{1-6}$ alkylene-O- or -$X_1$-PH(O)-$X_2$-; the hydrogen atom on the -$X_1$-PH(O)-$X_2$- may be substituted with Ra, wherein the Ra is each independently selected from hydroxyl, $C_{6-12}$ aryl-O-, or $C_{6-12}$ aryl-$C_{1-6}$ alkylene-O-, -O-$C_{6-30}$ aryl-$C_{1-18}$ alkylene- or -O-$C_{3-30}$ heteroaryl-$C_{1-18}$ alkylene-; the Ra and $X_2$ may be connected to form a ring;

the $X_1$ is selected from a single bond or -$C_{1-6}$ alkylene-O-;

the $X_2$ is selected from a single bond or -O-;

the $R^1$ is selected from hydrogen, hydroxyl, amino, $C_{1-30}$ hydrocarbon radical, $C_{1-30}$ alkoxy, unsubstituted or methoxy-substituted $C_{12-17}$ tetracondensed cyclic group containing at least one heteroatom, oxocyclopentyl-$C_{1-6}$ alkylene-$C_{6-12}$ arylene-$C_{1-6}$ alkylene-, hydroxylcyclopentyl-$C_{1-6}$ alkylene-$C_{6-12}$ arylene-$C_{1-6}$ alkylene-, $C_{1-6}$ alkyl-O-C(O)-$C_{1-6}$ alkylene-imino-, $C_{6-12}$ aryl-$C_{1-6}$ alkylene-, -O-C(O)-$Y_1$, -$C_{1-12}$ alkylene-C(O)-O-$Y_2$, -O-$C_{1-12}$ alkylene-O-C(O)-$Y_3$, -C=CH-$Y_4$, or -$Y_5$-$Y_6$-$Y_7$;

the $Y_1$ is selected from $C_{1-6}$ alkyl, $C_{3-6}$ cycloalkyl or $C_{6-12}$ aryl which are unsubstituted or substituted with Re; wherein the Re is each independently selected from unsubstituted or halogen-substituted $C_{6-12}$ aryl;

the $Y_3$ is selected from $C_{1-6}$ alkyl or -O-$C_{1-6}$ alkyl;

the $Y_4$ is selected from unsubstituted or Rg-substituted $C_{6-12}$ aryl, wherein the Rg is each independently selected from hydroxyl or methoxy;

the $Y_5$ is selected from a single bond, unsubstituted or amino-substituted $C_{1-6}$ alkylene;

the $Y_6$ is selected from a single bond, -NH-C(NH)-, $-(O-CH_2-CH_2)_m-$ or $-CH_2-(O-CH_2-CH_2)_n-$, wherein the *m* and the *n* are each independently selected from any natural number of 2-10;

the $Y_7$ is selected from amino, $C_{1-6}$ alkoxy or hydroxyl.

In some embodiments of the present disclosure, the compound is selected from the compound of Formula III:

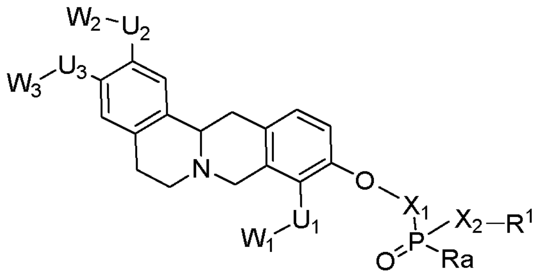

III ;

wherein various groups are defined as those for the compound of Formula II.

In some embodiments of the present disclosure, in the compound of Formula III, the Ra is each independently selected from hydroxyl, $C_{6-12}$ aryl-O-, $C_{6-12}$ aryl-$C_{1-6}$ alkylene-O- or -O-$C_{6-12}$ aryl-$C_{1-6}$ alkylene-; the Ra and $X_2$ may be connected to form a ring;

the $X_1$ is selected from a single bond or -$C_{1-6}$ alkylene-O-;
the $X_2$ is selected from a single bond, -O-, -NH-;
the $R^1$ is selected from absent, hydrogen, hydroxyl, $C_{1-6}$ alkyl-O-C(O)-$C_{1-6}$ alkylene-imino-, $C_{6-12}$ aryl-$C_{1-6}$ alkylene- or -O-$C_{1-12}$ alkylene-O-C(O)-$Y_3$; the $Y_3$ is selected from -O-$C_{1-6}$ alkyl.

In some embodiments of the present disclosure, the compound is selected from the compound of Formula IVa, Formula IVb, or Formula IVc:

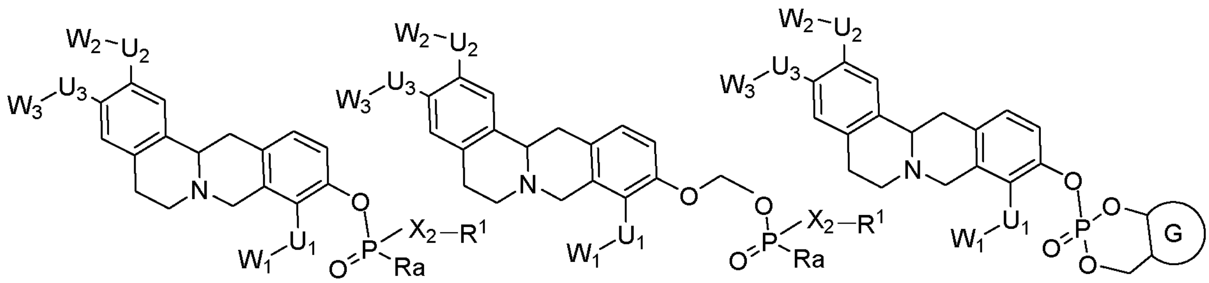

IVa IVb IVc

wherein, the ring G of Formula IVc is $C_{6-12}$ aryl ring; and various groups are defined as those for the compound of Formula III.

In some embodiments of the present disclosure, the compound is selected from the compound of Formula V:

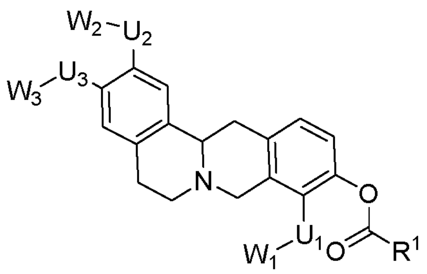

V ;

wherein various groups are defined as those for the compound of Formula II.

In some embodiments of the present disclosure, the compound is selected from the compound of Formula VIa or Formula VIb:

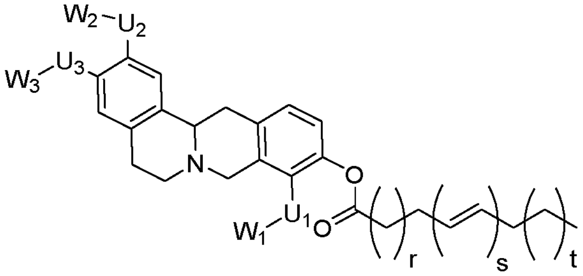

VIa

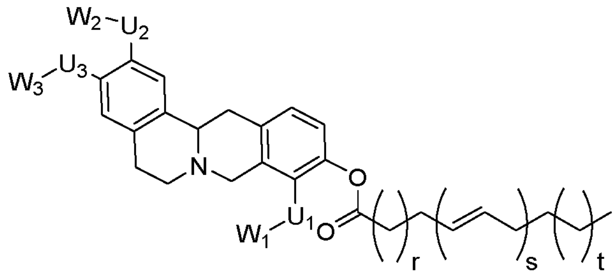

VIb

wherein *r* and *t* in Formula VIa and Formula VIb are each independently selected from any natural number of 0-10, s is each independently selected from any natural number of 0-9 (alternatively any natural number of 1-*6); r, s* and *t* are not 0 at the same time; various groups are defined as those for the compound of Formula V.

In some embodiments of the present disclosure, the compound is selected from the compound of Formula VII:

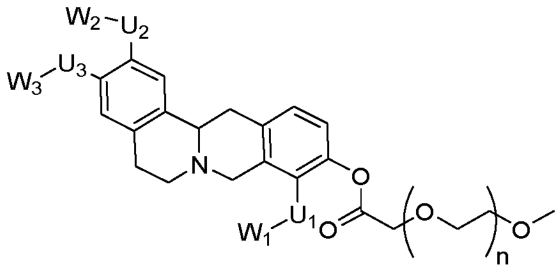

VII

wherein n is selected from any natural number of 2-20, alternatively any natural number of 2-10; various groups are defined as those for the compound of Formula V.

In some embodiments of the present disclosure, the compound is selected from the compound of Formula VIII:

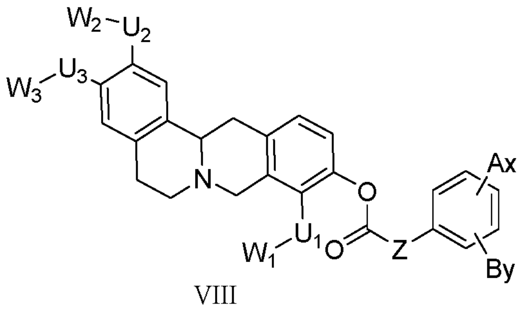

VIII

wherein Z is selected from $C_{1-30}$ alkylene, $C_{3-10}$ cycloalkylene, $C_{6-30}$ arylene, $C_{3-30}$ heteroarylene, or -C=CH-;
A is selected from hydroxyl, halogen, $C_{6-30}$ aryl or Re-substituted $C_{1-18}$ alkyl, wherein the Rc is each independently selected from $C_{3-10}$ cycloalkyl unsubstituted or substituted with hydroxyl, oxo, or thioxo;
B is selected from hydroxyl, halogen or $C_{6-30}$ aryl;
*x* is selected from 0 or 1, y is selected from 0, 1 or 2, and *x* + *y* is not 0;
various groups are defined as those for the compound of Formula V.

In some embodiments of the present disclosure, the compound is selected from the compound of Formula IX:

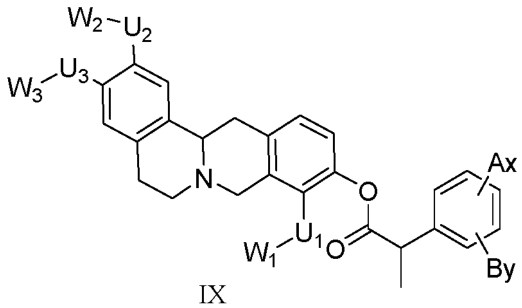

IX

A is selected from Rc-substituted $C_{1-18}$ alkyl, wherein the Rc is each independently selected from $C_{3-10}$ cycloalkyl substituted with oxo;
B is selected from halogen or $C_{6-30}$ aryl;
*x* is selected from 1, *y* is selected from 0 or 1;
various groups are defined as those for the compound of Formula VIII.

In some embodiments of the present disclosure, the compound is selected from the compound of Formula X:

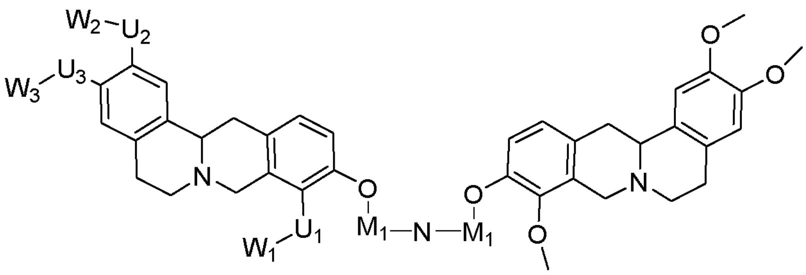

X

wherein $M_1$ is each independently selected from a single bond or -C(O)-;
$L_3$ is selected from $C_{1-12}$ alkylene;
various groups are defined as those for the compound of Formula I.

In some embodiments of the present disclosure, the compound is selected from the compound of Formula XI:

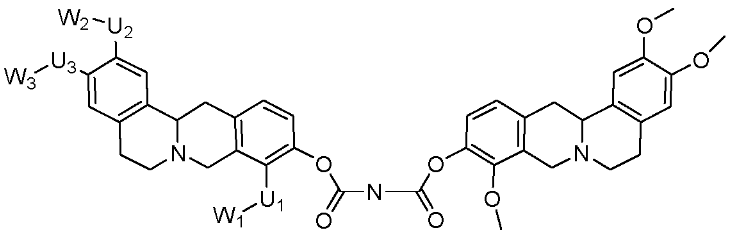

XI

wherein, $L_3$ is selected from $C_{1-12}$ alkylene;
alternatively, $U_1$, $U_2$, and $U_3$ are each independently selected from -O-, and $W_1$, $W_2$, and $W_3$ are each independently selected from methyl.

In some embodiments of the present disclosure, the compound is selected from the compound of Formula XII:

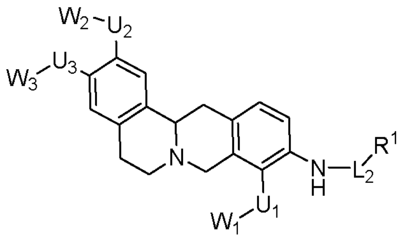

XII ;

wherein various groups are defined as those for the compound for Formula I.

In some embodiments of the present disclosure, in the compound of Formula XII, the $L_2$ is selected from a single bond or -O-; the $R^1$ is selected from hydrogen, amino, or $-Y_5-Y_6-Y_7$; and the $Y_5$ is selected from a single bond, unsubstituted or amino-substituted $C_{3-12}$ alkylene.

In some embodiments of the present disclosure, the compound is selected from the compound of Formula XIII:

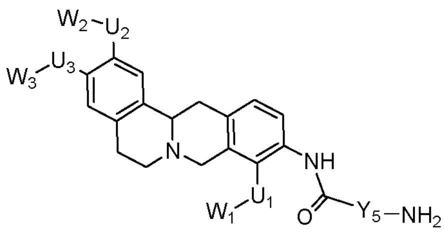

XIII

alternatively, the $Y_5$ is selected from unsubstituted or amino-substituted $C_{3-12}$ alkylene, alternatively unsubstituted or amino-substituted $C_{3-6}$ alkylene; various groups are defined as those for the compound of Formula XII.

In some embodiments of the present disclosure, the compound is selected from the compound of Formula XIV:

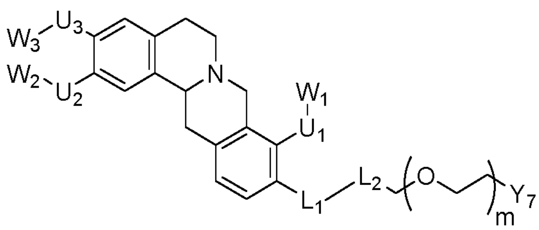

XIV ;

wherein various groups are defined as those for the compound for Formula I.

In some embodiments of the present disclosure, in the compound of Formula XIV: the $L_1$ is selected from - O- or -NH-; the $L_2$ is selected from -C(O)- or $C_{1-18}$ alkylene; and the $Y_7$ is selected from amino, $C_{1-30}$ alkyl, $C_{1-30}$ alkoxy or hydroxyl.

In some embodiments of the present disclosure, the compound is selected from the compound of Formula XVa or Formula XVb:

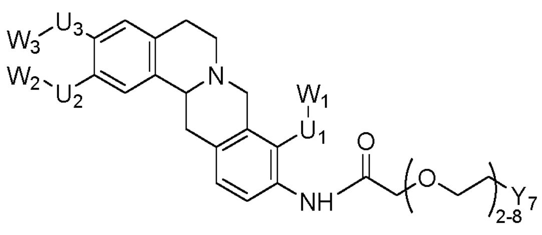

XVa

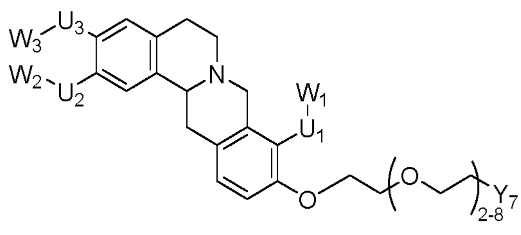

XVb

alternatively, the $Y_7$ is selected from methoxy or hydroxyl; and various groups are defined as those for the compound of Formula XIV.

In some embodiments of the present disclosure, in the compounds of Formulae I to XIV, $U_1$, $U_2$, and $U_3$ are each

independently selected from -O-;

$W_1$, $W_2$, and $W_3$ are each independently selected from $C_{1-18}$ alkyl, alternatively $C_{1-12}$ alkyl, yet alternatively $C_{1-6}$ alkyl, further alternatively methyl.

In some embodiments of the present disclosure, the compound is selected from the compound of Formula Ia:

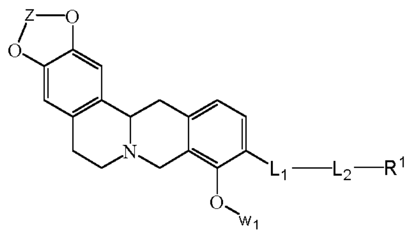

Ia

,

alternatively

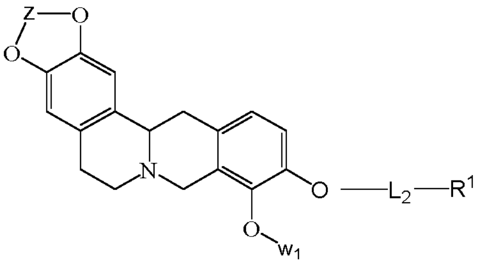

Ia-1

, or

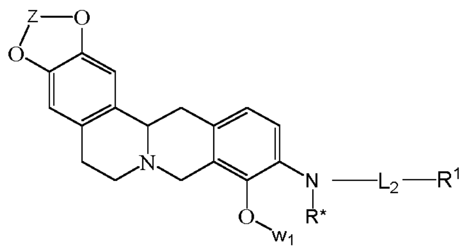

Ia-2

wherein $L_1$, $L_2$, $W_1$, $R^1$, and R* are defined as those for the compound of Formula I.

In some embodiments, the present disclosure relates to the compound of Formula XVI, XVI-1, XVI-2, XVI-3, or XVI-4, or a pharmaceutically acceptable salt, ester, stereoisomer, polymorph, solvate, N-oxide, isotope-labeled compound, metabolite, or prodrug thereof, and a mixture thereof:

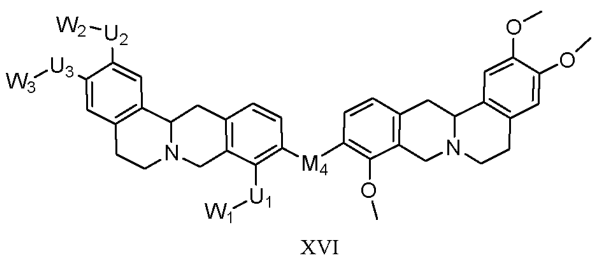

XVI

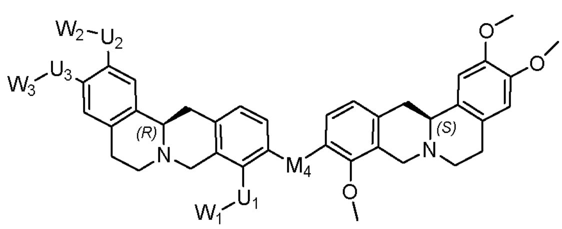

XVI-1

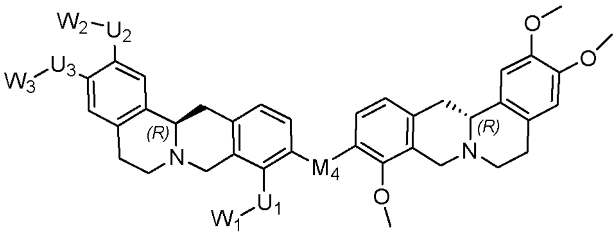

XVI-2

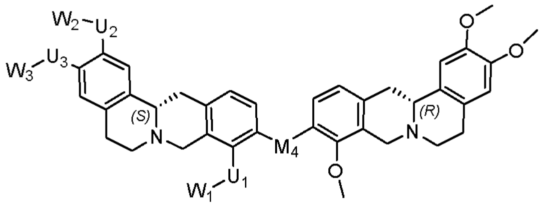

XVI-3

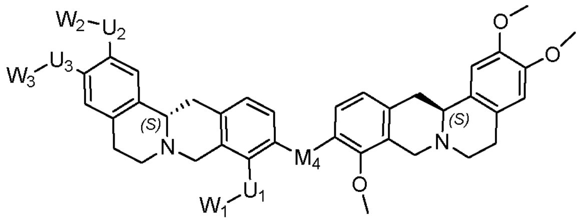

XVI-4

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-18}$ alkyl; and
$M_4$ is selected from a chemical bond, -O-, -NH-, -C(O)-, -C(O)O-, -OC(O)-, -NHC(O)O-, -OC(O)NH- or - $C_{1-10}$ alkylene-.

In some embodiments, the present disclosure relates to the compound of Formula XVI, XVI-1, XVI-2, XVI-3, or XVI-4, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are independently selected from -O-C(O)- or -O-;
$W_1$, $W_2$, and $W_3$ are independently selected from $C_{1-12}$ alkyl, alternatively $C_{1-6}$ alkyl; and
$M_4$ is selected from a chemical bond, -NH-, -C(O)O-, -OC(O)-, -NHC(O)O- or -OC(O)NH-.

In some embodiments, the present disclosure relates to the compound of Formula XVI, XVI-1, XVI-2, XVI-3, or XVI-4, or a pharmaceutically acceptable salt, ester, stereoisomer, polymorph, solvate, N-oxide, isotope-labeled compound, metabolite, or prodrug thereof, and a mixture thereof, wherein

$U_1$, $U_2$, and $U_3$ are -O-;
$W_1$, $W_2$, and $W_3$ are independently selected from $C_{1-6}$ alkyl, alternatively $C_{1-4}$ alkyl, yet alternatively methyl; and
$M_4$ is selected from -NHC(O)O- or -OC(O)NH-.

In some embodiments, the present disclosure relates to the compound of Formula VIII, VIII-1, or VIII-2, or a pharmaceutically acceptable salt, ester, stereoisomer, polymorph, solvate, N-oxide, isotope-labeled compound, metabolite, or prodrug thereof, and a mixture thereof:

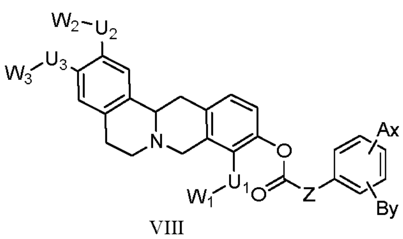

VIII

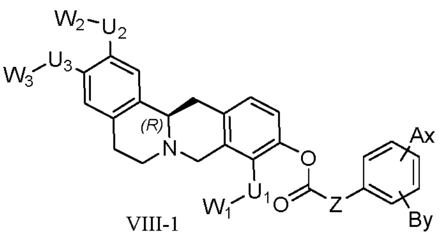

VIII-1

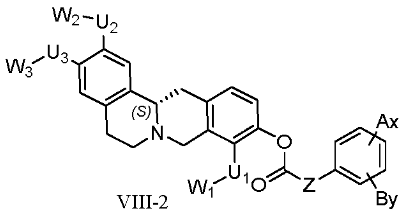

VIII-2

wherein

$U_1$, $U_2$, and $U_3$ are independently selected from -O-C(O)-, -NH-C(O)-, -O-$CH_2$-O- or -O;
$W_1$, $W_2$, and $W_3$ are independently selected from $C_{1-12}$ alkyl;
Z is selected from a chemical bond, -O-, -NH-, -$C_{1-12}$ alkylene-, -$C_{1-12}$ alkylene-O-$C_{1-12}$ alkylene-, -$C_{1-12}$ alkylene-NH-$C_{1-12}$ alkylene-, -$C_{1-18}$ alkylene-C(O)-$C_{1-12}$ alkylene-, -$C_{1-10}$ alkylene-C(O)O-$C_{1-10}$ alkylene-, -$C_{1-10}$ alkylene-NHC(O)-$C_{1-10}$ alkylene-, -$C_{1-10}$ alkylene-NHC(O)O-$C_{1-10}$ alkylene-, -$C_{1-10}$ alkylene-C(O)NH-$C_{1-10}$ alkylene- or -$C_{1-10}$ alkylene-OC(O)NH-$C_{1-10}$ alkylene-, alternatively a chemical bond or -$C_{1-6}$ alkylene-NHC(O)-$C_{1-6}$ alkylene-;
the Z is optionally substituted with 1, 2, 3, 4, or 5 substituents selected from halogen, -C(O)OH, -C(O)O-$C_{1-6}$ alkyl, -C(O)-$C_{1-6}$ alkyl, -O-$C_{1-6}$ alkyl, $C_{1-6}$ alkyl, or $C_{1-6}$haloalkyl;

A and B are independently selected from H, halogen, OH, -$L_A$-$C_{3-10}$ cycloalkyl, -$L_A$-3- to 10-membered heterocyclyl, -$L_A$-$C_{6-12}$ aryl, or -$L_A$-5- to 12-membered heteroaryl, alternatively halogen, $C_{6-10}$ aryl, or -NH-$C_{6-10}$ aryl;

$L_A$ is selected from a chemical bond, -NH-, -O-, -C(O)-, -C(O)O-, -NHC(O)-, -NHC(O)O-, -OC(O)NH- or - C(O)NH-;
the A and B are optionally further substituted with halogen, $C_{1-6}$ alkyl, or $C_{1-6}$ haloalkyl;
*x is* 0, 1 or 2; and
*y* is 0, 1, 2, or 3.

In some embodiments, the present disclosure relates to the compound of Formula VIII, VIII-1, or VIII-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are independently selected from -O-C(O)- or -O-;
$W_1$, $W_2$, and $W_3$ are independently selected from $C_{1-6}$ alkyl;
Z is selected from a chemical bond, -O-, -NH-, -$C_{1-6}$ alkylene-, -$C_{1-6}$ alkylene-C(O)-$C_{1-6}$ alkylene-, -$C_{1-6}$ alkylene-C(O)O-$C_{1-6}$ alkylene-, -$C_{1-6}$ alkylene-NHC(O)-$C_{1-6}$ alkylene-, -$C_{1-6}$ alkylene-C(O)NH-$C_{1-6}$ alkylene-, alternatively a chemical bond, -$C_{1-6}$ alkylene-NHC(O)-$C_{1-6}$ alkylene- or -$C_{1-6}$ alkylene-C(O)NH-$C_{1-6}$ alkylene-;
the Z is optionally substituted with 1, 2, or 3 substituents selected from halogen, C(O)OH, C(O)O-$C_{1-6}$ alkyl, $C_{1-6}$ alkyl, or $C_{1-6}$ haloalkyl;
A and B are independently selected from H, halogen, $C_{6-10}$ aryl, -NH-$C_{6-10}$ aryl, -O-$C_{6-10}$ aryl, -C(O)-$C_{6-10}$ aryl, and -C(O)O-$C_{6-10}$ aryl, alternatively halogen, $C_{6-10}$ aryl, or -NH-$C_{6-10}$ aryl;
the A and B are optionally further substituted with halogen, $C_{1-6}$ alkyl, or $C_{1-6}$ haloalkyl;
*x is* 0, 1 or 2; and
*y* is 0, 1, 2, or 3.

In some embodiments, the present disclosure relates to the compound of Formula VIII, VIII-1, or VIII-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are -O-;
$W_1$, $W_2$, and $W_3$ are independently selected from $C_{1-4}$ alkyl, alternatively methyl;
Z is selected from a chemical bond, -$C_{1-4}$ alkylene-NHC(O)-$C_{1-4}$ alkylene- or -$C_{1-4}$ alkylene-C(O)NH-$C_{1-4}$ alkylene-, alternatively a chemical bond or -$C_{1-4}$ alkylene-NHC(O)-$C_{1-4}$ alkylene-;
the Z is optionally substituted with 1, 2, or 3 substituents selected from halogen, C(O)OH, C(O)O-$C_{1-4}$ alkyl, or $C_{1-4}$ alkyl;
A and B are independently selected from H, halogen, phenyl, or -NH-phenyl, alternatively F, phenyl, or NH-phenyl;
the A and B are optionally further substituted with halogen;
*x* and *y* are 0, 1 or 2.

In some embodiments, the present disclosure relates to the compound of Formula II, II-1, II-2, Formula I-1, I-2, or I-3, or a pharmaceutically acceptable salt, ester, stereoisomer, polymorph, solvate, N-oxide, isotope-labeled compound, metabolite, or prodrug thereof, and a mixture thereof:

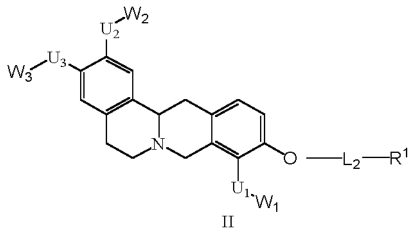
II

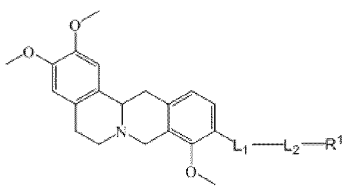
I-1

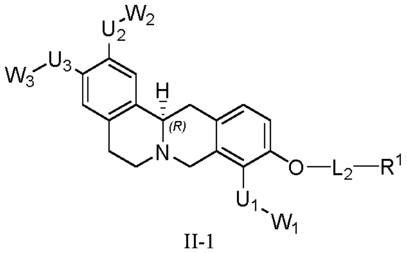
II-1

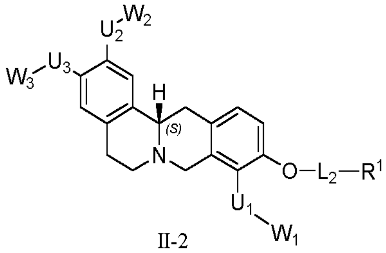
II-2

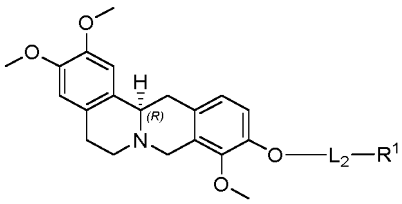
I-2

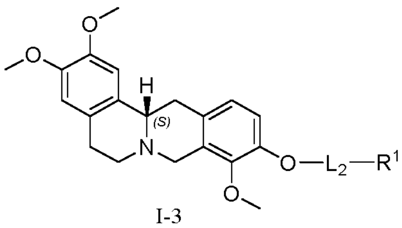
I-3

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
$L_1$ is selected from a single bond, -O- or -NH-;
$L_2$ is -$C_{1-12}$ alkylene-, and the alkylene may be further substituted with $C_{1-6}$ alkyl;
$R^1$ is selected from -OC(O)-$Y_1$;
$Y_1$ is selected from $C_{1-12}$ alkyl, $C_{3-10}$ cycloalkyl, $C_{6-18}$ aryl, or $C_{3-18}$ heteroaryl, and the $Y_1$ may be further substituted with $C_{1-6}$ alkyl;
$Y_1$ is alternatively $C_{1-6}$ alkyl, yet alternatively $CH_3$.

In some embodiments, the present disclosure relates to the compound of Formula II, II-1, II-2, Formula I-1, I-2, or I-3, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are selected from -O- or -OC(O)-;
$W_1$, $W_2$, and $W_3$ are independently selected from $C_{1-6}$ alkyl;
$L_1$ is selected from -O- or -NH-;
$L_2$ is -$C_{1-6}$ alkylene-, and the alkylene may be further substituted with $C_{1-6}$ alkyl;
$R^1$ is selected from -OC(O)-$Y_1$;
$Y_1$ is selected from $C_{1-6}$ alkyl, $C_{3-6}$ cycloalkyl or $C_{6-12}$ aryl, and the $Y_1$ may be further substituted with $C_{1-6}$ alkyl; and
$Y_1$ is alternatively $CH_3$.

In some embodiments, the present disclosure relates to the compound of Formula II, II-1, II-2, Formula I-1, I-2, or I-3, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are -O-;
$W_1$, $W_2$, and $W_3$ are $CH_3$;
$L_1$ is -O-;
$L_2$ is -$CH_2$-, -$CH(CH_3)$-,

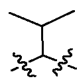 ;

and
$R^1$ is selected from

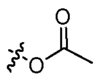 , 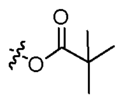 , 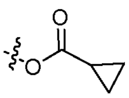 , or 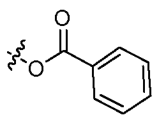 .

In some embodiments, the present disclosure relates to the compound of Formula III, III-1, or III-2, or a pharmaceutically acceptable salt, ester, stereoisomer, polymorph, solvate, N-oxide, isotope-labeled compound, metabolite, or prodrug thereof, and a mixture thereof:

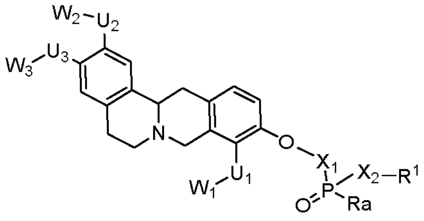

III

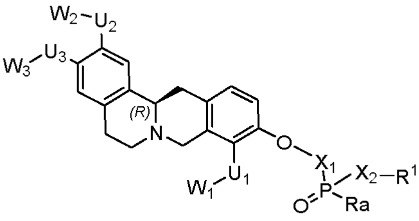

III-1

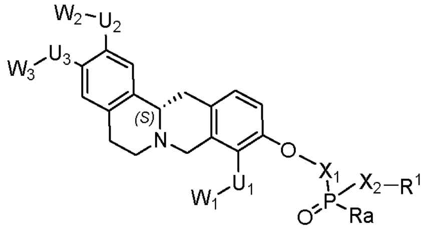

III-2

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
$X_1$ is selected from a chemical bond or -$C_{1-6}$ alkylene-O-;
$X_2$ is a chemical bond, -O- or -C(O)-;
$R^1$ is absent, or selected from H, -NH-$C_{1-12}$ alkylene-C(O)-O-$C_{1-12}$ alkyl, -$C_{1-12}$ alkylene-$C_{6-18}$ aryl, -O-$C_{1-12}$ alkylene-O-C(O)-$Y_3$, wherein the hydrogen atom(s) in the alkylene of $R^1$ is(are) optionally substituted with Rd;
$Y_3$ is -O-$C_{1-6}$ alkyl, and the $C_{1-6}$ alkyl may be further substituted with $C_{1-6}$ alkyl;
Ra is selected from OH, -O-$C_{6-18}$ aryl, -O-$C_{1-12}$ alkylene-$C_{6-18}$ aryl, or -O-$C_{6-18}$ aryl-$C_{1-12}$ alkyl;
or Ra and $X_2$ are connected to form a ring; and
Rd is selected from $C_{1-12}$ alkyl or $C_{1-12}$ alkoxy.

In some embodiments, the present disclosure relates to the compound of Formula III, III-1, or III-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are selected from -O- or -OC(O)-;
$W_1$, $W_2$, and $W_3$ are independently selected from $C_{1-6}$ alkyl;
$X_1$ is selected from a chemical bond or -$C_{1-6}$ alkylene-O-;
$X_2$ is a chemical bond, -O- or -C(O)-;
$R^1$ is absent, or selected from H, -NH-$C_{1-6}$ alkylene-C(O)-O-$C_{1-6}$ alkyl, -$C_{1-6}$ alkylene-$C_{6-10}$ aryl, -O-$C_{1-6}$ alkylene-O-C(O)-$Y_3$, wherein the hydrogen atom(s) in the alkylene of $R^1$ is(are) optionally substituted with Rd;
$Y_3$ is -O-$C_{1-6}$ alkyl, and the $C_{1-6}$ alkyl may be further substituted with $C_{1-6}$ alkyl;
Ra is selected from OH, -O-$C_{6-12}$ aryl, -O-$C_{1-6}$ alkylene-$C_{6-12}$ aryl, or -O-$C_{6-12}$ aryl-$C_{1-6}$ alkyl;
or Ra and $X_2$ are connected to form a ring; and
Rd is $C_{1-6}$ alkyl.

In some embodiments, the present disclosure relates to the compound of Formula III, III-1, or III-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are O;
$W_1$, $W_2$, and $W_3$ are $CH_3$;
$X_1$ is selected from a chemical bond or -$CH_2$-O-;
$X_2$ is a chemical bond or -O-;
$R^1$ is absent, or selected from H,

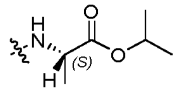

,

$-CH_2$-phenyl or

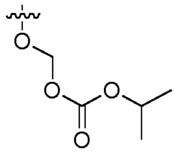

;

Ra is selected from OH, -O-phenyl, or $-O-CH_2$-phenyl, or $-O$-phenyl-$CH_3$;
or Ra and $X_2$ are connected to form a ring.

In some embodiments, the present disclosure relates to the compound of Formula IVa, IVa-1, IVa-2, IVb, IVb-1, IVb-2, IVc, IVc-1, or IVc-2, or a pharmaceutically acceptable salt, ester, stereoisomer, polymorph, solvate, N-oxide, isotope-labeled compound, metabolite, or prodrug thereof, and a mixture thereof:

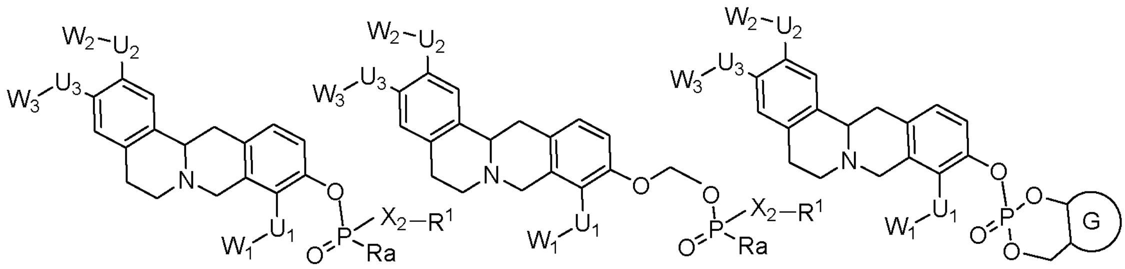

IVa IVb IVc

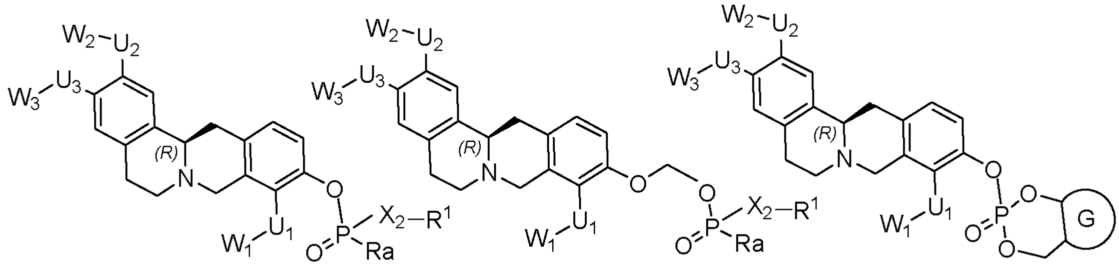

IVa-1 IVb-1 IVc-1

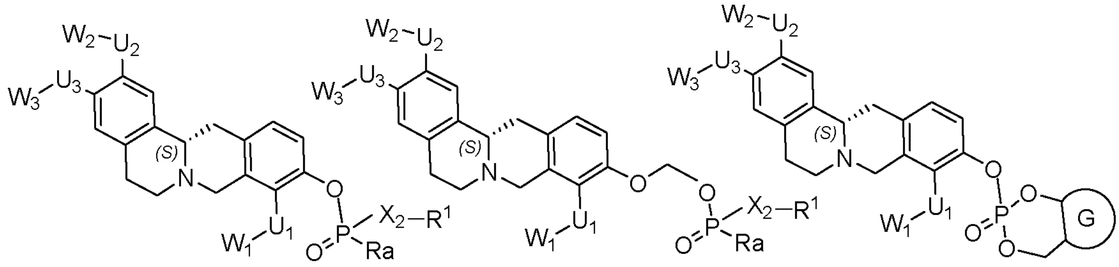

IVa-2 IVb-2 IVc-2

wherein the ring G is $C_{6-12}$ aryl, alternatively $C_{6-10}$ aryl, yet alternatively phenyl; and
various other variables are as defined above.

In some embodiments, the present disclosure relates to the compound of Formula V, V-1, or V-2, or a pharmaceutically acceptable salt, ester, stereoisomer, polymorph, solvate, N-oxide, isotope-labeled compound, metabolite, or prodrug thereof, and a mixture thereof:

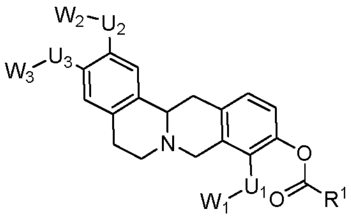
V

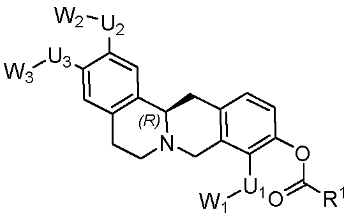
V-1

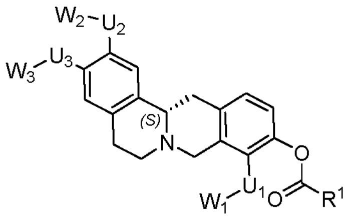
V-2

wherein

- $U_1$, $U_2$, and $U_3$ are independently selected from -O-, -O-C(O)-, -NH-C(O)- or -O-$CH_2$-O-;
- $W_1$, $W_2$, and $W_3$ are independently selected from $C_{1-12}$ alkyl;
- $R^1$ is selected from $C_{1-30}$ alkyl, $C_{2-30}$ alkenyl, $C_{1-20}$ alkoxy, and -O-$C_{1-12}$ alkylene-O-C(O)-$Y_3$ which is unsubstituted or has a hydrogen atom replaced with Rd in the alkylene;
- Rd is selected from $C_{1-12}$ alkyl;
- $R^1$ is alternatively $C_{6-10}$ alkyl or $C_{10-30}$ alkenyl;
- $Y_3$ is selected from $C_{1-12}$ alkyl or O-$C_{1-12}$ alkyl.

In some embodiments, the present disclosure relates to the compound of Formula V, V-1, or V-2, or a pharmaceutically acceptable salt, ester, stereoisomer, polymorph, solvate, N-oxide, isotope-labeled compound, metabolite, or prodrug thereof, and a mixture thereof, wherein:

- $U_1$, $U_2$, and $U_3$ are selected from -O- or -OC(O)-;
- $W_1$, $W_2$, and $W_3$ are independently selected from $C_{1-6}$ alkyl;
- $R^1$ is selected from $C_{1-30}$ alkyl, $C_{2-30}$ alkenyl, $C_{1-6}$ alkoxy, and -O-$C_{1-6}$ alkylene-O-C(O)-$Y_3$ which is unsubstituted or has a hydrogen atom replaced with Rd in the alkylene;
- Rd is selected from $C_{1-6}$ alkyl;
- $R^1$ is alternatively $C_{6-10}$ alkyl or $C_{10-30}$ alkenyl;
- $Y_3$ is $C_{1-6}$ alkyl.

In some embodiments, the present disclosure relates to the compound of Formula V, V-1, or V-2, or a pharmaceutically acceptable salt, ester, stereoisomer, polymorph, solvate, N-oxide, isotope-labeled compound, metabolite, or prodrug thereof, and a mixture thereof, wherein:

- $U_1$, $U_2$, and $U_3$ are O;
- $W_1$, $W_2$, and $W_3$ are $CH_3$;
- $R^1$ is selected from $CH_3$,

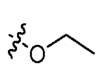, 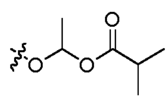,

$C_7$ alkyl, $C_8$ alkyl, $C_9$ alkyl, $C_{10}$ alkyl, $C_{11}$ alkyl, $C_{12}$ alkyl, $C_{13}$ alkyl, $C_{15}$ alkyl, $C_{16}$ alkyl, $C_{17}$ alkyl, $C_{19}$ alkyl, $C_{21}$ alkyl, $C_{23}$ alkyl, $C_{25}$ alkyl,

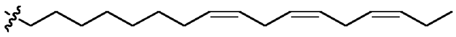, 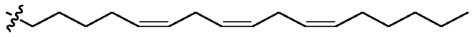,

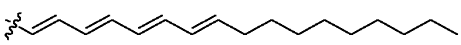, 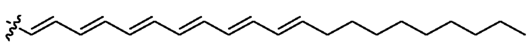,

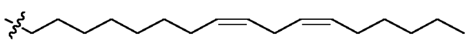, 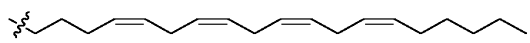,

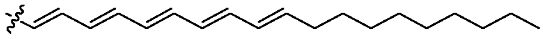,
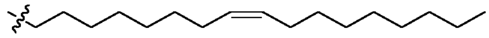,

and

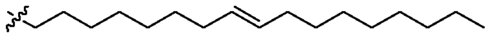.

In some embodiments, the present disclosure relates to the compound of Formula VIa, VIa-1, VIa-2, VIb, VIb-1, VIb-2, VIc, VIc-1, or VIc-2, or a pharmaceutically acceptable salt, ester, stereoisomer, polymorph, solvate, N-oxide, isotope-labeled compound, metabolite, or prodrug thereof, and a mixture thereof:

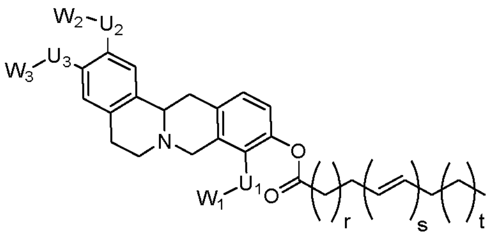
VIa

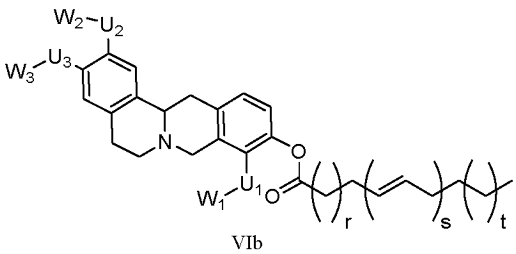
VIb

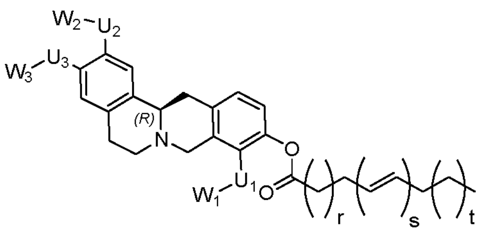
VIa-1

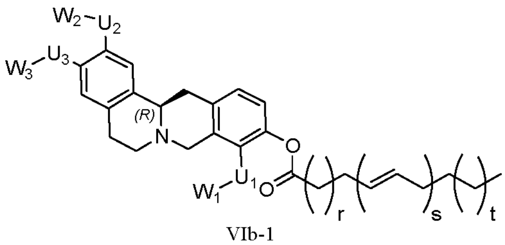
VIb-1

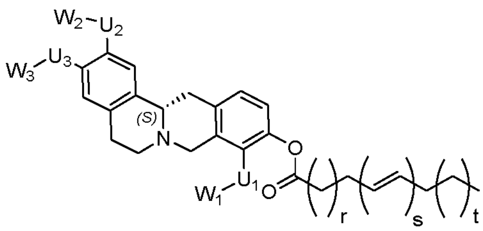
VIa-2

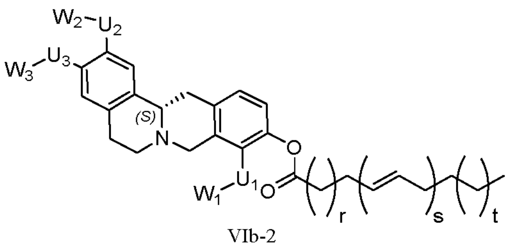
VIb-2

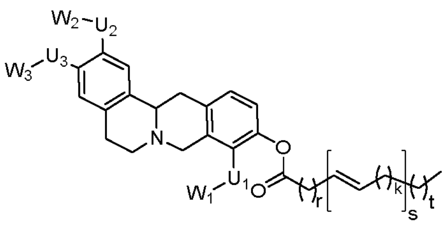
VIc

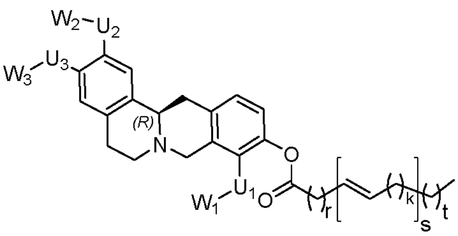
VIc-1

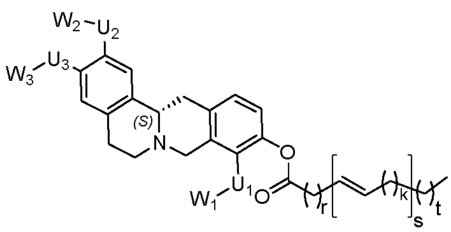
VIc-2

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
*r* and *t* are selected from 0, 1, 2, 3, 4, 5, 6, 7, 8, 9 or 10;
*s* is selected from 0, 1, 2, 3, 4, 5, 6, 7, 8 or 9;
*k* is selected from 0, 1, 2, 3, 4 or 5.

In some embodiments, the present disclosure relates to the compound of Formula VIa, VIa-1, VIa-2, VIb, VIb-1, VIb-2, VIc, VIc-1, or VIc-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are selected from -O- or -OC(O)-;
$W_1$, $W_2$, and $W_3$ are independently selected from $C_{1-6}$ alkyl;
*r* and *t* are selected from 0, 1, 2, 3, 4, 5, 6, 7, 8, 9 or 10;
*s* is selected from 0, 1, 2, 3, 4, 5, 6, 7, 8 or 9;
*k* is selected from 0, 1, 2, or 3.

In some embodiments, the present disclosure relates to the compound of Formula VIa, VIa-1, VIa-2, VIb, VIb-1, VIb-2, VIc, VIc-1, or VIc-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are O;
$W_1$, $W_2$, and $W_3$ are $CH_3$;
*r is* 0, 2, 3, 6, or 7;
*s* is 1, 2, 4, 5, or 6;
*t* is 0, 2, 5, 6, or 8;
*k* is 0 or 1.

In some embodiments, the present disclosure relates to the compound of Formula VII, VII-1, VII-2, XIV, XIV-1, or XIV-2, or a pharmaceutically acceptable salt, ester, stereoisomer, polymorph, solvate, N-oxide, isotope-labeled compound, metabolite, or prodrug thereof, and a mixture thereof:

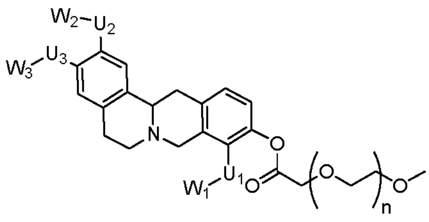
VII

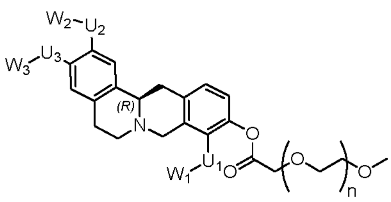
VII-1

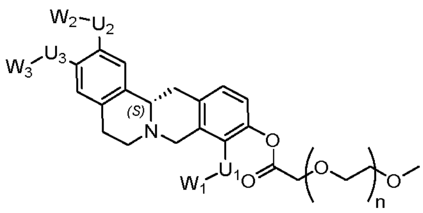
VII-2

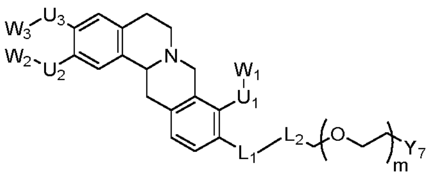
XIV

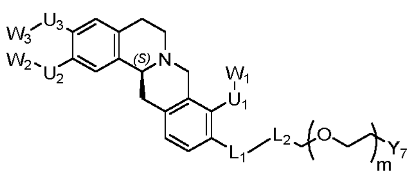
XIV-1

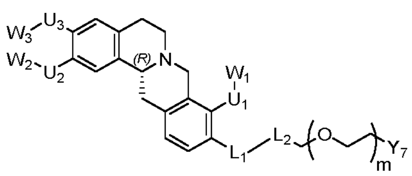
XIV-2

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
*m* and *n* are selected from 2, 3, 4, 5, 6, 7, 8, 9, 10, 11, 12, 13, 14 or 15, alternatively 6, 7, 8, 9 or 10, yet alternatively 7, 8 or 9;
$L_1$ is a chemical bond, -O- or -NH-;
$L_2$ is -C(O)-, -C(O)O-, -C(O)-$C_{1-18}$ alkylene- or -O-$C_{1-18}$ alkylene-;

$Y_7$ is $C_{1-12}$ alkoxy.

In some embodiments, the present disclosure relates to the compound of Formula VII, VII-1, VII-2, XIV, XIV-1, or XIV-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are selected from -O- or -OC(O)-;
$W_1$, $W_2$, and $W_3$ are independently selected from $C_{1-6}$ alkyl;
*m* and *n* are selected from 2, 3, 4, 5, 6, 7, 8, 9 or 10, alternatively 7, 8 or 9;
$L_1$ is -O- or -NH-, alternatively -O-;
$L_2$ is -C(O)- or -C(O)-$C_{1-6}$ alkylene-;
$Y_7$ is $C_{1-3}$ alkoxy.

In some embodiments, the present disclosure relates to the compound of Formula VII, VII-1, VII-2, XIV, XIV-1, or XIV-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are O;
$W_1$, $W_2$, and $W_3$ are $CH_3$;
*m* and *n* are 3, 4, 5, 6, 7, 8, alternatively 3 or 8;
$L_1$ is O;
$L_2$ is -C(O)- or -C(O)-$C_{1-4}$ alkylene-, alternatively -C(O)-;
$Y_7$ is $C_{1-3}$ alkoxy, alternatively $OCH_3$.

In some embodiments, the present disclosure relates to the compound of Formula VIII, VIII-1, or VIII-2, or a pharmaceutically acceptable salt, ester, stereoisomer, polymorph, solvate, N-oxide, isotope-labeled compound, metabolite, or prodrug thereof, and a mixture thereof:

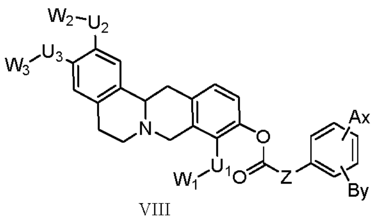
VIII

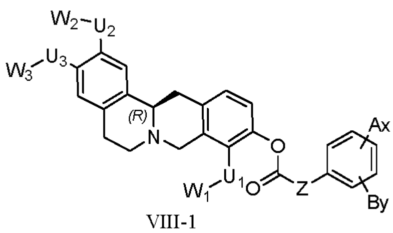
VIII-1

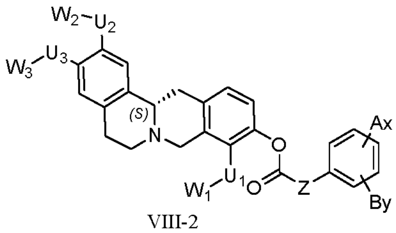
VIII-2

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
Z is selected from $C_{1-30}$ alkylene or $C_{2-30}$ alkenylene and the Z is optionally substituted with 1, 2, or 3 $R^3$;
$R^3$ is $C_{1-6}$ alkyl;
A is selected from halogen, OH, $C_{1-18}$ alkyl or $C_{6-30}$ aryl which are optionally substituted with Rc, alternatively OH, $C_{1-18}$ alkyl or $C_{6-30}$ aryl which are optionally substituted with Rc, yet alternatively $C_{6-30}$ aryl;
B is selected from halogen, OH or $C_{6-30}$ aryl, alternatively halogen or OH, yet alternatively halogen;
Rc is selected from $C_{3-10}$ cycloalkyl optionally substituted with OH, oxo, or thioxo, alternatively $C_{3-10}$ cycloalkyl substituted with oxo;
*x is* 0, 1 or 2; and
*y* is 0, 1, 2, or 3.

In some embodiments, the present disclosure relates to the compound of Formula VIII, VIII-1, or VIII-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are selected from -O- or -OC(O)-;
$W_1$, $W_2$, and $W_3$ are independently selected from $C_{1-6}$ alkyl;
Z is selected from $C_{1-6}$ alkylene or $C_{2-6}$ alkenylene, alternatively $C_{1-3}$ alkylene; and the Z is optionally substituted with

1, 2, or 3 $R^3$;
$R^3$ is $C_{1-6}$ alkyl;
Ais selected from halogen, OH, $C_{1-6}$ alkyl or $C_{6-12}$ aryl which are optionally substituted with Rc, alternatively OH, $C_{1-6}$ alkyl or $C_{6-12}$ aryl which are optionally substituted with Rc, yet alternatively $C_{6-12}$ aryl;
B is selected from halogen, OH or $C_{6-12}$ aryl, alternatively halogen or OH, yet alternatively halogen;
Rc is selected from $C_{3-10}$ cycloalkyl optionally substituted with OH or oxo;
*x is* 0, 1 or 2; and
*y* is 0, 1, 2, or 3.

In some embodiments, the present disclosure relates to the compound of Formula VIII, VIII-1, or VIII-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are O;
$W_1$, $W_2$, and $W_3$ are $CH_3$;
Z is selected from $-CH(CH_3)-$, $-CH=CH-$ or $-CH_2-CH_2-$;
A is selected from F, OH, phenyl,

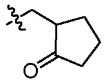 , or 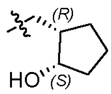 ,

alternatively OH, phenyl, or

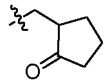 ,

yet alternatively phenyl;
B is selected from F, OH or phenyl, alternatively F or OH, yet alternatively F;
*x* and *y* are 0, 1 or 2.

In some embodiments, the present disclosure relates to the compound of Formula IX, IX-1, or IX-2, or a pharmaceutically acceptable salt, ester, stereoisomer, polymorph, solvate, N-oxide, isotope-labeled compound, metabolite, or prodrug thereof, and a mixture thereof:

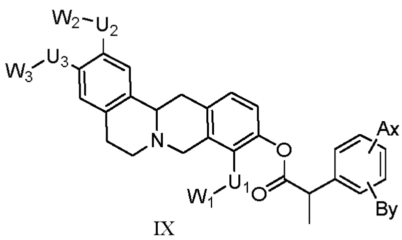
IX

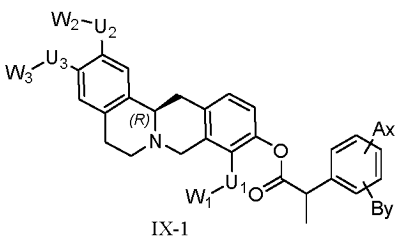
IX-1

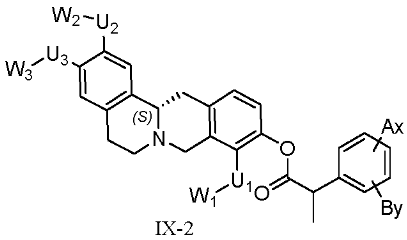
IX-2

wherein

$U_1$, $U_2$, and $U_3$ are independently selected from -O-, -O-C(O)-, -NH-C(O)- or $-O-CH_2-O-$;
$W_1$, $W_2$, and $W_3$ are independently selected from $C_{1-12}$ alkyl;
A is selected from H or Re-substituted $C_{1-18}$ alkyl;
B is selected from halogen or $C_{6-30}$ aryl, alternatively halogen or $C_{6-12}$ aryl;
Rc is selected from $C_{3-10}$ cycloalkyl optionally substituted with OH, oxo, or thioxo, alternatively $C_{3-10}$ cycloalkyl substituted with oxo;
*x is* 0, 1 or 2; and
*y* is 0, 1, 2, or 3.

In some embodiments, the present disclosure relates to the compound of Formula IX, IX-1, or IX-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are selected from -O- or -OC(O)-;
$W_1$, $W_2$, and $W_3$ are independently selected from $C_{1-6}$ alkyl;
A is selected from H or Re-substituted $C_{1-6}$ alkyl, alternatively H;
B is selected from halogen or $C_{6-10}$ aryl, alternatively F or phenyl;
Rc is selected from $C_{3-10}$ cycloalkyl substituted with oxo or OH, alternatively $C_{3-10}$ cycloalkyl substituted with oxo;
*x is* 0, 1 or 2; and
*y* is 0, 1, 2, or 3.

In some embodiments, the present disclosure relates to the compound of Formula IX, IX-1, or IX-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are -O-;
$W_1$, $W_2$, and $W_3$ are $CH_3$;
A is selected from

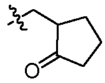 or 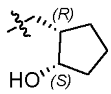 ;

B is selected from F or phenyl;
*x is 0 or* 1;
*y is 0 or 1.*

In some embodiments, the present disclosure relates to the compound of Formula X, X-1, X-2, X-3 or X-4, or a pharmaceutically acceptable salt, ester, stereoisomer, polymorph, solvate, N-oxide, isotope-labeled compound, metabolite, or prodrug thereof, and a mixture thereof:

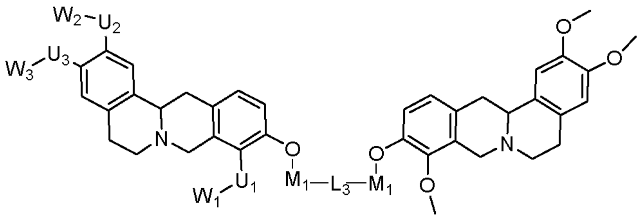

X

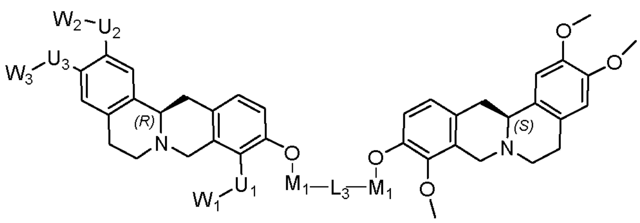

X-1

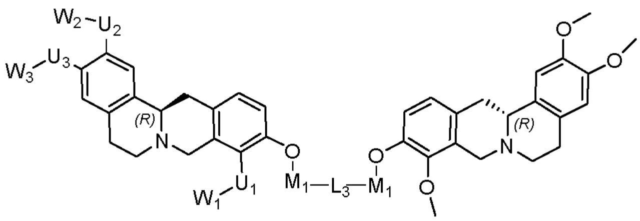

X-2

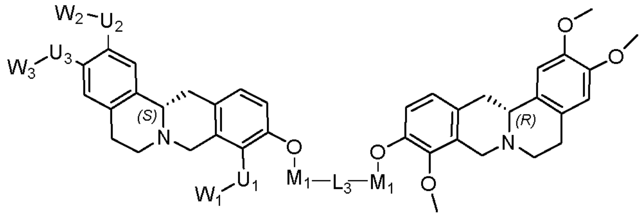

X-3

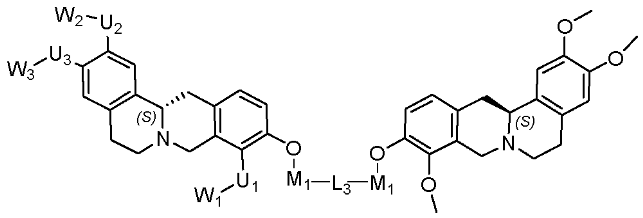

X-4

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
each of $M_1$ is each independently a chemical bond or -C(O)-, alternatively, two $M_1$ are not both chemical bonds;
$L_3$ is a chemical bond or -$C_{1-18}$- alkylene-, alternatively a chemical bond.

In some embodiments, the present disclosure relates to the compound of Formula X, X-1, X-2, X-3 or X-4, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are selected from -O- or -OC(O)-;
$W_1$, $W_2$, and $W_3$ are independently selected from $C_{1-6}$ alkyl;
each of $M_1$ is each independently a chemical bond or -C(O)-, alternatively, two $M_1$ are not both chemical bonds;
$L_3$ is selected from a chemical bond or -$C_{1-12}$- alkylene-, alternatively a chemical bond.

In some embodiments, the present disclosure relates to the compound of Formula X, X-1, X-2, X-3 or X-4, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are -O-;
$W_1$, $W_2$, and $W_3$ are $CH_3$;
each of $M_1$ is each independently a chemical bond or -C(O)-, alternatively, two $M_1$ are not both chemical bonds;
$L_3$ is selected from a chemical bond or -$C_{1-6}$- alkylene-, e.g., a chemical bond, -$CH_2$-, -$CH_2CH_2CH_2$- or -$CH_2CH_2CH_2CH_2CH_2CH_2$-, alternatively a chemical bond.

In some embodiments, the present disclosure relates to the compound of Formula XI, XI-1, XI-2, XI-3 or XI-4, or a pharmaceutically acceptable salt, ester, stereoisomer, polymorph, solvate, N-oxide, isotope-labeled compound, metabolite, or prodrug thereof, and a mixture thereof:

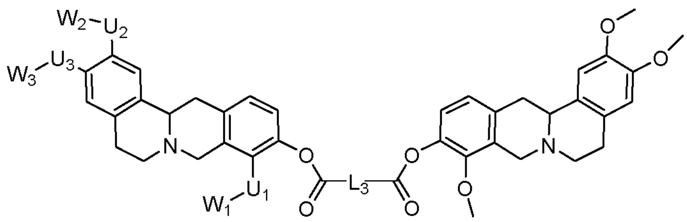

XI

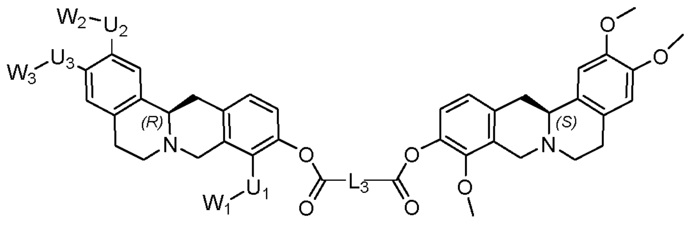

XI-1

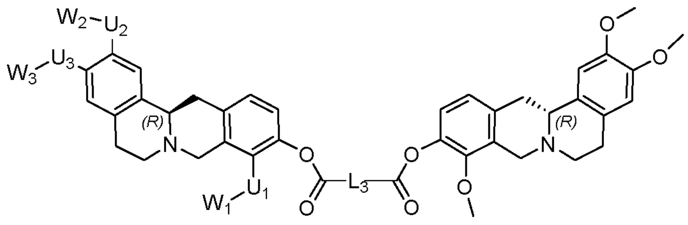

XI-2

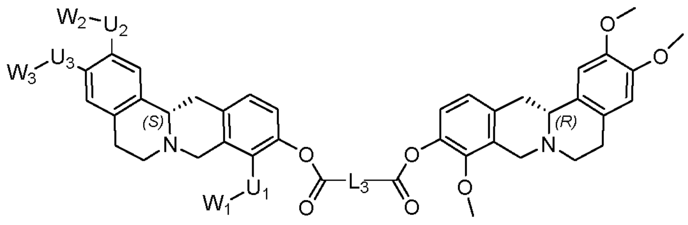

XI-3

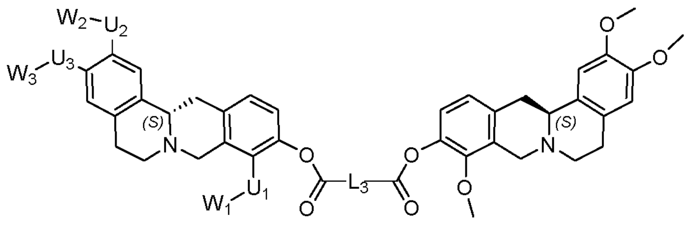

XI-4

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
$L_3$ is -$C_{1-18}$- alkylene-.

In some embodiments, the present disclosure relates to the compound of Formula XI, XI-1, XI-2, XI-3 or XI-4, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are selected from -O- or -OC(O)-;
$W_1$, $W_2$, and $W_3$ are independently selected from $C_{1-6}$ alkyl;
$L_3$ is -$C_{1-12}$- alkylene-.

In some embodiments, the present disclosure relates to the compound of Formula XI, XI-1, XI-2, XI-3 or XI-4, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are O;
$W_1$, $W_2$, and $W_3$ are $CH_3$;

$L_3$ is -$C_{1-6}$- alkylene-, e.g., -$CH_2CH_2CH_2$- or -$CH_2CH_2CH_2CH_2CH_2CH_2$-.

In some embodiments, the present disclosure relates to the compound of Formula XII, XII-1, or XII-2, or a pharmaceutically acceptable salt, ester, stereoisomer, polymorph, solvate, N-oxide, isotope-labeled compound, metabolite, or prodrug thereof, and a mixture thereof:

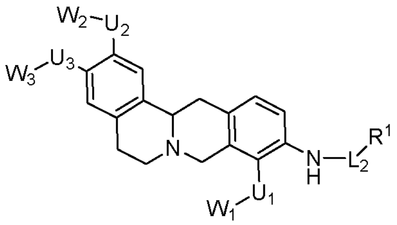
XII

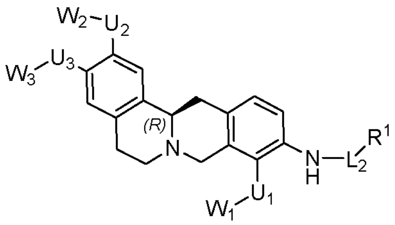
XII-1

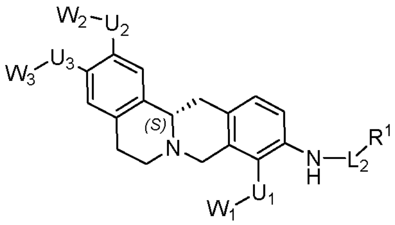
XII-2

wherein

$U_1$, $U_2$, and $U_3$ are independently selected from -O-, -O-C(O)-, -NH-C(O)- or -O-$CH_2$-O-;
$W_1$, $W_2$, and $W_3$ are $C_{1-12}$ alkyl;
$L_2$ is a chemical bond, -C(O)- or -C(O)O-;
$R^1$ is selected from H, OH, or -$Y_5$-$Y_6$-$Y_7$
$Y_5$ is unsubstituted or $NH_2$-substituted $C_{1-12}$ alkylene;
$Y_6$ is a single bond or -NH-C(NH)-;
$Y_7$ is $NH_2$, OH or $C_{1-12}$ alkoxy.

In some embodiments, the present disclosure relates to the compound of Formula XII, XII-1, or XII-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are selected from -O- or -OC(O)-;
$W_1$, $W_2$, and $W_3$ are $C_{1-6}$ alkyl;
$L_2$ is a chemical bond or -C(O)-;
$R^1$ is selected from H, OH, or -$Y_5$-$Y_6$-$Y_7$;
$Y_5$ is unsubstituted or $NH_2$-substituted $C_{3-6}$ alkylene;
$Y_6$ is a single bond or -NH-C(NH)-;
$Y_7$ is $NH_2$ or OH.

In some embodiments, the present disclosure relates to the compound of Formula XII, XII-1, or XII-2, or a pharmaceutically acceptable salt, ester, stereoisomer, polymorph, solvate, N-oxide, isotope-labeled compound, metabolite, or prodrug thereof, and a mixture thereof, wherein:

$U_1$, $U_2$, and $U_3$ are O;
$W_1$, $W_2$, and $W_3$ are $CH_3$;
$L_2$ is a chemical bond or -C(O)-;
$R^1$ is selected from H,

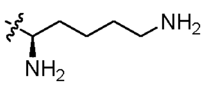 , 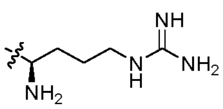 , 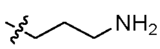 , or 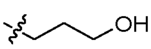 .

In some embodiments, the present disclosure relates to the compound of Formula XIII, XIII-1, or XIII-2, or a pharmaceutically acceptable salt, ester, stereoisomer, polymorph, solvate, N-oxide, isotope-labeled compound, metabolite, or prodrug thereof, and a mixture thereof:

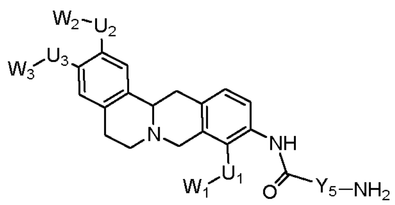
XIII

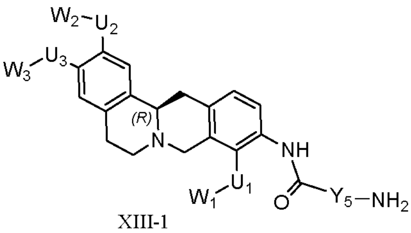
XIII-1

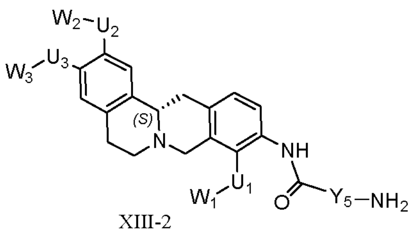
XIII-2

wherein
various variables are as defined above.

In some embodiments of the present disclosure, the compound is selected from the following compounds:

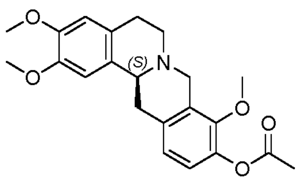
A1

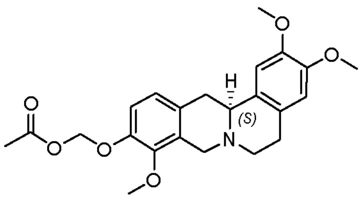
A2

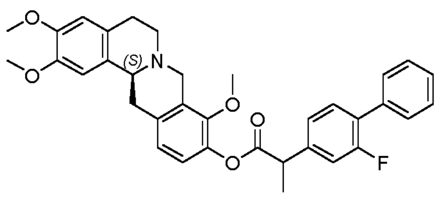
A3

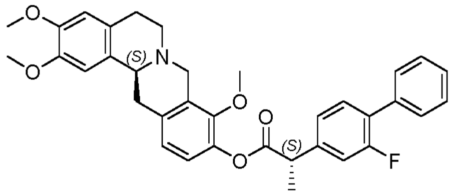
A4

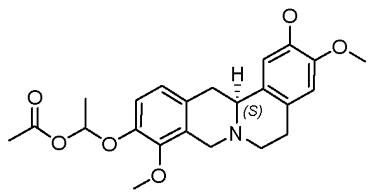
A5

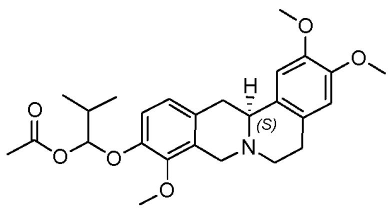
A6

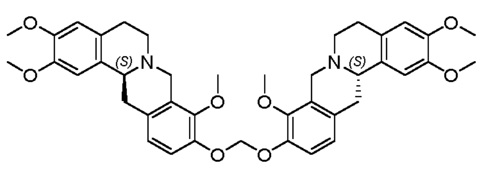
A7

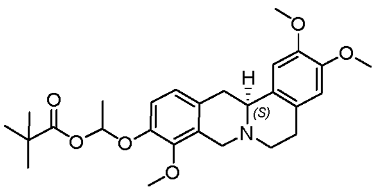
A8

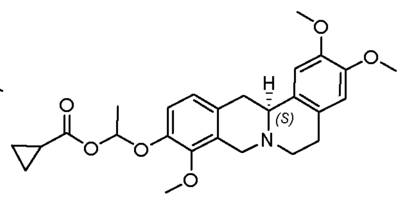
A9

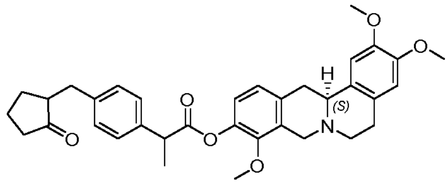
A10

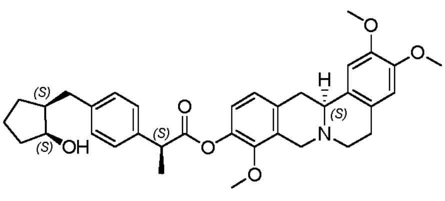
A11

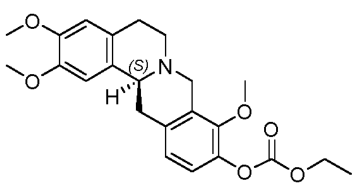
A12

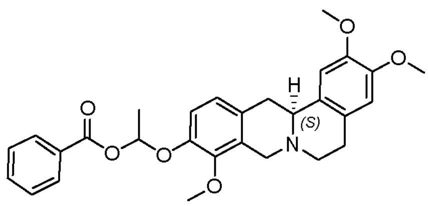
A13

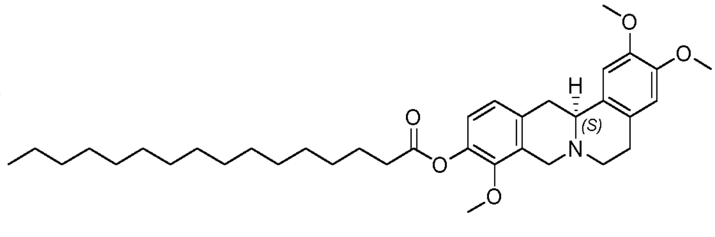
A14

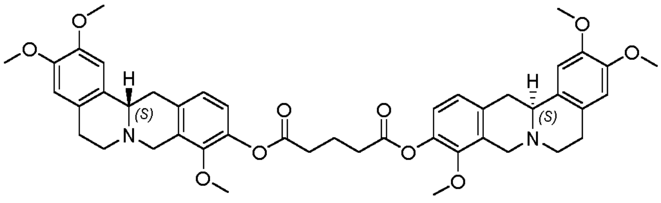

A15

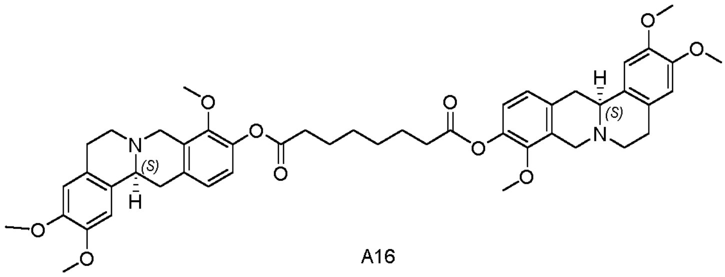

A16

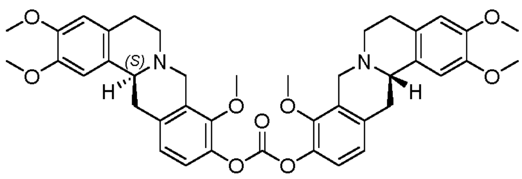

A17

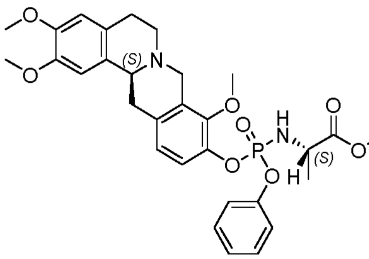

A18

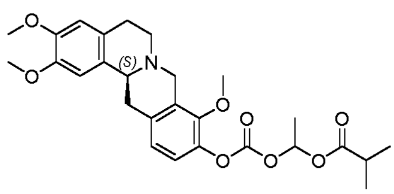

A19

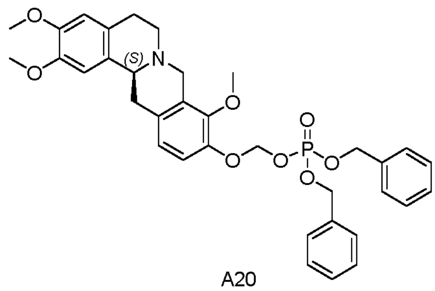

A20

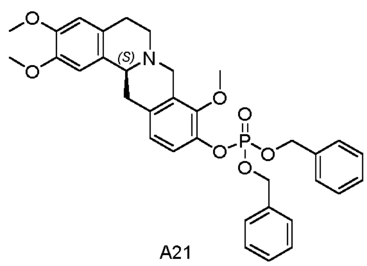

A21

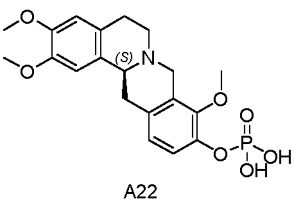

A22

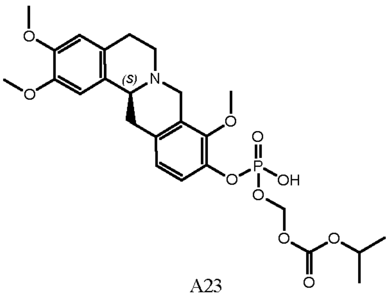

A23

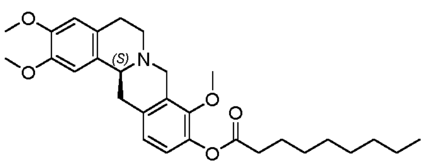

A24

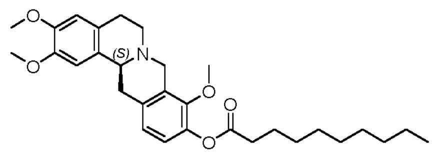

A25

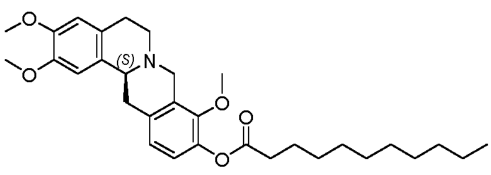

A26

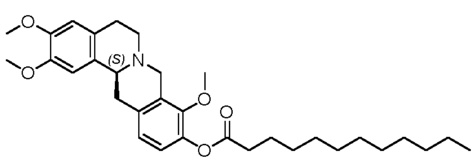

A27

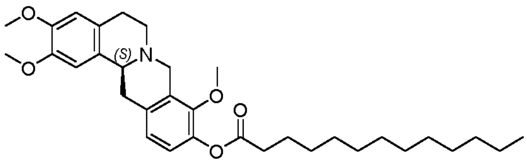
A28

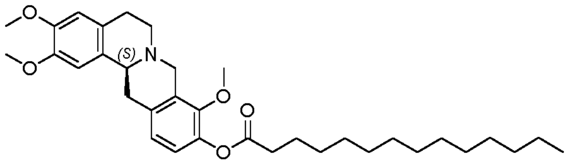
A29

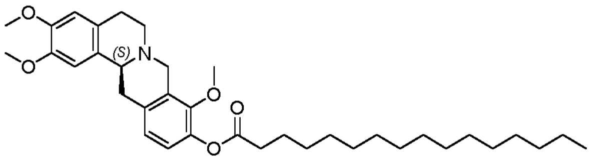
A30

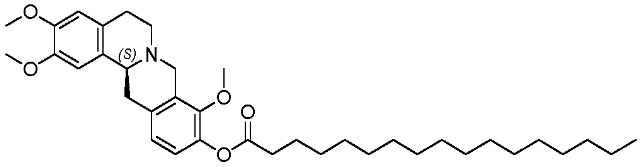
A31

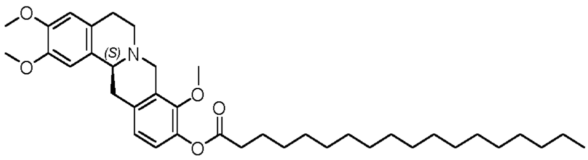
A32

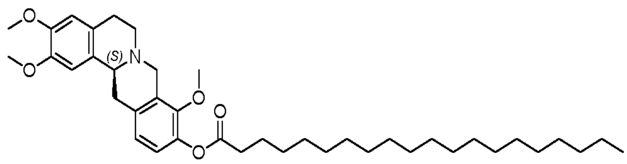
A33

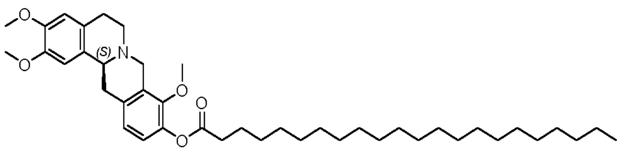
A34

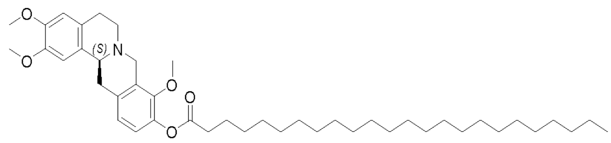
A35

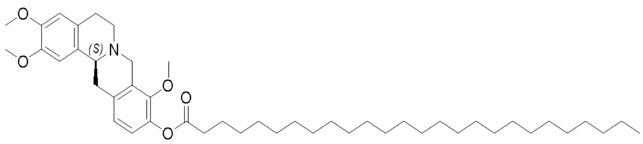
A36

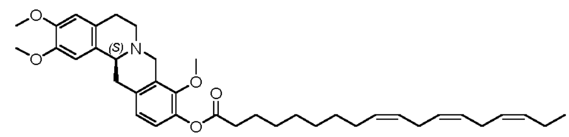
A37

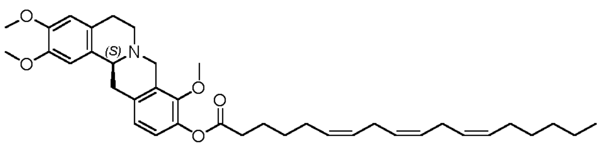
A38

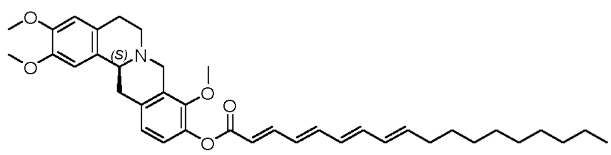
A39

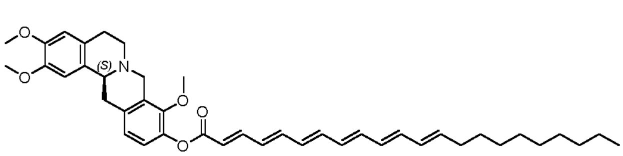
A40

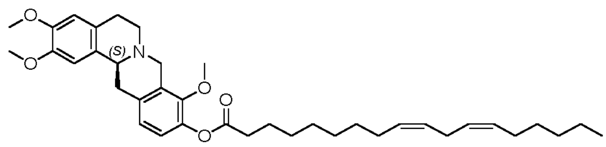
A41

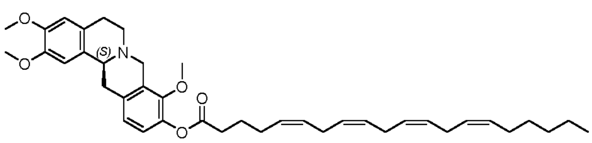
A42

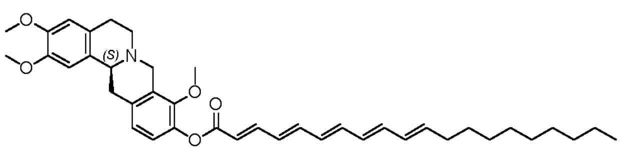
A43

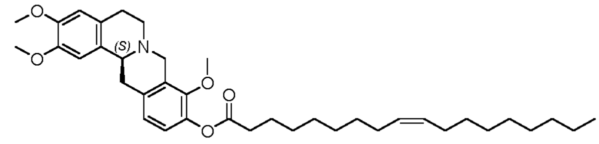

A44

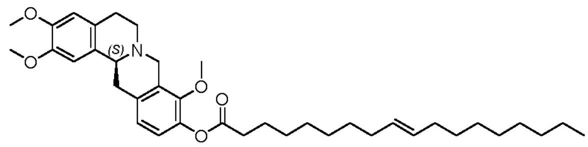

A45

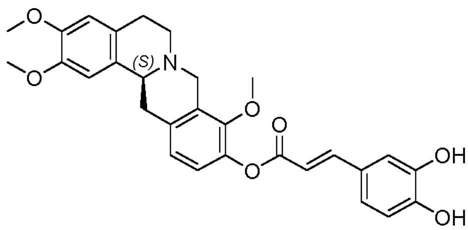

A46

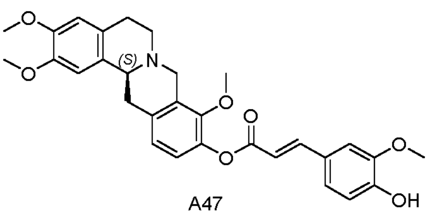

A47

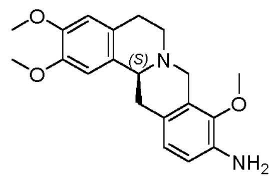

A48

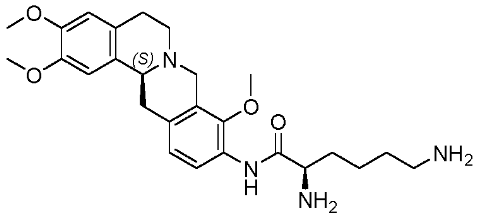

A49

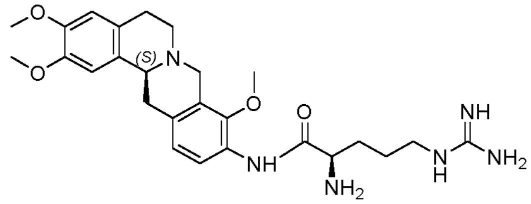

A50

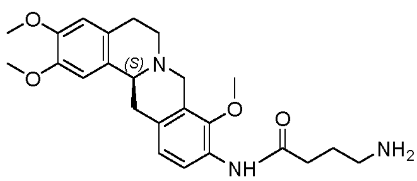

A51

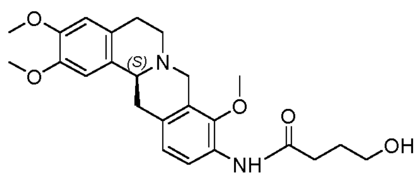

A52

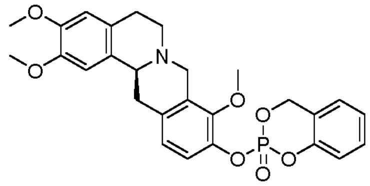

A53

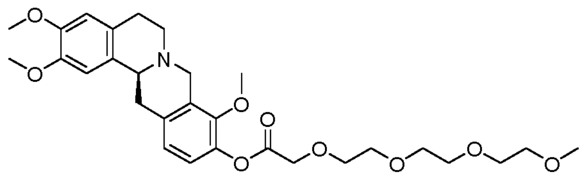

A54

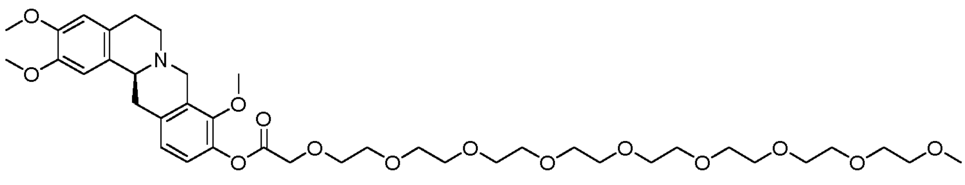

A55

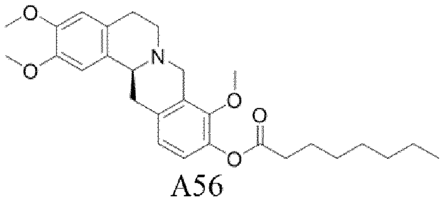

A56

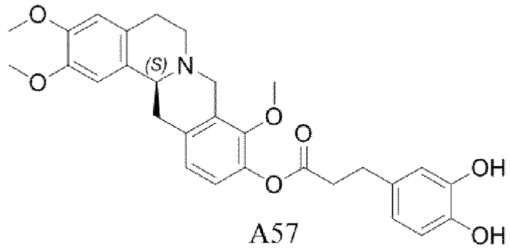

A57

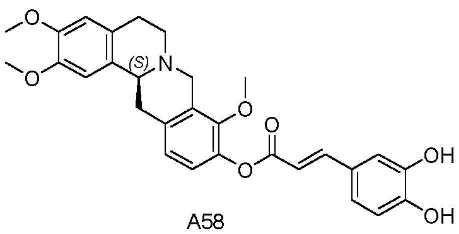

A58

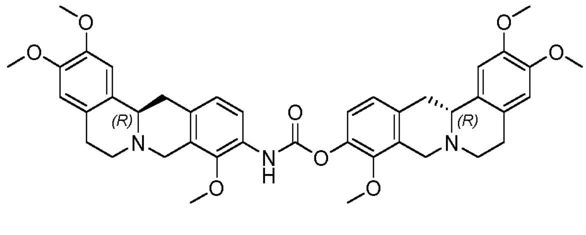

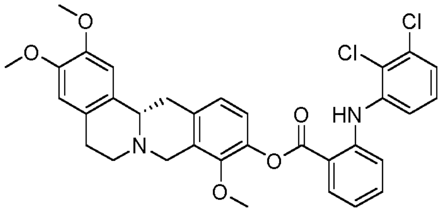

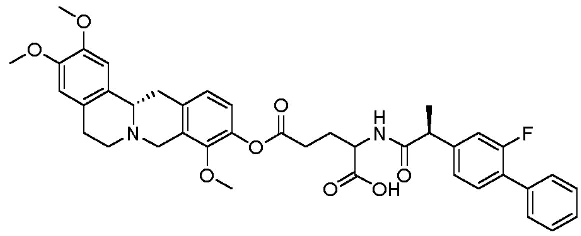

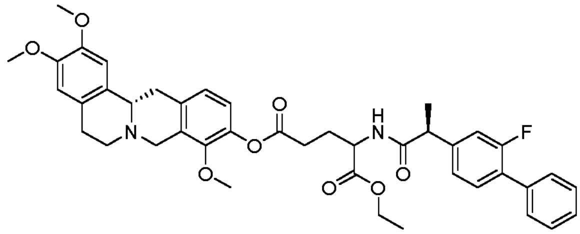

.

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

The compounds of the present disclosure may exist in tautomeric forms. Tautomers are functional group isomers produced by the rapid movement of an atom in a molecule between two positions. The tautomers are special functional group isomers. A pair of tautomers can be converted into each other, but usually a relatively stable isomer is the main form of existence. The most important examples are the enol and keto tautomers.

It will be understood by those skilled in the art that the organic compounds can form complexes with solvents in which they are reacted or from which they are precipitated or crystallized. These complexes are known as "solvates." When the solvent is water, the complex is known as "hydrate." The present disclosure encompasses all solvates of the compounds of the present disclosure.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates

and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula R·x $H_2O$, wherein R is the compound, and x is a number greater than 0. Given compounds can form more than one type of hydrates, including, for example, monohydrates (x is 1), lower hydrates (x is a number greater than 0 and smaller than 1, for example, hemihydrates (R·0.5 $H_2O$)) and polyhydrates (x is a number greater than 1, for example, dihydrates (R·2 $H_2O$) and hexahydrates (R·6 $H_2O$)).

Compounds of the present disclosure may be in an amorphous or a crystalline form (polymorph). Furthermore, the compounds of the present disclosure may exist in one or more crystalline forms. Therefore, the present disclosure includes all amorphous or crystalline forms of the compounds of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes (isotopic variants), which are equivalent to those described in formula (A) or (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain isotope-labeled compounds of the present disclosure, such as those incorporating radioactive isotopes (e.g., $^{3}H$ and $^{14}C$), can be used for the measurement of the distribution of drug and/or substrate in tissue. Tritium, which is $^{3}H$ and carbon-14, which is $^{14}C$ isotope, are yet alternative, because they are easy to prepare and detect. Furthermore, replaced by heavier isotopes, such as deuterium, which is $^{2}H$, may provide therapeutic benefits due to the higher metabolic stability, such as prolonging the half-life in vivo or decreasing the dosage requirements, and thus may be alternative in some cases. Isotope-labeled compounds of formula (A) or (I) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted into an active form that has medical effects in vivo by, for example, hydrolysis in blood. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which is incorporated herein by reference.

The prodrugs are any covalently bonded compounds of the present disclosure, which release the parent compound in vivo when the prodrug is administered to a patient. Prodrugs are typically prepared by modifying functional groups in such a way that the modifications can be cleaved either by routine manipulation or decompose in vivo to yield the parent compound. Prodrugs include, for example, compounds of the present disclosure wherein the hydroxyl, amino or sulfhydryl groups are bonded to any group that, when administered to a patient, cleaves to form the hydroxyl, amino or sulfhydryl groups. Thus, representative examples of prodrugs include (but are not limited to) the acetate/acetamide, formate/formamide and benzoate/benzamide derivatives of the hydroxyl, sulfhydryl or amino functional groups of the compounds of formula (A) or (I). Furthermore, in the case of carboxylic acid (-COOH), esters such as methyl esters and ethyl esters, etc. can be employed. The ester itself may be active in their own and/or hydrolyzable under in vivo conditions in the human body. Suitable pharmaceutically acceptable in vivo hydrolysable ester groups include those groups that can readily break down in the human body to release the parent acids or salts thereof.

The present disclosure also provides a pharmaceutical formulation comprising a therapeutically effective amount of a compound of formula (A) or (I), or therapeutically acceptable salts thereof, and pharmaceutically acceptable carriers, diluents or excipients thereof. All of these forms belong to the present disclosure.

In a second aspect, the present disclosure provides a stereoisomer, pharmaceutically acceptable salt, ester, optical isomer, polymorph, solvate, N-oxide, isotope-labeled compound, metabolite, chelate, complex, inclusion, or prodrug of the compound described in the first aspect of the present disclosure.

In a third aspect, the present disclosure provides a composition including the compound described in the first aspect of the present disclosure and/or the stereoisomer, pharmaceutically acceptable salt, ester, optical isomer,

polymorph, solvate, N-oxide, isotope-labeled compound, metabolite, chelate, complex, inclusion, or prodrug described in the second aspect of the present disclosure.

The composition of the present disclosure may further include pharmaceutically acceptable carrier, adjuvant, excipient, etc.

Use of the compound of the present disclosure, and a stereoisomer, pharmaceutically acceptable salt, ester, optical isomer, polymorph, solvate, N-oxide, isotope-labeled compound, metabolite, chelate, complex, inclusion, or prodrug thereof, or the composition including the compound in the manufacture of a medicament for preventing and/or treating a CNS-associated disease.

Use of the compound or a stereoisomer, pharmaceutically acceptable salt, ester, optical isomer, polymorph, solvate, N-oxide, isotope-labeled compound, metabolite, chelate, complex, inclusion, or prodrug thereof, or the composition in the manufacture of a medicament for analgesia, anti-depression, or anti-addiction.

In some embodiments of the present disclosure, the drug is used for pains caused by postoperative trauma, postoperative incision, or organ metastasis of cancer.

In a fifth aspect, the present disclosure provides a method for analgesia, anti-depression, or anti-drug addiction including administering to a subject in need thereof an effective dose of the compound described in the first aspect of the present disclosure, the stereoisomer, pharmaceutically acceptable salt, ester, optical isomer, polymorph, solvate, N-oxide, isotope-labeled compound, metabolite, chelate, complex, inclusion, or prodrug described in the second aspect, or the composition described in the third aspect, wherein the administration is carried out by ways of oral, rectal, nasal, topical, or parenteral administration.

In some embodiments of the present disclosure, the effective dose is 0.1 mg/day to 1000 mg/day, alternatively 3 mg/day to 300 mg/day, yet alternatively 5 mg/day to 50 mg/day, based on the compound.

In a sixth aspect, the present disclosure provides the compound described in the first aspect of the present disclosure, the stereoisomer, pharmaceutically acceptable salt, ester, optical isomer, polymorph, solvate, N-oxide, isotope-labeled compound, metabolite, chelate, complex, inclusion, or prodrug described in the second aspect, or the composition described in the third aspect of the present disclosure for use as a drug or for use for therapy.

In the present disclosure, the compound of Formula (I), and a stereoisomer, pharmaceutically acceptable salt, ester, optical isomer, polymorph, solvate, N-oxide, isotope-labeled compound, metabolite, chelate, complex, inclusion, or prodrug thereof are obtained by modification(s) on different active sites of the s-corydalmine parent structure, may be used to prevent and/or treat a CNS-associated disease such as pain, drug addiction, and depression, have good analgesic, anti-depressive, and anti-addiction effects, and may be used in the manufacture of a medicament for analgesia, anti-depression, or anti-addiction.

Of course, it is not necessary for the implementation of any product or method of the present disclosure to achieve all the advantages as set forth above at the same time.

## BRIEF DESCRIPTION OF DRAWINGS

In order to illustrate the technical solutions of the present disclosure or the prior art more clearly, the drawings required in the examples and the prior art are briefly described below. Obviously, the drawings described below are only some examples of the present disclosure, and those of ordinary skill in the art can obtain other examples on the basis of these drawings without any creative effort.

Fig. 1 is a graph showing the results of mechanical pain threshold in mechanical pain test of inflammatory pain model induced by complete Freund's adjuvant in mice;

Fig. 2 is a graph showing the results of plantar thickness in mechanical pain test of inflammatory pain model induced by complete Freund's adjuvant in mice;

Fig. 3 is a graph showing the results of writhing start time of mice in various groups in the analgesic test of writhing model caused by acetic acid in mice; and

Fig. 4 is a graph showing the results of total writhing number of mice in various groups in the analgesic test of writhing model caused by acetic acid in mice.

## DETAILED DESCRIPTION

Hereinafter the technical solutions in the present disclosure are clearly and completely described with reference to the drawings in the present disclosure. Obviously, the described examples are only some of the examples instead of all the examples of the present disclosure. Based on the examples of the present disclosure, all other examples obtained by those of ordinary skill in the art based on the present disclosure are within the scope of the present disclosure.

Abbreviates:

PE: petroleum ether;
EA: ethyl acetate;
DMAP: 4-dimethylaminopyridine;
DCM: dichloromethane;
DCC: dicyclohexylcarbodiimide
EDCI, EDC: 1-ethyl-(3-dimethylaminopropyl)carbodiimide;
DIPEA, DIEA: N,N-diisopropylethylamine;
EtOH: ethanol;
Et3N: triethylamine;
ACN: acetonitrile
TBAB: tetrabutylammonium bromide;
NMP: N-methyl pyrrolidinone;
THF: tetrahydrofuran;
rt: room temperature
LCMS: liquid chromatography-mass spectrometry;
TLC: thin layer chromatography;
Chemical Formula: chemical formula;
Molecular Weight: relative molecular mass;
Exact Mass: exact mass.
Calculation of Yield:

$$\text{Yield} = \text{Actual Quality of Synthetic Product} / \text{Theoretical Quality of Synthetic Product} * 100\%.$$

Hereinafter the present disclosure is described in detail with reference to specific examples. The compound of the present disclosure is not particularly limited in terms of synthesis method, and may be synthesized by any method known to those skilled in the art.

Example 1: Synthesis of Compound A7

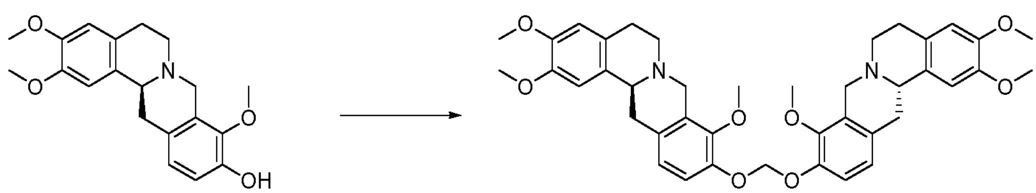

$C_{20}H_{23}NO_4$
Molecular Weight: 341.41
**A10-0**

$C_{41}H_{46}N_2O_8$
Molecular Weight: 694.83
**A7**

A10-0 (34 mg), NaOH (20 mg), and DCM (0.5 mL) were added into a reaction flask. Chloromethyl pivalate (45 mg) and DCM (0.5 mL) were added into a separate vial, mixed well, and then slowly added dropwise into the reaction flask at room temperature. After addition, the reaction mixture was stirred at room temperature overnight, and monitored by LCMS. The peak at 695 nm was the main peak. The reaction mixture was separated by TLC with the developing agent of PE/EA (3/1, v/v) to give a light-yellow oily matter, which was freeze-dried to give A7 as a light-yellow solid (24 mg).

$^1$H-NMR(400MHz, CDCl-$d_3$) $\delta$ 7.103(d, $J$ = 8.4 Hz, 2H), 6.879(d, $J$ = 8.4 Hz, 2H), 6.723(s, 2H), 6.625(s, 2H), 5.755(d, 2H), 4.265(d, $J$ = 16.0 Hz, 2H), 3.890(s, 6H), 3.873(s, 6H), 3.826(s, 6H), 3.707~3.575(m,4H), 3.314~3.231(m, 6H), 2.922~2.865(m, 2H), 2.709(d, $J$ = 10.4 Hz, 4H).

Example 2: Synthesis of Compound A10

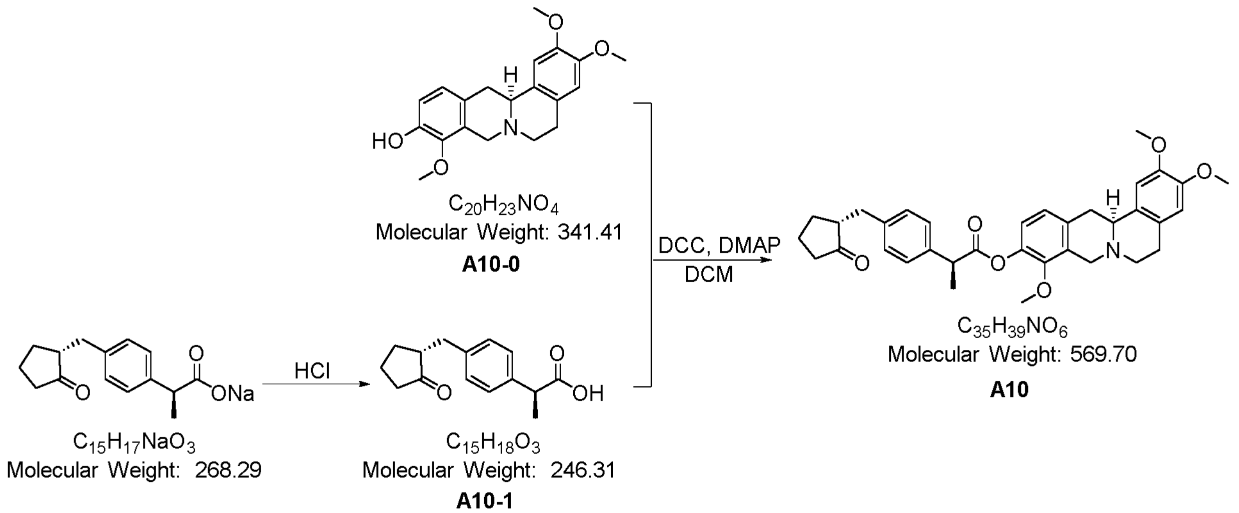


Step 1: Loxoprofen sodium (1.52 g) and water (5 mL) was added into a reaction flask, and adjusted to pH 2 by addition of 1N (i.e., 1 mol/L) hydrochloric acid dropwise under stirring. The reaction liquor was extracted with EA. The organic phase was dried, filtered, and concentrated to dryness under reduced pressure to give A10-1 as a colorless oily matter (1.3 g).

$^1$H-NMR(400MHz, DMSO-$d_6$) 12.234(s, 1H), 7.188(d, $J$ = 8.4 Hz, 2H), 7.128(d, $J$ = 8.0 Hz, 2H), 3.623(q, 7.2 Hz, 1H), 2.951(dd, $J_1$ = 13.2 Hz, $J_2$ = 3.6 Hz, 1H), 2.463~2.325(m, 2H), 2.270~2.202(m, 1H), 2.112~2.018(m, 1H), 1.966~1.815(m, 2H), 1.744~10624(m, 1H), 1.525~1.426(m, 1H), 1.336(d, $J$ = 7.2 Hz, 3H).

Step 2: A10-1 (360 mg of), A10-0 (500 mg), DMAP (18 mg), and ultradry DCM (44 mL) were added into a reaction flask, and DCC (604 mg) was added at room temperature under stirring. The reaction was stirred at room temperature overnight. The reaction liquor was washed three times with water, dried, filtered, and the mother liquor was concentrated to dryness under reduced pressure. The residue was purified by column chromatography to give a light-yellow solid. DCM (3 mL) was added, and the mixture was cooled in a refrigerator for 1h, filtered, and the filtrate was concentrated to dryness under reduced pressure to give A10 as a yellow solid (596 mg).

$^1$H-NMR(400MHz, DMSO-$d_6$) $\delta$ 5.332(d, $J$ = 8.0 Hz, 2H), 7.211(d, $J$ = 8.0 Hz, 2H), 6.933(d, $J$ = 8.0 Hz, 1H), 6.890~6.860(m, 2H), 6.681(s, 1H), 4.084(q, $J$ = 7.2 Hz, 1H), 4.009(dd, $J_1$ = 15.6 Hz, $J_2$ = 11.2 Hz, 1H), 3.743(s, 3H), 3.722(s, 3H), 3.455~3.370(m, 3H), 3.352(d, $J$ = 2.8 Hz, 3H), 3.104~3.086(m, 1H), 3.004~2.886(m, 2H), 2.632~3.542(m, 2H), 2.479~2.390(m, 3H), 2.247(dd, $J_1$ = 18.0 Hz, $J_2$ = 8.0 Hz, 1H), 2.117~2.024(m,1H), 1.954~1.833(m, 2H), 1.760~1.688(m, 1H), 1.536~1.466(m,4H).

Example 3: Synthesis of Compound A12

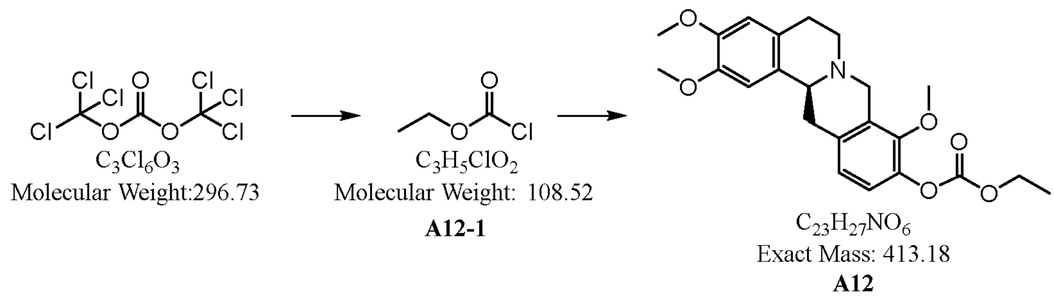


Step 1: Absolute ethanol (138 mg), pyridine (237 mg), and DCM (1 mL) were added into a reaction flask, and cooled in an ice-water bath. A solution of triphosgene (di(trichloromethyl) carbonate, 297 mg) in DCM (1 mL) was added dropwise into the reaction liquor. After addition, the mixture was stirred for additional 1.5h. The reaction liquor was diluted

with DCM. The organic phase was washed with water three times, dried, filtered, and the filtrate was concentrated to dryness under reduced pressure to give A12 as a colorless oily matter (scented).

Step 2: A10-0 (34 mg), triethylamine (20 mg), and DCM (1 mL) were added into a reaction flask, and cooled in an ice-water bath. A solution of A12-1 (12 mg) in DCM (1 mL) was added dropwise into the reaction flask. After addition, the mixture was stirred at room temperature. LCMS showed that the main peak was the target product. The reaction liquor was diluted with DCM, and purified by column chromatography to give a light-yellow oily matter (24 mg). The oily matter was dissolved in a small amount of DCM, and purified by TLC with the developing agent of DCM/EA (1/1, v/v) to give a light-yellow oily matter (17 mg), which was freeze-dried to give A12 as a light-yellow solid (15 mg).

$^1$H-NMR(400MHz, DMSO-$d_6$) 7.067(d, $J$ = 8.4 Hz, 1H), 6.967(d, $J$ = 8.4 Hz, 1H), 6.890(s, 1H), 6.692(s, 1H), 4.261(q, $J$ = 7.2 Hz, 2H), 4.073(d, $J$ = 16.0 Hz, 1H), 3.752(s, 3H), 3.737(s, 3H), 3.729(s, 3H), 3.489~3.429(m, 3H), 3.138~3.122(m, 1H), 2.931~2.902(m, 1H), 2.646~2.562(m, 2H), 1.296(t, $J$ = 7.2 Hz, 3H).

Example 4: Synthesis of Compound A14

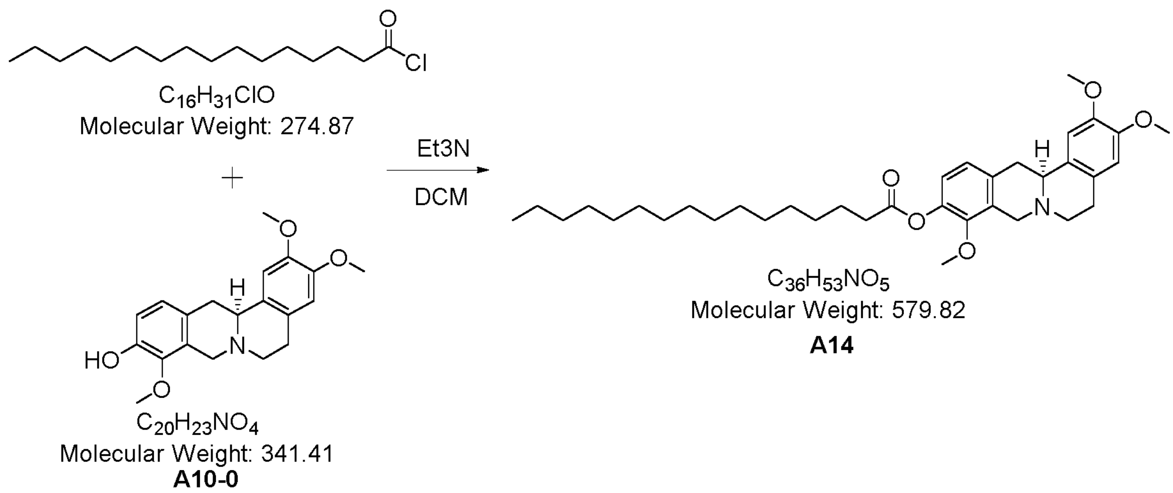


A10-0 (34 mg), DCM (1 mL), and triethylamine (20 mg) were added into a reaction flask, and a solution of palmitoyl chloride (27 mg) in DCM (1 mL) was added dropwise into the reaction flask. The reaction was detected by TLC until completion. The reaction liquor was purified by TLC with the developing agent of DCM/EA (1/1, v/v) to give a yellow solid, which was freeze-dried to give A14 (56 mg).

$^1$H-NMR(400MHz, CDCl-$d_3$) 6.949~6.897(m, 2H), 6.709(s, 1H), 6.630(s, 1H), 4.291(m, 1H), 3.894(s, 3H), 3.875(s, 3H), 3.814(s, 3H), 3.711~3.631(m, 2H), 3.350~3.280(m, 3H), 2.964(s, 1H), 2.733(s, 1H), 2.590(t, $J$ = 7.2 Hz, 2H), 1.814~1.739(m, 2H), 1.360~1.263(m, 24H), 0.881(t, J= 6.8 Hz, 3H).

Example 5: Synthesis of Compound A15

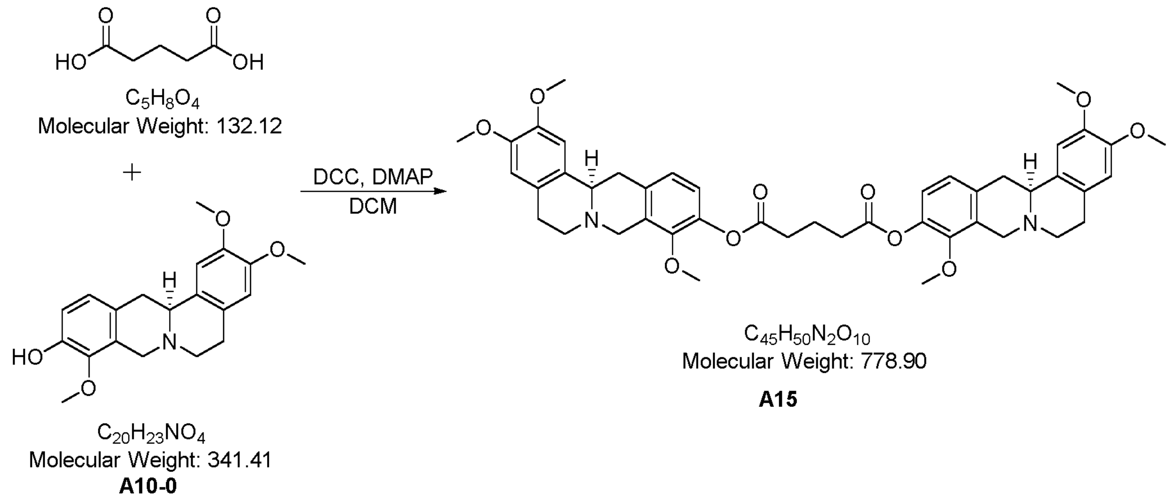


A10-0 (68 mg), glutaric acid (13 mg), DMAP (12 mg), and DCM (2 mL) were added into a reaction flask, and cooled in an ice-water bath. DCC (82 mg) was added into the reaction flask. After addition, the reaction mixture was stirred at room temperature overnight. The reaction liquor was filtered, and the filter cake was washed with DCM. The filtrate was purified by TLC to give a colorless oily matter, which was freeze-dried to give A15 as a light-yellow solid (61 mg).

$^1$H-NMR(400MHz, CDCl-$d_3$) 6.965~6.917(m, 4H), 6.715(s, 2H), 6.631(s, 2H), 4.284(d, $J$ = 14.8 Hz, 2H), 3.897(s, 6H), 3.877(s, 6H), 3.824(s, 6H), 3.694(s, 4H), 3.359~3.256(m, 6H), 2.956(s, 2H), 2.816~2.742(m, 8H), 2.280~2.208(m, 2H).

Example 6: Synthesis of Compound A16

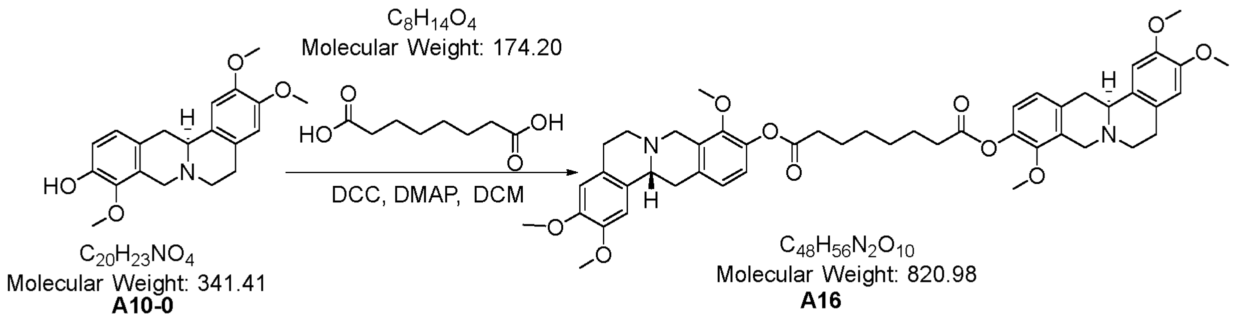


A10-0 (50 mg), suberic acid (12.7 mg), DMAP (2 mg), and DCM (2 mL) were added into a reaction flask, and cooled in an ice-water bath. DCC (60 mg) was added into the reaction flask. After addition, the reaction mixture was stirred at room temperature overnight. The reaction liquor was filtered, and the filtrate was purified by TLC with the developing agent as EA to give A16 as a while solid (42 mg).

$^1$H-NMR(400MHz, CDCl-$d_3$) $\delta$ 6.953~6.901(m, 4H), 6.710(s, 2H), 6.629(s, 2H), 4.272(d, $J$ = 14.0 Hz, 2H), 3.893(s, 6H), 3.875(s, 6H), 3.816(s, 6H), 3.724~3.633(m, 4H), 3.349~3.261(m, 6H), 2.964(s, 2H), 2.742(s, 4H), 2.621(t, $J$ = 7.2 Hz, 4H), 1.824(t, 6.8 Hz, 4H), 1.535~1.500(m, 4H).

Example 7: Synthesis of Compound A17

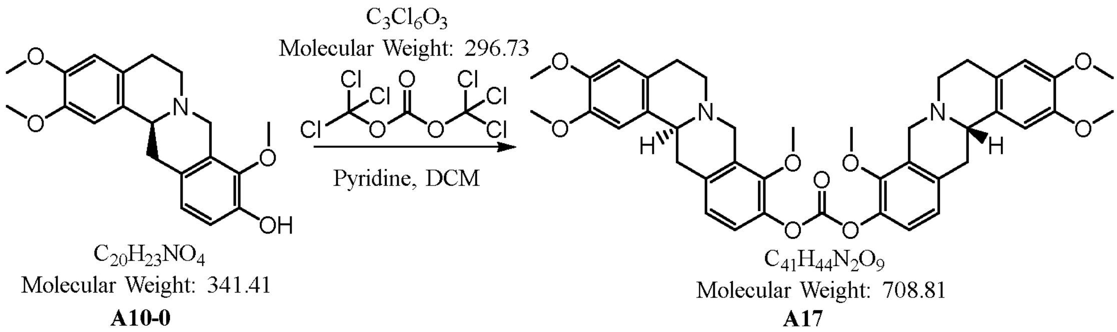


A10-0 (50 mg), triphosgene (7.2 mg), and DCM (1 mL) were added into a reaction flask, and cooled in an ice-water bath. A solution of pyridine (17 mg) in DCM (1 mL) was added dropwise into the flask. After addition, the reaction mixture was stirred at room temperature overnight. The reaction was detected by TLC until completion. The reaction mixture was filtered to remove insoluble matters, and the filtrate was purified by TLC to give a colorless oily matter, which was freeze-dried to give A17 as a solid (42 mg).

$^1$H-NMR(400MHz, CDCl-$d_3$) $\delta$ 7.117(d, $J$ = 8.4 Hz, 2H), 6.967(d, $J$ = 8.4 Hz, 2H), 6.711(s, 2H), 6.634(s, 2H), 4.306(s, 2H), 3.945(s, 6H), 3.893(s, 6H), 3.876(s, 6H), 3.761~3.636(m, 4H), 3.373~3.279(m, 6H), 2.987(s, 2H), 2.746(s, 4H).

Example 8: Synthesis of Compound A18

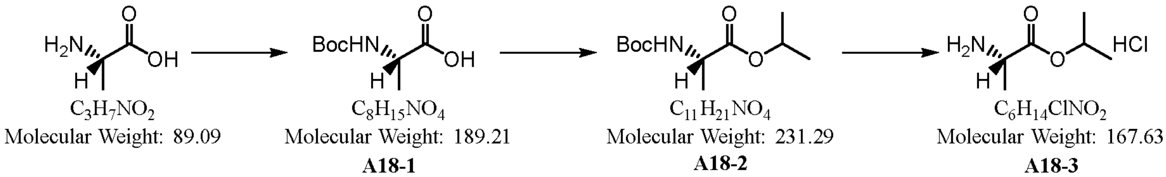


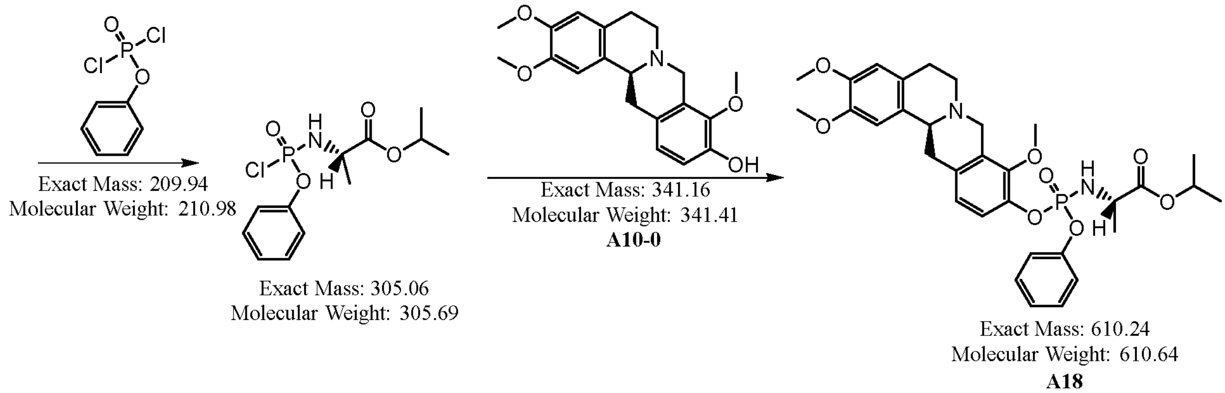


Step 1: L-alanine (5.0 g) and THF (25 mL) were added into a reaction flask. Sodium hydroxide (2.45 g) was added into a separate vial, and dissolved by addition of water (50 mL). After cooling, the sodium hydroxide solution was added into the reaction flask, and stirred at room temperature. $(Boc)_2O$ (13.5 g) was added dropwise into the flask. After addition, the reaction mixture was stirred at room temperature overnight, and the obtained reaction liquor was concentrated under reduced pressure to remove THF. The residue was adjusted to pH 3 with 1N HCl, and the aqueous phase was extracted with EA three times. The combined organic phase was dried, filtered, and concentrated to dryness under reduced pressure to give A18-1 as a colorless oily matter (3.4 g).

1H-NMR(400MHz, CDCl-d3) $\delta$ 8.819(s, 1H), 5.075(s, 1H), 4.342(s, 1H), 1.528(s, 1.454(s, 9H).

Step 2: A18-1 (3.4 g), isopropanol (1.18 g), DMAP (0.22 g) were added into DCM (34 mL), and cooled in an ice-water bath. EDCI (4.13 g) was added, and then the ice-water bath was removed. The reaction mixture was stirred at room temperature overnight. TLC detected that no raw material remained. The reaction liquor was diluted with DCM. The mixture was washed with water three times. The organic phase was dried over anhydrous sodium sulfate, filtered, concentrated, and purified by column chromatography using EA/PE (1/1, v/v) to give A18-2 as a colorless oily matter (2.1

g).

$^1$H-NMR(400MHz, CDCl-$d_3$) $\delta$ 5.090~4.997(m, 2H), 4.250(s, 1H), 1.447(s, 9H), 1.364(d, $J$ = 7.2 Hz, 3H), 1.255(t, $J$ = 6.8 Hz, 6H).

Step 3: A18-2 (2.1 g) was dissolved in hydrochloric acid/dioxane (6 mL). The reaction was stirred at room temperature overnight. TLC detected that no raw material remained. The reaction liquor was concentrated to dryness under reduced pressure to give a colorless oily matter. Isopropyl ether (10 mL) was added into the oily matter to precipitate a white solid. The reaction mixture was filtered, and the filter cake was dried at 50°C under vacuum to give A18-3 as a white solid (1.3g).

$^1$H-NMR(400MHz, CDCl-$d_3$) $\delta$ 8.708(s, 2H), 5.089(hept, $J$ = 6.0 Hz, 1H), 4.181(quint, $J$ = 6.0 Hz, 1H), 1.705(d, $J$ = 7.2 Hz, 3H), 1.278(dd, $J_1$ = 6.4 Hz, $J_2$ = 4.4 Hz, 6H).

Step 4: A18-3 (37 mg) was added into a reaction flask under $N_2$ protection. Phenyl dichlorophosphate (46 mg) was added into a separate vial, and DCM (1 mL) was added. The phenyl dichlorophosphate solution was transferred into the reaction flask, and cooled under stirring at -40°C. Triethylamine (89 mg) was added into a separate vial, and dissolved by addition of DCM (1 mL). The solution was slowly added dropwise into the reaction flask. After addition, the reaction mixture was stirred at room temperature for 10 min, and then moved to cool under stirring at -40°C for 1h. A10-0 (68 mg) was added into a separate vial, and dissolved by addition of DCM (1 mL). The solution was slowly added dropwise into the reaction flask. After addition, the reaction mixture was moved to stir at room temperature for 2h, and then detected by TLC. The mixture was purified by reverse phase column chromatography to give a yellow oily matter, which was freeze-dried to give A18 as a light-yellow solid (53 mg).

$^1$H-NMR(400MHz, CDCl-$d_3$) $\delta$ 7.358~7.282(m, 5H), 7.171(t, $J$ = 7.2 Hz, 1H), 6.885(d, $J$ = 8.4 Hz, 1H), 6.707(s, 1H), 6.623(s, 1H), 5.502(sext, $J$ = 6.4 Hz, 1H), 4.299~4.254(m, 1H), 4.126~4.064(m, 1H), 3.924~3.871(m, 7H), 3.808~3.763(m, 3H), 3.595(s, 2H), 3.318~3.276(m, 3H), 2.902(s, 1H), 2.708(s, 2H), 1.332(dd, $J_1$ = 21.2 Hz, $J_2$ = 6.8 Hz, 3H), 1.244~1.199(m, 6H).

Example 9: Synthesis of Compound A19

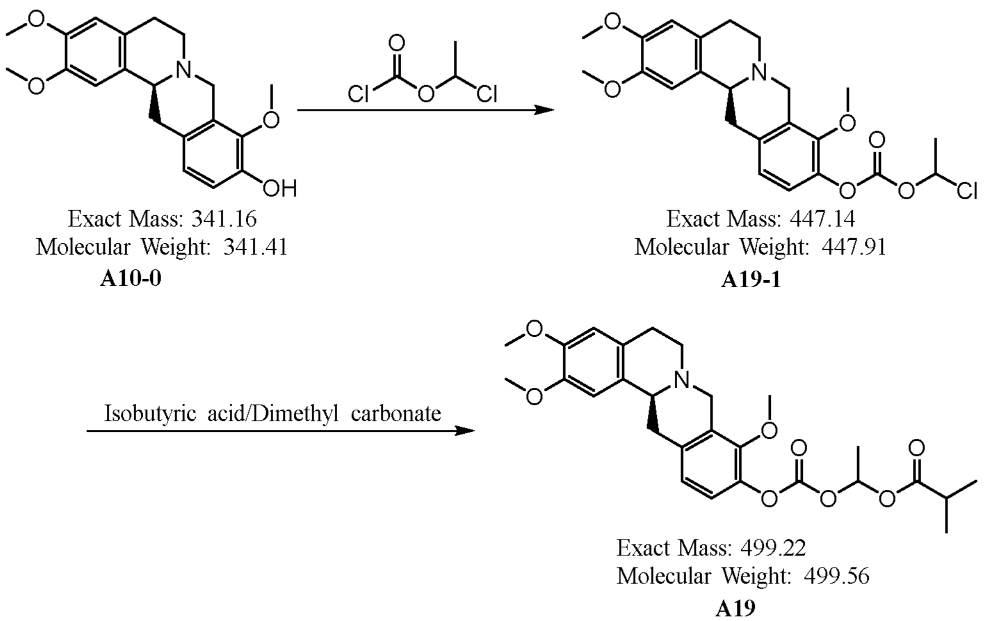


Exact Mass: 341.16
Molecular Weight: 341.41
**A10-0**

Exact Mass: 447.14
Molecular Weight: 447.91
**A19-1**

Exact Mass: 499.22
Molecular Weight: 499.56
**A19**

Step 1: A10-0 (136 mg), DIPEA (77 mg), and ultradry DCM (1 mL) were added into a reaction flask. 1-Chloroethyl chloroformate (57 mg) and DCM (1 mL) were added into a separate vial to give a solution of 1-chloroethyl chloroformate. Under stirring at room temperature, the solution of 1-chloroethyl chloroformate was added dropwise into the reaction flask. After addition, the reaction mixture was stirred at room temperature for 3h. The reaction was detected by TLC until completion. The reaction mixture was isolated by TLC, with the developing agent of PE/EA (1/1, v/v) to give A19-1 as a light-yellow solid (178 mg).

$^1$H-NMR(400MHz, CDCl-$d_3$) $\delta$ 7.029 (dd, $J_1$ = 8.4 Hz, $J_2$ = 3.2 Hz, 1H), 6.946(d, $J$ = 8.4 Hz, 1H), 6.714(s, 1H), 6.630(s, 1H), 6.517(q, $J$ = 6.0 Hz, 1H), 4.263(d, $J$ = 16.0 Hz, 1H), 3.894(s, 3H), 3.875(s, 3H), 3.857(d, $J$ = 2.0 Hz, 3H), 3.711~3.618(m, 2H), 3.355~3.165(m, 3H), 2.975~2.891(m, 1H), 2.727(d, $J$ = 11.6 Hz, 2H), 1.912(d, $J$ = 5.6 Hz, 3H).

Step 2: A19-1 (45mg), dimethyl carbonate (0.25 mL) and isobutyric acid (0.75 mL) were added into a vial. Under stirring in an ice-water bath, DIEA (26 mg) was slowly added dropwise into the vial. After addition, the reaction was heated at 80°C overnight, and monitored by TLC. The product had the same Rf value as that of A19, which was isolated by TLC with the developing agent of PE/EA (4/1, v/v) to give A19 as a light-yellow oily matter (22 mg).

$^1$H-NMR(400MHz, CDCl-$d_3$) $\delta$ 7.016(d, $J$ = 8.4Hz, 1H), 6.926(d, $J$ = 8.0 Hz, 1H), 6.824(td, $J_1$ = 5.6 Hz, $J_2$ = 2.8 Hz, 1H), 6.709(s, 1H), 6.627(s, 1H), 4.262(d, $J$ = 15.2 Hz, 1H), 3.892(s, 3H), 3.873(s, 3H), 3.854(s, 3H), 3.709~3.592(m, 2H), 3.321(dd, $J_1$ = 16.4 Hz, $J_2$ = 3.6 Hz, 1H), 3.276~3.147(m, 2H), 2.996~2.887(m, 1H), 2.735~2.699(m, 2H), 2.592(sep, $J$ = 7.2 Hz, 1H), 1.602(d, $J$ = 5.6 Hz, 3H), 1.196(d, $J$ = 6.8 Hz, 6H).

Example 10: Synthesis of Compound A21

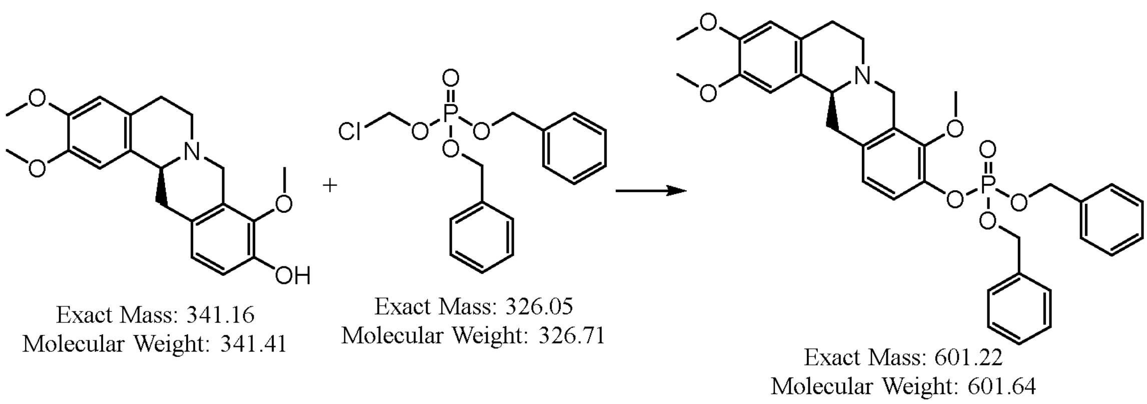


**A21**

A10-0 (34 mg), NaOH powder (16 mg), and DCM (1 mL) were added into a reaction flask, and stirred at room temperature for 10 min. Solids precipitated in the flask. Then, dibenzyl chloromethylphosphate (98 mg) was added, and stirred at room temperature overnight. The reaction liquor was isolated by TLC with the developing agent of PE/EA=1:1 to give A21 as a light-yellow solid (39 mg, the product is prone to yellow due to oxidization).

$^1$H-NMR(400MHz, CDCl-$d_3$) $\delta$ 7.328~7.307(m, 10H), 7.151(d, J = 8.4Hz, 1H), 6.841(d, J = 8.4Hz, 1H), 6.719(s, 1H), 6.623(s, 1H), 5.164~5.133(m, 4H), 4.213(d, J = 16.0 Hz, 1H), 3.893(s, 3H), 3.872(s, 3H), 3.836(s, 3H), 3.587~3.511(m, 2H), 2.375(dd, J2 = 16.4 Hz, J2 = 3.6 Hz, 1H), 3.212~3.120(m, 2H), 2.853(t, J = 13.4 Hz, 1H), 2.706~2.635(m, 2H).

Example 11: Synthesis of Compound A22

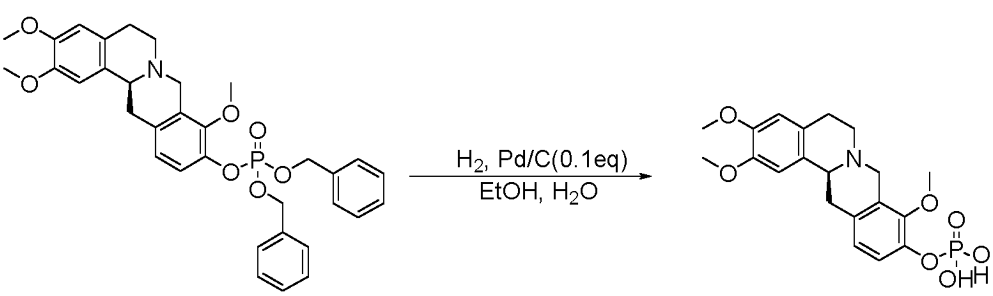


Exact Mass: 601.22
Molecular Weight: 601.64
**A21**

$C_{20}H_{24}NO_7P$
Molecular Weight: 421.39
**A22**

A21 (130 mg), ethanol (10 mL), water (5 mL), and Pd/C (13 mg) were added into a reaction flask. The reaction mixture was subjected to $H_2$ replacement, and stirred at room temperature overnight. The reaction was monitored by LCMS until completion. The reaction liquor was filtered, and the filtrate was concentrated under reduced pressure. The residue was extracted with ethyl acetate (5 mL x 2), and the aqueous phase was freeze-dried to give A22 as a light-yellow

solid (53 mg).

$^1$H-NMR(400MHz, $D_2O$-$d_2$) $\delta$ 7.300(d, $J$ = 8.4 Hz, 1H), 7.020(d, $J$ = 8.4 Hz, 1H), 6.941(s, 1H), 6.899(s, 1H), 4.783~4.743(m, 2H), 4.415(d, $J$ = 16.0 Hz, 1H), 3.889~3.790(m, 11H), 3.556~3.480(m, 1H), 3.296~3.207(m, 1H), 3.102~3.004(m, 2H).

Example 12: Synthesis of Compound A1

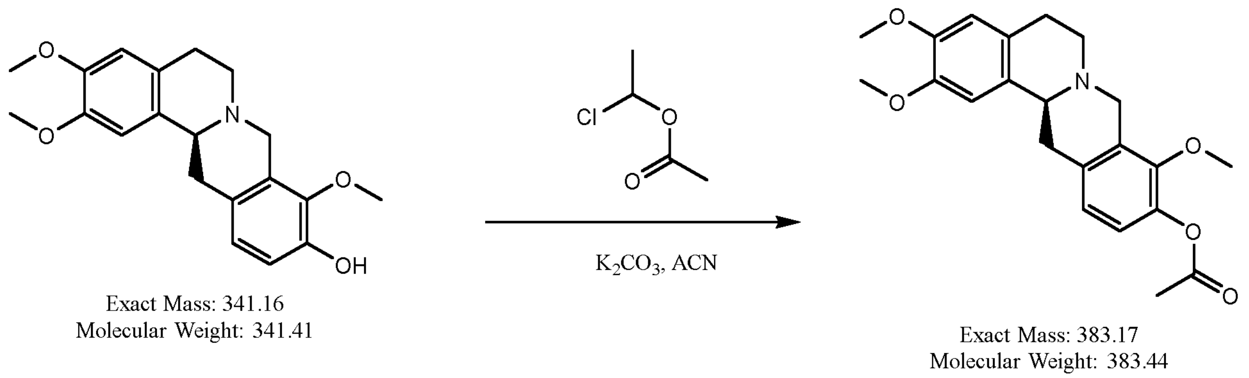


The raw material A10-0 was dissolved into acetonitrile (3 mL), and $K_2CO_3$ and 1-chloroethyl acetate (47 mg) were added sequentially. After being stirred at 60°C for 2h, the reaction mixture was diluted by addition of ethyl acetate (30 mL), sequentially washed with water twice and saturated brine once, dried over anhydrous sodium sulfate, filtered, concentrated, and purified by silica gel column to give A1 as a light-yellow solid (34 mg). MS: [M+H] 384.

$^1$H-NMR(400MHz, DMSO-$d_6$) $\delta$ 6.95(s, 2H), 6.89(s, 1H), 6.69(s, 1H), 4.06(d, $J$ = 15.9 Hz, 1H), 3.74(d, $J$ = 9.6 Hz, 9H), 3.44(dd, $J$ = 16.5, 8.2 Hz, 3H), 3.13(dd, $J$ = 10.9, 3.7 Hz, 1H), 2.97-2.87(m, 1H), 2.69-2.55(m, 2H), 2.31(s, 3H).

Example 13: Synthesis of Compound A3

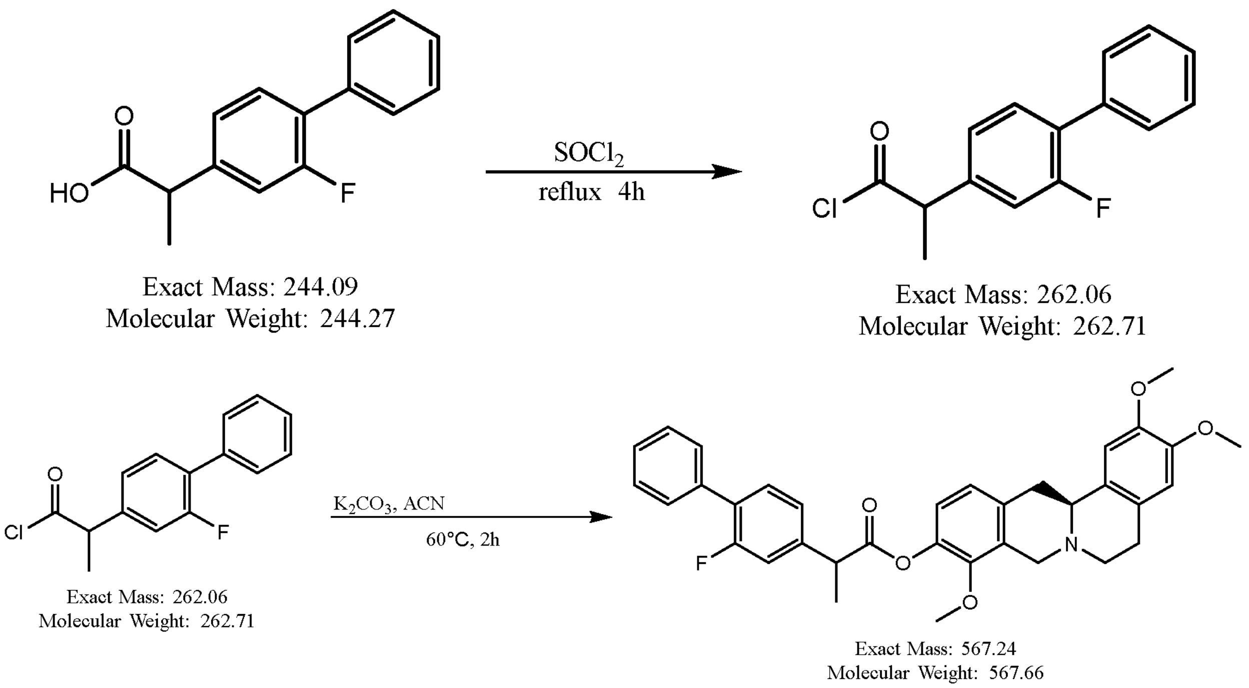


Step 1: The raw material (200 mg) was dissolved into thionyl chloride (10 mL), and heated to reflux for 4h. Then, the reaction mixture was cooled, and concentrated to give a concentrated product T-004-1 (218 mg in total), which was

directly used in the next step.

Step 2: A10-0 (218 mg) and T-004-1 (201 mg) were dissolved into acetonitrile (10 mL), and anhydrous potassium carbonate (176 mg) were added. After being stirred at 60°C for 2h, the reaction mixture was cooled. The reaction liquor was diluted by addition of ethyl acetate (50 mL), washed with water (30 mL) twice and saturated brine once, dried over anhydrous sodium sulfate, filtered, concentrated, and purified by silica gel column to give A3 as a light-yellow solid (26 mg). MS: [M+H] 568.

$^1$H-NMR(400 MHz, DMSO-$d_6$) δ 6.95(s, 2H), 6.89(s, 1H), 6.69(s, 1H), 4.06(d, *J* = 15.9 Hz, 1H), 3.74(d, *J* = 9.6 Hz, 9H), 3.44(dd, *J* = 16.5, 8.2 Hz, 3H), 3.13(dd, *J* = 10.9, 3.7 Hz, 1H), 2.97-2.87(m, 1H), 2.69 - 2.55(m, 2H), 2.31(s, 3H).

Example 14: Synthesis of Compound A30

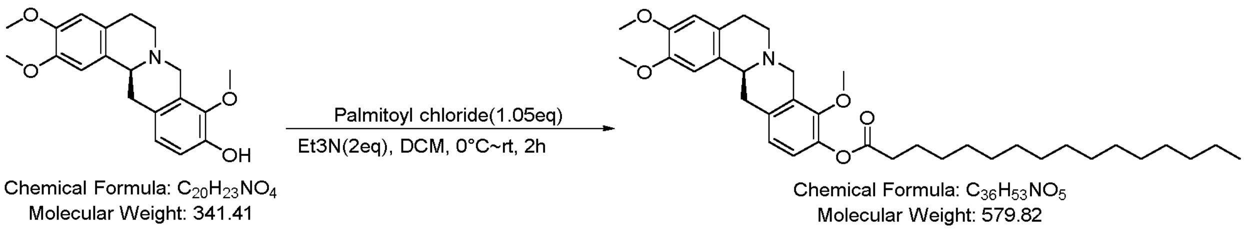


A10-0 (150 mg), triethylamine (89 mg), and DCM (4 mL) were added into a reaction flask, and cooled in an ice-water bath. Palmitoyl chloride (127 mg) and DCM (1 mL) were added into a separate vial, and the palmitoyl chloride solution was added dropwise into the reaction flask. After addition, the reaction mixture was stirred at room temperature for 2h, and detected by TLC. After the reaction was completed, the reaction liquor was isolated by column chromatography, and purified to give a light-yellow solid (245 mg), which was freeze-dried to give A30 as a light-yellow solid (228 mg, yield 83.4%). MS: [M+H] 580.

$^1$H-NMR($CDCl_3$, 400 MHz) $\delta$ 6.931(d, *J* = 8.4 Hz, 1H), 6.894(d, *J* = 8.4 Hz, 1H), 6.716(s, 1H), 6.625(s, 1H), 4.234(d, *J* = 15.6 Hz, 1H), 3.894(s, 3H), 3.873(s, 3H), 3.806(s, 3H), 3.711-3.589(m, 2H), 3.311(dd, $J_1$ = 16.4 Hz, $J_2$ = 3.2 Hz, 1H), 3.231-3.173(m, 2H), 2.942-2.873(m, 1H), 2.720-2.688(m, 2H), 2.587(t, *J* = 7.6 Hz, 2H), 1.777(quint, *J* = 7.6 Hz, 2H), 1.461-1.263(m, 24H), 0.897-0.864(m, 3H).

Example 15: Synthesis of Compound A20

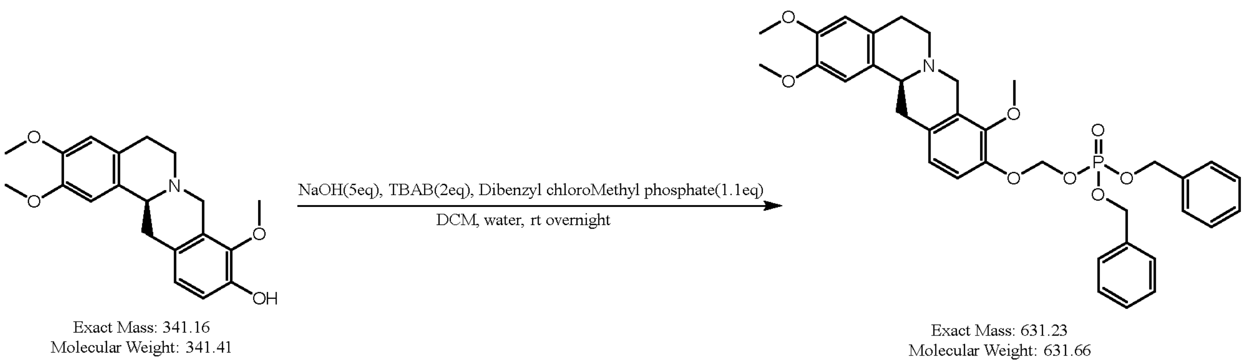


A10-0 (340 mg) and TBAB (640 mg) were added into a reaction flask, and dissolved by addition of DCM (5 mL). NaOH (200 mg) was added into a separate vial, and dissolved by addition of water (5 mL). At room temperature, the NaOH solution was added dropwise into the reaction flask. Dibenzyl chloromethyl phosphate (360 mg) was added into a separate vial, and dissolved by addition of DCM (5 mL). At room temperature, the solution was added dropwise into the reaction flask. After addition, the reaction mixture was continued to stir overnight, and purified by TLC isolation to give A20 as a light-yellow oily matter (210 mg, with a purity of 97%). MS: [M+H] 632.

Example 16: Synthesis of Compound A23

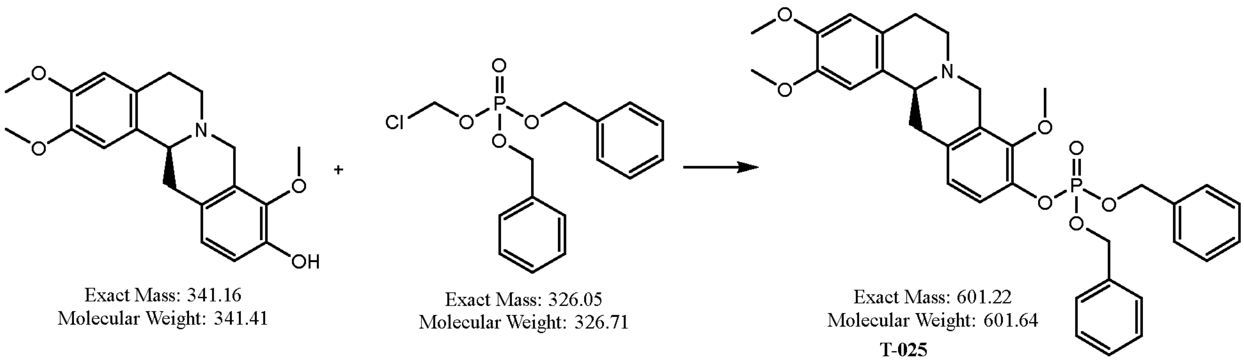

T-025

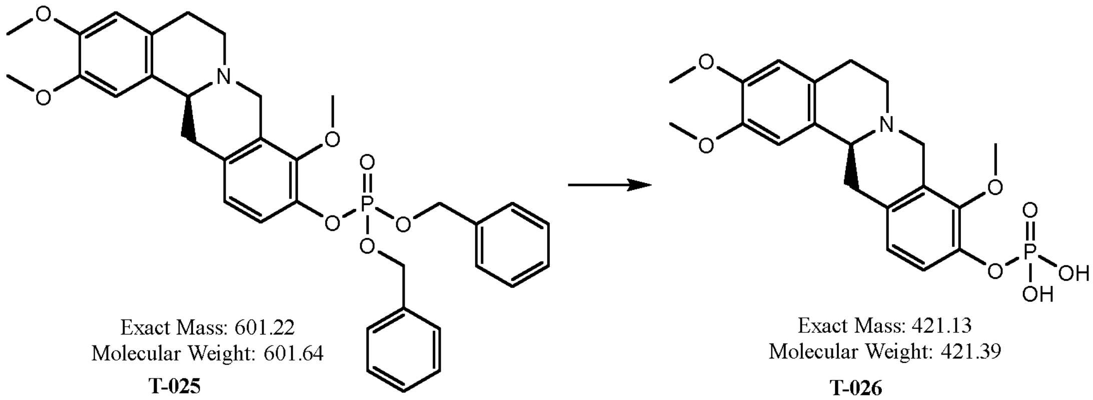

T-025

T-026

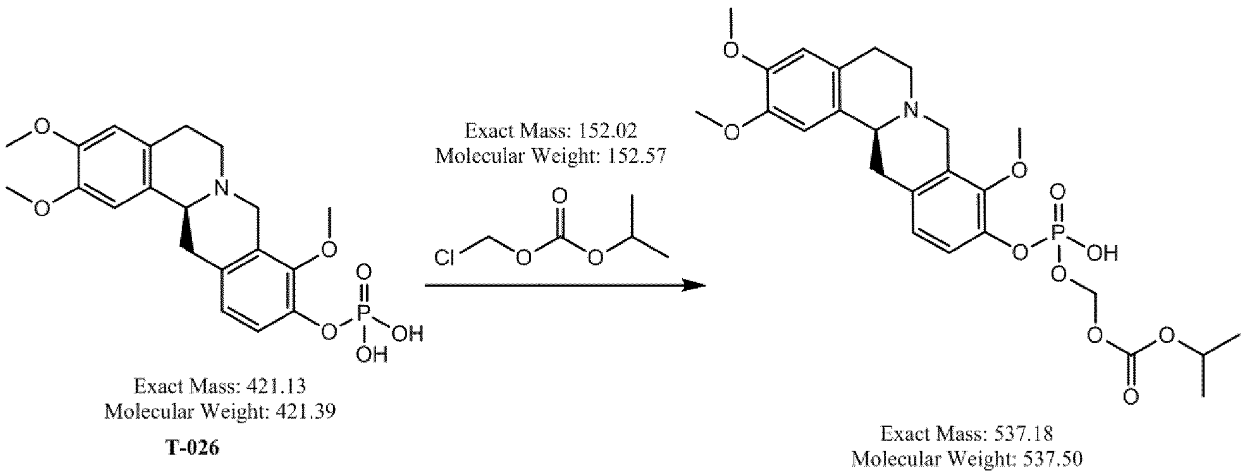

T-026

Step 1: A10-0 (341 mg), sodium hydroxide (160 mg, 4 eq), and DCM (6 mL) were added into a 20 mL reaction flask. Dibenzyl chloromethyl phosphate (490 mg, 1.5 eq) was added into a separate vial, and diluted by addition of DCM (4 mL). The reaction mixture was stirred at room temperature for 10 min, and solids were precipitated in the reaction flask. The dibenzyl chloromethyl phosphate solution was added dropwise in to the reaction flask, stirred at room temperature overnight. The separated aqueous phase was extracted with DCM. The combined organic phase was purified by column chromatography to give T-025 as a light-yellow solid (462 mg, yield 77%). MS: [M+H] 602. $^1$H-NMR(400MHz, CDCl-$d_3$) $\delta$ 7.328~7.307(m, 10H), 7.151(d, $J$ = 8.4Hz, 1H), 6.841(d, $J$ = 8.4Hz, 1H), 6.719(s, 1H), 6.623(s, 1H), 5.164~5.133(m, 4H), 4.213(d, $J$ = 16.0 Hz, 1H), 3.893(s, 3H), 3.872(s, 3H), 3.836(s, 3H), 3.587~3.511(m, 2H), 2.375(dd, $J_1$ = 16.4 Hz, $J_2$ = 3.6 Hz, 1H), 3.212~3.120(m, 2H), 2.853 (t, $J$ = 13.4 Hz, 1H), 2.706~2.635(m, 2H).

Step 2: T-025 (462 mg), absolute ethanol (10 mL), purified water (5 mL), and Pd/C (46 mg, 10%) were added into a 50 mL single-neck flask, and stirred intensely. The reaction mixture was subjected to hydrogen replacement three times, and stirred at room temperature overnight. The reaction liquor was filtered, and the filtrate was concentrated under

reduced pressure. The residue was filtered. The filtrate was extracted with EA twice, and the aqueous phase was freeze-dried to give T-026 as a light-yellow solid (212 mg, yield 66%). MS: [M+H] 422.

$^1$H-NMR(400MHz, $D_2O$-$d_2$) $\delta$ 7.300(d, $J$ = 8.4 Hz, 1H), 7.020(d, $J$ = 8.4 Hz, 1H), 6.941(s, 1H), 6.899(s, 1H), 4.783~4.743(m, 2H), 4.415(d, $J$ = 16.0 Hz, 1H), 3.889~3.790(m, 11H), 3.556~3.480(m, 1H), 3.296~3.207(m, 1H), 3.102~3.004(m, 2H).

Step 3: T-026 (60 mg) and NMP (1 mL) were added into a vial, and heated to 45°C. Triethylamine (60 mg, 4 eq) and TBAB (48 mg) were added, and heated to 50°C. Chloromethyl isopropyl carbonate (114 mg, 5 eq) was added, and the reaction mixture was stirred at 50°C overnight. The reaction mixture was purified by column chromatography. The fractions were concentrated to dryness under reduced pressure to give a yellow solid, which was dissolved in water and then freeze-dried to give A23 as a yellow solid (49 mg, yield 48.0%). MS: [M+H] 538.

$^1$H-NMR ($CDCl_3$, 400 MHz) $\delta$ 7.198(d, $J$ = 8.8 Hz, 1H), 6.686(s, 1H), 6.648-6.612(m, 2H), 5.645-5.563(m, 2H), 4.845(sept, $J$ = 6.4 Hz, 1H), 4.531(s, 1H), 4.067-3.706(m, 13H), 3.309(dd, $J_1$ = 16.4Hz, $J_2$ = 6.4 Hz, 1H), 3.149(s, 3H), 2.968(s, 1H), 2.809(d, $J$ = 15.6 Hz, 1H), 1.300(t, $J$ = 7.6 Hz, 6H).

$^{31}$P-NMR ($CDCl_3$, 160 MHz) $\delta$ -7.7546(s).

Example 17: Synthesis of Compound A56

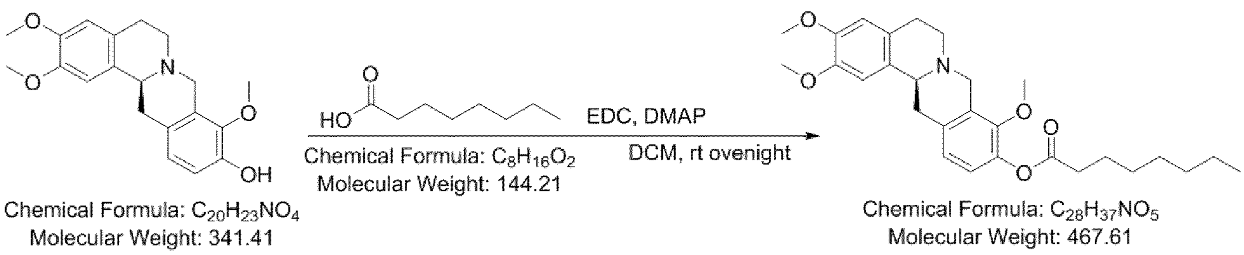


A10-0

A10-0 (68 mg), n-caprylic acid (32 mg), DMAP (2 mg), and dichloromethane (1 mL) were added into a reaction flask, and stirred at room temperature. EDCI (76 mg, 2 eq) were added into the flask, and the reaction mixture was stirred at room temperature overnight. The reaction was detected by TLC until completion. The reaction mixture was purified by silica gel column chromatography to give a light-yellow oily matter, which was freeze-dried to give the product A56 (65 mg, yield 69.8%). MS: [M+H] 468.

$^1$H-NMR($CDCl_3$, 400 MHz) $\delta$ 6.880(d, $J$ = 8.4 Hz, 1H), 6.890(d, $J$ = 8.4 Hz, 1H), 6.720(s, 1H), 6.623(s, 1H), 4.218(d, $J$ = 15.6 Hz, 1H), 3.894(s, 3H), 3.873(s, 3H), 3.803(s, 3H), 3.620-3.565(m, 2H), 3.304(dd, $J_1$ = 16.4 Hz, $J_2$ = 3.2 Hz, 1H), 3.227-3.108(m, 2H), 2.884(dd, $J_1$ = 14.8 Hz, $J_2$ = 12.0 Hz, 1H), 2.709-2.850(m, 2H), 2.589(t, $J$ = 7.2 Hz, 3H), 1.781(quint, $J$ = 7.6 Hz, 2H), 1.465-1.393(m, 2H), 1.374-1.313(m, 6H), 0.914-0.881(m, 3H).

Example 18: Synthesis of Compound A44

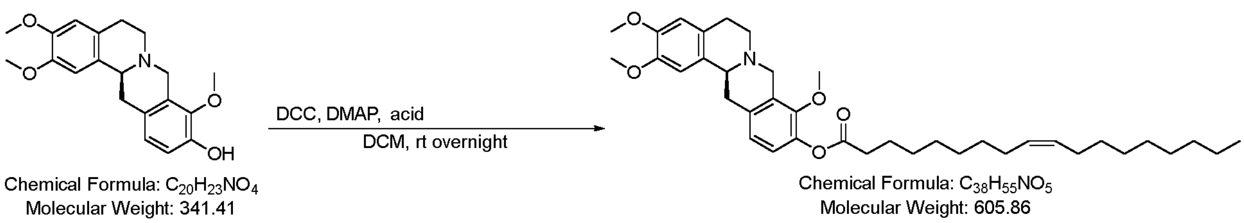


A10-0 (68 mg), oleic acid (57 mg, 1.01 eq), DMAP (3 mg, 0.1 eq), and DCM (1 mL) were added into a reaction flask. DCC (82 mg, 2 eq) was added into a separate vial, and diluted by addition of DCM (1 mL). Under stirring at room temperature, the DCC solution was added dropwise into the reaction flask. After addition, the reaction mixture was stirred at room temperature overnight. The reaction was detected by TLC until completion, and the reaction mixture was purified to give a light-yellow oily matter, which was freeze-dried to give A 44 as a light-yellow solid (60.53 mg). MS: [M+H] 606.

$^1$H-NMR($CDCl_3$, 400 MHz) $\delta$ 6.882(d, $J$ = 8.0 Hz, 1H), 6.898(d, $J$ = 8.4 Hz, 1H), 6.714(s, 1H), 6.625(s, 1H), 5.396-5.312(m, 2H), 4.254(d, $J$ = 15.6 Hz, 1H), 3.893(s, 3H), 3.873(s, 3H), 3.807(s, 3H), 3.701-3.609(m, 2H),

3.340-3.183(m, 3H), 2.960-2.894(m, 1H), 2.738-2.706(m, 2H), 2.587(t, $J$ = 7.6 Hz, 2H), 2.025-2.012(m, 5H), 1.779(quint, $J$ = 7.2 Hz, 2H), 1.433-1.271(m, 19H), 0.897-0.864(m, 3H).

Example 19: Synthesis of Compound A24

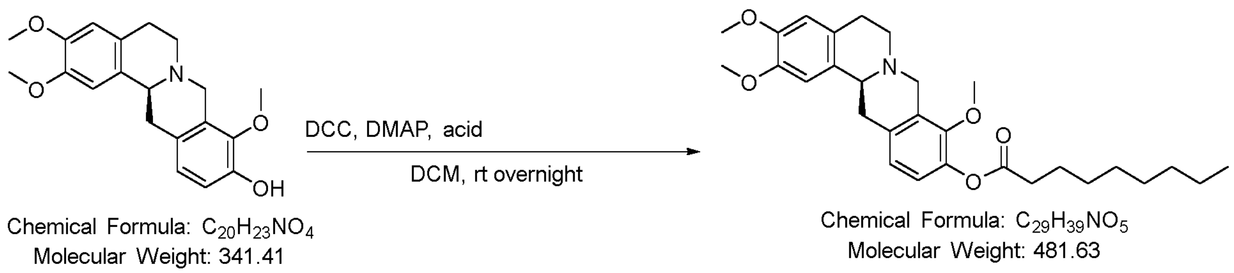


A10-0 (68 mg), DMAP (3 mg), n-nonanoic acid (32 mg), and DCM (1 mL) were added into a reaction flask. DCC (82 mg) and DCM (1 mL) were added into a separate vial, and the DCC solution was added dropwise into the reaction flask under stirring at room temperature. The reaction mixture was stirred at room temperature overnight. The reaction was detected by TLC until completion, and the reaction mixture was purified to give a colorless oily matter, which was freeze-dried to give A24 as a light-yellow solid (63.44 mg). MS: [M+H] 482.

$^1$H-NMR($CDCl_3$, 400 MHz) $\delta$ 6.882(d, $J$ = 8.4 Hz, 1H), 6.896(d, $J$ = 8.4 Hz, 1H), 6.716(s, 1H), 6.625(s, 1H), 4.242(d, $J$ = 16.0 Hz, 1H), 3.893(s, 3H), 3.872(s, 3H), 3.807(s, 3H), 3.710-3.601(m, 2H), 3.313(dd, $J_1$ = 16.0 Hz, $J_2$ = 3.6 Hz, 1H), 3.245-3.145(m, 2H), 2.950-2.886(m, 1H), 2.730-2.697(m, 2H), 2.588(t, $J$ = 7.6 Hz, 2H), 1.779(quint, $J$ = 7.6 Hz, 2H), 1.362-1.256(m, 10H), 0.908-0.874(m, 3H).

Example 20: Synthesis of Compound A25

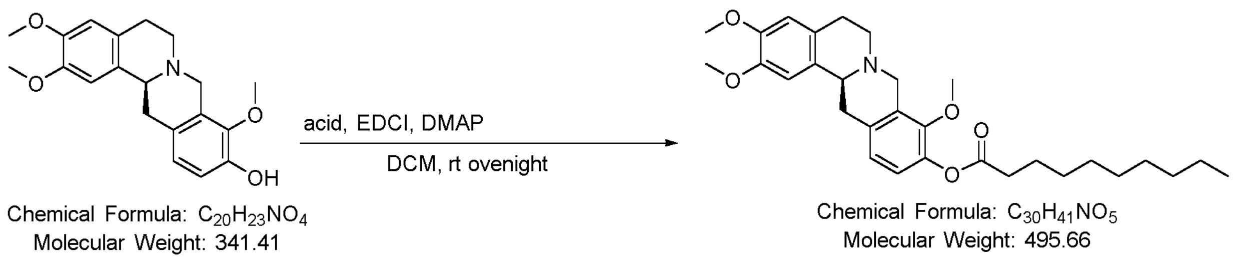


A10-0 (68 mg), DMAP (3 mg, 0.1 eq), n-decanoic acid (38 mg), and DCM (1 mL) were added into a reaction flask. EDCI (77 mg) and DCM (0.5 mL) were added into a separate vial, and the solid were not dissolved. Under stirring, the EDCI suspension was added into the reaction flask with a syringe, and the solid was dissolved immediately. The reaction mixture was stirred at room temperature overnight. The reaction was detected by TLC until completion. The reaction mixture was purified to give a light-yellow oily matter, which was freeze-dried to give A25 as a light-yellow solid (61.91 mg). MS: [M+H] 496.

$^1$H-NMR($CDCl_3$, 400 MHz) $\delta$ 6.934(d, $J$ = 8.4 Hz, 1H), 6.900(d, $J$ = 8.0 Hz, 1H), 6.713(s, 1H), 6.627(s, 1H), 4.252(d, $J$ = 15.2 Hz, 1H), 3.893(s, 3H), 3.873(s, 3H), 3.810(s, 3H), 3.710-3.627(m, 2H), 3.343-3.160(m, 3H), 2.937(s, 1H), 2.723-2.695(m, 2H), 2.588(t, $J$ = 7.6 Hz, 2H), 1.778(quint, 7.6 Hz, 2H), 1.448-1.283(m, 14), 0.904-0.871(m, 3H).

Example 21: Synthesis of Compound A57

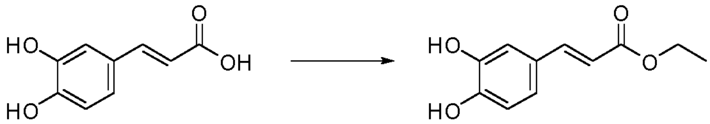

Chemical Formula: $C_9H_8O_4$
Molecular Weight: 180.16

Chemical Formula: $C_{11}H_{12}O_4$
Molecular Weight: 208.21
T-039-1

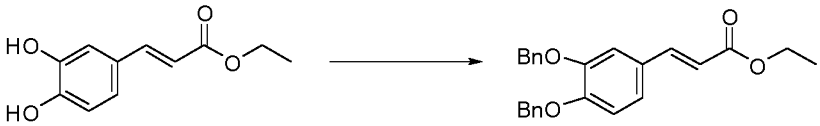

Chemical Formula: $C_{11}H_{12}O_4$
Molecular Weight: 208.21
T-039-1

Chemical Formula: $C_{25}H_{24}O_4$
Molecular Weight: 388.46
T-039-2

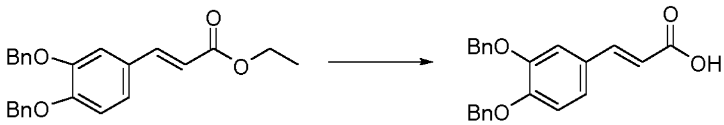

Chemical Formula: $C_{25}H_{24}O_4$
Molecular Weight: 388.46
T-039-2

Chemical Formula: $C_{23}H_{20}O_4$
Molecular Weight: 360.41
T-039-3

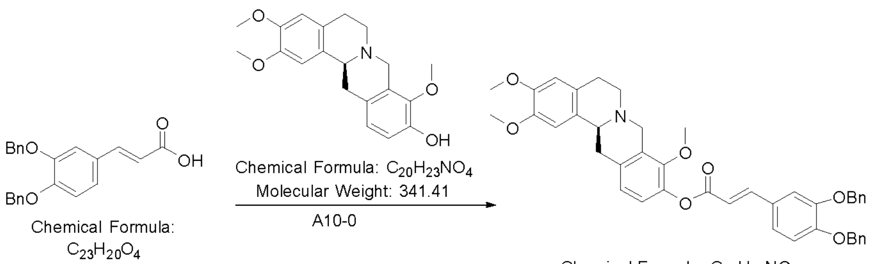

Chemical Formula: $C_{20}H_{23}NO_4$
Molecular Weight: 341.41
A10-0

Chemical Formula: $C_{23}H_{20}O_4$
Molecular Weight: 360.41
T-039-3

Chemical Formula: $C_{43}H_{41}NO_7$
Molecular Weight: 683.80
T-039-4

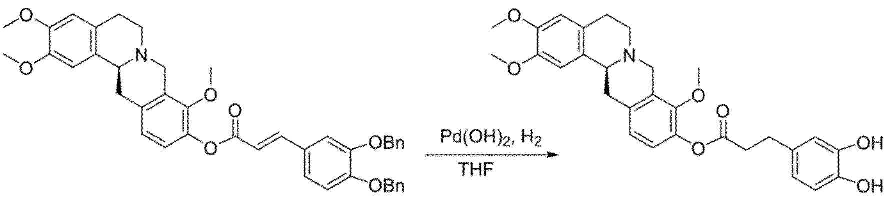


Chemical Formula: $C_{43}H_{41}NO_7$
Molecular Weight: 683.80
T-039-4

Chemical Formula: $C_{29}H_{31}NO_7$
Molecular Weight: 505.57

Step 1: Caffeic acid (1.8 g) and absolute ethanol (20 mL) were added into a 30-mL flask, and concentrated sulfuric acid (5 drops) was added dropwise. After addition, the reaction mixture was heated and stirred under reflux overnight. TLC detected that no raw material remained. The reaction liquor was diluted by addition of water, and the mixture was extracted with EA twice. The combined organic phase was washed with water twice, dried, and filtered. The filtrate was concentrated to dryness under elevated pressure to give T-039-1 as a brown solid (2.12 g).

$^1$H-NMR(DMSO-$d_6$, 400MHz) $\delta$ 9.565(s, 1H), 9.116(s, 1H), 7.470(d, $J$ = 16.0 Hz, 1H), 7.047(d, $J$ = 1.6 Hz, 1H), 6.999(dd, $J_1$ = 8.0 Hz, $J_2$ = 2.0 Hz, 1H), 6.763(d, $J$ = 8.4 Hz, 1H), 6.253(d, $J$ = 16.0 Hz, 1H), 4.155(q, $J$ = 7.2 Hz, 2H), 1.241(t, $J$ = 7.2 Hz, 3H).

Step 2: T-039-1 (1.04 g), $K_2CO_3$ (2.07 g), and acetone (15 mL) were added into a vial, and stirred at room temperature. Benzyl bromide (1.79 g) was added dropwise. After addition, the reaction mixture was stirred at 60°C overnight. TLC detected that no raw material remained. The reaction liquor was filtered. The filtrate was concentrated to dryness under reduced pressure to give T-039-2 as a colorless oily matter. The crude was directly used in the next step.

Step 3: T-039-2 obtained in the last step was dissolved in THF (30 mL), and water (5.5 mL) and sodium hydroxide

solid (1.62g, 8.1 eq) were added. The reaction mixture was stirred at room temperature for 36h. LCMS showed that the reaction was completed. The reaction liquor was concentrated under reduced pressure, and the aqueous phase was adjusted to pH 2. The mixture was extracted with EA once, and the EA phase was dried, filtered, and concentrated to dryness under reduced pressure to give T-039-3 as a beige solid (1.741 g).

$^1$H-NMR(DMSO-$d_6$, 400MHz) $\delta$ 12.202(s, 1H), 7.516-7.437(m, 6H), 7.406-7.363(m, 4H), 7.335-7.301(m, 2H), 7.195(dd, $J_1$ = 8.4 Hz, $J_2$ = 1.6 Hz, 1H), 7.073(d, $J$ = 8.4 Hz, 1H), 6.418(d, $J$ = 16.0 Hz, 1H), 5.196(s, 2H), 5.184(s, 2H).

Step 4: To a vial were added A10-0 (102 mg), T-039-3 (129 mg), DMAP (3 mg), and DCM (3 mL). The solids were not dissolved fully. EDCI (115 mg) was added, and the solids were dissolved immediately. The reaction mixture was stirred at room temperature for 48h. TLC detected that substantially no raw material remained. The reaction liquor was isolated by silica gel chromatography with the developing agent of PE/EA=1/1 to give T-039-4 as a yellow oily matter (208 mg).

$^1$H-NMR(DMSO-$d_6$, 400MHz) $\delta$ 7.792(d, $J$ = 16.0 Hz, 1H), 7.620(s, 1H), 7.496-7.449(m, 4H), 7.416-7.375(m, 4H), 7.348-7.311(m, 3H), 7.127(d, $J$ = 8.4 Hz, 1H), 7.028-6.947(m, 2H), 6.899(s, 1H), 6.826(d, $J$ = 16.0 Hz, 1H), 6.697(s, 1H), 5.223-5.220(m, 4H), 4.086(d, $J$ = 16.0 Hz, 1H), 3.761(s, 3H), 3.744(s, 3H), 3.733(s, 3H), 3.497-3.438(m, 3H), 3.156-3.118(m, 1H), 2.953-2.913(m, 1H), 2.653-2.583(m, 2H), 2.537-2.529(m, 1H).

Step 5: T-039-4 (208 mg) was dissolved in THF (15 mL), and palladium hydroxide (20 mg, 10%) was added. The reaction mixture was subjected to hydrogen replacement, and stirred at room temperature overnight. TLC detected that no raw material remained. The reaction liquor was filtered, and concentrated to dryness under reduced pressure. The residue was isolated by Prep TLC with the developing agent of EA to give A57 as an offwhite solid (70 mg). MS: [M+H] 506.

$^1$H-NMR($CDCl_3$, 400 MHz) $\delta$ 6.941-6.863(m, 2H), 6.716(s, 1H), 6.678(d, $J$ = 8.0 Hz, 1H), 6.635(s, 1H), 6.567(dd, $J_1$ = 8.0 Hz, $J_2$ = 1.6 Hz, 1H), 6.418(d, $J$ = 1.6 Hz, 1H), 4.293(d, $J$= 16.0 Hz, 1H), 3.891(s, 3H), 3.876(s, 3H), 3.767-3.736(m,1H), 3.710(s, 3H), 3.649(d, $J$ = 16.0 Hz, 1H), 3.369(dd, $J_1$ = 16.4 Hz, $J_2$= 3.6 Hz, 1H), 3.312-3.256(m, 2H), 2.990(dd, $J_1$ = 16.0 Hz, $J_2$ = 12.0 Hz, 1H), 2.848-2.712(m,6H).

Example 22: Synthesis of Compound A53

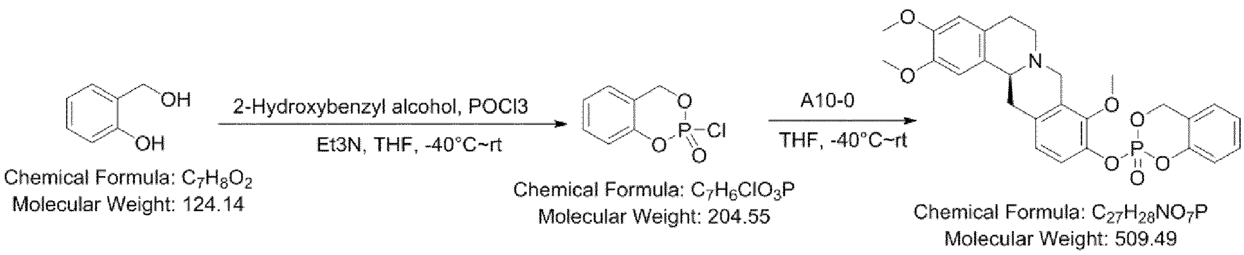


Phosphorus oxychloride (101 mg), triethylamine (302 mg), and THF (2 mL) were added into a reaction flask, and cooled at -40°C. A solution of 2-hydroxybenzyl alcohol (74 mg) in THF (2 mL) was slowly added dropwise at -40°C. After addition, the reaction was stirred at -40°C for 1h, moved to room temperature to stir for additional 2h, and then cooled at -40°C. At a temperature of -40°C, a solution of A10-0 (102 mg) in THF (0.5 mL) was slowly added dropwise. After addition, the reaction was moved to stir at room temperature overnight. LCMS showed that no A10-0 remained in the reaction, and the main peak was 510. The reaction liquor was filtered, and the filtrate was isolated by preparative thin layer chromatography with the developing agent of EA to give a colorless oily matter, which was freeze-dried to give A53 as a light-yellow solid (97.78 mg). MS: [M+H] 510.

$^1$H-NMR($CDCl_3$, 400 MHz) $\delta$ 7.369(t, $J$ = 7.2 Hz, 1H), 7.265(s, 1H), 7.195(t, $J$ = 7.2 Hz, 1H), 7.140(d, $J$ = 7.6 Hz, 2H), 7.927(d, $J$ = 8.4 Hz, 1H), 6.741(s, 1H), 6.650(s, 1H), 5.608-5.553(m, 1H), 5.499-5.417(m, 1H), 4.234(d, $J$ = 14.8 Hz, 1H), 3.920(d, $J$ = 2.0 Hz, 3H), 3.299(d, $J$ = 1.6 Hz, 3H), 3.751(dd, $J_1$ = 13.6 Hz, $J_2$ = 2.4 Hz, 3H), 3.647-3.566(m, 2H), 3.320(d, $J$ = 16.4 Hz, 1H), 3.243-3.157(m, 2H), 2.933-2.869(m, 1H), 2.741-2.707(m, 2H).

$^{13}$P-NMR($CDCl_3$, 160 MHz): $\delta$ -15.448(s), -15.548(s).

Example 23: Synthesis of Compound A43

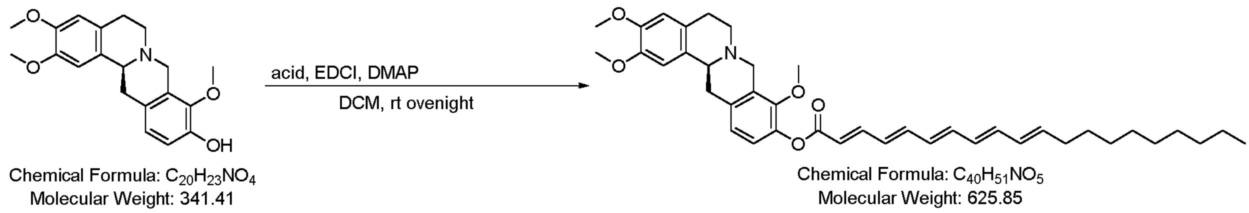


A10-0 (68 mg), DMAP (2 mg), all-cis-eicosapentaenoic acid (120 mg), and DCM (2 mL) were added into a reaction flask. EDCI (153 mg) was added at room temperature. After addition, the reaction mixture was stirred at room temperature overnight. The reaction was detected by TLC until completion. The reaction mixture was isolated by column chromatography to give a yellow oily matter, which was freeze-dried to give A43 as a light-yellow solid (93.01 mg). MS: [M+H] 626.

$^1$H-NMR($CDCl_3$, 400 MHz) $\delta$ 6.928(d, $J$ = 8.4 Hz, 1H), 6.893(d, $J$ = 8.4 Hz, 1H), 6.715(s, 1H), 6.623(s, 1H), 5.548-5.291(m, 10 H), 4.233(d, $J$ = 16.0 Hz, 1H), 3.890(s, 3H), 3.870(s, 3H), 3.802(s, 3H), 3.631-3.593(m, 2H), 3.308(dd, $J_1$ = 16.4 Hz, $J_2$ = 2.8 Hz, 1H), 3.236-3.145(m, 2H), 2.987-2.815(m, 9H), 2.724-2.692(m, 2H), 2.608(t, $J$ = 7.2 Hz, 2H), 2.225(q, $J$ = 6.4 Hz, 2H), 2.076(quint, $J$ = 7.2 Hz, 2H), 1.862(quint, $J$ = 7.2 Hz, 2H), 0.974(t, $J$ = 7.2 Hz, 3H).

Example 24: Synthesis of Compound A54

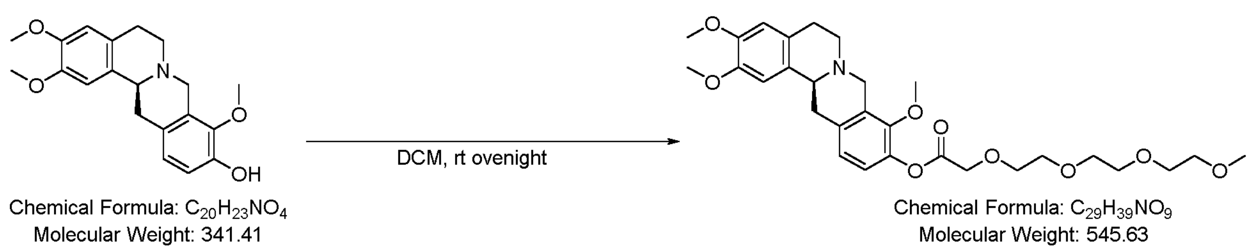


A10-0 (68 mg), DMAP (2 mg), 3,6,9,12-tetraoxotridecanoic acid (49 mg), and DCM (2 mL) were added into a reaction flask. EDCI (76mg) was added at room temperature. After addition, the reaction mixture was stirred at room temperature overnight. TLC detected that no raw material remained. The reaction liquor was isolated by column chromatography to give a colorless oily matter (75 mg), which was freeze-dried to give A54 as a light-yellow solid (65 mg). MS: [M+H] 546.

$^1$H-NMR(DMSO, 400 MHz) $\delta$ 6.999(d, $J$ = 8.0 Hz, 1H), 6.963(d, $J$ = 8.4 Hz, 1H), 6.887(s, 1H), 6.691(s, 1H), 4.282(s, 2H), 4.069(d, $J$ = 16.0 Hz, 1H), 3.753(s, 3H), 3.729-3.694(s, 8H), 3.605-3.582(m, 2H), 3.554-3.419(m, 12H), 3.238(s, 3H), 3.144-3.108(m, 1H), 2.970-2.891(m, 1H), 2.640-2.565(m, 2H).

Example 25: Synthesis of Compound A40

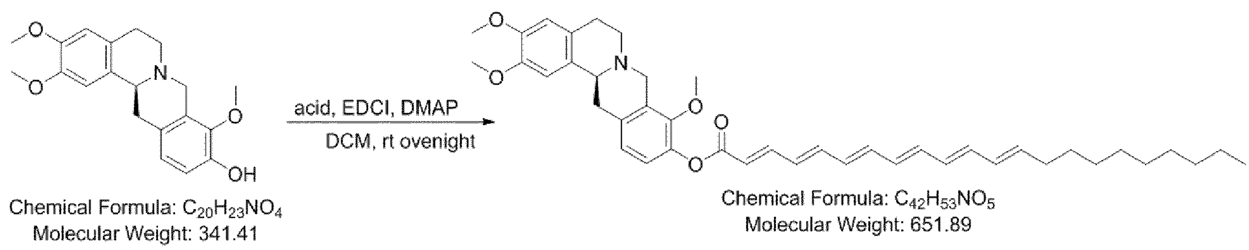


A10-0 (68 mg), DMAP (2 mg), docosahexenoic acid (79 mg), and DCM (2 mL) were added into a reaction flask. EDCI (76mg) was added at room temperature. The reaction mixture was stirred at room temperature overnight. After TLC detected that the reaction was completed, the reaction mixture was purified to give a light-yellow oily matter, which was freeze-dried to give A40 as a light-yellow oil matter (103 mg). MS: [M+H] 652.

$^1$H-NMR(CDC13, 400 MHz) $\delta$ 6.929(d, $J$ = 8.4 Hz, 1H), 6.896(d, $J$ = 8.4 Hz, 1H), 6.714(s, 1H), 6.625(s, 1H), 5.484-5.294(m, 12H), 4.239(m, 16.0 Hz, 1H), 3.892(s, 3H), 3.872(s, 3H), 3.804(s, 3H), 3.709-3.599(m, 2H), 3.311(dd, $J_1$ =

16.0 Hz, $J_2$ = 3.2 Hz, 1H), 3.241-3.142(m, 2H), 2.995-2.797(m, 11H), 2.728-2.644(m, 4H), 2.566-2.518(m, 2H), 2.073(quint, $J$ = 7.2 Hz, 2H), 0.972(t, $J$ = 7.6 Hz, 3H).

Example 26: Synthesis of Compound A55

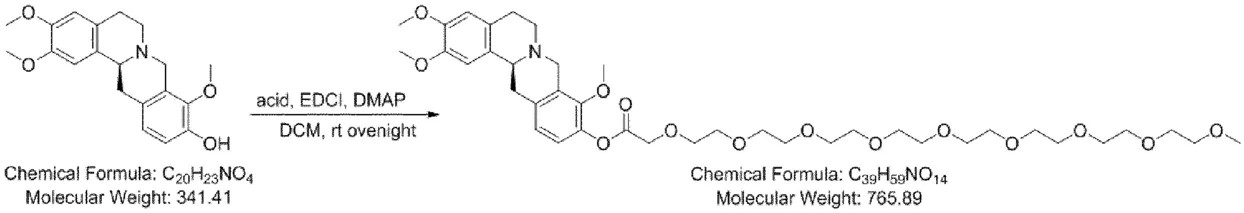


A10-0 (68 mg), DMAP (2 mg), 2,5,8,11,14,17,20,23,26-nonaoxaoctacosan-28-oic acid (101 mg), and DCM (2 mL) were added into a reaction flask. EDCI (76 mg) was added under stirring at room temperature. The reaction mixture was stirred at room temperature overnight. TLC detected that no raw material remained. The reaction mixture was isolated by column chromatography to give a light-yellow oily matter, which was freeze-dried to give A55 as a light-yellow solid (135 mg). MS: [M+H] 766.5.

$^1$H-NMR($CDCl_3$, 400 MHz) $\delta$ 6.973-3.929(m, 2H), 6.709(s, 1H), 6.632(s, 1H), 4.459(s, 2H), 4.308-4.276(m, 1H), 3.894(s, 3H), 3.875(s, 3H), 3.852-3.814(m, 5H), 3.747-3.724(m, 2H), 3.690-3.629(m, 27H), 3.557-3.533(m, 2H), 3.376(s, 3H), 3.322-3.198(m, 2H), 2.987(s, 1H), 2.760(s, 2H), 1.759(s, 2H).

Example 27: Synthesis of Compound A45

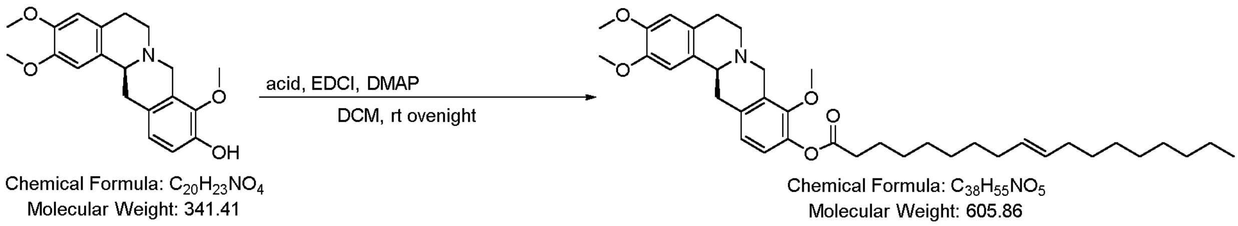


**A10-0 (68** mg), DMAP (2 mg), elaidic acid (68 mg), and DCM (2 mL) were added into a reaction flask. EDCI (76mg) was added under stirring at room temperature. The reaction mixture was stirred at room temperature overnight. After TLC detected that the reaction was completed, the reaction mixture was purified by column chromatography to give a yellow oily matter (102 mg), which was freeze-dried to give A45 as a light-yellow solid (95 mg). MS: [M+H] 606.

$^1$H-NMR($CDCl_3$, 400 MHz) $\delta$ 6.932(d, $J$ = 8.4 Hz, 1H), 6.898(d, $J$ = 8.4 Hz, 1H), 6.713(s, 1H), 6.626(s, 1H), 5.390(s, 2H), 4.251(d, $J$ = 15.6 Hz, 1H), 3.892(s, 3H), 3.873(s, 3H), 3.808(s, 3H), 3.7093.626(m, 2H), 3.334-3.232(m, 3H), 2.938(s, 1H), 2.712(s, 2H), 2.585(t, $J$ = 7.6 Hz, 2H), 2.019-1.974(m, 4H), 1.776(quint, $J$ = 7.2 Hz, 2H), 1.443-1.265(m, 20H), 0.895-0.862(m, 3H).

Example 28: Synthesis of Compound A4

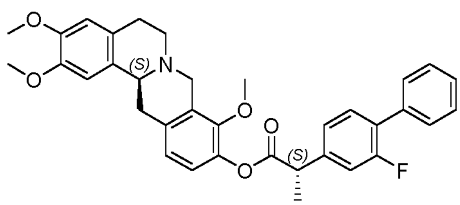

A4

S-conformational raw material (200 mg) was used as the starting material, and the procedure was the same as that of the compound A3 to finally give A4 as a light-yellow solid (25 mg)

MS: [M+H] 568.

1H-NMR(400 MHz, DMSO-*d*6) $\delta$ 6.95(s, 2H), 6.89(s, 1H), 6.69(s, 1H), 4.06(d, *J* = 15.9 Hz,1H), 3.74(d, *J* = 9.6 Hz, 9H), 3.44(dd, *J* = 16.5, 8.2 Hz, 3H), 3.13(dd, *J* = 10.9, 3.7 Hz, 1H),2.97-2.87(m, 1H), 2.69 - 2.55(m, 2H), 2.31(s, 3H).

Example 29: Synthesis of Compound A48

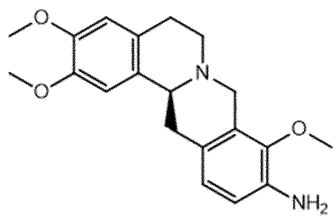

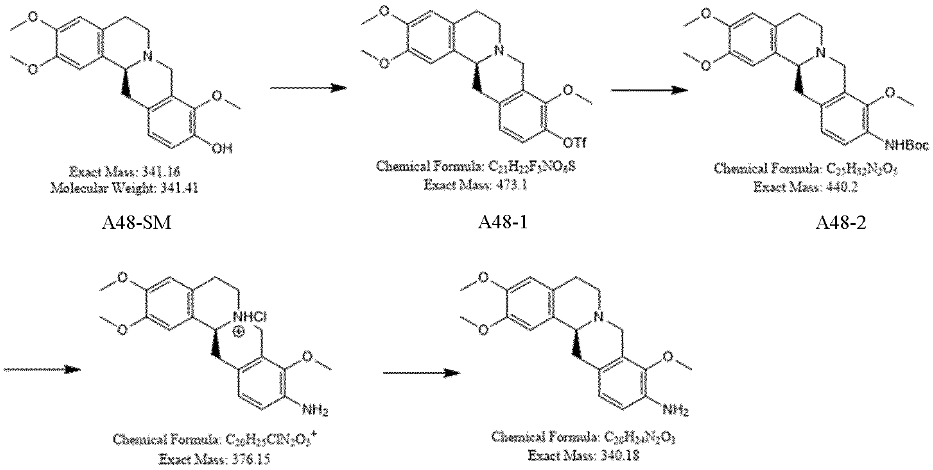


A48-SM A48-1 A48-2

A48-3 A48-0

1. Synthesis of A48-1

Steps: A48-SM (20.0 g, 1.0 eq) and dichloromethane (200 mL) were added into a 500-mL flask. Triethylamine (8.88 g) was added under nitrogen protection, and triflic anhydride (19.8 g) was added dropwise in an ice bath. The reaction mixture was warmed to stir at room temperature overnight. LC/MS detected that the reaction was completed. Water (200 mL) was added into the reaction liquor, and stirred for layer separation. The aqueous phase was extracted with dichloromethane (100 mL). The combined dichloromethane phase was washed with saturated solution chloride (150 mL), dried with anhydrous sodium sulfate, filtered, and concentrated. The sample residue was purified by silica gel column/330 g (PE / EA, 0~30%, 0~40min, gradient elution), to give A48-1 as a yellow solid (26.97 g, yield 94.8%). HPLC purity: 98.93%. MS:474[M+H]. $^1$H NMR (400 MHz, DMSO-$d_6$) $\delta$ 7.30 (d, *J* = 8.5 Hz, 1H), 7.11 (d, *J* = 8.6 Hz, 1H), 6.89 (s, 1H), 6.70 (s, 1H), 4.13 (d, *J* = 16.1 Hz, 1H), 3.83 (s, 3H), 3.74 (d, *J* = 9.4 Hz, 6H), 3.53 (dd, *J* = 16.5, 6.6 Hz, 3H), 3.15 (dd, *J* = 11.1, 3.7 Hz, 1H), 2.99 - 2.86 (m, 1H), 2.63 (dd, *J* = 15.6, 9.6 Hz, 3H).

2. Synthesis of A48-2

Step: A48-1 (20.0 g, 1.0 eq), tert-butyl carbamate, tripotassium phosphate, tri(dibenzylideneacetone) dipalladium(0), 2-(biphenyl) di-tert-butyl phosphine, and 1,4-dioxane (200 mL) were added into a 1L flask. The reaction mixture was heated to 90°C under nitrogen protection, and stirred overnight. LC/MS detected that the reaction was completed. The reaction liquor was cooled to room temperature, and filtered. The filter cake was rinsed with EA (50 mL). The liquor was concentrated to dryness. The residue was purified by silica gel column to give A48-2 as a yellow solid (22.0 g, yield 100%).

3. Synthesis of A48

Step: A48-2 (2.5 g) and dichloromethane (10 mL) were added into a 50-mL flask, and a solution of hydrochloric acid in dioxane (10 mL) was added dropwise. The reaction mixture was stirred at room temperature overnight. LC/MS detected that the reaction was completed. The reaction mixture was concentrated to dryness, and then DCM (20 mL) was added. A saturated sodium carbonate solution (20 mL) was added to adjust the pH to basic. Layers were separated, and the aqueous phase was extracted with DCM (20 mL). The combined DCM phase was washed with saturated sodium chloride (20 mL), dried over anhydrous sodium sulfate, filtered, and concentrated. The sample residue was purified by silica gel column to give A48 as a yellow solid (1.05 g, yield 54.4%).
MS: [M+H] 326.
$^1H$ NMR (400 MHz, DMSO-$d_6$) δ 6.85 (s, 1H), 6.67(s, 1H), 6.65 (d, *J* = 8.0 Hz, 1H), 6.55 (d, *J* = 8.0 Hz, 1H), 4.65 (s, 2H), 4.05 - 4.01 (m, 1H), 3.74 (s, 3H), 3.72 (s, 3H), 3.63 (s, 3H), 3.41 - 3.35 (m, 2H), 3.27 (dd, *J* = 16.0 Hz, 4.0 Hz, 1H), 3.10 (dd, *J* = 12.0 Hz, 4.0 Hz, 1H), 2.97 - 2.89 (m, 1H), 2.60 (d, *J* = 16.0 Hz, 1H), 2.48-2.42 (m, 2H).

Example 30: Synthesis of Compound N12

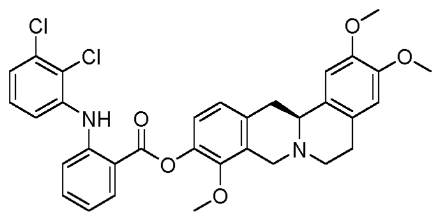

**Synthesis of N12-1**

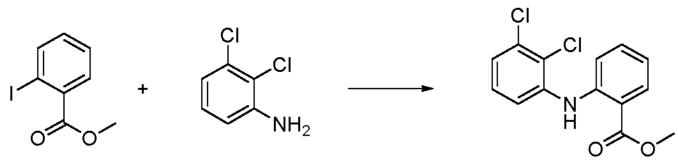

N12-SM1 N12-SM2 N12-1

N12-SM2 (2 g, 1 eq) was added into a single-neck flask, and N12-SM1 (6.47 g, 2 eq), potassium phosphate (8.05 g, 3 eq), BrettPhos Pd G3(2.2g, 0.2eq) were added at room temperature. Dioxane (30 mL) was added into the system. The system was heated to 80°C, and stirred overnight. The reaction was monitored by LCMS. The reaction was subjected to post-treatment. The reaction liquor was cooled to room temperature, quenched by addition of water, extracted with ethyl acetate, washed with saturated sodium chloride solution, dried over anhydrous sodium sulfate, concentrated, and rotation-dried. Preparation was carried at medium pressure to give the product N12-1 (624 mg).
MS: 296, $^1H$ NMR (400 MHz, DMSO-$d_6$) $\delta$ 9.64 (s, 1H), 7.96 (dd, *J* = 8.0, 1.5 Hz, 1H), 7.55 - 7.47 (m, 2H), 7.36 - 7.27 (m, 3H), 6.97 (t, *J* = 7.6 Hz, 1H), 3.88 (s, 3H).

**Synthesis of N12-2**

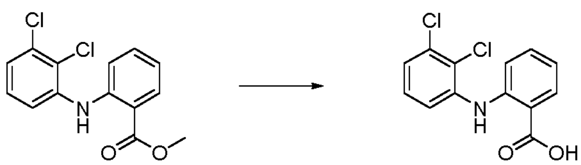

N12-1 N12-2

N12-1 (624 mg, 1.0 eq) was added into MeOH (10 mL), and a solution of sodium hydroxide (140 mg, 1.8 eq) in water (13 mL) was added. The reaction mixture was heated and stirred at 60°C for 1h. LCMS detected that no raw material remained. The reaction mixture was subjected to post-treatment, concentrated, adjusted to pH 3~4, and filtered. The filter

cake was collected, and freeze-dried to give the product (540 mg), which was directly used in the next step. MS: 341.

**Synthesis of Compound N12**

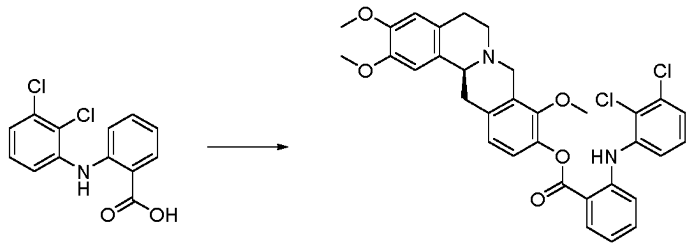

N12-2 N12

N12-2 (200 mg, 1 eq) was added into DCM. After being fully dissolved, A48 (266 mg, 1.1 eq), EDCI (203 mg, 1.5 eq), and DMAP (9 mg, 0.1 eq) were added. The reaction mixture was stirred at room temperature for 1h to give the desired product. The reaction liquor was extracted with ethyl acetate. The organic phase was washed with water and saturated brine, and concentrated to dryness under reduced pressure. The residue was purified by column chromatography to give the product Compound N12 (250 mg).

MS: 605.

$^1$H NMR (400 MHz, DMSO-$d_6$) δ 9.57 (s, 1H), 8.28 (d, *J* = 8.0 Hz, 1H), 7.62 - 7.56 (m, 2H), 7.38-7.32 (m, 3H), 7.14 (d, *J* = 8.0 Hz, 1H), 7.08 - 7.02 (m, 2H), 6.91 (s, 1H), 6.70 (s, 1H), 4.11 (d, *J* = 16.0Hz, 1H), 3.79 - 3.72 (m, 9H), 3.53 - 3.46(m, 3H), 3.14 (dd, *J* = 8.0 Hz, 4.0 Hz, 1H), 2.99 - 2.91 (m, 1H), 2.68 -2.61 (m, 2H), 2.53 -2.48 (m, 1H).

Example 31

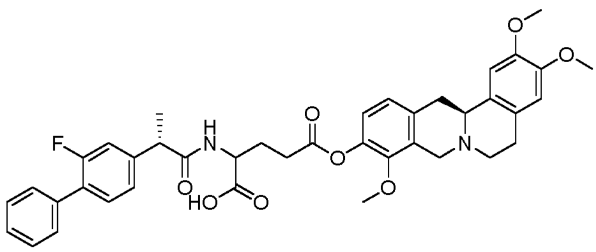

Synthesis of N16-1

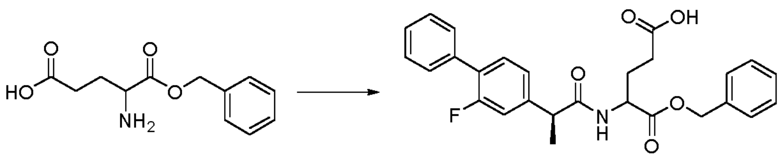

N16-SM N16-1

S-flurbiprofen (400 mg, 1 eq) was added into DMF, and fully dissolved. Then, HATU (700 mg, 1.1 eq) and DIEA (800 mg, 4 eq) were added. The reaction mixture was stirred at 40°C for 1h. N16-SM (400 mg, 1 eq) was added into the reaction system. The reaction mixture was detected by mass spectrometry after being stirred for 2h, and the desired product was obtained. The reaction mixture was extracted with ethyl acetate, back-extracted with water and saturated brine, rotation-dried, and subjected to column chromatography with petroleum ether/ethyl acetate to give the desired product N16-1. MS: 464.

**Synthesis of N16-2**

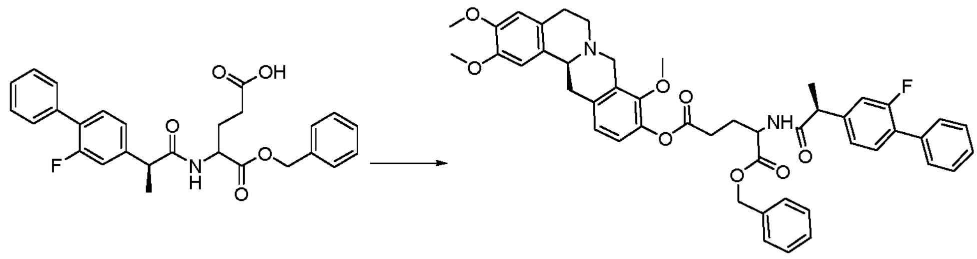

N16-1 N16-2

N16-1 (400 mg, 1 eq), A10-0 (400 mg, 1.3 eq), and DMAP (10 mg, 0.1 eq) were dissolved into DCM, and EDCI (300 mg, 1.6 eq) was added. The reaction mixture was stirred for 2h. The reaction mixture was extracted with dichloromethane, rotation-dried, and purified by column chromatography to give the product N16-2. MS: 787.

**Synthesis of Compound N16**

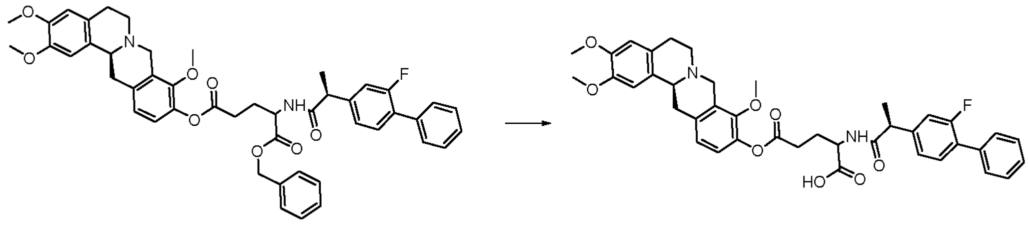

N16-2 N16

N16-2 (400 mg) was dissolved in methanol, and palladium hydroxide on carbon was added. The reaction mixture was stirred for 3h to give the desired product (detected by mass spectrometry). The reaction mixture was filtered and rotation-dried to give the crude product, which was subjected to acid and alkali treatment, and freeze-dried to give Compound N16 (400 mg). MS: 697.

**Synthesis of Compound N17**

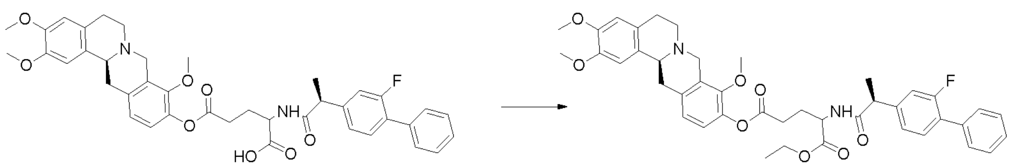

N16 N17

Compound N16 was dissolved in ethanol, and thionyl chloride was added dropwise at 0°C. The reaction mixture was stirred for 30 min to give the desired product (detected by mass spectrometry). The reaction mixture was extracted with ethyl acetate, and rotation-dried. The residue was subjected to column chromatography to give the desired crude product. The crude product was recrystallized from ethanol to give the desired product.

MS: [M+H] 725.

$^1$H NMR (400 MHz, DMSO-$d_6$) $\delta$ 8.57 (t, J = 8.0 Hz, 1H), 7.54 - 7.36 (m, 6H), 7.29 - 7.24 (m, 2H), 6.96 (s, 1H), 6.93 - 6.86 (m, 2H), 6.69 (s, 1H), 4.36 - 4.29 (m, 1H), 4.14 - 4.01 (m, 3H), 3.83 - 3.77 (m, 1H), 3.75 - 3.71 (m, 9H), 3.60 (s, 1H), 3.48 - 3.38 (m, 3H), 3.15 - 3.08 (m, 1H), 2.97 - 2.89 (m, 1H), 2.75 - 2.59 (m, 4H), 2.17 - 2.11 (m, 1H), 1.99 - 1.90 (m, 1H), 1.39 (d, *J* = 8.0 Hz, 3H), 1.14(dt, *J* = 40 Hz, 8.0 Hz, 3H).

Mechanical Pain Test of Inflammatory Pain Model Induced by Complete Freund’s Adjuvant in Mice

1. Test Method

1) The animal mice (C57BL/6J, 8-week-old, male, purchased from Zhejiang Charles River Experimental Animal Technology Co., Ltd.; Certificate Number: 20220617Abzz0619000690; License Number: SCXK (Zhejiang) 2019-0001; Issued by: Zhejiang Charles River Experimental Animal Technology Co., Ltd.; 18-22g) were adaptively reared for one week after purchase, during which the mice were fed ad libitum;
2) In the first 3 days of the test, the mice were placed on a metal pain measuring rack to adapt for 40-60 min, during which the experimenter contacted the animals adaptively (contacting the mice for 3-5 min, Day 1 to Day 3) to reduce the stress reaction of the experimental animals to the experimenters;
3) After the completion of the environmental adaptation (Day 4), the Baseline values of the animals were tested (Up-down testing method), and the animals were randomly divided into 4 groups, including Model Group (Model), Control Group I (Morphine), Control Group II (A10-0, Corydalmine), Test Group (A3) with 4 mice in a group (n = 4);
4) The inflammatory pain model was induced by complete Freund's adjuvant, including the steps of: pipetting 20 μL of the complete Freund's adjuvant (CFA) solution with a sample microinjector, inserting the needle from the toe of one hind paw of the animal, slowly injecting the CFA solution into the subcutaneous position of the palm to form a bulge under the skin, keeping the needle *in situ* for 20s, and slowly withdrawing the needle in rotatory manner to avoid any liquid leakage;
5) The mechanical pain thresholds and the plantar thicknesses of the mice were tested 0.5h, 2h, 8h, and 24h after the first administration (in Control Group I, morphine was subcutaneously administered; and in other groups, the agents were administered by oral gavage at intervals of 8h); wherein the dosage was 2 mg/kg in Control Group I (Morphine), 5 mg/kg in Control Group II (A10-0, Corydalmine), and 8.4 mg/kg in Test Group (A3).

The measurement method of mechanical pain (Up-down testing method) includes the following steps:

a. An experimental animal was placed on a mechanical pain metal net rack and stood for 30 min to 60 min. After the animal stopped looking, exploring, and being quiet, the test began.
b. After adaptation, the middle plantaris of hind paw was stimulated with a fiber.
The experimenter placed the Von Frey fiber perpendicular to the plantar surface, applied a sufficient force to slightly bend the fiber relative to the paw for about 6-8s;
c. The contact should be gentle (no puncture), and the force was slowly applied until the fibers began to bend.
d. The mouse experiment started from 1.0 g, and then the researcher would use a fiber with smaller or larger force according to the up-down method according to the presence or absence of reaction;
e. The first data point after the first inflection point was taken as Reading 1, and the test was stopped after the obtainment of 4 readings. In the mouse experiment, the maximum force applied was 2.0 g, and the minimum force was 0.16 g.
f. If the animal had reactions such as paw lifting, hiding, or paw licking due to stimulation, it was marked as positive (X), except paw withdrawal reaction due to body actions, and if no behavior described above occurred, it was marked as negative (O).
g. Grams of fiber: grams of mouse: 0.16, 0.40, 0.60, 1.0, 1.4, 2.0, with the cut-off value of 2.0 g.

2. Test Results

The above-obtained data were summarized and counted, analyzed by the SPSS data statistics software, and plotted by the Graph Pad software according to the SPSS analytic results. The results are shown in Fig. 1 and Fig. 2.
Fig. 1 is a graph showing the results of mechanical pain threshold in mechanical pain test of inflammatory pain model induced by complete Freund's adjuvant in mice, wherein the values are all Mean ± SEM (mean ± standard error), n=4; *: vs. Group Model, *-P<0.05, **-P<0.01, ***-p<0.001, #: vs. Morphine-2mg/kg, ###-P<0.001.
Fig. 2 is a graph showing the results of plantar thickness in mechanical pain test of inflammatory pain model induced by complete Freund's adjuvant in mice, wherein the values are all Mean ± SEM (mean ± standard error), n = 4; *: vs. Model Group, *-P<0.05.
As seen from Fig. 1 and Fig. 2, the inflammatory pain model induced by Freund's adjuvant (CFA) was successfully established. The positive drug Morphine (with a dose of 2 mg/kg) exhibited a good analgesic effect on the inflammatory pain mode in mice, but the effect cannot be maintained for a long term, and the positive drug Morphine (with a dose of 2 mg/kg) did not have an effect of reducing the sole-swelling degree in mice. Compound A10-0 (with a dose of 5 mg/kg) did not have a significant analgesic effect on the inflammatory pain model in mice, and had an effect of reducing the sole-welling degree in mice, but the effect was maintained for a short time. Compared with the positive drug Morphine,

Compound A10-0 has different effects in terms of analgesic effect and sole-swelling-reducing effect. Compound A3 (with a dose of 8.4 mg/kg) had a strong analgesic effect on the inflammatory pain model in mice, but the effect was maintained for a short term, and Compound A3 (with a dose of 8.4 mg/kg) produced an effect of reducing the sole-swelling degree after several drug treatment. Compared with the positive drug Morphine, Compound A3 has different effects in terms of analgesic effect and sole-swelling-reducing effect.

Analgesia Test in Acetic Acid-induced Writhing Model

1. Test Method

1) The animal mice (CD-1 mice, age: 6-8 weeks, sex: male, body weight: 20-24 g, number: 120, animal supplier: SPF (Beijing) Biotechnology Co., Ltd.) underwent a 7-day quarantine period, during which the routine health examination was conducted by veterinarians. The animals with abnormalities were excluded from the study prior to experimentation.

The animal mice were housed in a SPF-grade laminar flow clean room with constant temperature and constant humidity at the Pharmaron (Beijing) New Drug Technology Co., Ltd. Animal Center (an AAALAC-accredited facility), with 5 mice per cage. Feeding room was maintained at a temperature of 22 ± 3°C and a humidity of 40-70%, with a light radiation of 12-hour light/dark cycle. Cages were made from polycarbonate. Autoclaved clean beddings were made from soft corncob and changed twice weekly. Diet and drinking water: Clean-grade rodent diet (purchased from Beijing Keao Xieli Feed Co., Ltd.). The drinking water was autoclaved, and the food was irradiated with cobalt 60 ray. The animals were free to ingest sterile food and drinking water.

Each cage was provided with a cage label, indicating the animal number, sex, line, receipt time, grouping, and start time of experiment. Animal number: Each animal was marked with a unique animal number at its tail.

The animals were randomly divided to groups according to their weight, and the specific groups and administration modes were listed in Table 1 below, wherein fentanyl, an existing drug commonly used for analgesia, was used as positive control in the present disclosure.

Table 1. Grouping and Administration Modes of Animals

| Grouping | Animal Number | Treatment Drug | Number of Administration Times | Administration Dose (mg/kg) | Acetic Acid-induced Writhing Model | Administration Route |
|---|---|---|---|---|---|---|
| 1 | 5 | Vehicle | Single | N/A | Y | Oral Gavage |
| 2 | 5 | Fentanyl | Single | 0.008 | Y | Intravenous |
| 3 | 5 | **A10-0** | Single | 18.0 | Y | Oral Gavage |
| 4 | 5 | **A3** | Single | 30.0 | Y | Oral Gavage |
| 5 | 5 | **A10** | Single | 30.1 | Y | Oral Gavage |
| 6 | 5 | **A14** | Single | 30.6 | Y | Oral Gavage |
| 7 | 5 | **A15** | Single | 20.6 | Y | Oral Gavage |
| 8 | 5 | **A17** | Single | 18.7 | Y | Oral Gavage |
| 9 | 5 | **A18** | Single | 32.3 | Y | Oral Gavage |
| 10 | 5 | **A21** | Single | 31.8 | Y | Oral Gavage |
| 11 | 5 | **A22** | Single | 22.3 | Y | Oral Gavage |
| 12 | 5 | **A7** | Single | 40.7 | Y | Oral Gavage |
| 13 | 5 | **A19** | Single | 18.4 | Y | Oral Gavage |
| 14 | 5 | **A23** | Single | 28.4 | Y | Oral Gavage |
| 15 | 5 | **A44** | Single | 32.0 | Y | Oral Gavage |
| 16 | 5 | **A24** | Single | 25.5 | Y | Oral Gavage |

(continued)

| Grouping | Animal Number | Treatment Drug | Number of Administration Times | Administration Dose (mg/kg) | Acetic Acid-induced Writhing Model | Administration Route |
|---|---|---|---|---|---|---|
| 17 | 5 | **A53** | Single | 26.9 | Y | Oral Gavage |
| 18 | 5 | **A40** | Single | 34.5 | Y | Oral Gavage |
| 19 | 5 | **A55** | Single | 40.5 | Y | Oral Gavage |
| 20 | 5 | **A57** | Single | 26.7 | Y | Oral Gavage |
| 21 | 5 | **A4** | Single | 30.0 | Y | Oral Gavage |

Prior to the administration, the mice were fasted for 16 hours. According to Table 1, the administration modes in various groups were as follows: Positive drug fentanyl: intravenous injection; other drug or blank groups: oral gavage; administration volume: 10 mL/kg; number of administration times: single.

2) All the mice were weighed and recorded in detail before the administration. Various groups were administered in accordance with the administration modes listed in Table 1. After the administration, various groups were injected with 0.6% glacial acetic acid, wherein Group 2 was injected 10 min after the administration, and other groups were injected 2h after the administration. 0.6% glacial acetic acid was injected into the abdominal cavity of mice and deposited in the visceral and parietal peritoneum, causing inflammatory pain in a large area for a long term in deep, so that the mice exhibited behavior reactions including abdominal depression, trunk and hind limb stretching, and hip rising, called writhing reaction. The writhing number of mice were taken as the index of pain reaction, evaluating the analgesic effect of the compound. The writhing reaction occurred more frequently within 30 min after the injection of 0.6% glacial acetic acid. The number of "writhing reactions" occurred were observed and recorded within 5-15 min and 15-30 min after the injection of 0.6% glacial acetic acid.

2. Test Results

The writhing test results of mice in various groups were represented as "mean ± standard error". The data of various groups were subjected to data statistics using SPSS16.0 software package with the statistical method of One-way ANOVA. The groups were compared to determine if there was a statistical difference therebetween, wherein $P<0.05$ indicated a statistical difference. The recorded results of the writhing start time of the mice in various groups were shown in Fig. 3, and the writhing test results of the mice in various groups were shown in Table 2. The total writhing numbers of mice in various groups were shown in Table 2 and Fig. 4.

Table 2. Writhing Test Results of Mice in Various Groups

| Group | Weight (g) | Time to start writhing (s) | Number of Writhing | | |
|---|---|---|---|---|---|
| | | | 5-15 min | 15-30 min | Total |
| Blank | 22.4±0.5 | 241±95 | 17±15 | 20±14 | 37±26 |
| Fentanyl | 22.6±0.5 | 470±494 | 4±5* | 3±2 | 8±6* |
| **A10-0** | 22.5±0.3 | 545±221** | 4±3 * | 5±3 | 9±2* |
| **A3** | 22.6±0.4 | 351±227 | 4±7* | 2±5 | 6±12* |
| **A10** | 22.6±0.3 | 421±383 | 5±8* | 6±8 | 11±17 |
| **A14** | 22.1±1.0 | 421±210* | 8±5 | 7±6 | 14±9 |
| **A15** | 22.1±0.6 | 377±222 | 8±10 | 8±10 | 16±18 |
| **A17** | 22.2±1.1 | 420±134** | 7±5 | 6±4 | 13±8 |
| **A18** | 22.4±0.8 | 275±97 | 16±8 | 15±10 | 31±15 |
| **A21** | 22.9±1.2 | 481±192** | 10±7 | 10±8 | 20±15 |
| **A22** | 21.9±0.7 | 435±296* | 3±3* | 4±3 | 7±5 * |
| **A7** | 22.1±2.1 | 319±91 | 14±10 | 11±7 | 25±15 |

(continued)

| Group | Weight (g) | Time to start writhing (s) | Number of Writhing | | |
|---|---|---|---|---|---|
| | | | 5-15 min | 15-30 min | Total |
| **A19** | 22.0±0.4 | 694±486** | 8±7 | 6±7 | 14±14 |
| **A23** | 22.5±1.4 | 465±161** | 6±5* | 6±5 | 12±9 |
| **A44** | 21.8±1.9 | 275±66 | 12±10 | 10±10 | 23±19 |
| **A24** | 22.0±0.8 | 493±484 | 15±15 | 13±10 | 28±24 |
| **A53** | 22.3±1.0 | 442±238* | 6±5* | 13±14 | 19±17 |
| **A40** | 22.9±1.3 | 310±210 | 5±6* | 5±3 | 10±9* |
| **A55** | 22.3±0.4 | 294±223 | 5±7* | 3±7 | 8±13* |
| **A57** | 22.7±1.1 | 716±538 | 9±9 | 8±8 | 17±17 |
| **A4** | 22.6±0.8 | 154±151 | 14±14 | 12±14 | 26±27 |

As seen according to the results in Table 2, the mice to which the compounds of the present disclosure were administrated had less writhing number than that of the blank group, indicating that the compounds of the present disclosure have good analgesic effect.

**Detection of Activity**

The animal mice (CD-1 mice, age: 6-8 weeks, sex: male, body weight: 20-24 g, number: 120, animal supplier: SPF (Beijing) Biotechnology Co., Ltd.) underwent a 7-day quarantine period, during which the routine health examination was conducted by veterinarians. The animals with abnormalities were excluded from the study prior to experimentation.

The animal mice were housed in a clean room, with 5 mice per cage. Feeding room was maintained at a temperature of 22 ± 3°C and a humidity of 40-70%, with a light radiation of 12-hour light/dark cycle. Cages were made from polycarbonate. Autoclaved clean beddings were made from soft corncob and changed twice weekly. Diet and drinking water: Clean-grade rodent diet (purchased from Beijing Keao Xieli Feed Co., Ltd.). The drinking water was autoclaved, and the food was irradiated with cobalt 60 ray. The animals were free to ingest sterile food and drinking water. All the mice were weighed and recorded in detail before the administration.

Before the administration, the mice were fasted for 16h. The administration was conducted by intravenous injection with an administration volume of 10 mL/kg and a dose of 10 mpk. 15 min after the administration, 0.6% glacial acetic acid was injected into the abdominal cavity of mice and deposited in the visceral and parietal peritoneum, causing inflammatory pain in a large area for a long term in deep, so that the mice exhibited behavior reactions including abdominal depression, trunk and hind limb stretching, and hip rising, called writhing reaction. The writhing number occurred in mice was taken as the index of pain reaction to evaluate the analgesic effect of the compound. The number of "writhing reactions" occurred within 20 min after the injection of 0.6% glacial acetic acid and the number of "writhing reactions" in the control group without administration were observed, and a ratio thereof was calculated. The ratio was subtracted from 100% to give a value which was recorded as the inhibition rate.

Table 3

| | Inhibition Rate (%) |
|---|---|
| Compound N16 | 55.66 |
| Compound N17 | 60.33 |

Various examples in this specification were described in related ways, and the same and similar parts between the examples can be referred to each other, wherein each example was focused on the differences from other examples.

The above are only the alternative examples of the present disclosure, and are not intended to limit the protection scope of the present disclosure. Any modification, equivalent substitution, improvement, etc. made within the spirit and principle of the disclosure are encompassed in the protection scope of the disclosure.

## Claims

**1.** A compound of Formula A, or a pharmaceutically acceptable salt, ester, stereoisomer, polymorph, solvate, N-oxide, isotope-labeled compound, metabolite, or pro-drug thereof, and a mixture thereof:

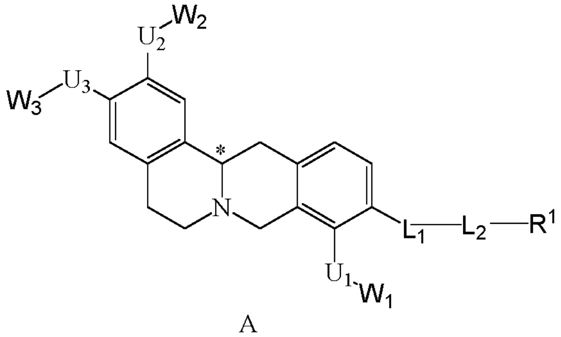

A

wherein

* represents a chiral center, and is selected from (S)-configuration or (R)-configuration;
$L_1$ is selected from a single bond, -O-, -NH-, -S- or -P(O)OH-; alternatively a single bond, -O-, -NH-;
$L_2$ is selected from a single bond, -O-, -NH-, -C(O)-, -C(O)-O-, -C(O)NH-, -OC(O)-,
$C_{1-18}$ alkylene (alternatively $C_{1-12}$ alkylene, and yet alternatively $C_{1-6}$ alkylene),
$C_{3-10}$ cycloalkylene (alternatively $C_{3-8}$ cycloalkylene, and yet alternatively $C_{3-6}$ cycloalkylene),
$C_{6-30}$ arylene (alternatively $C_{6-18}$ arylene, and yet alternatively $C_{6-12}$ arylene),
$C_{3-30}$ heteroarylene (alternatively $C_{3-18}$ heteroarylene, and yet alternatively $C_{3-12}$ heteroarylene),
-$C_{1-18}$ alkylene-O- (alternatively -$C_{1-12}$ alkylene-O-, and yet alternatively -$C_{1-6}$ alkylene-O-),
-$C_{6-30}$ arylene-O- (alternatively -$C_{6-18}$ arylene-O-, and yet alternatively -$C_{6-12}$ arylene-O-),
-$C_{3-30}$ heteroarylene-O- (alternatively -$C_{3-18}$ heteroarylene-O-, and yet alternatively -$C_{3-12}$ heteroarylene-O-),
-C(O)-$C_{1-18}$ alkylene-,
-C(O)-$C_{1-18}$ alkylene-NHC(O)-$C_{1-18}$ alkylene-,
-C(O)-$C_{1-18}$ alkylene-C(O)NH-$C_{1-18}$ alkylene-,
$C_{1-30}$ alkyleneoxy, or
-$X_1$-PRa(O)-$X_2$-; the Ra is each independently selected from hydrogen, hydroxyl, halogen (alternatively F, Cl or Br), $C_{6-30}$ aryl (alternatively $C_{6-18}$ aryl, and yet alternatively $C_{6-12}$ aryl), $C_{3-30}$ heteroaryl (alternatively $C_{3-18}$ heteroaryl, and yet alternatively $C_{3-12}$ heteroaryl), $C_{1-30}$ hydrocarbon radical (alternatively $C_{1-30}$ alkyl, and yet alternatively $C_{1-18}$ alkyl, and further alternatively $C_{1-6}$ alkyl), $C_{3-10}$ cycloalkyl (alternatively $C_{3-8}$ cycloalkyl, and yet alternatively $C_{3-6}$ cycloalkyl), $C_{1-30}$ alkoxy (alternatively $C_{1-18}$ alkoxy, and yet alternatively $C_{1-6}$ alkoxy), $C_{6-30}$ aryl-O- (alternatively $C_{6-18}$ aryl-O-, and yet alternatively $C_{6-12}$ aryl-O-), $C_{3-30}$ heteroaryl-O- (alternatively $C_{3-18}$ heteroaryl-O-, and yet alternatively $C_{3-12}$ heteroaryl-O-), $C_{6-30}$ aryl-$C_{1-18}$ alkylene-O- (alternatively $C_{6-18}$ aryl-$C_{1-12}$ alkylene-O-, and yet alternatively $C_{6-12}$ aryl-$C_{1-6}$ alkylene-O-), $C_{3-30}$ heteroaryl-$C_{1-18}$ alkylene-O- (alternatively $C_{3-18}$ heteroaryl-$C_{1-12}$ alkylene-O-, and yet alternatively $C_{3-12}$ heteroaryl-$C_{1-6}$ alkylene-O-), -O-$C_{6-30}$ aryl-$C_{1-18}$ alkylene- (alternatively -O-$C_{6-18}$ aryl-$C_{1-12}$ alkylene-) or -O-$C_{3-30}$ heteroaryl-$C_{1-18}$ alkylene- (alternatively -O-$C_{3-18}$ heteroaryl-$C_{1-12}$ alkylene-); the Ra and $X_2$ may be connected to form a ring;
the $X_1$ is selected from a single bond, -O-, -$C_{1-18}$ alkylene-O- (alternatively -$C_{1-12}$ alkylene-O-, and yet alternatively -$C_{1-6}$ alkylene-O-), -$C_{6-30}$ arylene-O- (alternatively -$C_{6-18}$ arylene-O-, and yet alternatively -$C_{6-12}$ arylene-O-), or -$C_{3-30}$ heteroarylene-O- (alternatively -$C_{3-18}$ heteroarylene-O-, and yet alternatively -$C_{3-12}$ heteroarylene-O-);
the $X_2$ is selected from a single bond, -O- or -NR*-; wherein R* is selected from H, $C_{1-6}$ alkyl, and $C_{1-6}$ alkoxy;
the $L_2$ is optionally substituted with 1, 2, 3, 4, or 5 substituents selected from halogen, OH, CN, C(O)OH, C(O)O-$C_{1-6}$ alkyl, $C_{1-6}$ alkyl, $C_{1-6}$ haloalkyl, $C_{1-6}$ alkoxy, or $C_{1-6}$ haloalkoxy;
$R^1$ is selected from absent or hydrogen, hydroxyl, amino unsubstituted or substituted with 1 or 2 Rb, unsubstituted or Rb-substituted $C_{1-30}$ hydrocarbon radical (alternatively $C_{1-30}$ alkyl or $C_{2-30}$ alkenyl, yet alternatively $C_{1-18}$ alkyl or $C_{2-18}$ alkenyl, further alternatively $C_{1-6}$ alkyl or $C_{2-12}$ alkenyl, wherein the alkenyl has a double-bond number of 1-10, alternatively 1-6), $C_{3-10}$ cycloalkyl (alternatively $C_{3-8}$ cycloalkyl, yet alternatively $C_{3-6}$ cycloalkyl) unsubstituted or substituted with 1, 2, 3, 4 or 5 Rb, unsubstituted or Rb-substituted $C_{1-30}$ alkoxy (alternatively $C_{1-20}$ alkoxy, yet alternatively $C_{1-12}$ alkoxy), $C_{12-30}$ condensed cyclic group (alternatively $C_{12-17}$ condensed cyclic group, yet alternatively $C_{12-17}$ condensed cyclic group containing at least one heteroatom, further alternatively

$C_{12-17}$ tetracondensed cyclic group containing at least one heteroatom) unsubstituted or substituted with 1, 2, 3, 4 or 5 Rb, $C_{1-18}$ alkyl-$C_{6-30}$ arylene-$C_{1-18}$ alkylene- (alternatively $C_{1-12}$ alkyl-$C_{6-18}$ arylene-$C_{1-12}$ alkylene-) which is unsubstituted or has a hydrogen atom replaced with Rc in the alkyl, $C_{1-12}$ alkyl-O-C(O)-$C_{1-12}$ alkylene-imino-, which is unsubstituted or has a hydrogen atom replaced with Rd in the alkyl, $C_{6-30}$ aryl-$C_{1-18}$ alkylene- (alternatively $C_{6-18}$ aryl-$C_{1-12}$ alkylene-), -O-C(O)-$Y_1$, -$C_{1-12}$ alkylene-C(O)-O- $Y_2$ which is unsubstituted or has a hydrogen atom replaced with Rd in the alkylene, -O-$C_{1-12}$ alkylene-O-C(O)-$Y_3$ which is unsubstituted or has a hydrogen atom replaced with Rd in the alkylene, -C=CH-$Y_4$ or -$Y_5$-$Y_6$-$Y_7$, -CH$R^{\#}R^{\#\#}$, -Z-$C_{6-30}$ aryl (alternatively -Z-$C_{6-18}$ aryl, yet alternatively -Z-$C_{6-12}$ aryl, e.g., -Z-phenyl) optionally substituted with *x* A; wherein $R^{\#}$ and $R^{\#\#}$ are each independently selected from H, hydroxyl, amino, halogen, alkyl, cycloalkyl, aryl, heteroaryl, -C(O)-O$R^2$, -OC(O)-O$R^2$, -$C_{1-18}$ alkylene-C(O)-O$R^2$, -$C_{1-18}$ alkylene-$R^2$, or -$C_{6-10}$ aryl-$C_{1-6}$ alkyl;

$R^2$ is selected from H, hydroxyl, amino, halogen, alkyl, cycloalkyl, aryl, heteroaryl, unsubstituted or Rb-substituted $C_{12-30}$ condensed cyclic group, or -NHC(NH)NH-;

the Rb is each independently selected from $C_{1-30}$ alkoxy (alternatively $C_{1-20}$ alkoxy, yet alternatively $C_{1-12}$ alkoxy, and further alternatively $C_{1-3}$ alkoxy), $C_{6-30}$ aryl (alternatively $C_{6-18}$ aryl, yet alternatively $C_{6-12}$ aryl), $C_{3-30}$ heteroaryl (alternatively $C_{3-18}$ heteroaryl, yet alternatively $C_{3-12}$ heteroaryl), or $C_{1-30}$ alkyl (alternatively $C_{1-18}$ alkyl, yet alternatively $C_{1-6}$ alkyl), which are unsubstituted or substituted with halogen (alternatively F, Cl, or Br);

the Rc is each independently selected from $C_{3-10}$ cycloalkyl (alternatively $C_{3-8}$ cycloalkyl, and yet alternatively $C_{3-6}$ cycloalkyl, and further alternatively cyclopentyl), which is unsubstituted or substituted with hydroxyl, oxo, or thioxo;

the Rd is each independently selected from $C_{1-18}$ alkyl (alternatively $C_{1-12}$ alkyl, and yet alternatively $C_{1-6}$ alkyl), $C_{1-30}$ alkoxy (alternatively $C_{1-20}$ alkoxy, and yet alternatively $C_{1-12}$ alkoxy, and further alternatively $C_{1-3}$ alkoxy), $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with halogen (alternatively F, Cl or Br);

the $Y_1$ is selected from $C_{1-30}$ alkyl (alternatively $C_{1-12}$ alkyl, and yet alternatively $C_{1-6}$ alkyl), $C_{3-10}$ cycloalkyl (alternatively $C_{3-8}$ cycloalkyl, and yet alternatively $C_{3-6}$ cycloalkyl), $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with Re; the Re is each independently selected from $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with halogen (alternatively F, Cl or Br);

the $Y_2$ is selected from unsubstituted or Rf-substituted $C_{12-30}$ condensed cyclic group (alternatively $C_{12-17}$ condensed cyclic group, and yet alternatively $C_{12-17}$ condensed cyclic group containing at least one heteroatom, and further alternatively $C_{12-17}$ tetracondensed cyclic group containing at least one heteroatom); wherein the Rf is each independently selected from $C_{1-30}$ alkoxy (alternatively $C_{1-20}$ alkoxy, and yet alternatively $C_{1-12}$ alkoxy, and further alternatively $C_{1-3}$ alkoxy), or $C_{6-30}$ aryl (alternatively $C_{6-18}$ aryl, and yet alternatively $C_{6-12}$ aryl), which are unsubstituted or substituted with halogen (alternatively F, Cl or Br);

the $Y_3$ is selected from $C_{1-18}$ alkyl (alternatively $C_{1-12}$ alkyl, and yet alternatively $C_{1-6}$ alkyl) or -O-$C_{1-18}$ alkyl (alternatively -O-$C_{1-12}$ alkyl, and yet alternatively -O-$C_{1-6}$ alkyl);

the $Y_4$ is selected from $C_{6-30}$ aryl (alternatively $C_{6-18}$ aryl, and yet alternatively $C_{6-12}$ aryl) or $C_{3-30}$ heteroaryl (alternatively $C_{3-18}$ heteroaryl, and yet alternatively $C_{3-12}$ heteroaryl), which are unsubstituted or substituted with Rg, wherein the Rg is each independently selected from hydroxyl and $C_{1-30}$ alkoxy (alternatively $C_{1-20}$ alkoxy, and yet alternatively $C_{1-12}$ alkoxy, and further alternatively $C_{1-3}$ alkoxy);

the $Y_5$ is selected from a single bond, unsubstituted or amino-substituted $C_{1-18}$ alkylene (alternatively $C_{1-12}$ alkylene, and yet alternatively $C_{3-6}$ alkylene);

the $Y_6$ is selected from a single bond, -NH-C(NH)-, $-(O-CH_2-CH_2)_m-$ or $-CH_2-(O-CH_2-CH_2)_n-$, and the *m* and the *n* are each independently selected from any natural number of 2-20 (alternatively any natural number of 2-10);

the $Y_7$ is selected from amino, $C_{1-30}$ alkoxy (alternatively $C_{1-20}$ alkoxy, and yet alternatively $C_{1-12}$ alkoxy, and further alternatively $C_{1-3}$ alkoxy) or hydroxyl;

*x* is selected from 0, 1, 2, 3, 4, or 5;

Z is selected from a chemical bond, -O-, -NH-, -$C_{1-12}$ alkylene-, -$C_{1-12}$ alkylene-O-$C_{1-12}$ alkylene-, -$C_{1-12}$ alkylene-NH-$C_{1-12}$ alkylene-, -$C_{1-18}$ alkylene-C(O)-$C_{1-12}$ alkylene-, -$C_{1-10}$ alkylene-C(O)O-$C_{1-10}$ alkylene-, -$C_{1-10}$ alkylene-NHC(O)-$C_{1-10}$ alkylene-, -$C_{1-10}$ alkylene-NHC(O)O-$C_{1-10}$ alkylene-, -$C_{1-10}$ alkylene-C(O)NH-$C_{1-10}$ alkylene- or -$C_{1-10}$ alkylene-OC(O)NH-$C_{1-10}$ alkylene-, alternatively a chemical bond or -$C_{1-6}$ alkylene-NHC(O)-$C_{1-6}$ alkylene-;

the Z is optionally substituted with 1, 2, 3, 4, or 5 substituents selected from halogen, -C(O)OH, -C(O)O-$C_{1-6}$ alkyl, -C(O)-$C_{1-6}$ alkyl, -O-$C_{1-6}$ alkyl, $C_{1-6}$ alkyl, or $C_{1-6}$haloalkyl;

A is independently selected from H, halogen, OH, -$L_A$-$C_{3-10}$ cycloalkyl, -$L_A$-3- to 10-membered heterocyclyl, -$L_A$-$C_{6-12}$ aryl or -$L_A$-5- to 12-membered heteroaryl, alternatively halogen, $C_{6-10}$ aryl or -NH-$C_{6-10}$ aryl;

$L_A$ is selected from a chemical bond, -NH-, -O-, -C(O)-, -C(O)O-, -NHC(O)-, -NHC(O)O-, -OC(O)NH- or - C(O) NH-;
the A is optionally further substituted with halogen, $C_{1-6}$ alkyl or $C_{1-6}$ haloalkyl;
when the $L_1$ and the $L_2$ are both selected from a single bond, the $R^1$ is not hydroxyl;
$U_1$, $U_2$ and $U_3$ are each independently selected from -O-C(O)-, -NH-C(O)-, -O-$CH_2$-O-, and -O-; or an available ring atom on $U_2$ and an available ring atom on $U_3$ are connected via Q group to form a 4- to 8-membered ring, alternatively a 5- to 6-membered ring,
Q is selected from a single bond; $NR^3$; $C_{1-3}$ alkylene in which 1 or 2 $CH_2$ are optionally independently replaced with a group selected from O, S and $NR^3$; and $C_{2-3}$ alkenylene in which any CH for forming the C=C double bond is optionally replaced with N;
$W_1$, $W_2$ and $W_3$ are each independently selected from $C_{1-18}$ alkyl (alternatively $C_{1-12}$ alkyl, and yet alternatively $C_{1-6}$ alkyl) or -(O-$C_{2-6}$ alkylene), (alternatively -(O-$C_{2-4}$ alkylene)$_p$, and yet alternatively -(O-$CH_2$-$CH_2$)$_p$), wherein the *p* is selected from any natural number of 1-18 alternatively any natural number of 1-10;
the above alkyl, alkylene, alkenyl, alkenylene, alkynyl, cyclic hydrocarbon radical, heterocyclyl, aryl, heteroaryl, and aralkyl at each occurrence are each optionally substituted with 1, 2, 3 or more $R^3$, wherein the $R^3$ at each occurrence is independently selected from halogen, cyano, nitro, $C_{1-6}$ alkyl, $C_{3-10}$ cyclic hydrocarbon radical, 3- to 10-membered heterocyclyl, $C_{6-10}$ aryl, 5- to 14-membered heteroaryl, and $C_{6-12}$ aralkyl; and wherein the alkyl, alkylene, cyclic hydrocarbon radical, heterocyclyl, aryl, heteroaryl, and aralkyl in relation to the substituent $R^3$ are further optionally substituted with 1, 2, 3 or more substituents independently selected from halogen, OH, oxo, amino, cyano, nitro, $C_{1-6}$ alkyl, $C_{1-6}$ haloalkyl, $C_{3-6}$ cyclic hydrocarbon radical, 3- to 10-membered heterocyclyl, $C_{6-10}$ aryl, 5- to 14-membered heteroaryl, and $C_{6-12}$ aralkyl.

**2.** A compound of Formula I, or a pharmaceutically acceptable salt, ester, stereoisomer, polymorph, solvate, N-oxide, isotope-labeled compound, metabolite, or pro-drug thereof, and a mixture thereof:

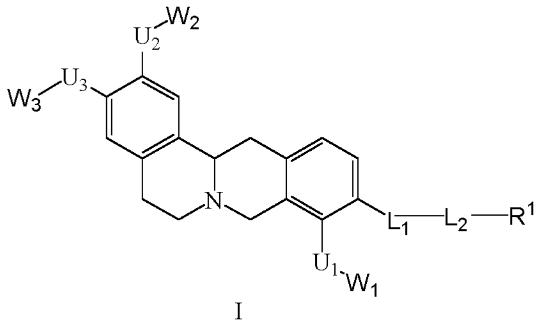

I

wherein

$L_1$ is selected from a single bond, -O-, -NH-, -S- or -P(O)OH-; alternatively a single bond, -O-, -NH-;
$L_2$ is selected from a single bond, -O-, -NH-, -C(O)-, -C(O)-O-,
$C_{1-18}$ alkylene (alternatively $C_{1-12}$ alkylene, and yet alternatively $C_{1-6}$ alkylene),
$C_{3-10}$ cycloalkylene (alternatively $C_{3-8}$ cycloalkylene, and yet alternatively $C_{3-6}$ cycloalkylene),
$C_{6-30}$ arylene (alternatively $C_{6-18}$ arylene, and yet alternatively $C_{6-12}$ arylene),
$C_{3-30}$ heteroarylene (alternatively $C_{3-18}$ heteroarylene, and yet alternatively $C_{3-12}$ heteroarylene),
-$C_{1-18}$ alkylene-O- (alternatively -$C_{1-12}$ alkylene-O-, and yet alternatively -$C_{1-6}$ alkylene-O-),
-$C_{6-30}$ arylene-O- (alternatively -$C_{6-18}$ arylene-O-, and yet alternatively -$C_{6-12}$ arylene-O-),
-$C_{3-30}$ heteroarylene-O- (alternatively -$C_{3-18}$ heteroarylene-O-, and yet alternatively -$C_{3-12}$ heteroarylene-O-),
-C(O)-$C_{1-18}$ alkyl-,
$C_{1-30}$ alkoxy, or
-$X_1$-PRa(O)-$X_2$-; the Ra is each independently selected from hydrogen, hydroxyl, halogen (alternatively F, Cl or Br), $C_{6-30}$ aryl (alternatively $C_{6-18}$ aryl, and yet alternatively $C_{6-12}$ aryl), $C_{3-30}$ heteroaryl (alternatively $C_{3-18}$ heteroaryl, and yet alternatively $C_{3-12}$ heteroaryl), $C_{1-30}$ hydrocarbon radical (alternatively $C_{1-30}$ alkyl, and yet alternatively $C_{1-18}$ alkyl, and further alternatively $C_{1-6}$ alkyl), $C_{3-10}$ cycloalkyl (alternatively $C_{3-8}$ cycloalkyl, and yet alternatively $C_{3-6}$ cycloalkyl), $C_{1-30}$ alkoxy (alternatively $C_{1-18}$ alkoxy, and yet alternatively $C_{1-6}$ alkoxy), $C_{6-30}$ aryl-O- (alternatively $C_{6-18}$ aryl-O-, and yet alternatively $C_{6-12}$ aryl-O-), $C_{3-30}$ heteroaryl-O- (alternatively $C_{3-18}$ heteroaryl-O-, and yet alternatively $C_{3-12}$ heteroaryl-O-), $C_{6-30}$ aryl-$C_{1-18}$ alkylene-O- (alternatively $C_{6-18}$ aryl-$C_{1-12}$ alkylene-O-, and yet alternatively $C_{6-12}$ aryl-$C_{1-6}$ alkylene-O-), $C_{3-30}$ heteroaryl-$C_{1-18}$ alkylene-O- (alter-

natively $C_{3-18}$ heteroaryl-$C_{1-12}$ alkylene-O-, and yet alternatively $C_{3-12}$ heteroaryl-$C_{1-6}$ alkylene-O-), -O-$C_{6-30}$ aryl-$C_{1-18}$ alkylene- (alternatively -O-$C_{6-18}$ aryl-$C_{1-12}$ alkylene-) or -O-$C_{3-30}$ heteroaryl-$C_{1-18}$ alkylene- (alternatively -O-$C_{3-18}$ heteroaryl-$C_{1-12}$ alkylene-); the Ra and $X_2$ may be connected to form a ring;

the $X_1$ is selected from a single bond, -O-, -$C_{1-18}$ alkylene-O- (alternatively -$C_{1-12}$ alkylene-O-, and yet alternatively -$C_{1-6}$ alkylene-O-), -$C_{6-30}$ arylene-O- (alternatively -$C_{6-18}$ arylene-O-, and yet alternatively -$C_{6-12}$ arylene-O-), or -$C_{3-30}$ heteroarylene-O- (alternatively -$C_{3-18}$ heteroarylene-O-, and yet alternatively -$C_{3-12}$ heteroarylene-O-);

the $X_2$ is selected from a single bond, -O- or -NR*-; wherein R* is selected from H, $C_{1-6}$ alkyl, and $C_{1-6}$ alkoxy;

$R^1$ is selected from absent or hydrogen, hydroxyl, unsubstituted or Rb-substituted amino, unsubstituted or Rb-substituted $C_{1-30}$ hydrocarbon radical (alternatively $C_{1-30}$ alkyl or $C_{2-30}$ alkenyl, and yet alternatively $C_{1-18}$ alkyl or $C_{2-18}$ alkenyl, and further alternatively $C_{1-6}$ alkyl or $C_{2-12}$ alkenyl, wherein the alkenyl has a double-bond number of 1-10, alternatively 1-6), unsubstituted or Rb-substituted $C_{3-10}$ cycloalkyl (alternatively $C_{3-8}$ cycloalkyl, and yet alternatively $C_{3-6}$ cycloalkyl), unsubstituted or Rb-substituted $C_{1-30}$ alkoxy (alternatively $C_{1-20}$ alkoxy, and yet alternatively $C_{1-12}$ alkoxy), unsubstituted or Rb-substituted $C_{12-30}$ condensed cyclic group (alternatively $C_{12-17}$ condensed cyclic group, and yet alternatively $C_{12-17}$ condensed cyclic group containing at least one heteroatom, and further alternatively $C_{12-17}$ tetracondensed cyclic group containing at least one heteroatom), $C_{1-18}$ alkyl-$C_{6-30}$ arylene-$C_{1-18}$ alkylene- (alternatively $C_{1-12}$ alkyl-$C_{6-18}$ arylene-$C_{1-12}$ alkylene-) which is unsubstituted or has a hydrogen atom replaced with Rc in the alkyl, $C_{1-12}$ alkyl-O-C(O)-$C_{1-12}$ alkylene-imino-, which is unsubstituted or has a hydrogen atom replaced with Rd in the alkyl, $C_{6-30}$ aryl-$C_{1-18}$ alkylene- (alternatively $C_{6-18}$ aryl-$C_{1-12}$ alkylene-), -O-C(O)-$Y_1$, -$C_{1-12}$ alkylene-C(O)-O-$Y_2$ which is unsubstituted or has a hydrogen atom replaced with Rd in the alkylene, -O-$C_{1-12}$ alkylene-O-C(O)-$Y_3$ which is unsubstituted or has a hydrogen atom replaced with Rd in the alkylene, -C=CH-$Y_4$, -$Y_5$-$Y_6$-$Y_7$, or -$CHR^{\#}R^{\#\#}$;

wherein $R^{\#}$ and $R^{\#\#}$ are each independently selected from H, hydroxyl, amino, halogen, alkyl, cycloalkyl, aryl, heteroaryl,

-C(O)-$OR^2$, -OC(O)-$OR^2$, -$C_{1-18}$ alkylene-C(O)-$OR^2$, -$C_{1-18}$ alkylene-$R^2$, or -$C_{6-10}$ aryl-$C_{1-6}$ alkyl;

$R^2$ is selected from H, hydroxyl, amino, halogen, alkyl, cycloalkyl, aryl, heteroaryl, unsubstituted or Rb-substituted $C_{12-30}$ condensed cyclic group, or -NHC(NH)NH-;

the Rb is each independently selected from $C_{1-30}$ alkoxy (alternatively $C_{1-20}$ alkoxy, yet alternatively $C_{1-12}$ alkoxy, and further alternatively $C_{1-3}$ alkoxy), $C_{6-30}$ aryl (alternatively $C_{6-18}$ aryl, yet alternatively $C_{6-12}$ aryl), $C_{3-30}$ heteroaryl (alternatively $C_{3-18}$ heteroaryl, yet alternatively $C_{3-12}$ heteroaryl), or $C_{1-30}$ alkyl (alternatively $C_{1-18}$ alkyl, yet alternatively $C_{1-6}$ alkyl), which are unsubstituted or substituted with halogen (alternatively F, Cl, or Br);

the Rc is each independently selected from $C_{3-10}$ cycloalkyl (alternatively $C_{3-8}$ cycloalkyl, and yet alternatively $C_{3-6}$ cycloalkyl, and further alternatively cyclopentyl) which is unsubstituted or substituted with hydroxyl, oxo, or thioxo;

the Rd is each independently selected from $C_{1-18}$ alkyl (alternatively $C_{1-12}$ alkyl, and yet alternatively $C_{1-6}$ alkyl), $C_{1-30}$ alkoxy (alternatively $C_{1-20}$ alkoxy, and yet alternatively $C_{1-12}$ alkoxy, and further alternatively $C_{1-3}$ alkoxy), $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with halogen (alternatively F, Cl or Br);

the $Y_1$ is selected from $C_{1-30}$ alkyl (alternatively $C_{1-12}$ alkyl, and yet alternatively $C_{1-6}$ alkyl), $C_{3-10}$ cycloalkyl (alternatively $C_{3-8}$ cycloalkyl, and yet alternatively $C_{3-6}$ cycloalkyl), $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with Re; the Re is each independently selected from $C_{6-30}$ aryl (alternatively $C_{6-18}$ aryl, and yet alternatively $C_{6-12}$ aryl), or $C_{3-30}$ heteroaryl (alternatively $C_{3-18}$ heteroaryl, and yet alternatively $C_{3-12}$ heteroaryl), which are unsubstituted or substituted with halogen (alternatively F, Cl or Br);

the $Y_2$ is selected from unsubstituted or Rf-substituted $C_{12-30}$ condensed cyclic group (alternatively $C_{12-17}$ condensed cyclic group, and yet alternatively $C_{12-17}$ condensed cyclic group containing at least one heteroatom, and further alternatively $C_{12-17}$ tetracondensed cyclic group containing at least one heteroatom); wherein the Rf is each independently selected from $C_{1-30}$ alkoxy (alternatively $C_{1-20}$ alkoxy, and yet alternatively $C_{1-12}$ alkoxy, and further alternatively $C_{1-3}$ alkoxy), or $C_{6-30}$ aryl (alternatively $C_{6-18}$ aryl, and yet alternatively $C_{6-12}$ aryl), which are unsubstituted or substituted with halogen (alternatively F, Cl or Br);

the $Y_3$ is selected from $C_{1-18}$ alkyl (alternatively $C_{1-12}$ alkyl, and yet alternatively $C_{1-6}$ alkyl) or -O-$C_{1-18}$ alkyl (alternatively -O-$C_{1-12}$ alkyl, and yet alternatively -O-$C_{1-6}$ alkyl);

the $Y_4$ is selected from $C_{6-30}$ aryl (alternatively $C_{6-18}$ aryl, and yet alternatively $C_{6-12}$ aryl) or $C_{3-30}$ heteroaryl (alternatively $C_{3-18}$ heteroaryl, and yet alternatively $C_{3-12}$ heteroaryl), which are unsubstituted or substituted with Rg, wherein the Rg is each independently selected from hydroxyl and $C_{1-30}$ alkoxy (alternatively $C_{1-20}$ alkoxy, and yet alternatively $C_{1-12}$ alkoxy, and further alternatively $C_{1-3}$ alkoxy);

the $Y_5$ is selected from a single bond, unsubstituted or amino-substituted $C_{1-18}$ alkylene (alternatively $C_{1-12}$

alkylene, and yet alternatively $C_{3-6}$ alkylene);
the $Y_6$ is selected from a single bond, -NH-C(NH)-, $-(O-CH_2-CH_2)_m-$ or $-CH_2-(O-CH_2-CH_2)_n-$, and the *m* and the *n* are each independently selected from any natural number of 2-20 (alternatively any natural number of 2-10);
the $Y_7$ is selected from amino, $C_{1-30}$ alkoxy (alternatively $C_{1-20}$ alkoxy, and yet alternatively $C_{1-12}$ alkoxy, and further alternatively $C_{1-3}$ alkoxy) or hydroxyl;
when the $L_1$ and the $L_2$ are both selected from a single bond, the $R^1$ is not hydroxyl;
$U_1$, $U_2$ and $U_3$ are each independently selected from -O-C(O)-, -NH-C(O)-, $-O-CH_2-O-$, and -O-; or an available ring atom on $U_2$ and an available ring atom on $U_3$ are connected via Q group to form a 4- to 8-membered ring, alternatively a 5- to 6-membered ring,
Q is selected from a single bond; $NR^3$; $C_{1-3}$ alkylene in which 1 or 2 $CH_2$ are optionally independently replaced with a group selected from O, S and $NR^3$; and $C_{2-3}$ alkenylene in which any CH for forming the C=C double bond is optionally replaced with N;
$W_1$, $W_2$ and $W_3$ are each independently selected from $C_{1-18}$ alkyl (alternatively $C_{1-12}$ alkyl, and yet alternatively $C_{1-6}$ alkyl) or $-(O-C_{2-6}$ alkylene), (alternatively $-(O-C_{2-4}$ alkylene$)_p$, and yet alternatively $-(O-CH_2-CH_2)_p$), wherein the *p* is selected from any natural number of 1-18 alternatively any natural number of 1-10;
the above alkyl, alkylene, alkenyl, alkenylene, alkynyl, cyclic hydrocarbon radical, heterocyclyl, aryl, heteroaryl, and aralkyl at each occurrence are each optionally substituted with 1, 2, 3 or more $R^3$, wherein the $R^3$ at each occurrence is independently selected from halogen, cyano, nitro, $C_{1-6}$ alkyl, $C_{3-10}$ cyclic hydrocarbon radical, 3- to 10-membered heterocyclyl, $C_{6-10}$ aryl, 5- to 14-membered heteroaryl, $C_{6-12}$ aralkyl; and wherein the alkyl, alkylene, cyclic hydrocarbon radical, heterocyclyl, aryl, heteroaryl, and aralkyl in relation to the substituent $R^3$ are further optionally substituted with 1, 2, 3 or more substituents independently selected from halogen, OH, oxo, amino, cyano, nitro, $C_{1-6}$ alkyl, $C_{1-6}$ haloalkyl, $C_{3-6}$ cyclic hydrocarbon radical, 3- to 10-membered heterocyclyl, $C_{6-10}$ aryl, 5- to 14-membered heteroaryl, and $C_{6-12}$ aralkyl.

**3.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein

the heteroatoms in the heteroaryl, the heteroarylene, and the $C_{12-17}$ condensed cyclic group containing at least one heteroatom are each independently selected from O, N or S, alternatively N;
alternatively, the number of heteroatoms in the heteroaryl, the heteroarylene, and the $C_{12-17}$ condensed cyclic group containing at least one heteroatom are 1-3, alternatively 1-2;
alternatively, the $C_{12-17}$ tetracondensed cyclic group containing at least one heteroatom is

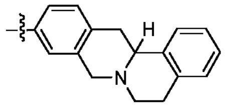

.

**4.** The compound of any one of claims 1-3, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula II:

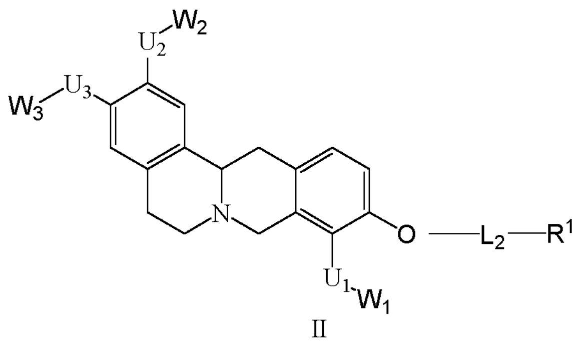

II

the $L_2$ is selected from a single bond, -C(O)-, -C(O)-O-, $C_{1-18}$ alkylene, $-C_{1-18}$ alkylene-O-, $-C(O)-C_{1-18}$ alkyl-, $C_{1-30}$ alkoxy, or $-X_1-PRa(O)-X_2-$; the Ra is each independently selected from hydrogen, hydroxyl, halogen, $C_{6-30}$ aryl, $C_{3-30}$ heteroaryl, $C_{1-30}$ hydrocarbon radical, $C_{3-10}$ cycloalkyl, $C_{1-30}$ alkoxy, $C_{6-30}$ aryl-O-, $C_{3-30}$ heteroaryl-O-, $C_{6-30}$ aryl-$C_{1-18}$ alkylene-O-, $C_{3-30}$ heteroaryl-$C_{1-18}$ alkylene-O-, $-O-C_{6-30}$ aryl-$C_{1-18}$ alkylene- or $-O-C_{3-30}$

heteroaryl-$C_{1-18}$ alkylene-; the Ra and $X_2$ may be connected to form a ring;
the $X_1$ is selected from a single bond or -$C_{1-18}$ alkylene-O-;
the $X_2$ is selected from a single bond, -O- or -NR*- (e.g., -NH-);
the $R^1$ is selected from hydrogen, hydroxyl, amino, $C_{1-30}$ hydrocarbon radical, $C_{1-30}$ alkoxy, unsubstituted or Rb-substituted $C_{12-30}$ condensed cyclic group, $C_{1-18}$ alkyl-$C_{6-30}$ arylene-$C_{1-18}$ alkylene- which is unsubstituted or has a hydrogen atom replaced with Rc in the alkyl, $C_{1-12}$ alkyl-O-C(O)-$C_{1-12}$ alkylene-imino-, $C_{6-30}$ aryl-$C_{1-18}$ alkylene-, -O-C(O)-$Y_1$, -$C_{1-12}$ alkylene-C(O)-O-$Y_2$, -O-$C_{1-12}$ alkylene-O-C(O)-$Y_3$, -C=CH-$Y_4$, or -$Y_5$-$Y_6$-$Y_7$, -$CHR^{\#}R^{\#\#}$;
wherein $R^{\#}$ and $R^{\#\#}$ are each independently selected from H, hydroxyl, amino, halogen, alkyl, cycloalkyl, aryl, heteroaryl,
-C(O)-$OR^2$, -OC(O)-$OR^2$, -$C_{1-18}$ alkylene-C(O)-$OR^2$, -$C_{1-18}$ alkylene-$R^2$, or -$C_{6-10}$ aryl-$C_{1-6}$ alkyl;
$R^2$ is selected from H, hydroxyl, amino, halogen, alkyl, cycloalkyl, aryl, heteroaryl, unsubstituted or Rb-substituted $C_{12-30}$ condensed cyclic group, or -NHC(NH)NH-;
the Rb is each independently selected from $C_{1-30}$ alkoxy, $C_{6-30}$ aryl, $C_{3-30}$ heteroaryl, or $C_{1-30}$ alkyl which are unsubstituted or substituted with halogen;
the Rc is each independently selected from $C_{3-10}$ cycloalkyl unsubstituted or substituted with hydroxyl, oxo, or thioxo;
the $Y_1$ is selected from $C_{1-30}$ alkyl, $C_{3-10}$ cycloalkyl, $C_{6-30}$ aryl, or $C_{3-30}$ heteroaryl, which are unsubstituted or substituted with Re; wherein the Re is each independently selected from $C_{6-30}$ aryl unsubstituted or substituted with halogen;
the $Y_2$ is selected from unsubstituted or Rf-substituted $C_{12-30}$ condensed cyclic group; wherein the Rf is each independently selected from $C_{1-30}$ alkoxy;
the $Y_3$ is selected from $C_{1-18}$ alkyl or -O-$C_{1-18}$ alkyl;
the $Y_4$ is selected from $C_{6-30}$ aryl or $C_{3-30}$ heteroaryl, which are unsubstituted or substituted with Rg, wherein the Rg is each independently selected from hydroxyl or $C_{1-30}$ alkoxy;
the $Y_5$ is selected from a single bond, unsubstituted or amino-substituted $C_{1-18}$ alkylene;
the $Y_6$ is selected from a single bond, -NH-C(NH)-, -$(O-CH_2-CH_2)_m$- or -$CH_2$-$(O-CH_2-CH_2)_n$-, wherein the *m* and the *n* are each independently selected from any natural number of 2-20;
the $Y_7$ is selected from amino, $C_{1-30}$ alkoxy or hydroxyl.

**5.** The compound of any one of claims 1-4, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula I-1:

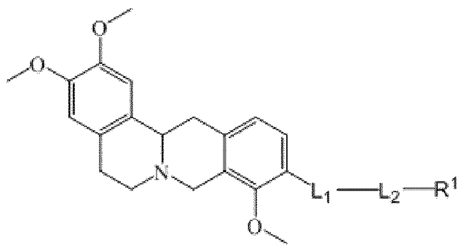

I-1

wherein $L_1$ is selected from a single bond, -O-, -NH-, -S- or -P(O)OH-; alternatively a single bond, -O- and -NH-;
the $L_2$ is selected from a single bond, -C(O)-, -C(O)-O-, -$C_{1-18}$ alkylene-, -$C_{1-18}$ alkylene-O- (e.g., -$(CH_2)_{1-5}$-O-), -C(O)-$C_{1-18}$ alkyl-, $C_{1-30}$ alkoxy (e.g., - $(OCH_2CH_2)_{2-8}$-), or -$X_1$-PRa(O)-$X_2$-;
the Ra is each independently selected from hydrogen, hydroxyl, halogen, $C_{6-30}$ aryl, $C_{3-30}$ heteroaryl, $C_{1-30}$ hydrocarbon radical, $C_{3-10}$ cycloalkyl, $C_{1-30}$ alkoxy, $C_{6-30}$ aryl-O-, $C_{3-30}$ heteroaryl-O-, $C_{6-30}$ aryl-$C_{1-18}$ alkylene-O-, $C_{3-30}$ heteroaryl-$C_{1-18}$ alkylene-O-, -O-$C_{6-30}$ aryl-$C_{1-18}$ alkylene- or -O-$C_{3-30}$ heteroaryl-$C_{1-18}$ alkylene-; wherein an available ring atom on the Ra may be directly connected with $X_2$ to form a 4- to 7-membered ring, alternatively a 5- or 6-membered ring;
the $X_1$ is selected from a single bond, -O- or -$C_{1-18}$ alkylene-O-; alternatively a single bond, -O- or -$(CH_2)_{1-3}$O- (e.g., -$CH_2$O-);
the $X_2$ is selected from a single bond, -O- or -NR*-; wherein R* is selected from H or $C_{1-6}$ alkyl (e.g., methyl, ethyl, isopropyl);
the $R^1$ is absent, or selected from hydrogen, hydroxyl, amino, $C_{1-30}$ hydrocarbon radical (in which the $C_{1-30}$

hydrocarbon radical contains 0-10 double bonds or triple bonds, alternatively 0-6 double bonds or triple bonds), $C_{1-30}$ alkoxy, unsubstituted or Rb-substituted $C_{12-30}$ condensed cyclic group, $C_{1-18}$ alkyl-$C_{6-30}$ arylene-$C_{1-18}$ alkylene- which is unsubstituted or has a hydrogen atom replaced with Rc in the alkyl, $C_{1-12}$ alkyl-O-C(O)-$C_{1-12}$ alkylene-imino-, $C_{6-30}$ aryl-$C_{1-18}$ alkylene-, -O-C(O)-$Y_1$, -$C_{1-12}$ alkylene-C(O)-O-$Y_2$, -O-$C_{1-12}$ alkylene-O-C(O)-$Y_3$, -CH=CH-$Y_4$, or -$Y_5$-$Y_6$-$Y_7$, -CHR$^{\#}$R$^{\#\#}$;

wherein R$^{\#}$ and R$^{\#\#}$ are each independently selected from H, hydroxyl, amino, halogen, alkyl, cycloalkyl, aryl, heteroaryl,

-C(O)-OR$^2$, -OC(O)-OR$^2$, -$C_{1-18}$ alkylene-C(O)-OR$^2$, -$C_{1-18}$ alkylene-R$^2$, or -$C_{6-10}$ aryl-$C_{1-6}$ alkyl;

R$^2$ is selected from H, hydroxyl, amino, halogen, alkyl, cycloalkyl, aryl, heteroaryl, unsubstituted or Rb-substituted $C_{12-30}$ condensed cyclic group, or -NHC(NH)NH-;

the Rb is each independently selected from $C_{1-30}$ alkoxy, $C_{6-30}$ aryl, $C_{3-30}$ heteroaryl, or $C_{1-30}$ alkyl which are unsubstituted or substituted with halogen;

the Rc is each independently selected from $C_{3-10}$ cycloalkyl unsubstituted or substituted with hydroxyl, oxo, or thioxo;

the $Y_1$ is selected from $C_{1-30}$ alkyl, $C_{3-10}$ cycloalkyl, $C_{6-30}$ aryl, or $C_{3-30}$ heteroaryl which are unsubstituted or substituted with Re; wherein the Re is each independently selected from $C_{6-30}$ aryl unsubstituted or substituted with halogen;

the $Y_2$ is selected from unsubstituted or Rf-substituted $C_{12-30}$ condensed cyclic group; wherein the Rf is each independently selected from $C_{1-30}$ alkoxy;

the $Y_3$ is selected from $C_{1-18}$ alkyl or -O-$C_{1-18}$ alkyl;

the $Y_4$ is selected from $C_{6-30}$ aryl or $C_{3-30}$ heteroaryl, which are unsubstituted or substituted with Rg, wherein the Rg is each independently selected from hydroxyl or $C_{1-30}$ alkoxy;

the $Y_5$ is selected from a single bond, unsubstituted or amino-substituted $C_{1-18}$ alkylene;

the $Y_6$ is selected from a single bond, -NH-C(NH)-, $-(O-CH_2-CH_2)_m-$ or $-CH_2-(O-CH_2-CH_2)_n-$, wherein the *m* and the *n* are each independently selected from any natural number of 2-20;

the $Y_7$ is selected from amino, $C_{1-30}$ alkoxy or hydroxyl.

**6.** The compound of any one of claims 1 to 5, or a pharmaceutically acceptable salt, ester, stereoisomer, polymorph, solvate, N-oxide, isotope-labeled compound, metabolite, or pro-drug thereof, and a mixture thereof, wherein

the $L_2$ is selected from a single bond, -C(O)-, -C(O)-O-, $C_{1-6}$ alkylene, -$C_{1-6}$ alkylene-O- or -$X_1$-PRa(O)-$X_2$-;

the Ra is each independently selected from hydrogen, hydroxyl, $C_{6-12}$ aryl-O-, or $C_{6-12}$ aryl-$C_{1-6}$ alkylene-O-, -O-$C_{6-30}$ aryl-$C_{1-18}$ alkylene- or -O-$C_{3-30}$ heteroaryl-$C_{1-18}$ alkylene-; the Ra and $X_2$ may be connected to form a ring;

the $X_1$ is selected from a single bond or -$C_{1-6}$ alkylene-O-;

the $X_2$ is selected from a single bond, -O-, -NH-;

the R$^1$ is selected from absent, hydrogen, hydroxyl, amino, $C_{1-30}$ alkyl, $C_{1-30}$ alkoxy, unsubstituted or methoxy-substituted $C_{12-17}$ tetracondensed cyclic group containing at least one heteroatom, oxocyclopentyl-$C_{1-6}$ alkylene-$C_{6-12}$ arylene-$C_{1-6}$ alkylene-, hydroxylcyclopentyl-$C_{1-6}$ alkylene-$C_{6-12}$ arylene-$C_{1-6}$ alkylene-, $C_{1-6}$ alkyl-O-C(O)-$C_{1-6}$ alkylene-imino-, $C_{6-12}$ aryl-$C_{1-6}$ alkylene-, -O-C(O)-$Y_1$, -$C_{1-12}$ alkylene-C(O)-O-$Y_2$, -O-$C_{1-12}$ alkylene-O-C(O)-$Y_3$, -C=CH-$Y_4$, or -$Y_5$-$Y_6$-$Y_7$, -CHR$^{\#}$R$^{\#\#}$;

wherein R$^{\#}$ and R$^{\#\#}$ are each independently selected from H, hydroxyl, amino, halogen, alkyl, cycloalkyl, aryl, heteroaryl,

-C(O)-OR$^2$, -OC(O)-OR$^2$, -$C_{1-18}$ alkylene-C(O)-OR$^2$, -$C_{1-18}$ alkylene-R$^2$, or -$C_{6-10}$ aryl-$C_{1-6}$ alkyl;

R$^2$ is selected from H, hydroxyl, amino, halogen, alkyl, cycloalkyl, aryl, heteroaryl, unsubstituted or Rb-substituted $C_{12-30}$ condensed cyclic group, or -NHC(NH)NH-;

the $Y_1$ is selected from $C_{1-6}$ alkyl, $C_{3-6}$ cycloalkyl or $C_{6-12}$ aryl which are unsubstituted or substituted with Re; wherein the Re is each independently selected from unsubstituted or halogen-substituted $C_{6-12}$ aryl;

the $Y_3$ is selected from $C_{1-6}$ alkyl or -O-$C_{1-6}$ alkyl;

the $Y_4$ is selected from unsubstituted or Rg-substituted $C_{6-12}$ aryl, wherein the Rg is each independently selected from hydroxyl or methoxy;

the $Y_5$ is selected from a single bond, unsubstituted or amino-substituted $C_{1-6}$ alkylene;

the $Y_6$ is selected from a single bond, -NH-C(NH)-, $-(O-CH_2-CH_2)_m-$ or $-CH_2-(O-CH_2-CH_2)_n-$, wherein the *m* and the *n* are each independently selected from any natural number of 2-10;

the $Y_7$ is selected from amino, $C_{1-6}$ alkoxy or hydroxyl.

**7.** The compound of any one of claims 1 to 6, or a pharmaceutically acceptable salt, ester, stereoisomer, polymorph, solvate, N-oxide, isotope-labeled compound, metabolite, or pro-drug thereof, and a mixture thereof, wherein

the $R^1$ is selected from absent, hydrogen, hydroxyl, amino, aryl (e.g., phenyl), $C_{1-30}$ alkyl, $C_{1-30}$ alkoxy (e.g., $-OCH_3$), $C_{12-30}$ condensed cyclic group unsubstituted or substituted with 1, 2, 3, 4, or 5 Rb, $-(CH_2)_{1-10}$-C(O)-O$C_{12-30}$ condensed cyclic group, -O-C(O)-$Y_1$, -$C_{1-12}$ alkylene-C(O)-O-$Y_2$, -O-$C_{1-12}$ alkylene-O-C(O)-$Y_3$, -C=CH-$Y_4$, or -$Y_5$-$Y_6$-$Y_7$, -CH$R^{\#}R^{\#\#}$, alternatively absent, hydrogen, hydroxyl, amino, aryl (e.g., phenyl), $C_{1-30}$ alkyl, $C_{1-30}$ alkoxy (e.g., $-OCH_3$), unsubstituted or Rb-substituted $C_{12-30}$ condensed cyclic group, $-(CH_2)_{1-10}$-C(O)-O$C_{12-30}$ condensed cyclic group, -O-C(O)-$Y_1$, -$C_{1-12}$ alkylene-C(O)-O-$Y_2$, -O-$C_{1-12}$ alkylene-O-C(O)-$Y_3$, -C=CH-$Y_4$, or - $Y_5$-$Y_6$-$Y_7$, -CH$R^{\#}R^{\#\#}$,

alternatively a structure of: H, -OH, $-NH_2$, $-OCH_3$, methyl, ethyl, isopropyl, cyclopropyl, $-(CH_2)_pCH_3$, $-(CH_2)_pOCH_3$, $-(CH_2)_pNH_2$, $-(CH_2)_pOH$, $-(OCH_2CH_2)qCH_3$, $-(OCH_2CH_2)qOCH_3$, $-(OCH_2CH_2)qOH$, $-(OCH_2CH_2)qNH_2$, phenyl,

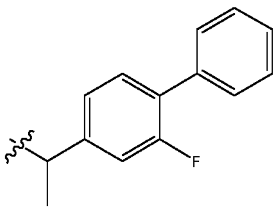 , 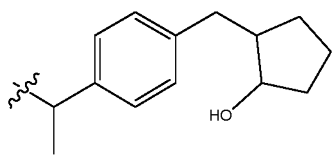 ,

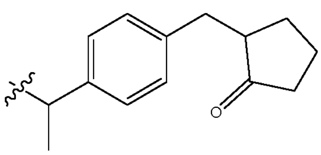 , 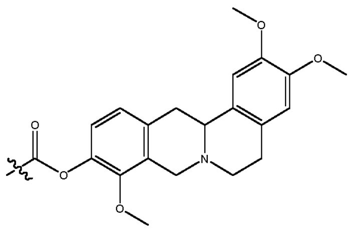 , 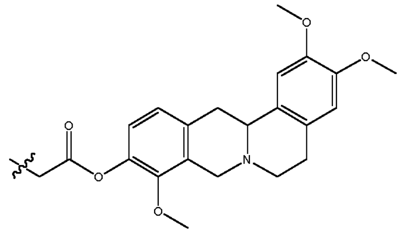 ,

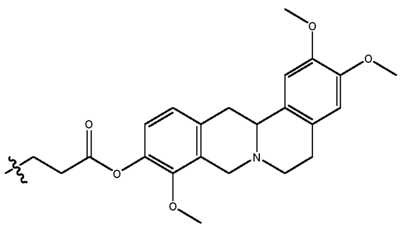 , 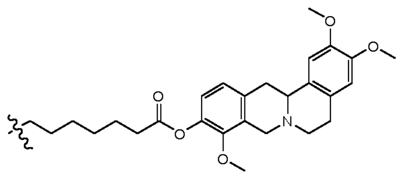 , 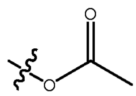 ,

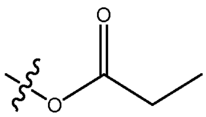 , 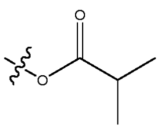 , 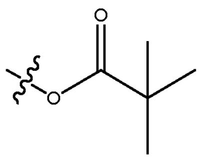 , 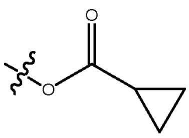 , 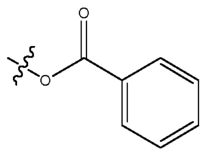 ,

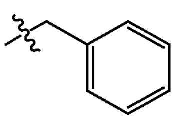 , 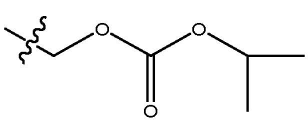 , 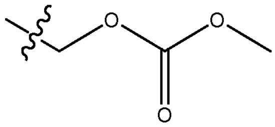 , 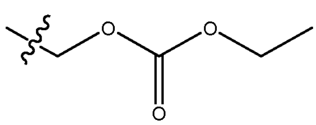 ,

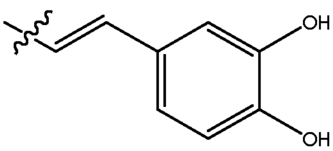 , 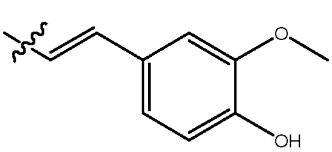 , 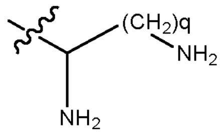

(e.g.,

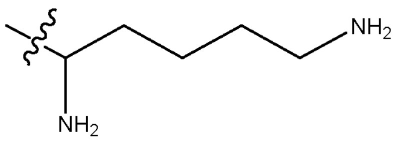 ), 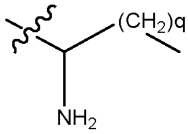 , 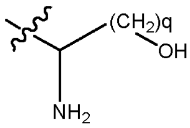 , 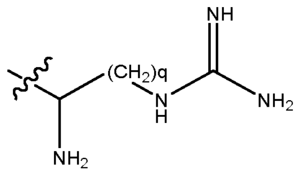

(e.g.,

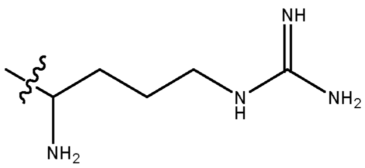 ), 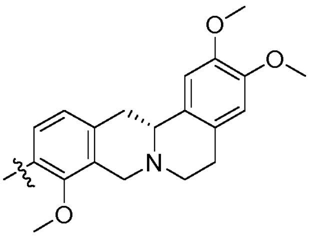 , 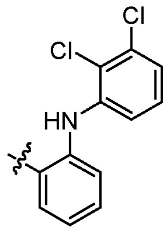 ,

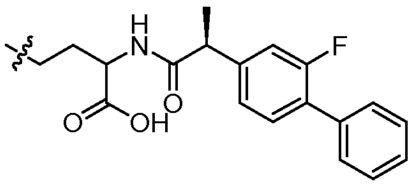 , 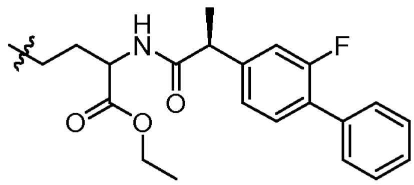 , or 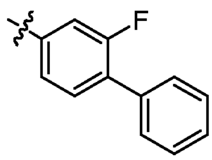 ;

alternatively a structure of: H, -OH, $-NH_2$, $-OCH_3$, methyl, ethyl, isopropyl, cyclopropyl, $-(CH_2)_pCH_3$, $-(CH_2)_pOCH_3$, $-(CH_2)_pNH_2$, $-(CH_2)_pOH$, $-(OCH_2CH_2)qCH_3$, $-(OCH_2CH_2)qOCH_3$, $-(OCH_2CH_2)qOH$, $-(OCH_2CH_2)qNH_2$, phenyl,

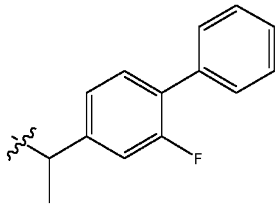 , 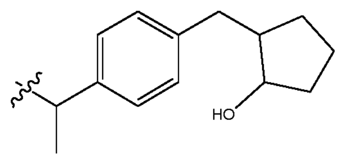 ,

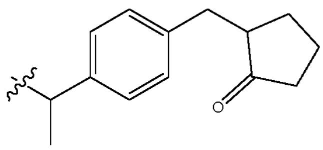 , 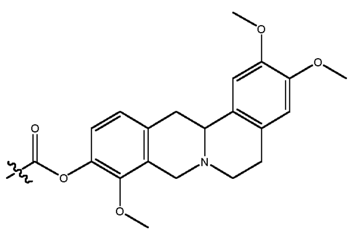 , 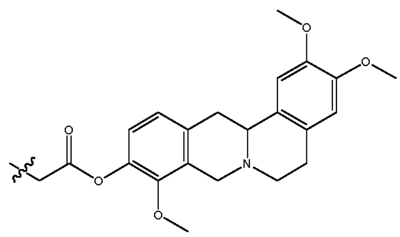 ,

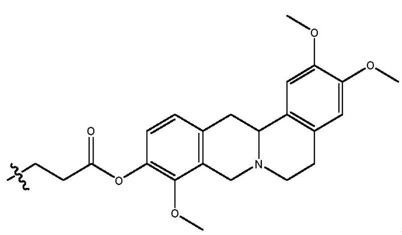 , 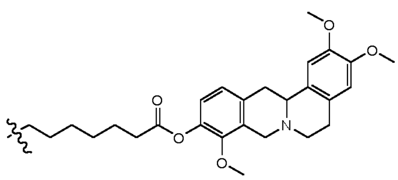 , 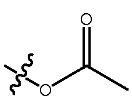 , 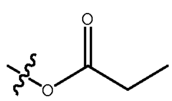 ,

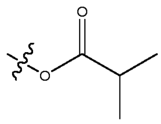 , 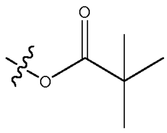 , 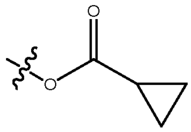 , 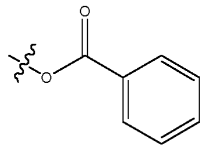 , 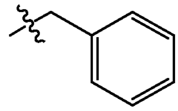 ,

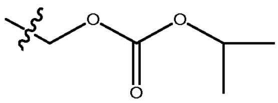 , 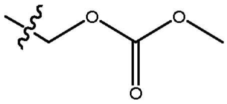 , 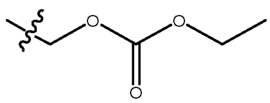 , 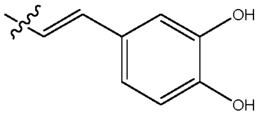 ,

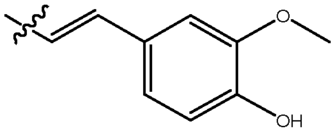 , 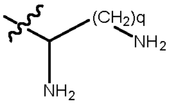

(e.g.,

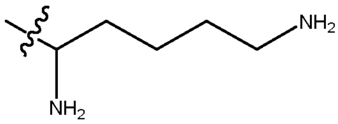 ), 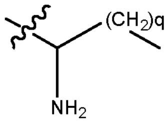 ,

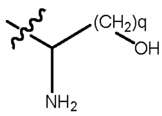 , 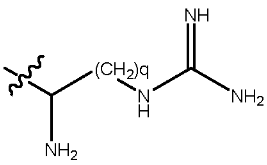

(e.g.,

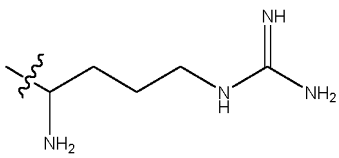 );

wherein *p* is selected from 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30;
q is selected from 0,1,2,3,4,5,6,7,8,9,10,11,12,13,14 or 15.

**8.** The compound of any one of claims 1-7, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula III:

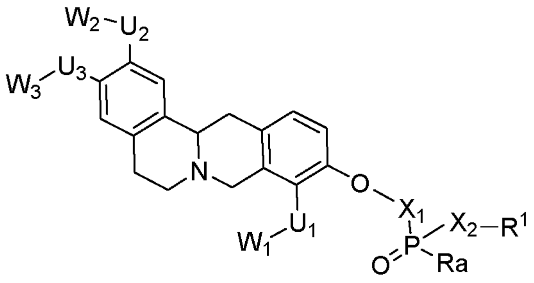

III

9. The compound of claim 8, or a pharmaceutically acceptable salt, ester, stereoisomer, polymorph, solvate, N-oxide, isotope-labeled compound, metabolite, or pro-drug thereof, and a mixture thereof, wherein

the Ra is each independently selected from hydroxyl, $C_{6-12}$ aryl-O-, $C_{6-12}$ aryl-$C_{1-6}$ alkylene-O- or -O-$C_{6-12}$ aryl-$C_{1-6}$ alkylene-; the Ra and $X_2$ may be connected to form a ring;
the $X_1$ is selected from a single bond or -$C_{1-6}$ alkylene-O-;
the $X_2$ is selected from a single bond, -O-, -NH-;
the $R^1$ is selected from absent, hydrogen, hydroxyl, $C_{1-6}$ alkyl-O-C(O)-$C_{1-6}$ alkylene-imino-, $C_{6-12}$ aryl-$C_{1-6}$ alkylene- or -O-$C_{1-12}$ alkylene-O-C(O)-$Y_3$; the $Y_3$ is selected from -O-$C_{1-6}$ alkyl.

10. The compound of claim 8 or 9, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula IVa, IVb or IVc:

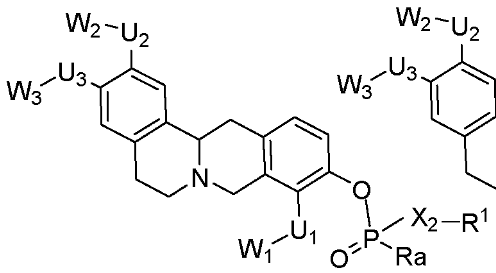

IVa

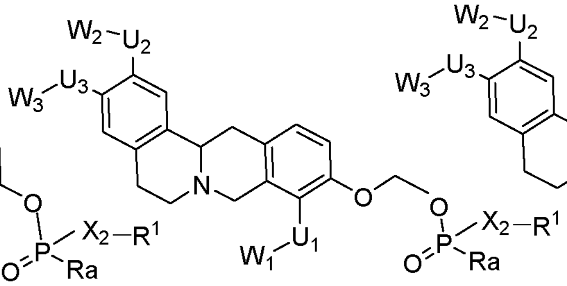

IVb

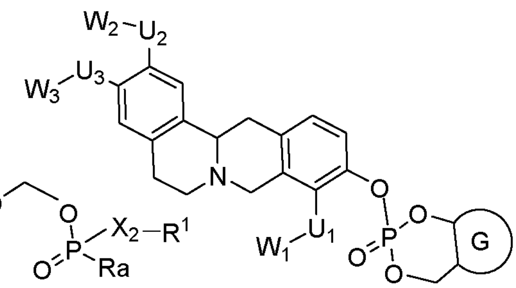

IVc

wherein, the ring G in Formula IVc is $C_{6-12}$ aryl ring.

11. The compound of any one of claims 1-5, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula V:

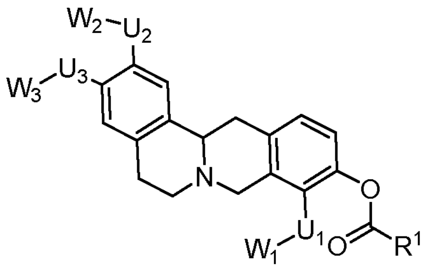

V

12. The compound of claim 11, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula VIa or VIb:

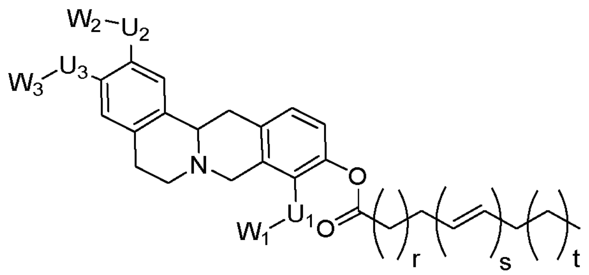

VIa

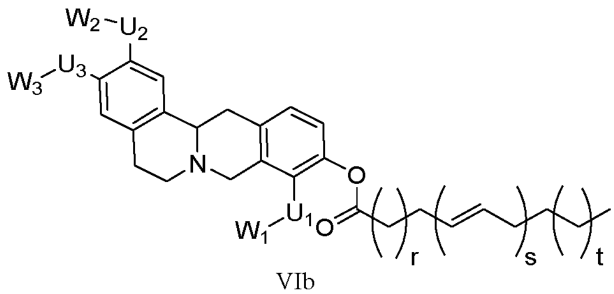

VIb

wherein *r* and *t* in Formula VIa and Formula VIb are each independently selected from any natural number of 0-10, s is each independently selected from any natural number of 0-9 (alternatively any natural number of 1-*6); r, s* and *t* are not 0 at the same time.

**13.** The compound of claim 11, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula VII:

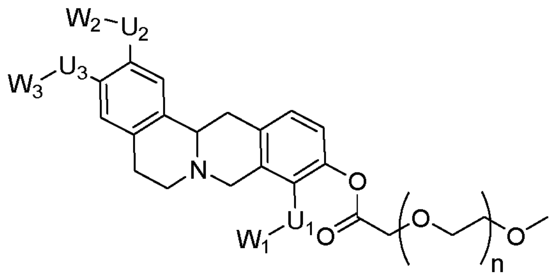

VII

wherein *n* is selected from any natural number of 2-20, alternatively any natural number of 2-10.

**14.** The compound of claim 11, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula VIII:

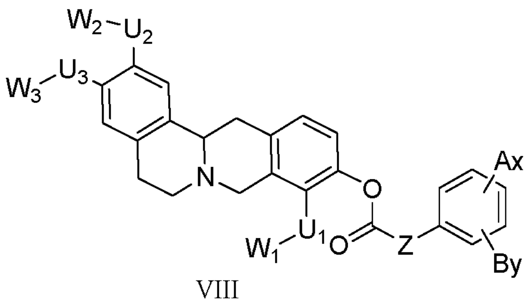

VIII

wherein Z is selected from $C_{1-30}$ alkylene, $C_{3-10}$ cycloalkylene, $C_{6-30}$ arylene, $C_{3-30}$ heteroarylene, or -C=CH-;
A is selected from hydroxyl, halogen, $C_{6-30}$ aryl or Re-substituted $C_{1-18}$ alkyl, wherein the Rc is each independently selected from $C_{3-10}$ cycloalkyl unsubstituted or substituted with hydroxyl, oxo, or thioxo;
B is selected from hydroxyl, halogen or $C_{6-30}$ aryl;
*x* is selected from 0 or 1, y is selected from 0, 1 or 2, and *x* + *y* is not 0.

**15.** The compound of claim 14, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula IX:

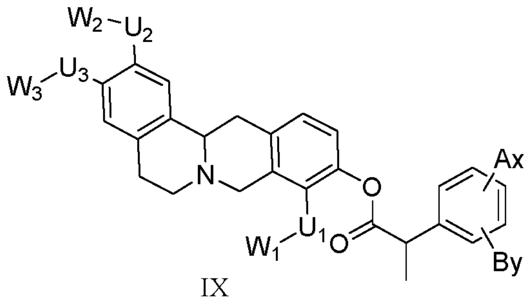

IX

A is selected from Rc-substituted $C_{1-18}$ alkyl, wherein the Rc is each independently selected from $C_{3-10}$ cycloalkyl substituted with oxo;
B is selected from halogen or $C_{6-30}$ aryl;
*x* is selected from 1, *y* is selected from 0 or 1.

**16.** The compound of claim 1, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula X:

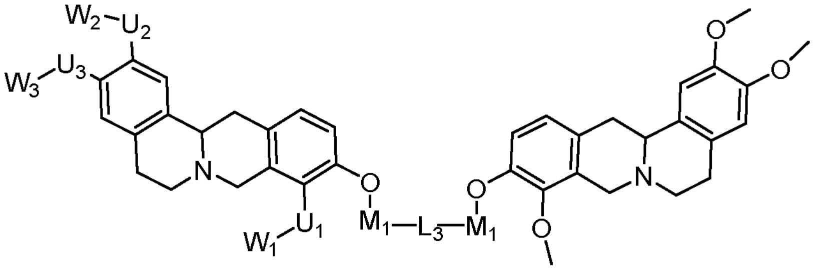

X

wherein $M_1$ is each independently selected from a single bond or -C(O)-;
$L_3$ is selected from $C_{1-12}$ alkylene.

**17.** The compound of claim 16, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula XI:

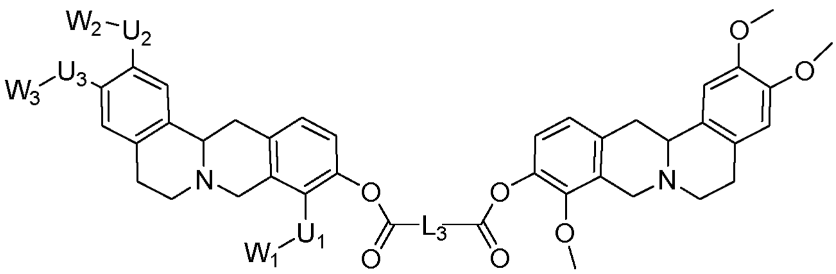

XI

wherein, $L_3$ is selected from $C_{1-12}$ alkylene;
alternatively, $U_1$, $U_2$, and $U_3$ are each independently selected from -O-, and $W_1$, $W_2$, and $W_3$ are each independently selected from methyl.

**18.** The compound of claim 1, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula XII:

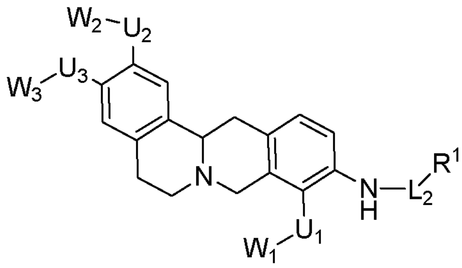

XII

.

**19.** The compound of claim 16, or a pharmaceutically acceptable salt, ester, stereoisomer, polymorph, solvate, N-oxide, isotope-labeled compound, metabolite, or pro-drug thereof, and a mixture thereof, wherein

the $L_2$ is selected from a single bond or -O-;
the $R^1$ is selected from hydrogen, amino, or $-Y_5-Y_6-Y_7$;
the $Y_5$ is selected from a single bond, unsubstituted or amino-substituted $C_{3-12}$ alkylene.

**20.** The compound of claim 16, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula XIII:

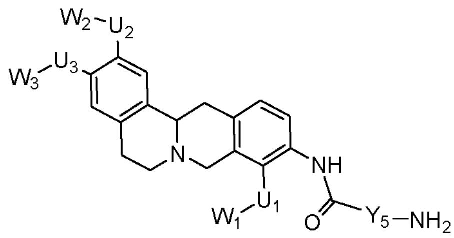

XIII

alternatively, the $Y_5$ is selected from unsubstituted or amino-substituted $C_{3-12}$ alkylene, alternatively unsubstituted or amino-substituted $C_{3-6}$ alkylene.

**21.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula XIV:

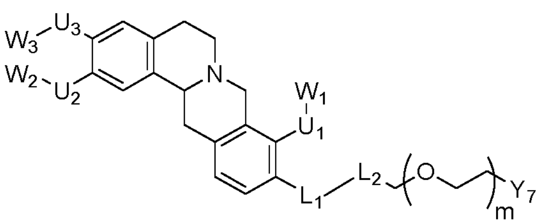

XIV

.

**22.** The compound of claim 19, or a pharmaceutically acceptable salt, ester, stereoisomer, polymorph, solvate, N-oxide, isotope-labeled compound, metabolite, or pro-drug thereof, and a mixture thereof, wherein:

the $L_1$ is selected from -O- or -NH-;
the $L_2$ is selected from -C(O)- or $C_{1-18}$ alkylene;
the $Y_7$ is selected from amino, $C_{1-30}$ alkyl, $C_{1-30}$ alkoxy or hydroxyl.

**23.** The compound of claim 19, or a pharmaceutically acceptable salt, ester, stereoisomer, polymorph, solvate, N-oxide, isotope-labeled compound, metabolite, or pro-drug thereof, and a mixture thereof, wherein the compound is a

compound of Formula XVa or XVb:

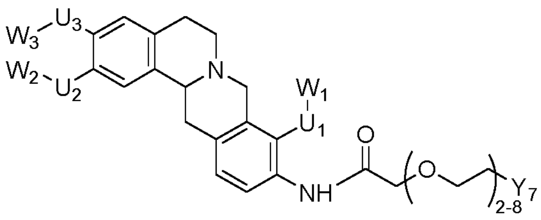

XVa

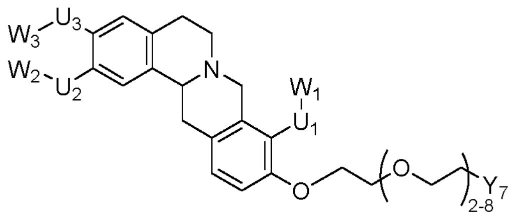

XVb

alternatively, the $Y_7$ is selected from methoxy or hydroxyl.

**24.** The compound of any one of claims 1 to 23, or a pharmaceutically acceptable salt, ester, stereoisomer, polymorph, solvate, N-oxide, isotope-labeled compound, metabolite, or pro-drug thereof, and a mixture thereof, wherein

$U_1$, $U_2$, and $U_3$ are each independently selected from -O-;
$W_1$, $W_2$, and $W_3$ are each independently selected from $C_{1-18}$ alkyl, alternatively $C_{1-12}$ alkyl, yet alternatively $C_{1-6}$ alkyl, further alternatively methyl.

**25.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula Ia:

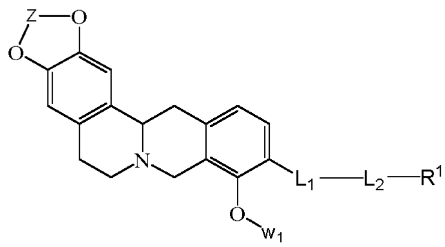

Ia ,

alternatively

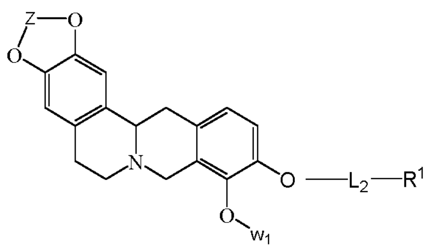

Ia-1 , or

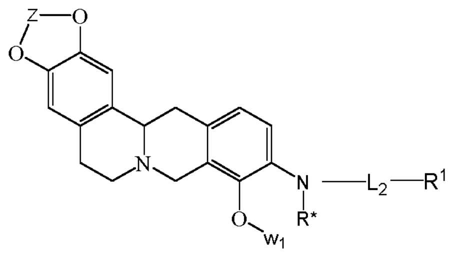

Ia-2

wherein $L_1$, $L_2$, $W_1$, $R^1$, and R* are as defined in claim 1 or 2.

**26.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula XVI, XVI-1, XVI-2, XVI-3, or XVI-4:

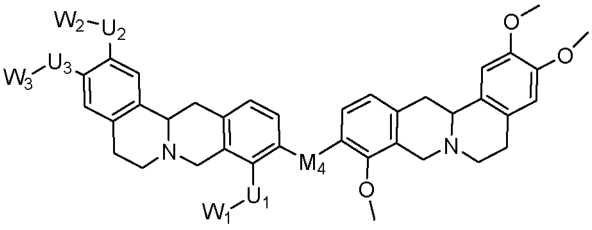

XVI

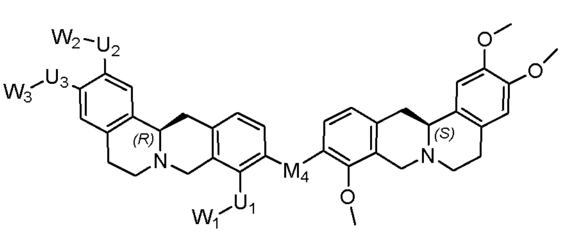

XVI-1

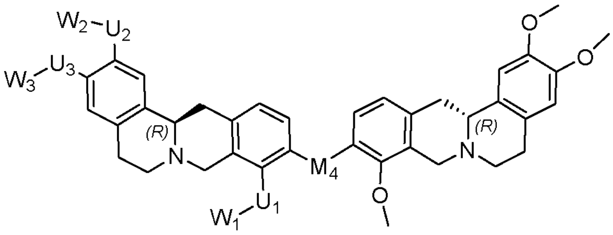

XVI-2

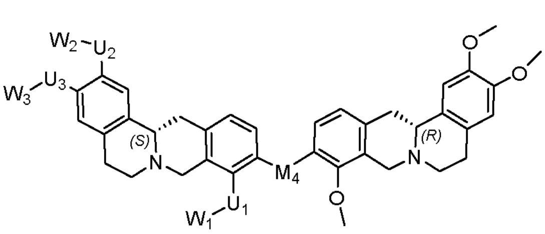

XVI-3

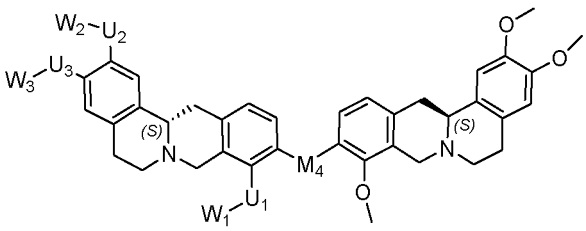

XVI-4

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-18}$ alkyl; and
$M_4$ is selected from a chemical bond, -O-, -NH-, -C(O)-, -C(O)O-, -OC(O)-, -NHC(O)O-, -OC(O)NH- or - $C_{1-10}$ alkylene-;
alternatively,
$U_1$, $U_2$, and $U_3$ are independently selected from -O-C(O)- or -O-;
$W_1$, $W_2$, and $W_3$ are independently selected from $C_{1-12}$ alkyl, alternatively $C_{1-6}$ alkyl; and
$M_4$ is selected from a chemical bond, -NH-, -C(O)O-, -OC(O)-, -NHC(O)O- or -OC(O)NH-;
alternatively,
$U_1$, $U_2$, and $U_3$ are -O-;
$W_1$, $W_2$, and $W_3$ are independently selected from $C_{1-6}$ alkyl, alternatively $C_{1-4}$ alkyl, yet alternatively methyl; and
$M_4$ is selected from -NHC(O)O- or -OC(O)NH-.

**27.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula VIII, VIII-1, or VIII-2:

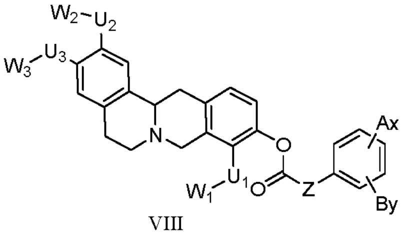

VIII

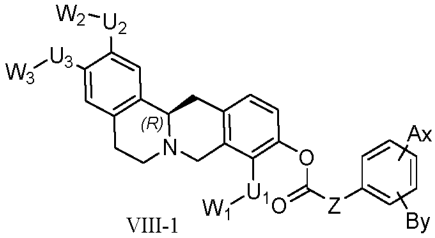

VIII-1

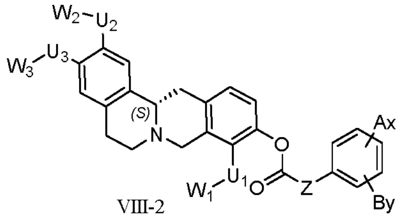

VIII-2

wherein

$U_1$, $U_2$, and $U_3$ are independently selected from -O-C(O)-, -NH-C(O)-, -O-$CH_2$-O- or -O;
$W_1$, $W_2$, and $W_3$ are independently selected from $C_{1-12}$ alkyl;
Z is selected from a chemical bond, -O-, -NH-, -$C_{1-12}$ alkylene-, -$C_{1-12}$ alkylene-O-$C_{1-12}$ alkylene-, -$C_{1-12}$ alkylene-NH-$C_{1-12}$ alkylene-, -$C_{1-18}$ alkylene-C(O)-$C_{1-12}$ alkylene-, -$C_{1-10}$ alkylene-C(O)O-$C_{1-10}$ alkylene-, -$C_{1-10}$ alkylene-NHC(O)-$C_{1-10}$ alkylene-, -$C_{1-10}$ alkylene-NHC(O)O-$C_{1-10}$ alkylene-, -$C_{1-10}$ alkylene-C(O)NH-$C_{1-10}$ alkylene- or -$C_{1-10}$ alkylene-OC(O)NH-$C_{1-10}$ alkylene-, alternatively a chemical bond or -$C_{1-6}$ alkylene-NHC(O)-$C_{1-6}$ alkylene-;
the Z is optionally substituted with 1, 2, 3, 4, or 5 substituents selected from halogen, -C(O)OH, -C(O)O-$C_{1-6}$ alkyl, -C(O)-$C_{1-6}$ alkyl, -O-$C_{1-6}$ alkyl, $C_{1-6}$ alkyl, or $C_{1-6}$ haloalkyl;
A and B are independently selected from H, halogen, OH, -$L_A$-$C_{3-10}$ cycloalkyl, -$L_A$-3- to 10-membered heterocyclyl, -$L_A$-$C_{6-12}$ aryl, or -$L_A$-5- to 12-membered heteroaryl, alternatively halogen, $C_{6-10}$ aryl, or -NH-$C_{6-10}$ aryl;
$L_A$ is selected from a chemical bond, -NH-, -O-, -C(O)-, -C(O)O-, -NHC(O)-, -NHC(O)O-, -OC(O)NH- or - C(O) NH-;
the A and B are optionally further substituted with halogen, $C_{1-6}$ alkyl, or $C_{1-6}$ haloalkyl;
*x* is 0, 1 or 2; and
*y* is 0, 1, 2, or 3;
alternatively,
$U_1$, $U_2$, and $U_3$ are independently selected from -O-C(O)- or -O-;
$W_1$, $W_2$, and $W_3$ are independently selected from $C_{1-6}$ alkyl;
Z is selected from a chemical bond, -O-, -NH-, -$C_{1-6}$ alkylene-, -$C_{1-6}$ alkylene-C(O)-$C_{1-6}$ alkylene-, -$C_{1-6}$ alkylene-C(O)O-$C_{1-6}$ alkylene-, -$C_{1-6}$ alkylene-NHC(O)-$C_{1-6}$ alkylene-, -$C_{1-6}$ alkylene-C(O)NH-$C_{1-6}$ alkylene-, alternatively a chemical bond, -$C_{1-6}$ alkylene-NHC(O)-$C_{1-6}$ alkylene- or -$C_{1-6}$ alkylene-C(O)NH-$C_{1-6}$ alkylene-;
the Z is optionally substituted with 1, 2, or 3 substituents selected from halogen, C(O)OH, C(O)O-$C_{1-6}$ alkyl, $C_{1-6}$ alkyl, or $C_{1-6}$ haloalkyl;
A and B are independently selected from H, halogen, $C_{6-10}$ aryl, -NH-$C_{6-10}$ aryl, -O-$C_{6-10}$ aryl, -C(O)-$C_{6-10}$ aryl, and -C(O)O-$C_{6-10}$ aryl, alternatively halogen, $C_{6-10}$ aryl, or -NH-$C_{6-10}$ aryl;
the A and B are optionally further substituted with halogen, $C_{1-6}$ alkyl, or $C_{1-6}$ haloalkyl;
*x is* 0, 1 or 2; and
*y* is 0, 1, 2, or 3;
yet alternatively,
$U_1$, $U_2$, and $U_3$ are -O-;
$W_1$, $W_2$, and $W_3$ are independently selected from $C_{1-4}$ alkyl, alternatively methyl;
Z is selected from a chemical bond, -$C_{1-4}$ alkylene-NHC(O)-$C_{1-4}$ alkylene- or -$C_{1-4}$ alkylene-C(O)NH-$C_{1-4}$ alkylene-, alternatively a chemical bond or -$C_{1-4}$ alkylene-NHC(O)-$C_{1-4}$ alkylene-;
the Z is optionally substituted with 1, 2, or 3 substituents selected from halogen, C(O)OH, C(O)O-$C_{1-4}$ alkyl, or $C_{1-4}$ alkyl;
A and B are independently selected from H, halogen, phenyl, or -NH-phenyl, alternatively F, phenyl, or NH-phenyl;
the A and B are optionally further substituted with halogen;
*x* and *y* are 0, 1 or 2.

**28.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula II, II-1, II-2, I-1, I-2, or I-3:

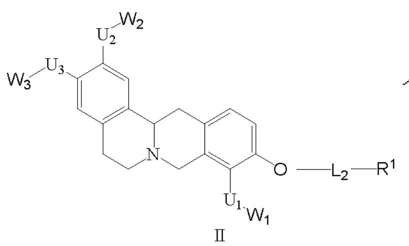
II

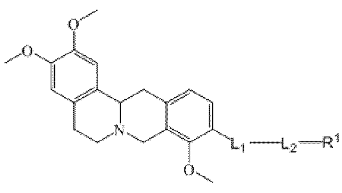
I-1

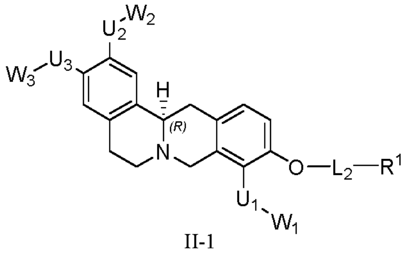

II-1

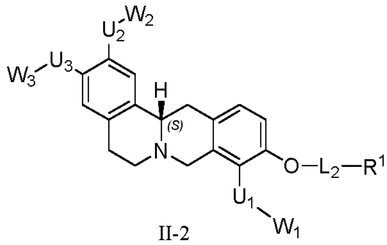

II-2

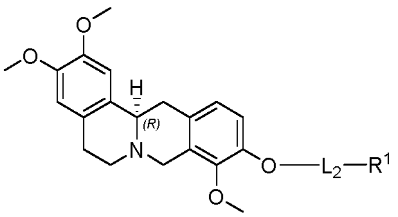

I-2

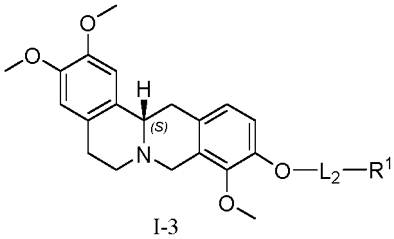

I-3

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
$L_1$ is selected from a single bond, -O- or -NH-;
$L_2$ is -$C_{1-12}$ alkylene-, and the alkylene may be further substituted with $C_{1-6}$ alkyl;
$R^1$ is selected from -OC(O)-$Y_1$;
$Y_1$ is selected from $C_{1-12}$ alkyl, $C_{3-10}$ cycloalkyl, $C_{6-18}$ aryl, or $C_{3-18}$ heteroaryl, and the $Y_1$ may be further substituted with $C_{1-6}$ alkyl;
$Y_1$ is alternatively $C_{1-6}$ alkyl, yet alternatively $CH_3$;
alternatively,
$U_1$, $U_2$, and $U_3$ are selected from -O- or -OC(O)-;
$W_1$, $W_2$, and $W_3$ are independently selected from $C_{1-6}$ alkyl;
$L_1$ is selected from -O- or -NH-;
$L_2$ is -$C_{1-6}$ alkylene-, and the alkylene may be further substituted with $C_{1-6}$ alkyl; $R^1$ is selected from -OC(O)-$Y_1$;
$Y_1$ is selected from $C_{1-6}$ alkyl, $C_{3-6}$ cycloalkyl or $C_{6-12}$ aryl, and the $Y_1$ may be further substituted with $C_{1-6}$ alkyl; and
$Y_1$ is alternatively $CH_3$;
yet alternatively,
$U_1$, $U_2$, and $U_3$ are -O-;
$W_1$, $W_2$, and $W_3$ are $CH_3$;
$L_1$ is -O-;
$L_2$ is -$CH_2$-, -$CH(CH_3)$-,

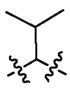;

and
$R^1$ is selected from

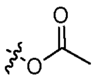, 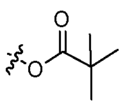, 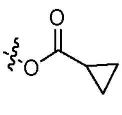, or 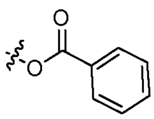.

**29.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula III, III-1, or III-2:

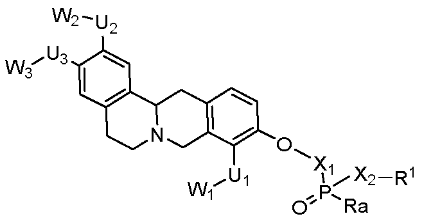

III

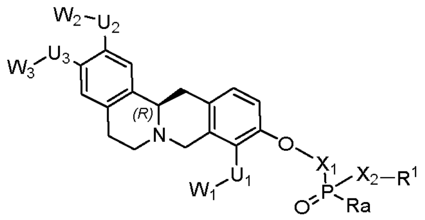

III-1

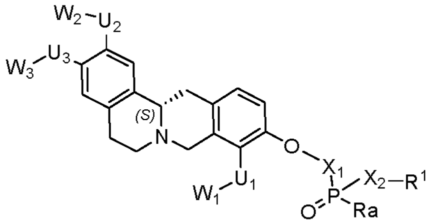

III-2

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
$X_1$ is selected from a chemical bond or -$C_{1-6}$ alkylene-O-;
$X_2$ is a chemical bond, -O- or -C(O)-;
$R^1$ is absent, or selected from H, -NH-$C_{1-12}$ alkylene-C(O)-O-$C_{1-12}$ alkyl, -$C_{1-12}$ alkylene-$C_{6-18}$ aryl, -O-$C_{1-12}$ alkylene-O-C(O)-$Y_3$, wherein the hydrogen atom(s) in the alkylene of $R^1$ is(are) optionally substituted with Rd;
$Y_3$ is -O-$C_{1-6}$ alkyl, and the $C_{1-6}$ alkyl may be further substituted with $C_{1-6}$ alkyl;
Ra is selected from OH, -O-$C_{6-18}$ aryl, -O-$C_{1-12}$ alkylene-$C_{6-18}$ aryl, or -O-$C_{6-18}$ aryl-$C_{1-12}$ alkyl;
or Ra and $X_2$ are connected to form a ring; and
Rd is selected from $C_{1-12}$ alkyl or $C_{1-12}$ alkoxy;
alternatively,
$U_1$, $U_2$, and $U_3$ are selected from -O- or -OC(O)-;
$W_1$, $W_2$, and $W_3$ are independently selected from $C_{1-6}$ alkyl;
$X_1$ is selected from a chemical bond or -$C_{1-6}$ alkylene-O-;
$X_2$ is a chemical bond, -O- or -C(O)-;
$R^1$ is absent, or selected from H, -NH-$C_{1-6}$ alkylene-C(O)-O-$C_{1-6}$ alkyl, -$C_{1-6}$ alkylene-$C_{6-10}$ aryl, -O-$C_{1-6}$ alkylene-O-C(O)-$Y_3$, wherein the hydrogen atom(s) in the alkylene of $R^1$ is(are) optionally substituted with Rd;
$Y_3$ is -O-$C_{1-6}$ alkyl, and the $C_{1-6}$ alkyl may be further substituted with $C_{1-6}$ alkyl;
Ra is selected from OH, -O-$C_{6-12}$ aryl, -O-$C_{1-6}$ alkylene-$C_{6-12}$ aryl, or -O-$C_{6-12}$ aryl-$C_{1-6}$ alkyl;
or Ra and $X_2$ are connected to form a ring;
Rd is $C_{1-6}$ alkyl;
yet alternatively,
$U_1$, $U_2$, and $U_3$ are O;
$W_1$, $W_2$, and $W_3$ are $CH_3$;
$X_1$ is selected from a chemical bond or -$CH_2$-O-;
$X_2$ is a chemical bond or -O-;
$R^1$ is absent, or selected from H,

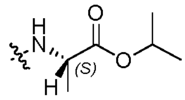

,

-$CH_2$-phenyl or

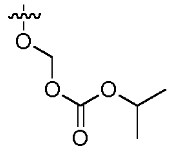

;

Ra is selected from OH, -O-phenyl, or -O-$CH_2$-phenyl, or -O-phenyl-$CH_3$; or Ra and $X_2$ are connected to form a ring.

**30.** The compound of claim 29, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula IVa, IVa-1, IVa-2, IVb, IVb-1, IVb-2, IVc, IVc-1, or IVc-2:

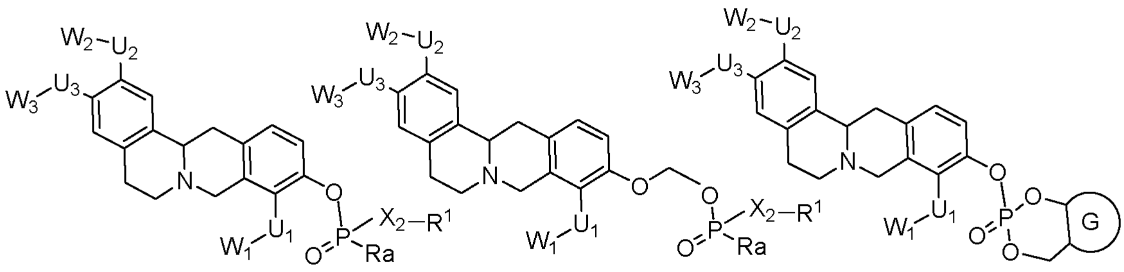

IVa IVb IVc

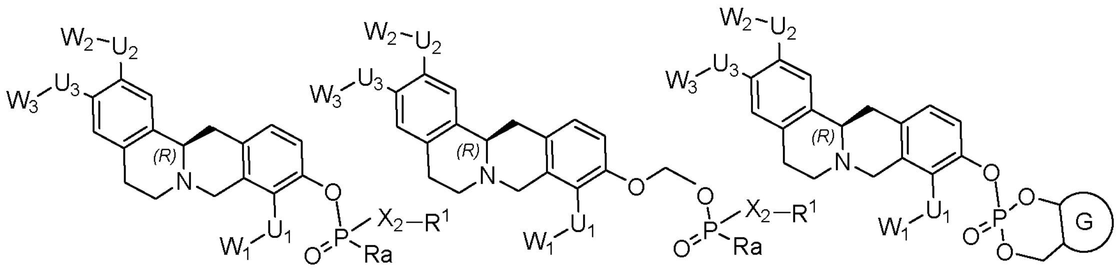

IVa-1 IVb-1 IVc-1

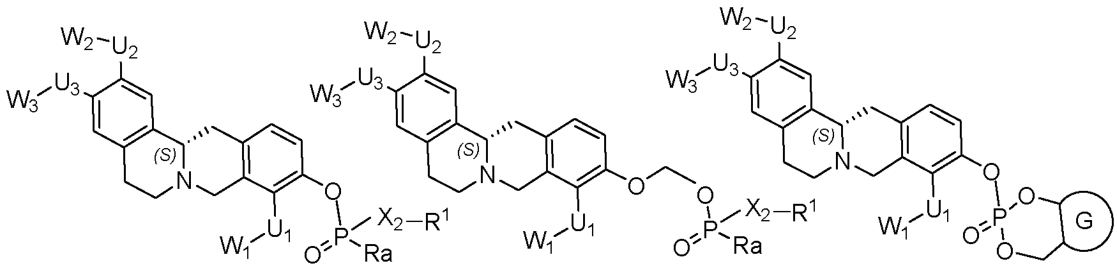

IVa-2 IVb-2 IVc-2

wherein the ring G is $C_{6-12}$ aryl, alternatively $C_{6-10}$ aryl, yet alternatively phenyl; and various other variables are as defined in claim 29.

**31.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula V, V-1, or V-2:

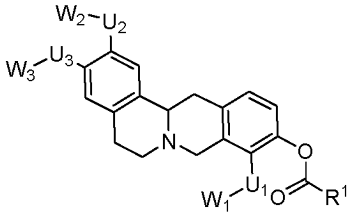

V

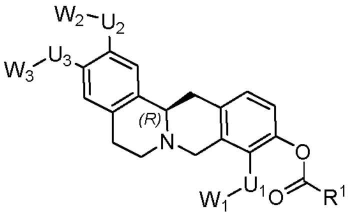

V-1

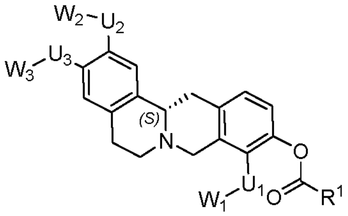

V-2

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
$R^1$ is selected from $C_{1-30}$ alkyl, $C_{2-30}$ alkenyl, $C_{1-20}$ alkoxy, and -O-$C_{1-12}$ alkylene-O-C(O)-$Y_3$ which is unsubstituted or has a hydrogen atom replaced with Rd in the alkylene;
Rd is selected from $C_{1-12}$ alkyl;
$R^1$ is alternatively $C_{6-10}$ alkyl or $C_{10-30}$ alkenyl;
$Y_3$ is selected from $C_{1-12}$ alkyl or O-$C_{1-12}$ alkyl;
alternatively,
$U_1$, $U_2$, and $U_3$ are selected from -O- or -OC(O)-;
$W_1$, $W_2$, and $W_3$ are independently selected from $C_{1-6}$ alkyl;
$R^1$ is selected from $C_{1-30}$ alkyl, $C_{2-30}$ alkenyl, $C_{1-6}$ alkoxy, and -O-$C_{1-6}$ alkylene-O-C(O)-$Y_3$ which is unsubstituted or has a hydrogen atom replaced with Rd in the alkylene;
Rd is selected from $C_{1-6}$ alkyl;
$R^1$ is alternatively $C_{6-10}$ alkyl or $C_{10-30}$ alkenyl;
$Y_3$ is $C_{1-6}$ alkyl;
yet alternatively,
$U_1$, $U_2$, and $U_3$ are O;
$W_1$, $W_2$, and $W_3$ are $CH_3$;
$R^1$ is selected from $CH_3$,

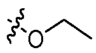 , 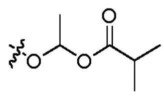 ,

$C_7$ alkyl, $C_8$ alkyl, $C_9$ alkyl, $C_{10}$ alkyl, $C_{11}$ alkyl, $C_{12}$ alkyl, $C_{13}$ alkyl, $C_{15}$ alkyl, $C_{16}$ alkyl, $C_{17}$ alkyl, $C_{19}$ alkyl, $C_{21}$ alkyl, $C_{23}$ alkyl, $C_{25}$ alkyl,

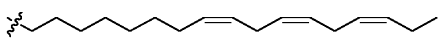 , 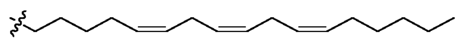 ,

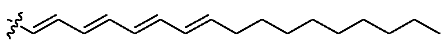 , 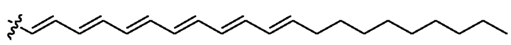 ,

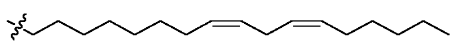 , 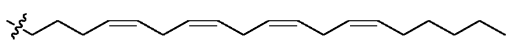 ,

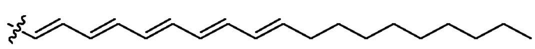 , 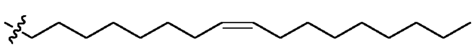 ,

and

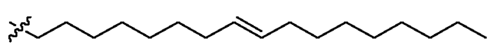 .

**32.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula VIa, VIa-1, VIa-2, VIb, VIb-1, VIb-2, VIc, VIc-1, or VIc-2:

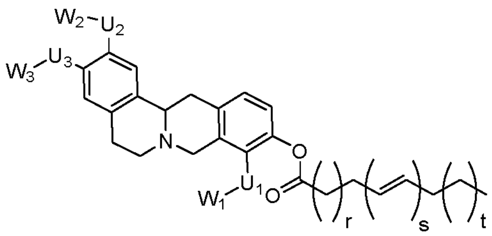

VIa

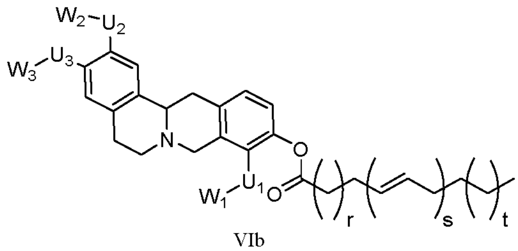

VIb

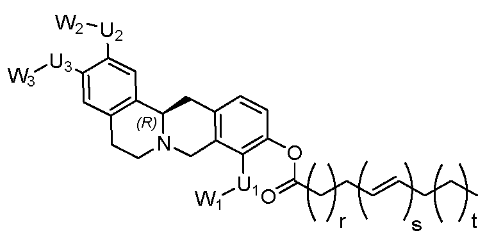

VIa-1

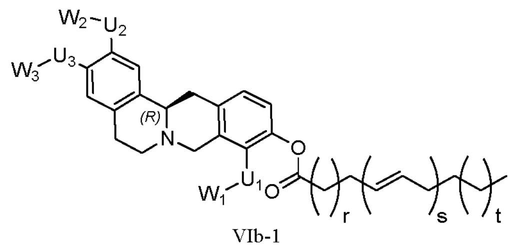

VIb-1

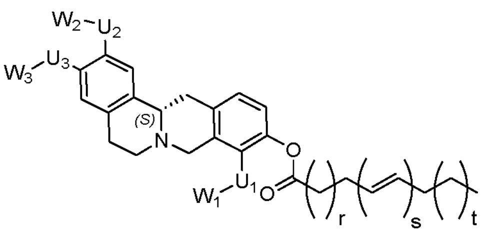

VIa-2

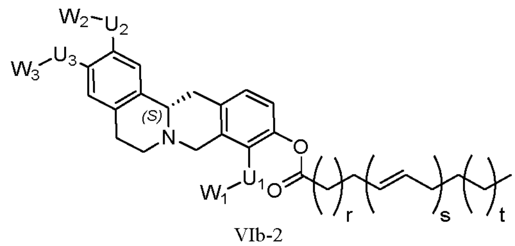

VIb-2

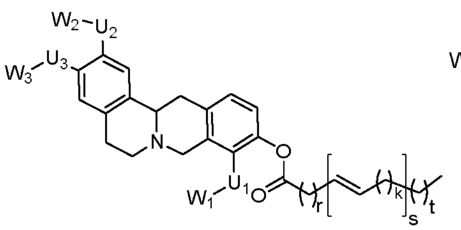

VIc

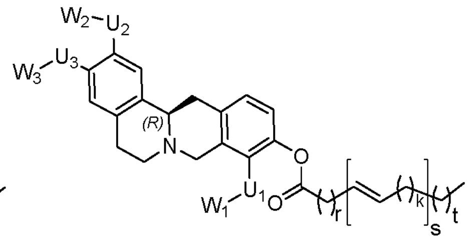

VIc-1

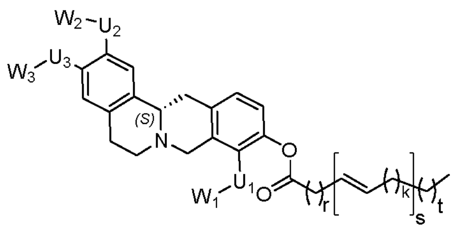

VIc-2

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
*r* and *t* are selected from 0, 1, 2, 3, 4, 5, 6, 7, 8, 9 or 10;
*s* is selected from 0, 1, 2, 3, 4, 5, 6, 7, 8 or 9;
k is selected from 0, 1, 2, 3, 4 or 5;
alternatively,
$U_1$, $U_2$, and $U_3$ are selected from -O- or -OC(O)-;
$W_1$, $W_2$, and $W_3$ are independently selected from $C_{1-6}$ alkyl;

*r* and *t* are selected from 0, 1, 2, 3, 4, 5, 6, 7, 8, 9 or 10;
*s* is selected from 0, 1, 2, 3, 4, 5, 6, 7, 8 or 9;
k is selected from 0, 1, 2, or 3;
yet alternatively,
$U_1$, $U_2$, and $U_3$ are O;
$W_1$, $W_2$, and $W_3$ are $CH_3$;
*r* is 0, 2, 3, 6, or 7;
*s* is 1, 2, 4, 5, or 6;
*t* is 0, 2, 5, 6, or 8;
*k* is *0 or* 1.

**33.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula VII, VII-1, VII-2, XIV, XIV-1, or XIV-2:

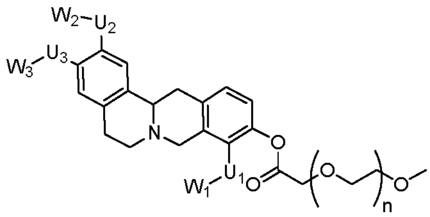
VII

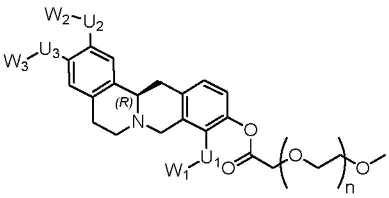
VII-1

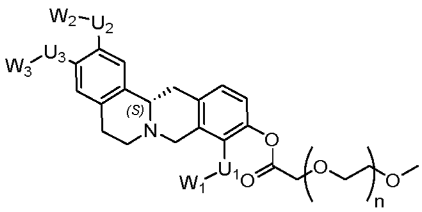
VII-2

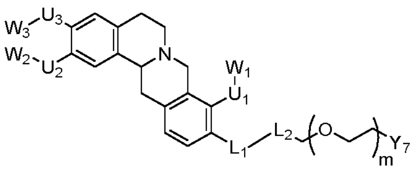
XIV

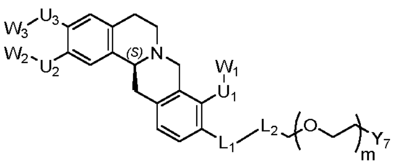
XIV-1

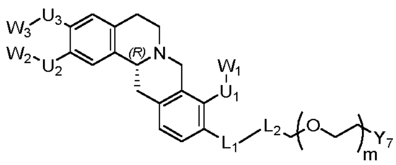
XIV-2

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
*m* and *n* are selected from 2, 3, 4, 5, 6, 7, 8, 9, 10, 11, 12, 13, 14 or 15, alternatively 6, 7, 8, 9 or 10, yet alternatively 7, 8 or 9;
$L_1$ is a chemical bond, -O- or -NH-;
$L_2$ is -C(O)-, -C(O)O-, -C(O)-$C_{1-18}$ alkylene- or -O-$C_{1-18}$ alkylene-;
$Y_7$ is $C_{1-12}$ alkoxy;
alternatively,
$U_1$, $U_2$, and $U_3$ are selected from -O- or -OC(O)-;
$W_1$, $W_2$, and $W_3$ are independently selected from $C_{1-6}$ alkyl;
*m* and *n* are selected from 2, 3, 4, 5, 6, 7, 8, 9 or 10, alternatively 7, 8 or 9;
$L_1$ is -O- or -NH-, alternatively -O-;
$L_2$ is -C(O)- or -C(O)-$C_{1-6}$ alkylene-;
$Y_7$ is $C_{1-3}$ alkoxy;
yet alternatively,
$U_1$, $U_2$, and $U_3$ are O;
$W_1$, $W_2$, and $W_3$ are $CH_3$;
*m* and *n* are 3, 4, 5, 6, 7, 8, alternatively 3 or 8;
$L_1$ is O;
$L_2$ is -C(O)- or -C(O)-$C_{1-4}$ alkylene-, alternatively -C(O)-;
$Y_7$ is $C_{1-3}$ alkoxy, alternatively $OCH_3$.

**34.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a

compound of Formula VIII, VIII-1, or VIII-2:

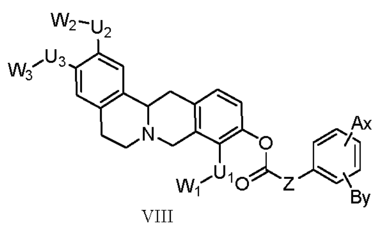
VIII
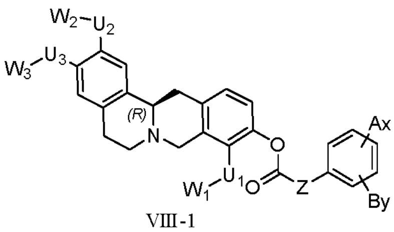
VIII-1
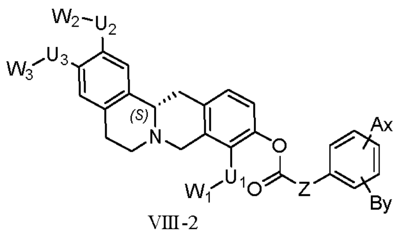
VIII-2

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
Z is selected from $C_{1-30}$ alkylene or $C_{2-30}$ alkenylene and the Z is optionally substituted with 1, 2, or 3 $R^3$;
$R^3$ is $C_{1-6}$ alkyl;
A is selected from halogen, OH, $C_{1-18}$ alkyl or $C_{6-30}$ aryl which are optionally substituted with Rc, alternatively OH, $C_{1-18}$ alkyl or $C_{6-30}$ aryl which are optionally substituted with Rc, yet alternatively $C_{6-30}$ aryl;
B is selected from halogen, OH or $C_{6-30}$ aryl, alternatively halogen or OH, yet alternatively halogen;
Rc is selected from $C_{3-10}$ cycloalkyl optionally substituted with OH, oxo, or thioxo, alternatively $C_{3-10}$ cycloalkyl substituted with oxo;
*x* is 0, 1 or 2; and
*y* is 0, 1, 2, or 3;
alternatively,
$U_1$, $U_2$, and $U_3$ are selected from -O- or -OC(O)-;
$W_1$, $W_2$, and $W_3$ are independently selected from $C_{1-6}$ alkyl;
Z is selected from $C_{1-6}$ alkylene or $C_{2-6}$ alkenylene, alternatively $C_{1-3}$ alkylene; and the Z is optionally substituted with 1, 2, or 3 $R^3$;
$R^3$ is $C_{1-6}$ alkyl;
Ais selected from halogen, OH, $C_{1-6}$ alkyl or $C_{6-12}$ aryl which are optionally substituted with Rc, alternatively OH, $C_{1-6}$ alkyl or $C_{6-12}$ aryl which are optionally substituted with Rc, yet alternatively $C_{6-12}$ aryl;
B is selected from halogen, OH or $C_{6-12}$ aryl, alternatively halogen or OH, yet alternatively halogen;
Rc is selected from $C_{3-10}$ cycloalkyl optionally substituted with OH or oxo;
*x* is 0, 1 or 2; and
*y* is 0, 1, 2, or 3;
yet alternatively,
$U_1$, $U_2$, and $U_3$ are O;
$W_1$, $W_2$, and $W_3$ are $CH_3$;
Z is selected from -CH($CH_3$)-, -CH=CH- or -$CH_2$-$CH_2$-;
A is selected from F, OH, phenyl,

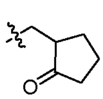, or 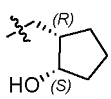,

alternatively OH, phenyl, or

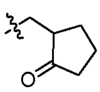,

yet alternatively phenyl;
B is selected from F, OH or phenyl, alternatively F or OH, yet alternatively F;
*x* and *y* are 0, 1 or 2.

**35.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a

compound of Formula IX, IX-1, or IX-2:

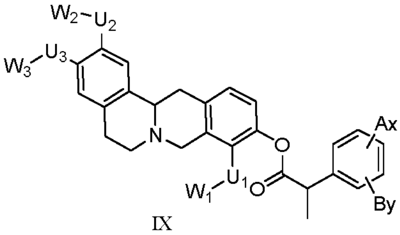
IX

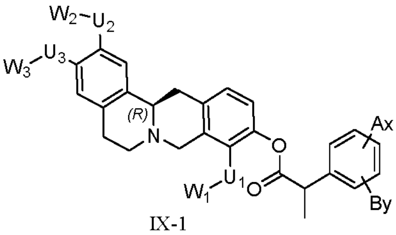
IX-1

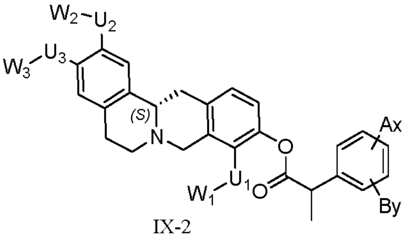
IX-2

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
A is selected from H or Re-substituted $C_{1-18}$ alkyl;
B is selected from halogen or $C_{6-30}$ aryl, alternatively halogen or $C_{6-12}$ aryl;
Rc is selected from $C_{3-10}$ cycloalkyl optionally substituted with OH, oxo, or thioxo, alternatively $C_{3-10}$ cycloalkyl substituted with oxo;
*x* is 0, 1 or 2; and
*y* is 0, 1, 2, or 3;
alternatively,
$U_1$, $U_2$, and $U_3$ are selected from -O- or -OC(O)-;
$W_1$, $W_2$, and $W_3$ are independently selected from $C_{1-6}$ alkyl;
A is selected from H or Re-substituted $C_{1-6}$ alkyl, alternatively H;
B is selected from halogen or $C_{6-10}$ aryl, alternatively F or phenyl;
Rc is selected from $C_{3-10}$ cycloalkyl substituted with oxo or OH, alternatively $C_{3-10}$ cycloalkyl substituted with oxo;
*x* is 0, 1 or 2; and
*y* is 0, 1, 2, or 3;
yet alternatively,
$U_1$, $U_2$, and $U_3$ are -O-;
$W_1$, $W_2$, and $W_3$ are $CH_3$;
A is selected from

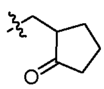 or 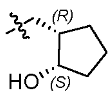 ;

B is selected from F or phenyl;
*x* is *0 or* 1;
*y* is *0 or 1.*

**36.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula X, X-1, X-2, X-3, or X-4:

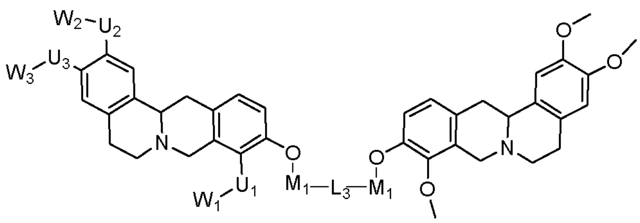
X

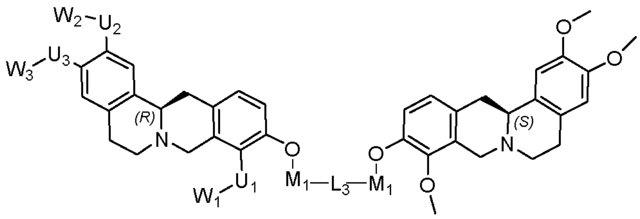

X-1

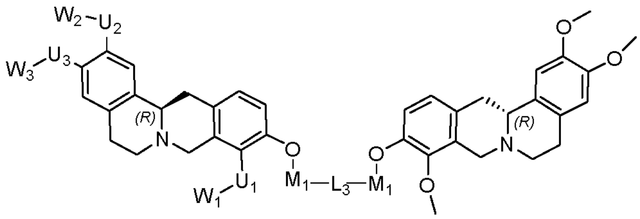

X-2

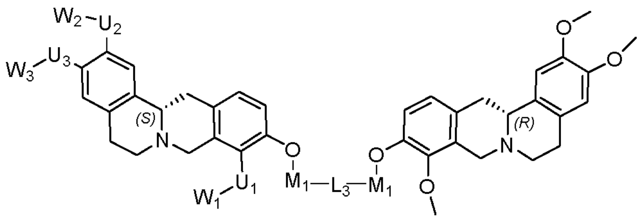

X-3

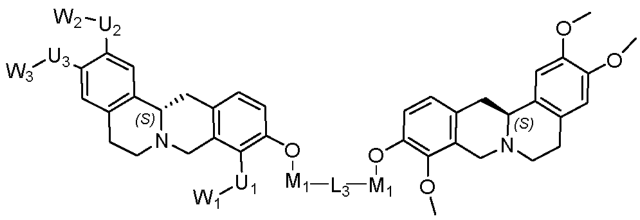

X-4

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
each of $M_1$is each independently a chemical bond or -C(O)-, alternatively, two $M_1$ are not both chemical bonds;
$L_3$ is a chemical bond or -$C_{1-18}$- alkylene-, alternatively a chemical bond;
alternatively,
$U_1$, $U_2$, and $U_3$ are selected from -O- or -OC(O)-;
$W_1$, $W_2$, and $W_3$ are independently selected from $C_{1-6}$ alkyl;
each of $M_1$is each independently a chemical bond or -C(O)-, alternatively, two $M_1$ are not both chemical bonds;
$L_3$ is selected from a chemical bond or -$C_{1-12}$- alkylene-, alternatively a chemical bond;
yet alternatively,
$U_1$, $U_2$, and $U_3$ are -O-;
$W_1$, $W_2$, and $W_3$ are $CH_3$;
each of $M_1$is each independently a chemical bond or -C(O)-, alternatively, two $M_1$ are not both chemical bonds;
$L_3$ is selected from a chemical bond or -$C_{1-6}$- alkylene-, e.g., a chemical bond, -$CH_2$-, -$CH_2CH_2CH_2$- or -$CH_2CH_2CH_2CH_2CH_2CH_2$-, alternatively a chemical bond.

**37.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula XI, XI-1, XI-2, XI-3, or XI-4:

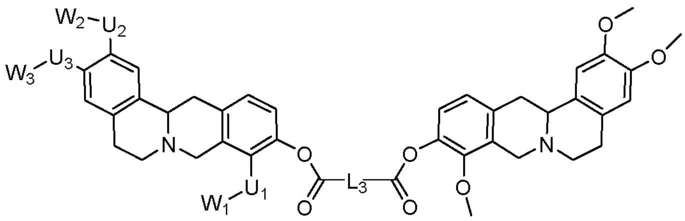

XI

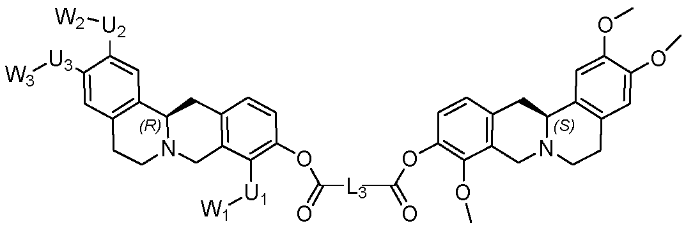

XI-1

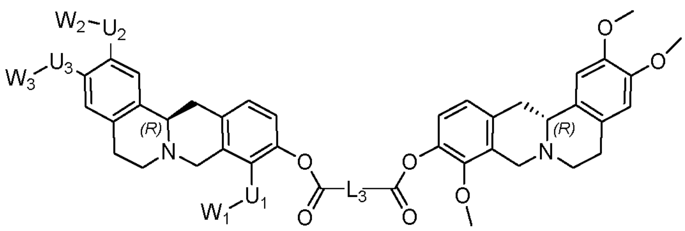

XI-2

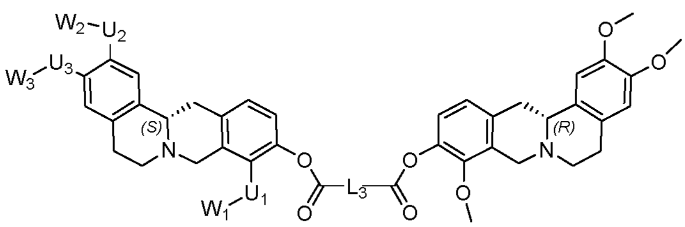

XI-3

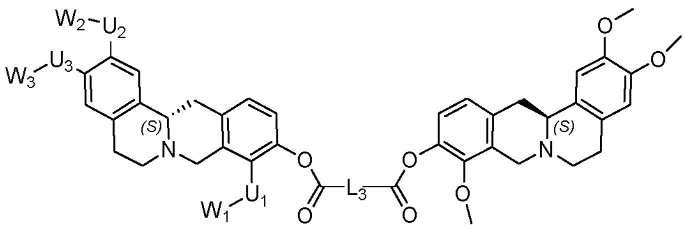

XI-4

wherein

$U_1$, $U_2$, and $U_3$ are independently selected from -O-, -O-C(O)-, -NH-C(O)- or -O-$CH_2$-O-;
$W_1$, $W_2$, and $W_3$ are independently selected from $C_{1-12}$ alkyl;
$L_3$ is -$C_{1-18}$- alkylene-;
alternatively,
$U_1$, $U_2$, and $U_3$ are selected from -O- or -OC(O)-;
$W_1$, $W_2$, and $W_3$ are independently selected from $C_{1-6}$ alkyl;
$L_3$ is -$C_{1-12}$- alkylene-;
yet alternatively,

$U_1$, $U_2$, and $U_3$ are O;
$W_1$, $W_2$, and $W_3$ are $CH_3$;
$L_3$ is -$C_{1-6}$- alkylene-, e.g., -$CH_2CH_2CH_2$- or -$CH_2CH_2CH_2CH_2CH_2CH_2$-.

**38.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula XII, XII-1, or XII-2:

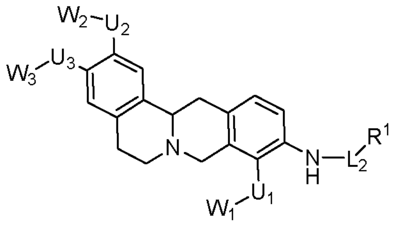
XII

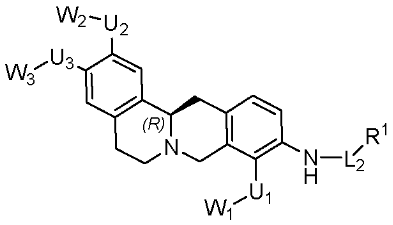
XII-1

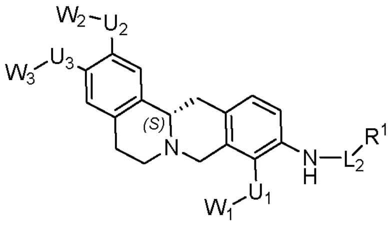
XII-2

wherein

$U_1$, $U_2$, and $U_3$ are independently selected from -O-, -O-C(O)-, -NH-C(O)- or -O-$CH_2$-O-;
$W_1$, $W_2$, and $W_3$ are $C_{1-12}$ alkyl;
$L_2$ is a chemical bond, -C(O)- or -C(O)O-;
$R^1$ is selected from H, OH, or -$Y_5$-$Y_6$-$Y_7$
$Y_5$ is unsubstituted or $NH_2$-substituted $C_{1-12}$ alkylene;
$Y_6$ is a single bond or -NH-C(NH)-;
$Y_7$ is $NH_2$, OH or $C_{1-12}$ alkoxy;
alternatively,
$U_1$, $U_2$, and $U_3$ are selected from -O- or -OC(O)-;
$W_1$, $W_2$, and $W_3$ are $C_{1-6}$ alkyl;
$L_2$ is a chemical bond or -C(O)-;
$R^1$ is selected from H, OH, or -$Y_5$-$Y_6$-$Y_7$;
$Y_5$ is unsubstituted or $NH_2$-substituted $C_{3-6}$ alkylene;
$Y_6$ is a single bond or -NH-C(NH)-;
$Y_7$ is $NH_2$ or OH;
yet alternatively,
$U_1$, $U_2$, and $U_3$ are O;
$W_1$, $W_2$, and $W_3$ are $CH_3$;
$L_2$ is a chemical bond or -C(O)-;
$R^1$ is selected from H,

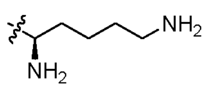 , 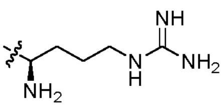 , 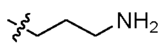 , or 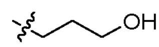 .

**39.** The compound of claim 38, or a pharmaceutically acceptable salt, ester, stereoisomer, polymorph, solvate, N-oxide, isotope-labeled compound, metabolite, or pro-drug thereof, and a mixture thereof, wherein the compound is a compound of Formula XIII, XIII-1, or XIII-2:

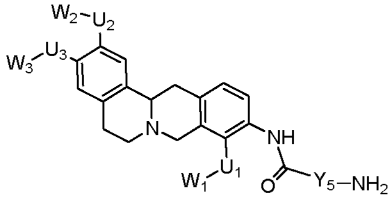
XIII

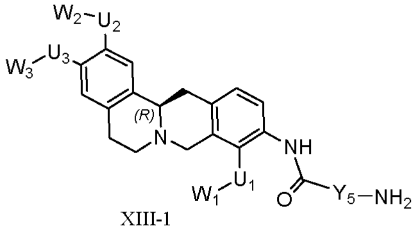
XIII-1

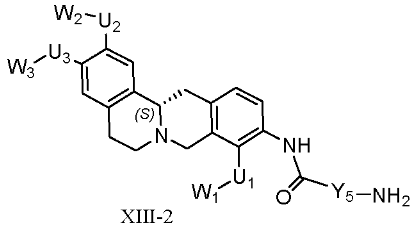
XIII-2

wherein
various variables are as defined in claim 38.

**40.** The compound of claim 1 or 2, or a pharmaceutically acceptable salt, ester, stereoisomer, polymorph, solvate, N-oxide, isotope-labeled compound, metabolite, or pro-drug thereof, and a mixture thereof, wherein the compound is selected from:

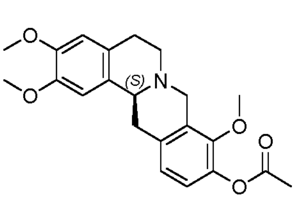
A1

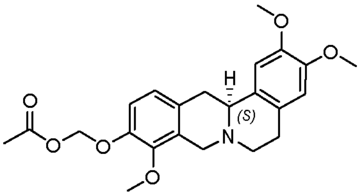
A2

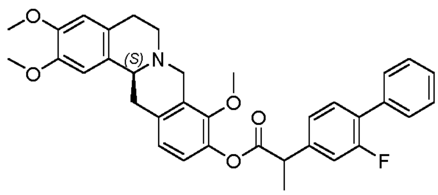
A3

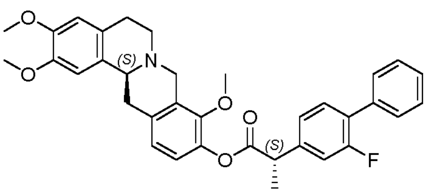
A4

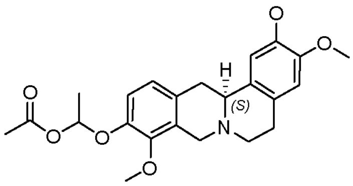
A5

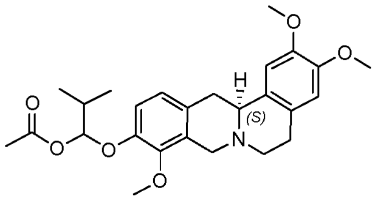
A6

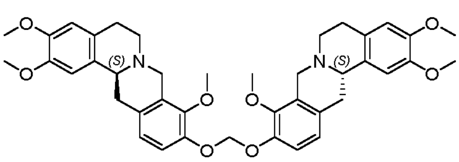
A7

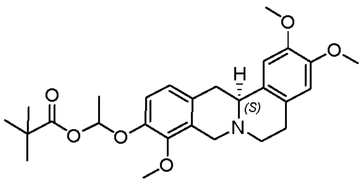
A8

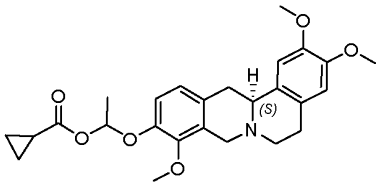
A9

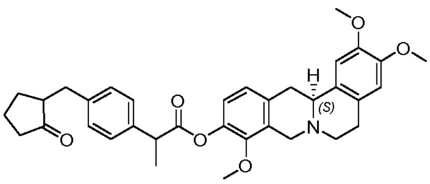
A10

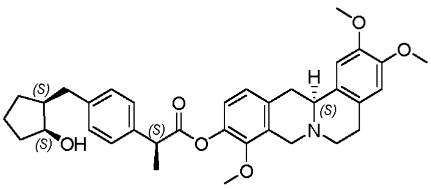
A11

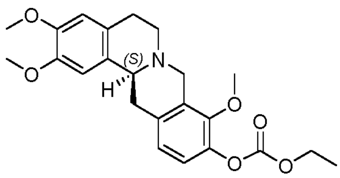
A12

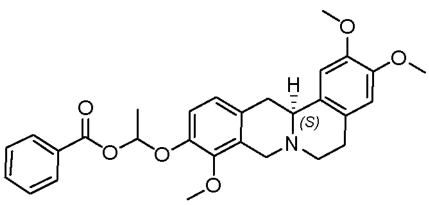
A13

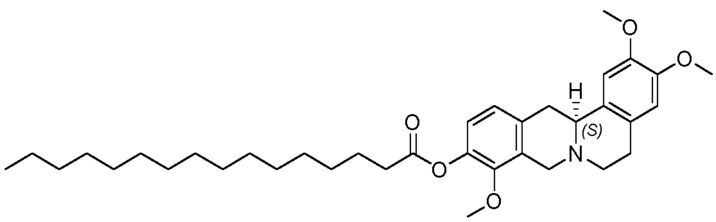
A14

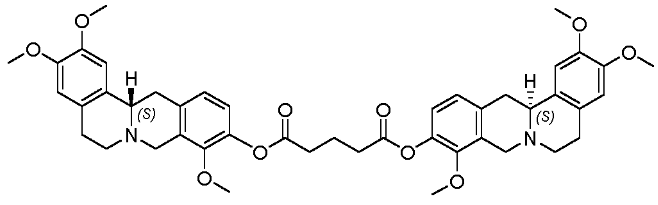

A15

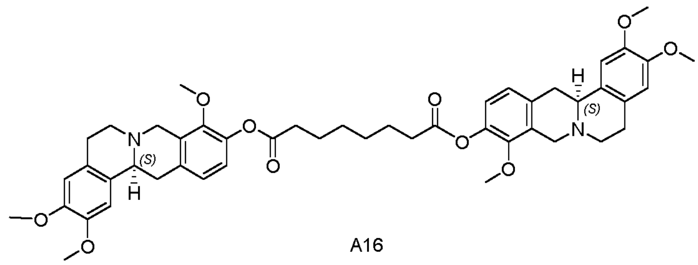

A16

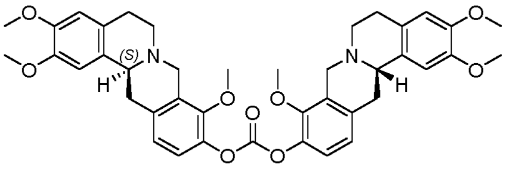

A17

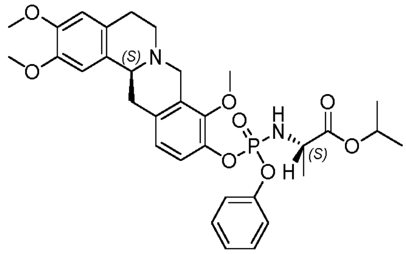

A18

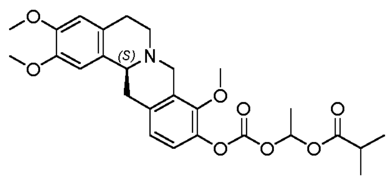

A19

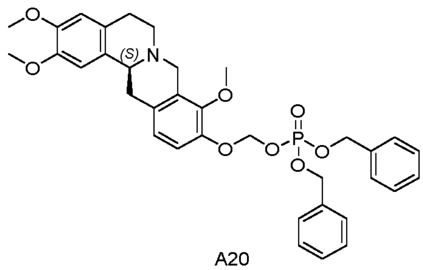

A20

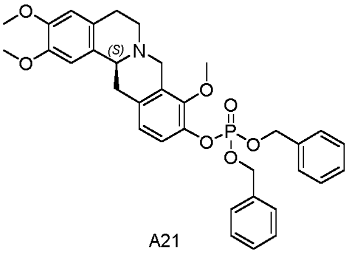

A21

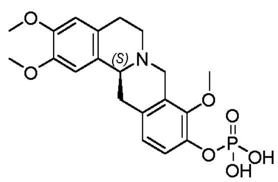

A22

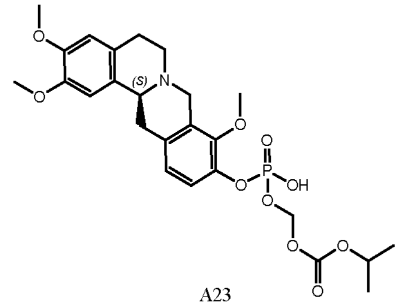

A23

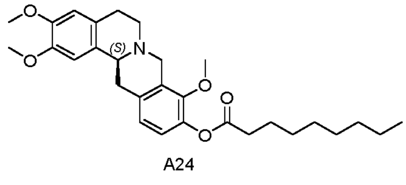

A24

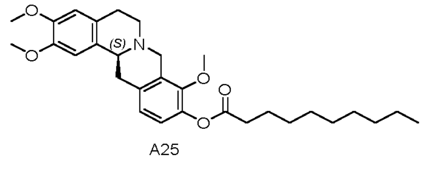

A25

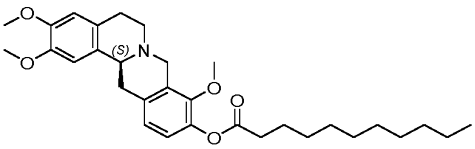

A26

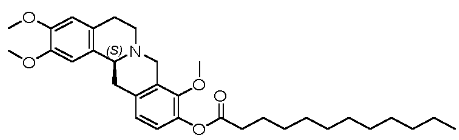

A27

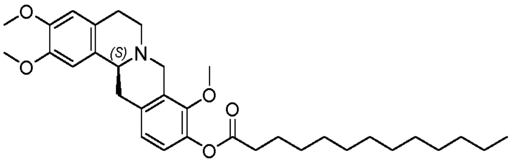

A28

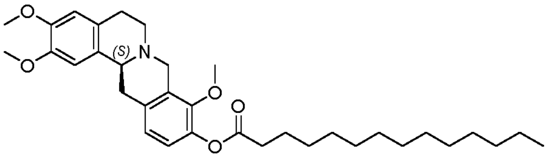

A29

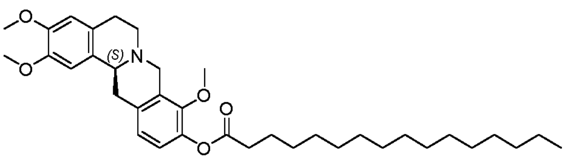

A30

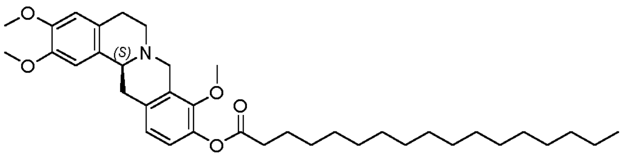

A31

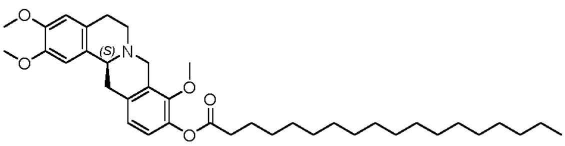

A32

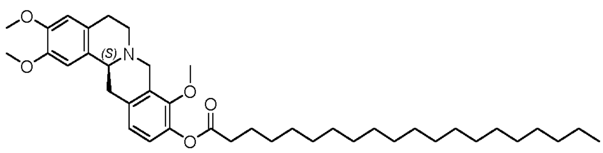

A33

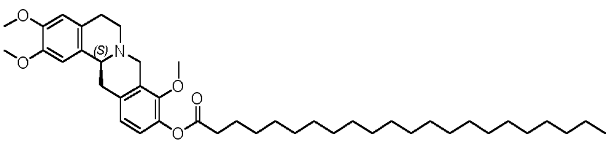

A34

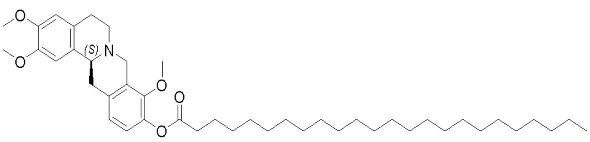

A35

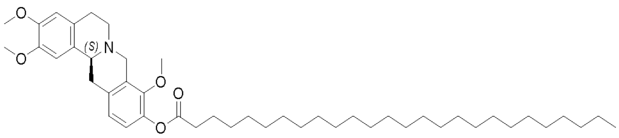

A36

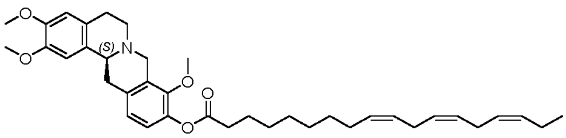

A37

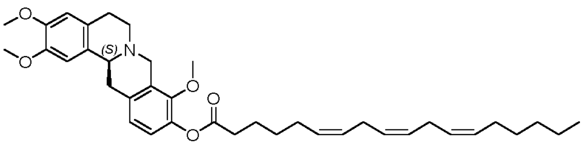

A38

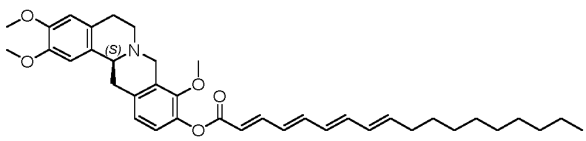

A39

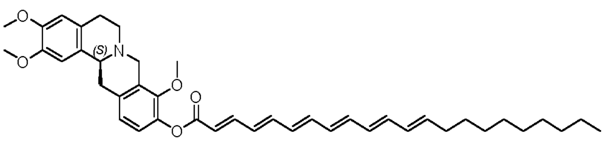

A40

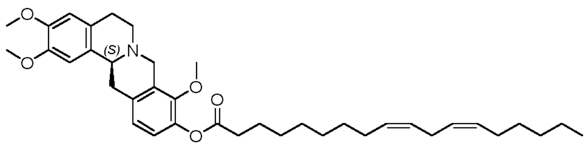

A41

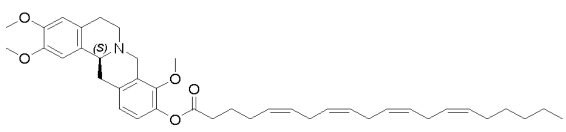

A42

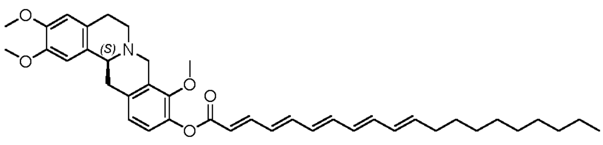

A43

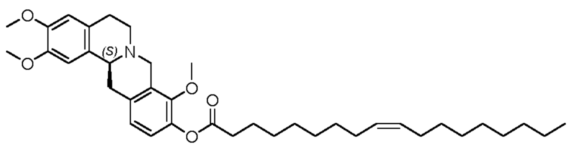

A44

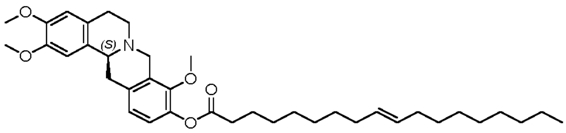

A45

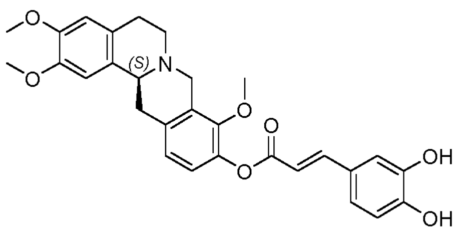

A46

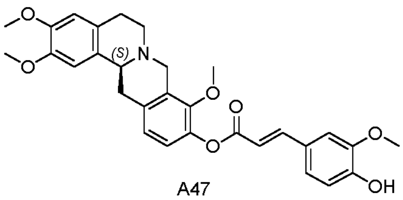

A47

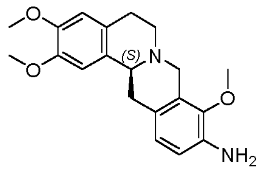

A48

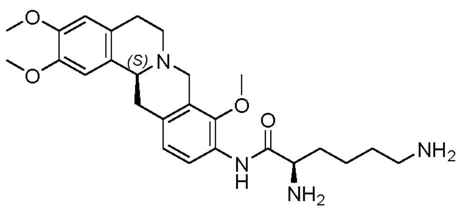

A49

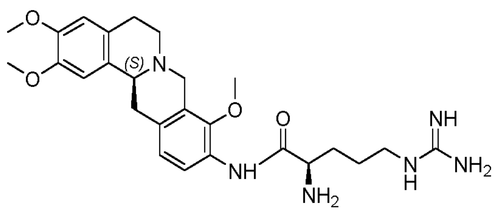

A50

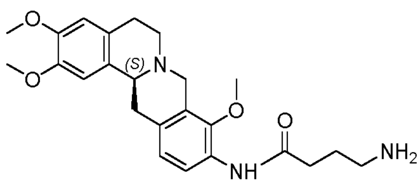

A51

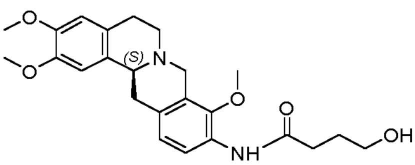

A52

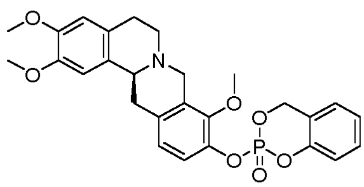

A53

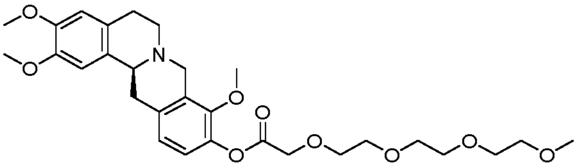

A54

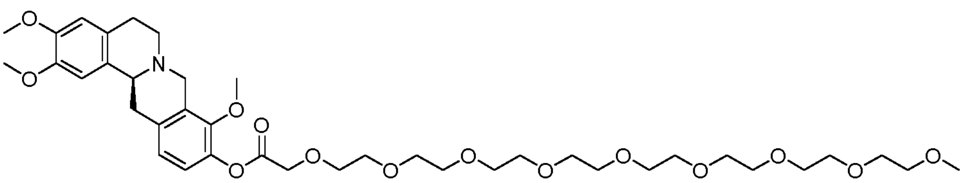

A55

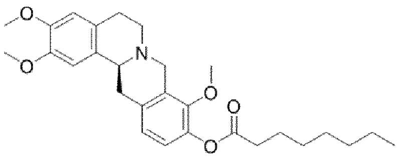
A56

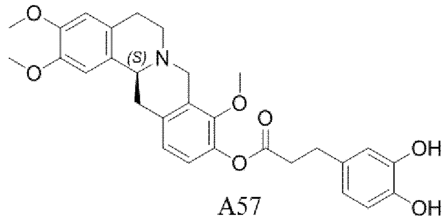
A57

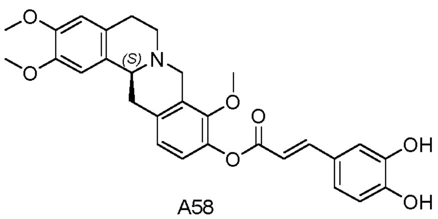
A58

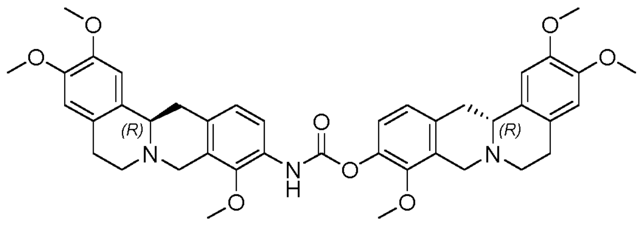

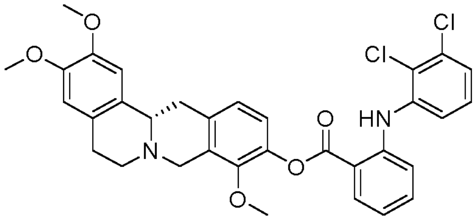

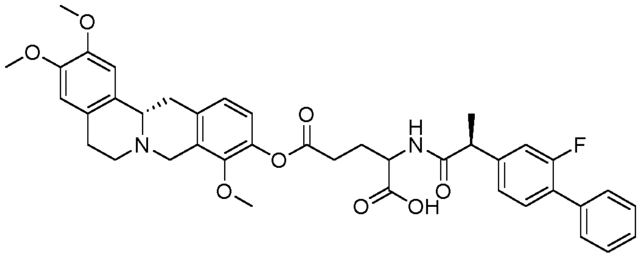

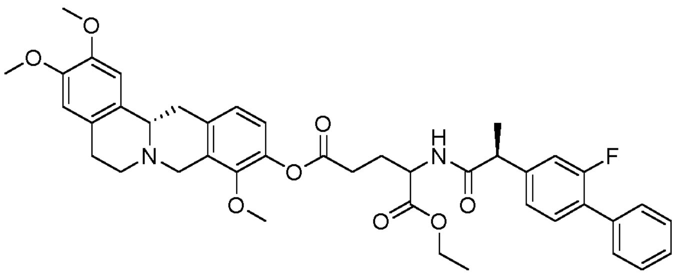
.

**41.** A stereoisomer, pharmaceutically acceptable salt, ester, optical isomer, polymorph, solvate, N-oxide, isotope-labeled compound, metabolite, chelate, complex, inclusion, or prodrug of the compound of any one of claims 1-40.

**42.** A composition comprising the compound of any one of claims 1-40 and/or the stereoisomer, pharmaceutically acceptable salt, ester, optical isomer, polymorph, solvate, N-oxide, isotope-labeled compound, metabolite, chelate, complex, inclusion, or prodrug of claim 41.

**43.** A pharmaceutical composition comprising the compound of any one of claims 1-40 and/or the stereoisomer, pharmaceutically acceptable salt, ester, optical isomer, polymorph, solvate, N-oxide, isotope-labeled compound, metabolite, chelate, complex, inclusion, or prodrug of claim 41, and optionally a pharmaceutically acceptable carrier or excipient.

**44.** Use of the compound of any one of claims 1-40, the stereoisomer, pharmaceutically acceptable salt, ester, optical isomer, polymorph, solvate, N-oxide, isotope-labeled compound, metabolite, chelate, complex, inclusion, or prodrug of claim 41, or the composition of claim 42 in the manufacture of a medicament for preventing and/or treating a central nervous system-associated disease.

**45.** Use of the compound of any one of claims 1-40, the stereoisomer, pharmaceutically acceptable salt, ester, optical isomer, polymorph, solvate, N-oxide, isotope-labeled compound, metabolite, chelate, complex, inclusion, or prodrug of claim 41, or the composition of claim 42 in the manufacture of a medicament for analgesia, anti-depression, or anti-addiction.

**46.** The use of claim 44, wherein the drug is used for pains caused by postoperative trauma, postoperative incision, or organ metastasis of cancer.

**47.** A method for analgesia, anti-depression, or anti-drug addiction, comprising administering to a subject in need thereof an effective dose of the compound of any one of claims 1-40, the stereoisomer, pharmaceutically acceptable salt, ester, optical isomer, polymorph, solvate, N-oxide, isotope-labeled compound, metabolite, chelate, complex, inclusion, or prodrug of claim 41, or the composition of claim 42, wherein the administration is carried out by ways of oral, rectal, nasal, topical, or parenteral administration.

**48.** The method of claim 47, wherein the effective dose is 0.1 mg/day to 1000 mg/day, alternatively 3 mg/day to 300 mg/day, yet alternatively 5 mg/day to 50 mg/day, based on the compound.

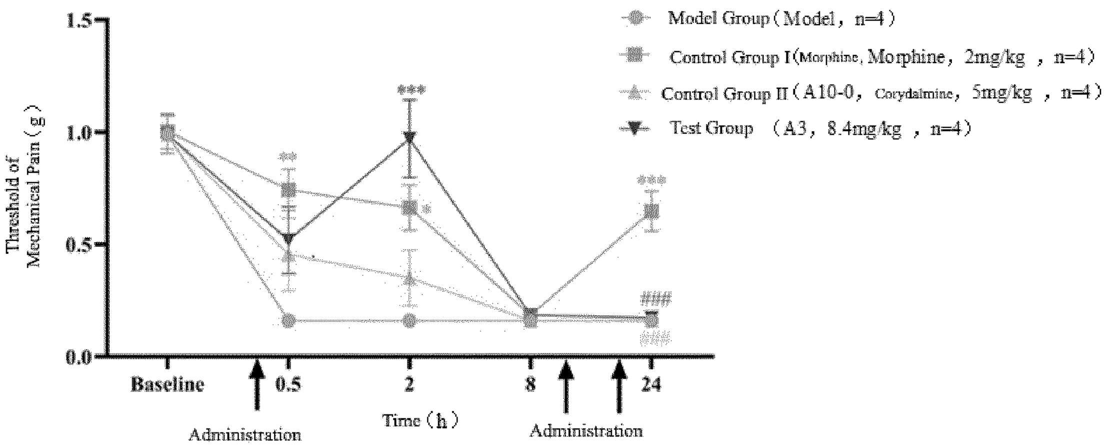
Model Group（Model，n=4）
Control Group I（Morphine, Morphine，2mg/kg ，n=4）
Control Group II（A10-0，Corydalmine，5mg/kg ，n=4）
Test Group （A3，8.4mg/kg ，n=4）
Threshold of Mechanical Pain（g）
1.5
1.0
0.5
0.0
Baseline
0.5
2
8
24
Time（h）
Administration
Administration
***
**
*
***
###
###

Fig. 1

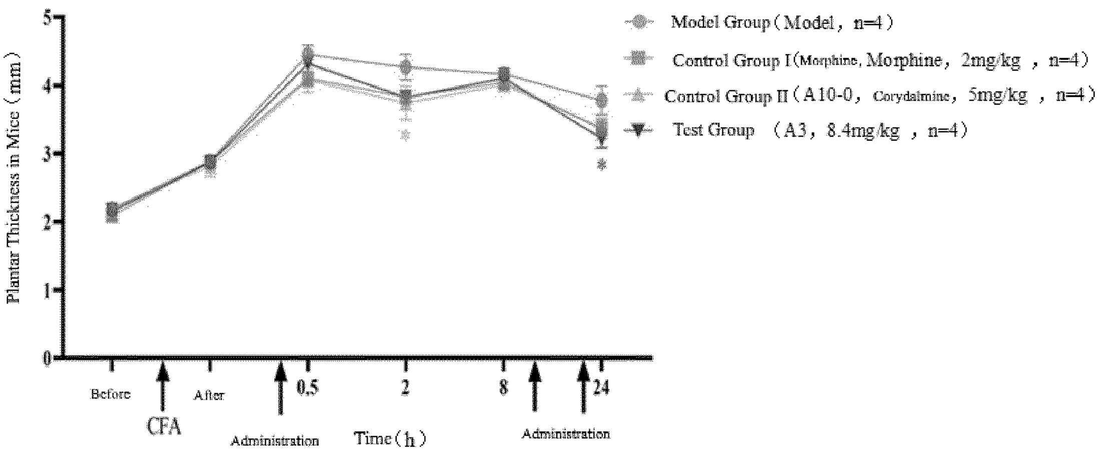
Model Group（Model，n=4）
Control Group I（Morphine, Morphine，2mg/kg ，n=4）
Control Group II（A10-0，Corydalmine，5mg/kg ，n=4）
Test Group （A3，8.4mg/kg ，n=4）
Plantar Thickness in Mice（mm）
5
4
3
2
1
0
Before
CFA
After
Administration
0.5
2
Time（h）
8
Administration
24
*
*

Fig. 2

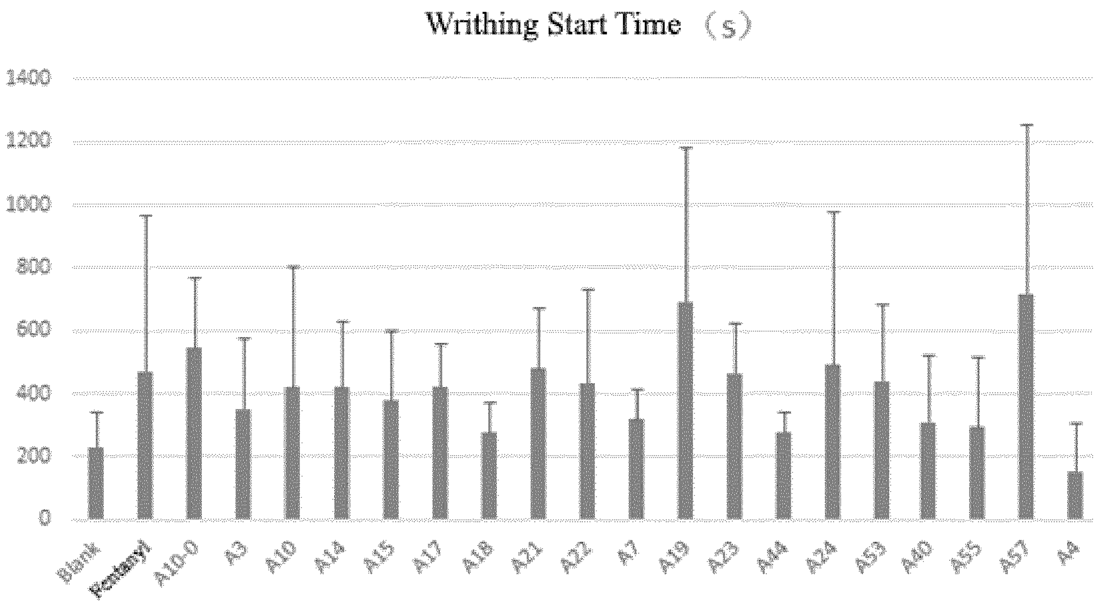
Writhing Start Time （s）
1400
1200
1000
800
600
400
200
0
Blank
Fentanyl
A10-0
A3
A10
A14
A15
A17
A18
A21
A22
A7
A19
A23
A44
A24
A53
A40
A55
A57
A4

Fig. 3

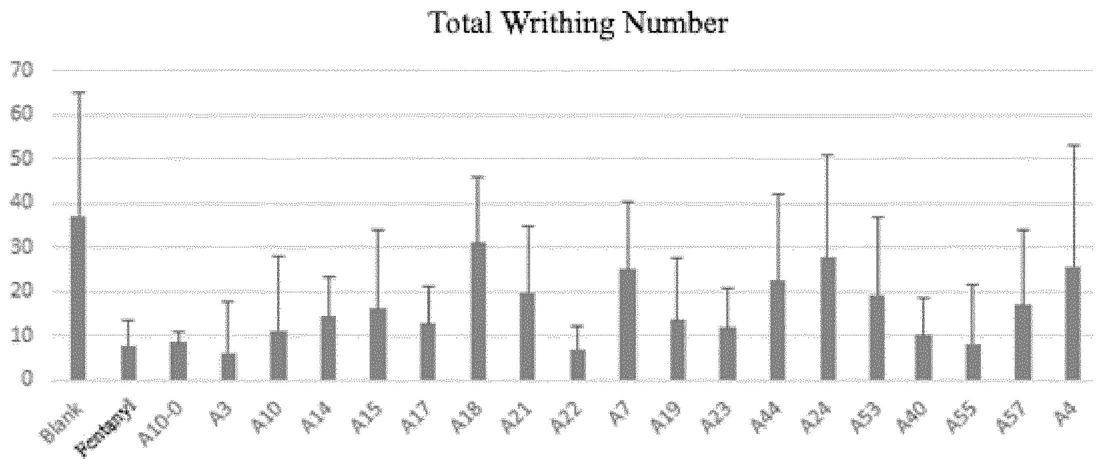
Total Writhing Number
70
60
50
40
30
20
10
0
Blank
Fentanyl
A10-0
A3
A10
A14
A15
A17
A18
A21
A22
A7
A19
A23
A44
A24
A53
A40
A55
A57
A4

Fig. 4

**INTERNATIONAL SEARCH REPORT**

International application No. **PCT/CN2023/125192**

**A. CLASSIFICATION OF SUBJECT MATTER**

C07D455/03(2006.01)i; C07D498/14(2006.01)i; A61K31/4375(2006.01)i; A61P25/00(2006.01)i

According to International Patent Classification (IPC) or to both national classification and IPC

**B. FIELDS SEARCHED**

Minimum documentation searched (classification system followed by classification symbols)

IPC: C07D,A61K,A61P

Documentation searched other than minimum documentation to the extent that such documents are included in the fields searched

Electronic data base consulted during the international search (name of data base and, where practicable, search terms used)

万方, WANFANG, cnki, cntxt, ven, stn-registry, caplus, marpat: 紫堇达明, 四氢原小檗碱, 四氢巴马汀, 疼痛, 中枢神经, 中枢系统, 1-DL, 1-CDL, corgdalmine, tetrahydrobeberruine, analgesic , pain, neuropathic pain, nervous system, stn-structure search

**C. DOCUMENTS CONSIDERED TO BE RELEVANT**

| Category* | Citation of document, with indication, where appropriate, of the relevant passages | Relevant to claim No. |
|---|---|---|
| X | CN 109384780 A (SHANGHAI INSTITUTE OF MATERIA MEDICA, CHINESE ACADEMY OF SCIENCES et al.) 26 February 2019 (2019-02-26)<br>see description, page 1, paragraph 2, and page 45, paragraph 490, and claims 8-10 | 1-7, 41-46 |
| Y | CN 109384780 A (SHANGHAI INSTITUTE OF MATERIA MEDICA, CHINESE ACADEMY OF SCIENCES et al.) 26 February 2019 (2019-02-26)<br>claim 1 | 8-15, 18-25, 27-35, 38-40 |
| Y | CN 1900076 A (SHANGHAI INSTITUTE OF MATERIA MEDICA, CHINESE ACADEMY OF SCIENCES) 24 January 2007 (2007-01-24)<br>see claim 1 | 8-15, 18-25, 27-35, 38-40 |
| Y | CN 101037436 A (SHANGHAI INSTITUTE OF MATERIA MEDICA, CHINESE ACADEMY OF SCIENCES) 19 September 2007 (2007-09-19)<br>see claim 1 | 8-15, 18-25, 27-35, 38-40 |
| X | KR 20120032660 A (DONG A PHARM CO., LTD.) 06 April 2012 (2012-04-06)<br>see description, page 15, table 1 | 1-7, 25 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 January 2024** | **30 January 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2023/125192**

**C. DOCUMENTS CONSIDERED TO BE RELEVANT**

| Category* | Citation of document, with indication, where appropriate, of the relevant passages | Relevant to claim No. |
|---|---|---|
| X | ZHANG, Hongwei et al. "Development of a Highly Sensitive and Specific ELISA Method for the Determination of l-corydalmine in SD Rats with Monoclonal Antibody" *Journal of Chromatography B,* Vol. vol. 1073, 14 September 2017 (2017-09-14), pages 163-169 | 1-7 |
| X | T. Kametani et al. "A Total Synthesis of Kikemanine" *Journal of the Chemical Society,* Vol. vol. 20, 01 January 1971 (1971-01-01), pages 3318-3321 | 1-7 |
| A | US 3420834 A (ROUSSEL UCLAF) 07 January 1969 (1969-01-07) see entire document | 1-46 |

Form PCT/ISA/210 (second sheet) (July 2022)

**INTERNATIONAL SEARCH REPORT**

International application No.
**PCT/CN2023/125192**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **47-48**
because they relate to subject matter not required to be searched by this Authority, namely:

The subject matter of claims 47 and 48 relates to a method for treatment of the human or animal body, and belongs to the cases set out in PCT Rule 39.1(iv) for which a search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/125192**

| Patent document cited in search report | | | Publication date (day/month/year) | Patent family member(s) | | | Publication date (day/month/year) |
|---|---|---|---|---|---|---|---|
| CN | 109384780 | A | 26 February 2019 | JP | 2020530483 | A | 22 October 2020 |
| | | | | JP | 7100693 | B2 | 13 July 2022 |
| | | | | WO | 2019034031 | A1 | 21 February 2019 |
| | | | | US | 2020255421 | A1 | 13 August 2020 |
| | | | | US | 11731965 | B2 | 22 August 2023 |
| | | | | EP | 3670511 | A1 | 24 June 2020 |
| | | | | EP | 3670511 | A4 | 02 September 2020 |
| CN | 1900076 | A | 24 January 2007 | None | | | |
| CN | 101037436 | A | 19 September 2007 | WO | 2008128431 | A1 | 30 October 2008 |
| KR | 20120032660 | A | 06 April 2012 | KR | 101155753 | B1 | 12 June 2012 |
| US | 3420834 | A | 07 January 1969 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211273550
- CN 202311089455


### Non-patent literature cited in the description


- **BERGE S. M. et al.** Pharmaceutical Salts. *J. Pharm. Sci.*, 1977, vol. 66, 1-19
- Prodrugs as Novel Delivery Systems. **T. HIGUCHI** ; **V. STELLA**. Bioreversible Carriers in Drug Design. American Pharmaceutical Association and Pergamon Press, 1987, vol. 14
- **D. FLEISHER** ; **S. RAMON** ; **H. BARBRA**. Improved oral drug delivery: solubility limitations overcome by the use of prodrugs. *Advanced Drug Delivery Reviews*, 1996, vol. 19 (2), 115-130